# EUROPEAN PATENT APPLICATION

(11) **EP 3 909 606 A1**
(43) Date of publication of application: **17.11.2021**
(21) Application number: 20737956.1
(22) Date of filing: 06.01.2020
(51) Int. Cl.: A61K 39/395, A61P 35/00, C07K 16/30, A61K 47/52, A61K 47/54, A61K 47/65, A61K 47/68, A61K 49/16, C12N 15/13, A61K 51/10, G01N 33/53, G01N 33/574

(54) **COMPLEX COMPRISING LIGAND AND CEACAM5 ANTIBODY FAB FRAGMENT**

(30) Priority: 07.01.2019 JP 2019000530; 14.11.2019 JP 2019206560
(71) Applicant: Astellas Pharma Inc., Chuo-ku Tokyo 103-8411 (JP)
(72) Inventor: AKAIWA, Michinori, Tokyo 103-8411 (JP); ISHIDA, Junya, Tokyo 103-8411 (JP); TOYA, Hiroki, Tokyo 103-8411 (JP); SHIRAISHI, Nobuyuki, Tokyo 103-8411 (JP); ASANO, Toru, Tokyo 103-8411 (JP); YOSHIKAWA, TOMOAKI, Tokyo 103-8411 (JP); SANO, Yorikata, Tokyo 103-8411 (JP); DOIHARA, Hitoshi, Tokyo 103-8411 (JP); SHIRAI, Hiroki, Tokyo 103-8411 (JP); HIRAYAMA, Kazunori, Tokyo 103-8411 (JP)
(74) Representative: Hoffmann Eitle
(86) International application number: PCT/JP2020/000037
(87) International publication number: WO 2020/145228

(57) **Abstract**

Provided is a conjugate comprising a ligand, a spacer, and a peptide linker useful for an in-vivo diagnostic drug and internal radiation therapy, using an anti-human CEACAM5 antibody Fab fragment whose binding activity is not attenuated even by labeling with a metal, a fluorescent dye, or the like. A conjugate comprising an anti-human CEACAM5 antibody Fab fragment and a ligand, the fragment comprising a heavy chain fragment including a heavy chain variable region consisting of a specific amino acid sequence and a light chain including a light chain variable region consisting of a specific amino acid sequence, or a conjugate comprising a ligand, a spacer, and a peptide linker, wherein the binding activity thereof is not attenuated even by labeling with a metal, a fluorescent dye, or the like, can be used as a diagnostic composition and/or a pharmaceutical composition.

SEQUENCE LISTING

<110>  Astellas Pharma Inc.

<120>  A conjugate comprising a ligand and Anti CEACAM5 antibody Fab fragment

<130>  A20001A00

<150> JP 2019-000530
<151> 2019-01-07

<150> JP 2019-206560
<151> 2019-11-14

<160>  19

<170>  PatentIn version 3.5

<210>  1
<211>  678
<212>  DNA
<213>  Artificial Sequence

<220>
<223>  DNA encoding PB009-01 Fab heavy chain


<220>
<221>  CDS
<222>  (1)..(678)

<400>  1
gaa gtg cag ctg gtg gaa tct ggc ggc gga ctg gtg cag cct ggc gga       48
Glu Val Gln Leu Val Glu Ser Gly Gly Gly Leu Val Gln Pro Gly Gly
1               5                   10                  15

tct ctg aga ctg agc tgt gcc gcc agc ggc ttc aac atc cgg gac acc       96
Ser Leu Arg Leu Ser Cys Ala Ala Ser Gly Phe Asn Ile Arg Asp Thr
            20                  25                  30

tac atg cac tgg gtg cgc cag gcc cct ggc aag gga ctg gaa tgg gtg      144
Tyr Met His Trp Val Arg Gln Ala Pro Gly Lys Gly Leu Glu Trp Val
        35                  40                  45

gcc aga atc gac ccc gcc aac ggc aac agc aga tac gtg ccc aag ttc      192
Ala Arg Ile Asp Pro Ala Asn Gly Asn Ser Arg Tyr Val Pro Lys Phe
    50                  55                  60

cag ggc cgg ttc acc atc agc gcc gac acc agc aga aac acc gcc tac      240
Gln Gly Arg Phe Thr Ile Ser Ala Asp Thr Ser Arg Asn Thr Ala Tyr
65                  70                  75                  80

ctg cag atg aac agc ctg cgg gcc gag gac acc gcc gtg tac tac tgt      288
Leu Gln Met Asn Ser Leu Arg Ala Glu Asp Thr Ala Val Tyr Tyr Cys
                85                  90                  95

gcc ccc ttc ggc tac tac gtg tcc gac tac gcc atg gcc tat tgg ggc      336
Ala Pro Phe Gly Tyr Tyr Val Ser Asp Tyr Ala Met Ala Tyr Trp Gly
            100                 105                 110

cag ggc acc ctc gtg aca gtg tcc tca gcc tcc acc aag ggc cca tcg      384
Gln Gly Thr Leu Val Thr Val Ser Ser Ala Ser Thr Lys Gly Pro Ser
        115                 120                 125

gtc ttc ccc ctg gca ccc tcc tcc aag agc acc tct ggg ggc aca gcg 432
Val Phe Pro Leu Ala Pro Ser Ser Lys Ser Thr Ser Gly Gly Thr Ala
    130                 135                 140

gcc ctg ggc tgc ctg gtc aag gac tac ttc ccc gaa ccg gtg acg gtg 480
Ala Leu Gly Cys Leu Val Lys Asp Tyr Phe Pro Glu Pro Val Thr Val
145                 150                 155                 160

tcg tgg aac tca ggc gcc ctg acc agc ggc gtg cac acc ttc ccg gct 528
Ser Trp Asn Ser Gly Ala Leu Thr Ser Gly Val His Thr Phe Pro Ala
                165                 170                 175

gtc cta cag tcc tca gga ctc tac tcc ctt agt agc gtg gtg acc gtg 576
Val Leu Gln Ser Ser Gly Leu Tyr Ser Leu Ser Ser Val Val Thr Val
            180                 185                 190

ccc tcc agc agc ttg ggc acc cag acc tac atc tgc aac gtg aat cac 624
Pro Ser Ser Ser Leu Gly Thr Gln Thr Tyr Ile Cys Asn Val Asn His
        195                 200                 205

aag ccc agc aac acc aag gtg gac aag aaa gtt gag ccc aaa tct tgt 672
Lys Pro Ser Asn Thr Lys Val Asp Lys Lys Val Glu Pro Lys Ser Cys
    210                 215                 220

gac tga 678
Asp
225

<210> 2
<211> 225
<212> PRT
<213> Artificial Sequence

<220>
<223> Synthetic Construct

<400> 2

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Asn Ile Arg Asp Thr
            20                  25                  30

Tyr Met His Trp Val Arg Gln Ala Pro Gly Lys Gly Leu Glu Trp Val
        35                  40                  45

Ala Arg Ile Asp Pro Ala Asn Gly Asn Ser Arg Tyr Val Pro Lys Phe
    50                  55                  60

Gln Gly Arg Phe Thr Ile Ser Ala Asp Thr Ser Arg Asn Thr Ala Tyr
65                  70                  75                  80

Leu Gln Met Asn Ser Leu Arg Ala Glu Asp Thr Ala Val Tyr Tyr Cys
                85                  90                  95

Ala Pro Phe Gly Tyr Tyr Val Ser Asp Tyr Ala Met Ala Tyr Trp Gly
            100                 105                 110

Gln Gly Thr Leu Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp
225

<210>  3
<211>  657
<212>  DNA
<213>  Artificial Sequence

<220>
<223>  DNA encoding PB009-01 light chain

<220>
<221>  CDS
<222>  (1)..(657)

<400>  3
gac atc cag ctg acc cag agc cct agc agc ctg tct gcc agc gtg ggc      48
Asp Ile Gln Leu Thr Gln Ser Pro Ser Ser Leu Ser Ala Ser Val Gly
1               5                   10                  15

gac aga gtg acc atc acc tgt aga gcc ggc gag agc gtg gac atc ttc      96
Asp Arg Val Thr Ile Thr Cys Arg Ala Gly Glu Ser Val Asp Ile Phe
            20                  25                  30

ggc gtg gga ttt ctg cac tgg tat cag cag aag ccc ggc aag gcc ccc      144
Gly Val Gly Phe Leu His Trp Tyr Gln Gln Lys Pro Gly Lys Ala Pro
        35                  40                  45

aag ctg ctg atc tac aga gcc agc aac ctg gaa agc ggc atc ccc agc 192
Lys Leu Leu Ile Tyr Arg Ala Ser Asn Leu Glu Ser Gly Ile Pro Ser
    50                  55                  60

aga ttc agc ggc agc ggc tcc aga acc gac ttc acc ctg acc atc agc 240
Arg Phe Ser Gly Ser Gly Ser Arg Thr Asp Phe Thr Leu Thr Ile Ser
65                  70                  75                  80

agc ctg cag ccc gag gac ttc gcc acc tac tac tgc cag cag acc aac 288
Ser Leu Gln Pro Glu Asp Phe Ala Thr Tyr Tyr Cys Gln Gln Thr Asn
                85                  90                  95

gag gac ccc tac acc ttt ggc cag ggc acc aag gtg gaa atc aag cgt 336
Glu Asp Pro Tyr Thr Phe Gly Gln Gly Thr Lys Val Glu Ile Lys Arg
            100                 105                 110

acg gtg gct gca cca tct gtc ttc atc ttc ccg cca tct gat gag cag 384
Thr Val Ala Ala Pro Ser Val Phe Ile Phe Pro Pro Ser Asp Glu Gln
        115                 120                 125

ttg aaa tct gga act gcc tct gtt gtg tgc ctg ctg aat aac ttc tat 432
Leu Lys Ser Gly Thr Ala Ser Val Val Cys Leu Leu Asn Asn Phe Tyr
    130                 135                 140

ccc aga gag gcc aaa gta cag tgg aag gtg gat aac gcc ctc caa tcg 480
Pro Arg Glu Ala Lys Val Gln Trp Lys Val Asp Asn Ala Leu Gln Ser
145                 150                 155                 160

ggt aac tcc cag gag agt gtc aca gag cag gac agc aag gac agc acc 528
Gly Asn Ser Gln Glu Ser Val Thr Glu Gln Asp Ser Lys Asp Ser Thr
                165                 170                 175

tac agc ctg agc agc acc ctg acg ctg agc aaa gca gac tac gag aaa 576
Tyr Ser Leu Ser Ser Thr Leu Thr Leu Ser Lys Ala Asp Tyr Glu Lys
            180                 185                 190

cac aaa gtc tac gcc tgc gaa gtc acc cat cag ggc ctg agc tcg ccc 624
His Lys Val Tyr Ala Cys Glu Val Thr His Gln Gly Leu Ser Ser Pro
        195                 200                 205

gtc aca aag agc ttc aac agg gga gag tgt tag 657
Val Thr Lys Ser Phe Asn Arg Gly Glu Cys
    210                 215

<210> 4
<211> 218
<212> PRT
<213> Artificial Sequence

<220>
<223> Synthetic Construct

<400> 4

Asp Ile Gln Leu Thr Gln Ser Pro Ser Ser Leu Ser Ala Ser Val Gly
1               5                   10                  15

Asp Arg Val Thr Ile Thr Cys Arg Ala Gly Glu Ser Val Asp Ile Phe
            20                  25                  30

Gly Val Gly Phe Leu His Trp Tyr Gln Gln Lys Pro Gly Lys Ala Pro
        35                  40                  45

Lys Leu Leu Ile Tyr Arg Ala Ser Asn Leu Glu Ser Gly Ile Pro Ser
    50                  55                  60

Arg Phe Ser Gly Ser Gly Ser Arg Thr Asp Phe Thr Leu Thr Ile Ser
65                  70                  75                  80

Ser Leu Gln Pro Glu Asp Phe Ala Thr Tyr Tyr Cys Gln Gln Thr Asn
                85                  90                  95

Glu Asp Pro Tyr Thr Phe Gly Gln Gly Thr Lys Val Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190

His Lys Val Tyr Ala Cys Glu Val Thr His Gln Gly Leu Ser Ser Pro
        195                 200                 205

Val Thr Lys Ser Phe Asn Arg Gly Glu Cys
    210                 215

<210>  5
<211>  669
<212>  DNA
<213>  Artificial Sequence

<220>
<223>  DNA encoding P10-1 Fab heavy chain fragment

<400>  5
caggtgcagc tggtgcagtc tggcgccgaa gtgaagaaac caggcgccag cgtgaaggtg      60

tcctgcaagg ccagcggcta caccttcacc aactacggcc tgagctgggt gcgccaggct     120

cctggacagg gactggaatg gatgggcgag aaccaccctg gcagcggcat catctaccac     180

aacgagaagt tccggggcag agtgaccctg accgccgacc ggagcaccag caccgcctac 240

atggaactga gcagcctgcg gagcgaggac accgccgtgt actactgtgc cagaagcagc 300

ggcaccagag gctttgccta ttggggacag ggcaccctcg tgaccgtgtc ctcagcctcc 360

accaagggcc catcggtctt ccccctggca ccctcctcca agagcacctc tgggggcaca 420

gcggccctgg gctgcctggt caaggactac ttccccgaac cggtgacggt gtcgtggaac 480

tcaggcgccc tgaccagcgg cgtgcacacc ttcccggctg tcctacagtc ctcaggactc 540

tactccctta gtagcgtggt gaccgtgccc tccagcagct tgggcaccca gacctacatc 600

tgcaacgtga atcacaagcc cagcaacacc aaggtggaca agaaagttga gcccaaatct 660

tgtgactga 669

<210> 6
<211> 222
<212> PRT
<213> Artificial Sequence

<220>
<223> P10-1 Fab heavy chain fragment

<400> 6

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Gly Leu Ser Trp Val Arg Gln Ala Pro Gly Gln Gly Leu Glu Trp Met
        35                  40                  45

Gly Glu Asn His Pro Gly Ser Gly Ile Ile Tyr His Asn Glu Lys Phe
    50                  55                  60

Arg Gly Arg Val Thr Leu Thr Ala Asp Arg Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Ser Ser Gly Thr Arg Gly Phe Ala Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp
    210                 215                 220

<210>  7
<211>  669
<212>  DNA
<213>  Artificial Sequence

<220>
<223>  DNA encoding P10-2 Fab heavy chain fragment

<400>  7
caggtgcagc tggtgcagtc tggcgccgaa gtgaagaaac caggcgccag cgtgaaggtg      60

tcctgcaagg ccagcggcta caccttcacc aactacggcc tgagctgggt gcgccaggct     120

cctggacagg gactggaatg gatgggcgag aaccaccctg gcagcggcat catctaccac     180

aacgagaagt tccggggcag agtgaccctg accgccgacc ggagcaccag caccgcctac     240

atggaactga gcagcctgcg gagcgaggac accgccgtgt actactgtgc cagaagcagc     300

ggcaccagag gctttgacta ttggggacag ggcaccctcg tgaccgtgtc ctcagcctcc     360

accaagggcc catcggtctt ccccctggca ccctcctcca agagcacctc tgggggcaca     420

gcggccctgg gctgcctggt caaggactac ttccccgaac cggtgacggt gtcgtggaac     480

tcaggcgccc tgaccagcgg cgtgcacacc ttcccggctg tcctacagtc ctcaggactc     540

tactccctta gtagcgtggt gaccgtgccc tccagcagct tgggcaccca gacctacatc     600

tgcaacgtga atcacaagcc cagcaacacc aaggtggaca agaaagttga gcccaaatct     660

tgtgactga                                                             669

<210>  8
<211>  222
<212>  PRT
<213>  Artificial Sequence

<220>
<223>  P10-2 Fab heavy chain fragment

<400>  8

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Gly Leu Ser Trp Val Arg Gln Ala Pro Gly Gln Gly Leu Glu Trp Met
        35                  40                  45

Gly Glu Asn His Pro Gly Ser Gly Ile Ile Tyr His Asn Glu Lys Phe
    50                  55                  60

Arg Gly Arg Val Thr Leu Thr Ala Asp Arg Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Ser Ser Gly Thr Arg Gly Phe Asp Tyr Trp Gly Gln Gly Thr
            100                 105                 110

Leu Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp
    210                 215                 220

<210>  9
<211>  660
<212>  DNA
<213>  Artificial Sequence

<220>
<223>  DNA encoding antibody light chain

<400>  9
gacgtcgtga tgacccagac ccctctgagc ctgagcgtga cacctggaca gcctgccagc      60

atcagctgca gatccagcca gagcatcgtg cacagcaacg gcaacaccta cctggaatgg     120

tatctgcaga agcccggcca gagcccccag ctgctgatct acagggtgtc caaccggttc     180

agcggcgtgc ccgacagatt ttctggcagc ggctccggca ccgacttcac cctgaagatc     240

tcccgggtgg aagccgagga cgtgggcgtg tactactgtt ttcaaggcag ccacggcccc     300

tggacctttg gccagggaac aaagctggaa atcaagcgta cggtggctgc accatctgtc     360

ttcatcttcc cgccatctga tgagcagttg aaatctggaa ctgcctctgt tgtgtgcctg     420

ctgaataact tctatcccag agaggccaaa gtacagtgga aggtggataa cgccctccaa     480

tcgggtaact cccaggagag tgtcacagag caggacagca aggacagcac ctacagcctg     540

agcagcaccc tgacgctgag caaagcagac tacgagaaac acaaagtcta cgcctgcgaa     600

gtcacccatc agggcctgag ctcgcccgtc acaaagagct tcaacagggg agagtgttag     660


<210>  10
<211>  219
<212>  PRT
<213>  Artificial Sequence

<220>
<223>  Antibody light chain

<400>  10

Asp Val Val Met Thr Gln Thr Pro Leu Ser Leu Ser Val Thr Pro Gly
1               5                   10                  15


Gln Pro Ala Ser Ile Ser Cys Arg Ser Ser Gln Ser Ile Val His Ser
            20                  25                  30


Asn Gly Asn Thr Tyr Leu Glu Trp Tyr Leu Gln Lys Pro Gly Gln Ser
        35                  40                  45


Pro Gln Leu Leu Ile Tyr Arg Val Ser Asn Arg Phe Ser Gly Val Pro
    50                  55                  60


Asp Arg Phe Ser Gly Ser Gly Ser Gly Thr Asp Phe Thr Leu Lys Ile
65                  70                  75                  80


Ser Arg Val Glu Ala Glu Asp Val Gly Val Tyr Tyr Cys Phe Gln Gly
                85                  90                  95


Ser His Gly Pro Trp Thr Phe Gly Gln Gly Thr Lys Leu Glu Ile Lys

            100                 105                 110

Arg Thr Val Ala Ala Pro Ser Val Phe Ile Phe Pro Pro Ser Asp Glu
        115                 120                 125

Gln Leu Lys Ser Gly Thr Ala Ser Val Val Cys Leu Leu Asn Asn Phe
    130                 135                 140

Tyr Pro Arg Glu Ala Lys Val Gln Trp Lys Val Asp Asn Ala Leu Gln
145                 150                 155                 160

Ser Gly Asn Ser Gln Glu Ser Val Thr Glu Gln Asp Ser Lys Asp Ser
                165                 170                 175

Thr Tyr Ser Leu Ser Ser Thr Leu Thr Leu Ser Lys Ala Asp Tyr Glu
            180                 185                 190

Lys His Lys Val Tyr Ala Cys Glu Val Thr His Gln Gly Leu Ser Ser
        195                 200                 205

Pro Val Thr Lys Ser Phe Asn Arg Gly Glu Cys
    210                 215

<210> 11
<211> 354
<212> DNA
<213> Artificial Sequence

<220>
<223> DNA encoding P10-1 Fab heavy chain variable region

<400> 11
caggtgcagc tggtgcagtc tggcgccgaa gtgaagaaac caggcgccag cgtgaaggtg 60

tcctgcaagg ccagcggcta caccttcacc aactacggcc tgagctgggt gcgccaggct 120

cctggacagg gactggaatg gatgggcgag aaccaccctg gcagcggcat catctaccac 180

aacgagaagt tccggggcag agtgaccctg accgccgacc ggagcaccag caccgcctac 240

atggaactga gcagcctgcg gagcgaggac accgccgtgt actactgtgc cagaagcagc 300

ggcaccagag gctttgccta ttggggacag ggcaccctcg tgaccgtgtc ctca 354

<210> 12
<211> 118
<212> PRT
<213> Artificial Sequence

<220>
<223> P10-1 Fab heavy chain variable region

<400> 12

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Gly Leu Ser Trp Val Arg Gln Ala Pro Gly Gln Gly Leu Glu Trp Met
        35                  40                  45

Gly Glu Asn His Pro Gly Ser Gly Ile Ile Tyr His Asn Glu Lys Phe
    50                  55                  60

Arg Gly Arg Val Thr Leu Thr Ala Asp Arg Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Ser Ser Gly Thr Arg Gly Phe Ala Tyr Trp Gly Gln Gly Thr
            100                 105                 110

Leu Val Thr Val Ser Ser
        115

<210>  13
<211>  354
<212>  DNA
<213>  Artificial Sequence

<220>
<223>  DNA encoding P10-2 Fab heavy chain variable region

<400>  13
caggtgcagc tggtgcagtc tggcgccgaa gtgaagaaac caggcgccag cgtgaaggtg      60

tcctgcaagg ccagcggcta caccttcacc aactacggcc tgagctgggt gcgccaggct     120

cctggacagg gactggaatg gatgggcgag aaccaccctg gcagcggcat catctaccac     180

aacgagaagt tccggggcag agtgaccctg accgccgacc ggagcaccag caccgcctac     240

atggaactga gcagcctgcg gagcgaggac accgccgtgt actactgtgc cagaagcagc     300

ggcaccagag gctttgacta ttggggacag ggcaccctcg tgaccgtgtc ctca           354

<210>  14
<211>  118
<212>  PRT
<213>  Artificial Sequence

<220>
<223>  P10-2 Fab heavy chain variable region

<400>  14

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Asn Tyr
            20                  25                  30

Gly Leu Ser Trp Val Arg Gln Ala Pro Gly Gln Gly Leu Glu Trp Met
        35                  40                  45

Gly Glu Asn His Pro Gly Ser Gly Ile Ile Tyr His Asn Glu Lys Phe
    50                  55                  60

Arg Gly Arg Val Thr Leu Thr Ala Asp Arg Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Ser Ser Gly Thr Arg Gly Phe Asp Tyr Trp Gly Gln Gly Thr
            100                 105                 110

Leu Val Thr Val Ser Ser
        115

<210>  15
<211>  339
<212>  DNA
<213>  Artificial Sequence

<220>
<223>  DNA encoding antibody light chain variable region

<400>  15
gacgtcgtga tgacccagac ccctctgagc ctgagcgtga cacctggaca gcctgccagc      60

atcagctgca gatccagcca gagcatcgtg cacagcaacg gcaacaccta cctggaatgg     120

tatctgcaga agcccggcca gagcccccag ctgctgatct acagggtgtc caaccggttc     180

agcggcgtgc ccgacagatt ttctggcagc ggctccggca ccgacttcac cctgaagatc     240

tcccgggtgg aagccgagga cgtgggcgtg tactactgtt ttcaaggcag ccacggcccc     300

tggacctttg gccagggaac aaagctggaa atcaagcgt                            339

<210>  16
<211>  113
<212>  PRT
<213>  Artificial Sequence

<220>
<223>  Antibody light chain variable region

<400>  16

Asp Val Val Met Thr Gln Thr Pro Leu Ser Leu Ser Val Thr Pro Gly
1               5                   10                  15

Gln Pro Ala Ser Ile Ser Cys Arg Ser Ser Gln Ser Ile Val His Ser
            20                  25                  30

Asn Gly Asn Thr Tyr Leu Glu Trp Tyr Leu Gln Lys Pro Gly Gln Ser
        35                  40                  45

Pro Gln Leu Leu Ile Tyr Arg Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Val Gly Val Tyr Tyr Cys Phe Gln Gly
                85                  90                  95

Ser His Gly Pro Trp Thr Phe Gly Gln Gly Thr Lys Leu Glu Ile Lys
            100                 105                 110

Arg

<210>  17
<211>  19
<212>  PRT
<213>  Artificial Sequence

<220>
<223>  Heavy chain signal sequence

<400>  17

Met Glu Trp Ser Trp Val Phe Leu Phe Phe Leu Ser Val Thr Thr Gly
1               5                   10                  15

Val His Ser

<210>  18
<211>  20
<212>  PRT
<213>  Artificial Sequence

<220>
<223>  Light chain signal sequence

<400>  18

Met Ser Val Pro Thr Gln Val Leu Gly Leu Leu Leu Leu Trp Leu Thr

1                   5                   10                  15

Asp Ala Arg Cys
            20

<210>  19
<211>  20
<212>  PRT
<213>  Artificial Sequence

<220>
<223>  Tandem repeat sequence of an extracellular domain of MUC1

<400>  19

His Gly Val Thr Ser Ala Pro Asp Thr Arg Pro Ala Pro Gly Ser Thr
1               5                   10                  15

Ala Pro Pro Ala
            20

## Description

### TECHNICAL FIELD

The present invention relates to a conjugate comprising an anti-human CEACAM5 antibody Fab fragment or a human MUC1 antibody Fab fragment and a ligand. The present invention also relates to a diagnostic composition and/or a pharmaceutical composition comprising the conjugate, a method for diagnosing and/or treating a cancer using the conjugate, and the like. Further, a conjugate comprising a ligand, a spacer, a peptide linker, and a biomolecule, a diagnostic composition and/or a pharmaceutical composition comprising the conjugate, a method for diagnosing and/or treating a disease associated with the biomolecule using the conjugate, and the like. In addition, the present invention relates to a conjugate comprising a complex formed from the ligand and a metal and the anti-human CEACAM5 antibody Fab fragment or the human MUC1 antibody Fab fragment. In addition, the present invention relates to a conjugate comprising the complex, a spacer, a peptide linker, and a biomolecule.

### BACKGROUND ART

A CEA (Carcinoembryonic antigen) or a CEACAM (Carcinoembryonic antigen-related cell adhesion molecule) is a tumor marker discovered in 1965 (J. Exp. Med.;1965;121:439-462, PNAS;1969;64:161-167), and 23 CEA-related molecules have been identified up to now (BioMed Central Biology;2010;8:12-33). Of these, CEACAM5 is rarely expressed in normal tissues, but is expressed in the fetal digestive tract and colorectal cancer (BBA;1990;1032:177-189, J. Clin. Mol. Pathol.;1999;52:174-178). In addition, CEACAM5 is known to also be expressed in breast cancer, lung cancer, and thyroid cancer (Diagn. Cytopathol.;1993;9:377-382, Cancer Res.;1990;50:6987-6994, Histopathology;2000;37:530-535).

The concentration of CEACAM5 in the blood is higher in colorectal cancer patients than in healthy subjects (J. Exp. Med.;1965;121:439-462), and CEACAM5 is used as a tumor marker. In a histological study of colorectal cancer patients, CEACAM5 is highly expressed in 90% or more of the tissues (British J. Cancer;2013; 108:662-667).

Early metastasis of colorectal cancer is localized to the liver, and thus the recurrence rate can be reduced if liver metastasis can be detected and treated at an early stage (Cell Mol. Gastroenterol. Hepatol.;2017;3:163-173).

Mucin 1 (Mucin 1: MUC1) is a membrane-bound glycoprotein expressed on the lumen side of epithelial cells constituting epithelial tissues of mammary glands, tracheas, the digestive tract, and the like (Nat. Rev. Cancer, 2004 Jan;4(1):45-60). MUC1 is overexpressed in cancer cells of breast cancer (Mod. Pathol., 2005 Oct;18(10):1295-304), lung cancer (Hum. Pathol., 2008 Jan;39(1):126-36), colorectal cancer (Int. J. Oncol., 2000 Jan;16(1):55-64), bladder cancer (PLoS One, 2014 Mar;9(3):e92742), skin cancer (Histopathology, 2000, Sep;37(3):218-23), thyroid cancer; (J. Pathol., 2003 Jul;200(3):357-69.), gastric cancer (J. Pathol., 2000 Mar;190(4):437-43), pancreatic cancer (Int. J. Oncol., 2004 Jan;24(1):107-13), kidney cancer (Mod. Pathol., 2004 Feb;17(2):180-8), ovarian cancer (Gynecol. Oncol., 2007 Jun;105(3):695-702), cervical cancer (Am. J. Clin. Pathol., 2004 Jul;122(1):61-9), and the like, and MUC1 is useful as a target molecule for detecting cancer lesions (Nat. Rev. Cancer, 2004 Jan;4(1):45-60, Pathol. Res. Pract., 2010 Aug15;206(8):585-9).

MUC1 is O-glycosylated at threonine 9 of HGVTSAPDTRPAPGSTAPPA (SEQ ID NO: 19 in the sequence listing of the present application), which is a tandem repeat sequence of 20 amino acids present in the extracellular domain. It is known that this O-glycosylation is incomplete in cancer cells, and that O-glycosylations such as T (Galβ1-3GalNAcα1-O-Ser/Thr), Tn (GalNAcα1-O-Ser/Thr), and 2,3ST (Neu5Aca2-3Galβ1-3GalNAcα-O-Ser/Thr) occur in a cancer-specific manner (PTL 1 and NPL 1). MUC1 in normal tissues does not undergo these cancer-specific O-glycosylations, and thus human cancer-specific MUC1 is particularly useful as a target molecule for treating various cancers in humans. As such an anti-human cancer-specific MUC1 antibody, for example, 1B2 antibody (PTL 1), PankoMab antibody (NPL 2), and 5E5 antibody (PTL 2) are known. Among these antibodies, the 1B2 antibody has been reported to have higher specificity for human cancer-specific MUC1 than the PankoMab antibody (PTL 1).

CT (computed tomography), MRI (nuclear magnetic resonance imaging), and FDG-PET (Fluorodeoxyglucose-positron emission tomography) are used for the diagnosis of liver metastasis. The detection sensitivities of CT, MRI, and FDG-PET are 74.4, 80.3, and 81.4%, respectively, and for tumors of 1 cm or less, the detection sensitivity is reduced to 47.3% for CT and 60.2% for MRI. (Radiology;2010;257:674-684). A liver-specific contrast-enhanced MRI is also used, and the detection sensitivity thereof is 29 to 38% for tumors of 1 cm or less (Radiology;2005;237:89-98).

Anti-cancer agents and antibodies bound to metal radioisotopes are used to diagnose and treat cancers. Targeting using an antibody is highly specific for tumor cells and has few side effects. To date, several metal radioisotope-labeled monoclonal antibodies have been clinically applied in diagnosis and treatment (Cancer Control;2012;19:196-203).

On the other hand, antibodies generally have a long half-life in the blood, and after they are administered into the body, it takes a long period of 4 days to 5 days to reach a tumor-to-blood ratio that gives a sufficient signal to visualize a cancer (Clin. Pharmacol. Ther.;2010;87:586-592). In addition, the Fc region of an antibody causes the pharmacological action of antibody-dependent cellular cytotoxicity (ADCC) or complement-dependent cytotoxicity (CDC) (Glycoconj. J.;2013;30:227-236, Curr. Opin. Biotechnol.;2002;13:609-614). In addition, antibodies are metabolized in the liver, and thus they are highly accumulated in the liver regardless of the target, but early metastasis of colorectal cancer is localized to the liver, and thus it is difficult to detect lesions of liver metastasis using an antibody (Clin. Pharmacol. Ther.;2010;87:586-592).

Low molecular weight recombinant antibody fragments such as Fab, scFv, and a diabody are highly tissue-penetrating and easily reach lesions, and can be expected to be produced at low cost using an expression system with Escherichia coli or a yeast and thus they are used as antibodies for treatment, whereas they are characterized by having a short half-life in the blood and being excreted by the kidneys, and thus they have been reported to be used as diagnostic drugs (Nat. Biotechnol.;2005;23:1126-1136).

As an anti-human CEACAM5 antibody applied as a diagnostic drug, M5A (PTL 3), which is a humanized antibody of the mouse monoclonal antibody T84.66, is known. For M5A labeled with ⁶⁴Cu, in a test using mice with cancer cells transplanted subcutaneously, it has been reported that an elapse of 22 hours or more is needed after administration in order to obtain a good PET image (NPL 3), and in addition, in a test using a mouse model of liver metastasis, it has been reported that the uptake into the normal tissues of the liver and the uptake into the lesion sites of the liver were about the same 3 hours after administration and that there was a significant difference after 24 hours (NPL 4).

For an anti-human CEACAM5 antibody fragment, it has been reported that CEA-Scan, which is a mouse monoclonal antibody NP-4 Fab' labeled with ^{99m}Tc, can be used for the diagnosis of colorectal cancer (NPL 5). However, the uptake of CEA-Scan into lesion sites does not exceed the uptake into the normal liver, and the detection sensitivity of liver metastasis is lower than that of FDG-PET (NPL 6). CEA-Scan was approved by FDA as a diagnostic drug for colorectal cancer in 1999, but it is no longer sold (NPL 7).

DOTA (1,4,7,10-tetraazacyclododecane-1,4,7,10-tetraacetic acid) has clinical results and is widely used as a chelator for a radioactive metal. In recent years, a study has been reported in which metal labeling is carried out using DOTA, followed by binding to a peptide and an antibody and targeting (NPL 8).

In cancer treatment, it has been reported that Satoreotide tetraxetan (NPL 9) is under development in phase I as a drug having DOTA. It has been reported that ⁹⁰Y-epratuzumab tetraxetan was administered to a patient having a hematological tumor (NPL 10).

In general, a conjugate to which a chelating agent such as DOTA and a low molecular weight antibody or peptide is bound is highly taken up, retained, or accumulated in the kidneys (NPLs 11 and 12).

As described above, the accumulation of the conjugate in the kidneys causes inconvenience in accurate diagnosis and treatment (NPL 13).

For example, in a test in mice given a conjugate of [¹¹¹In]DOTA-Rituxan Fab, it has been reported that it was highly accumulated in the kidneys (NPL 11).

In order to avoid such high accumulation in the kidneys, the first study of a conjugate modified to an antibody fragment such as Fab, scFV, Fab', or dsFV, and the second study of a conjugate having a linker (also referred to as a peptide linker) specifically cleaved in the kidneys between the chelate and the antibody can be mentioned (NPL 12).

It has been reported that initially, iodohippuric acid-Gly-Lys-Fab in which the peptide linker is Gly (glycine)-Lys (lysine) is cleaved by a renal brush border membrane enzyme, and iodohippuric acid is contained in urine as a metabolite and excreted (NPL 14). In addition, iodohippuric acid-Gly-Tys-Fab, in which the peptide linker is Gly-Tyr (tyrosine), has been reported (NPL 13).

On the other hand, [¹⁸⁸Re]CpTR-Gly-Lys-Fab, in which the peptide linker Gly-Lys is bound to the organorhenium complex CpTR-COOH instead of hippuric acid, has been reported (NPL 15).

In addition, ^{99m}Tc-PGGFML-IT-Fab, in which the peptide linker is Gly-Phe (phenylalanine)-Lys, has been reported (PTL 4).

In addition, focusing on NOTA as a chelate, a conjugate composed of the NOTA and a peptide linker has been reported (PTL 5).

The conjugate having a peptide linker was created, but depending on the type of a chelating agent, the problem of not being cleaved by the enzyme occurs, and a conjugate intended to solve the problem by introducing a linking portion (-CH2-Ph-CO-NH-) having a specific structure between the chelating agent and the peptide linker has been reported (PTL 6). The aim is to obtain a conjugate having a ligand that can coordinate an atom having a relatively large atomic radius, such as indium, which is generally used as a radioisotope. As a spacer via a chelate and a peptide linker, a spacer having a thiourea structure disclosed in PTL 5 was introduced, but it is mentioned that decomposition by a renal brush border membrane enzyme does not proceed.

The conjugate ⁶⁷Ga-NOTA-Met-Ile-Fab, in which the peptide linker aimed at cleavage by a lysosome is Met (methionine)-Ile (isoleucine), has been reported based on the following findings (NPL 16).

It has been reported that the metabolite ⁶⁷Ga-NOTA-Bn-Met produced by lysosomal cleavage of the conjugate NOTA-(p-SCN-Bz)-dsFv having no peptide linker is excreted in urine (NPL 17), and the second from the light chain N terminus of the dsFv of the above conjugate NOTA-(p-SCN-Bz)-dsFv is Ile, and because of these, the conjugate ⁶⁷Ga-NOTA-Met-Ile-Her2 (Herceptin) Fab was designed (NPL 19).

### CITATION LIST

### NON PATENT LITERATURE

NPL 1: Glycoconj. J., 2013 Apr;30(3):227-36.
NPL 2: Cancer Immunol Immunother, 2006 Nov; 55(11): 1337-47
NPL 3: Bioconjug. Chem.; 2008; 19: 89-96
NPL 4: PLOS ONE; 2014; 9(9): e106921
NPL 5: Ann. Surg.; 1997; 226: 621-631
NPL 6: J. Nucl. Med.; 2000; 41: 1657-1663
NPL 7: Kenneth T.Cheng, "99mTc-Arcitumomab", [online], Update: March 17, 2008., Molecular Imaging and Contrast Agent Database, [searched on May 17, 2017], Internet <URL: https://www.ncbi.nlm.nih.gov/books/NBK23676/>
NPL 8: Bioorg. Med. Chem.; 2019; 27: 3248-3253
NPL 9: Clinical Trials. gov Identifier: NCT02592707
NPL 10: Eur J Haematol. 2013 Dec; 91(6): 552-6
NPL 11: Bioconjugate Chem. 2001, 12, 264-270
NPL 12: Bioconjugate Chem. 2002, 13, 985-995
NPL 13: Bioconjugate Chem. 2013, 24, 291-299
NPL 14: Cancer Res. 1999, 59, 128-134
NPL 15: Bioconjugate Chem. 2007, 18, 190-198
NPL 16: Bioconjugate Chem. 2014, 25, 2038-2045
NPL 17: Bioconjugate Chem. 1997, 8, 365-369

### PATENT LITERATURE

PTL 1: International Publication No. WO2010/050528
PTL 2: International Publication No. WO2008/040362
PTL 3: International Publication No. WO2005/086875
PTL 4: International Publication No. WO2013/081091
PTL 5: International Publication No. WO2017/150549
PTL 6: International Publication No. WO2019/065774
PTL 7: International Publication No. WO2018/092885

### SUMMARY OF INVENTION

### TECHNICAL PROBLEM

A monovalent Fab fragment has a molecular weight of about 50 kDa, is smaller than an antibody having a molecular weight of about 150 kDa, is excreted by the kidneys, and has a short half-life in the blood. Because of this, within 2 to 32 hours after administration, a tumor-to-blood ratio that gives a sufficient signal to visualize a cancer is reached. The Fab fragment has no Fc region and thus does not cause ADCC or CDC. The Fab fragment is mainly excreted by the kidneys and thus does not interfere with the detection of liver metastasis. From these features, the Fab fragment can be expected to be more effective as an in-vivo diagnostic drug than an antibody.

However, in the Fab fragment, the binding activity of the Fab fragment is often attenuated because of being monovalent, not divalent as an antibody. Further, in order to use an antibody as an in-vivo diagnostic drug or an agent used in photoimmunotherapy, the antibody must be labeled with a metal, a fluorescent dye, or the like, but a problem is that by labeling with such a substance, the binding activity of the antibody is attenuated.

An object of the present invention is to provide a labeled conjugate useful for an in-vivo diagnostic drug and internal radiation therapy using an anti-human CEACAM5 antibody Fab fragment whose binding activity is not attenuated even by labeling with a metal, a fluorescent dye, or the like. An object of the present invention is to provide a conjugate comprising an anti-human MUC1 antibody Fab fragment (PTL 7), a peptide linker and a ligand, and a conjugate comprising an anti-human MUC1 antibody Fab fragment and a ligand. In addition, another object of the present invention is to provide a diagnostic composition comprising the above conjugate and a method for diagnosis using the same, and to provide a pharmaceutical composition comprising the above conjugate and a method for treatment using the same.

In addition, an object of the present invention is to provide a conjugate having a chelator and a biomolecule accumulating in the kidneys and excreted more rapidly.

### SOLUTION TO PROBLEM

The present inventors previously prepared an anti-human CEACAM5 antibody Fab fragment having a good affinity for human CEACAM5 (International Application PCT/JP2018/025618). As a result of further diligent studies, the present inventors prepared a conjugate wherein a ligand used for labeling is bound to the anti-human CEACAM5 antibody Fab fragment via (or without) a peptide linker, and have found that the conjugate has the same affinity for human CEACAM5 as the anti-human CEACAM5 antibody Fab fragment itself, that is, the binding activity is not attenuated even by the binding between the labeling portion and the Fab fragment, leading to completion of the present invention. That is, the present invention provides a conjugate comprising an anti-human CEACAM5 antibody Fab fragment, a peptide linker, and a ligand, and a conjugate comprising an anti-human CEACAM5 antibody Fab fragment and a specific ligand. It has been confirmed that the conjugate does not attenuate the binding activity to human CEACAM5 even by the binding between the labeling portion and the Fab fragment and retains a good binding activity to human CEACAM5, and based on these results, a mean for diagnosis and a mean for treatment using the conjugate of the present invention are provided.

In addition, the present inventors prepared an anti-human MUC1 antibody Fab fragment having a good affinity for human cancer-specific MUC1, and as a result of further diligent studies, prepared a conjugate wherein a ligand is bound to the anti-human MUC1 antibody Fab fragment via (or without) a peptide linker. The conjugate has the same affinity for human cancer-specific MUC1 as the anti-human MUC1 antibody Fab fragment itself. That is, the present invention provides a conjugate comprising an anti-human MUC1 antibody Fab fragment, a peptide linker, and a ligand, and a conjugate comprising an anti-human MUC1 antibody Fab fragment and a specific ligand. Further, it has been confirmed that the conjugate does not attenuate the binding activity to human cancer-specific MUC1 even by the binding of the labeling portion and retains a good binding activity to human cancer-specific MUC1, and based on these results, a mean for diagnosis and a mean for treatment using the conjugate of the present invention are provided.

In addition, the present inventors studied a conjugate that is excreted more rapidly because a conjugate consisting of a biomolecule such as an antibody useful as a medicament such as a contrast agent or an anticancer agent, a complex formed from a ligand and a metal (also referred to as a metal complex), a spacer, and a peptide linker may accumulate in the kidneys. As a result, the present inventors focused on DOTA (1,4,7,10-tetraazacyclododecane-1,4,7,10-tetraacetic acid), which has clinical results and is widely used as a chelator for a radioactive metal, and have found that a conjugate consisting of 3arm DOTA, a specific spacer, a specific peptide linker, and a biomolecule is decomposed in the kidneys and excreted.

Based on the above, the present invention relates to the following conjugate comprising a CEACAM5 Fab antibody, a diagnostic composition and/or a pharmaceutical composition comprising the conjugate, a method for diagnosing and/or treating a cancer using the conjugate, and the like. In addition, the present invention relates to the following conjugate comprising an MUC1 Fab antibody, a diagnostic composition and/or a pharmaceutical composition comprising the conjugate, a method for diagnosing and/or treating a cancer using the conjugate, and the like. In addition, the present invention relates to a conjugate comprising DOTA, a spacer, a peptide linker, and a biomolecule rapidly excreted by the kidneys, an intermediate of the conjugate, and a method for diagnosing and/or treating a disease associated with a biomolecule using the conjugate, and the like.
[1] A conjugate represented by the following formula (I):

   (Y-S₁-X)ₚ-Fab¹ (I)

   wherein
   Fab¹ is an anti-human CEACAM5 antibody Fab fragment selected from the group consisting of
   (a) an anti-human CEACAM5 antibody Fab fragment comprising a heavy chain fragment including a heavy chain variable region consisting of the amino acid sequence of amino acids 1 to 121 of SEQ ID NO: 2 and a light chain including a light chain variable region consisting of the amino acid sequence of amino acids 1 to 112 of SEQ ID NO: 4, and
   (b) an anti-human CEACAM5 antibody Fab fragment comprising a heavy chain fragment including a heavy chain variable region which consists of the amino acid sequence of amino acids 1 to 121 of SEQ ID NO: 2 and in which glutamic acid of amino acid 1 of SEQ ID NO: 2 is modified to pyroglutamic acid, and a light chain including a light chain variable region consisting of the amino acid sequence of amino acids 1 to 112 of SEQ ID NO: 4,
      the Fab¹ is bound to X via p amino groups or thiol groups in the Fab¹;
      X is a peptide linker or a bond;
      S₁ is a spacer or a bond;
      Y is a ligand; and
      p is a natural number of 1 to 25 and represents the number of a group (Y-S₁-X) bound to Fab¹;
      provided that when X is a bond, S₁ is -CH₂-(1,4-phenylene)-NH-C(=S)- or a bond, and Y is 1,4,7,10-tetraazacyclododecane-1,4,7,10-tetraacetic acid (hereinafter, sometimes abbreviated as DOTA).
[2] The conjugate according to [1], wherein
   Fab¹ is selected from the group consisting of
   (a) an anti-human CEACAM5 antibody Fab fragment comprising a heavy chain fragment consisting of the amino acid sequence shown in SEQ ID NO: 2 and a light chain consisting of the amino acid sequence shown in SEQ ID NO: 4, and
   (b) an anti-human CEACAM5 antibody Fab fragment comprising a heavy chain fragment which consists of the amino acid sequence shown in SEQ ID NO: 2 and in which glutamic
   acid of amino acid 1 of SEQ ID NO: 2 is modified to pyroglutamic acid, and a light chain consisting of the amino acid sequence shown in SEQ ID NO: 4.
[3] The conjugate according to [2], wherein Fab¹ comprises a Fab fragment comprising a heavy chain fragment consisting of the amino acid sequence shown in SEQ ID NO: 2 and a light chain consisting of the amino acid sequence shown in SEQ ID NO: 4 (hereinafter, referred to as Fab²).
[4] The conjugate according to any of [1] to [3], wherein X is a peptide linker including a peptide consisting of 2 to 4 amino acids having an amino acid sequence cleaved by a renal brush border membrane enzyme or a lysosomal enzyme.
[5] The conjugate according to any of [1] to [4], wherein
   S₁ is
   - C(=O)-CH₂O-(1,3-phenylene)-C(=O)-,
   - C(=O)-(CH₂CH₂O)₄-(1,3-phenylene)-C(=O)-,
   - C(=O)-(1,3-phenylene)-C(=O)-,
   - NH-CH₂-(1,3-phenylene)-C(=O)-,
   - NH-(CH₂)₂-C(=O)-,
   - CH₂-(1,4-phenylene)-NH-C(=S)-, -NH-(CH₂CH₂O)₃-CH₂-C(=O)-NH-CH₂-(1,3-phenylene)-C(=O)-,
   - NH-CH₂-(1,4-phenylene)-NH-C(=O)-,
   - NH-(CH₂)₃-C(=O)-,
   - NH-(CH₂CH₂O)₃-CH₂-C(=O)-,
   - NH-CH₂-(1,4-phenylene)-C(=O)-,
   - CH₂-(1,4-phenylene)-NH-C(=S)-NH-CH₂-(1,3-phenylene)-C(=O)-,
   - CH₂-(1,4-phenylene)-NH-C(=S)-NH-(CH₂CH₂O)₃-CH₂-C(=O)-NH-CH₂-(1,3-phenylene)-C(=O)-,
   a spacer represented by any of the following formulas (a) to (q), or a bond, 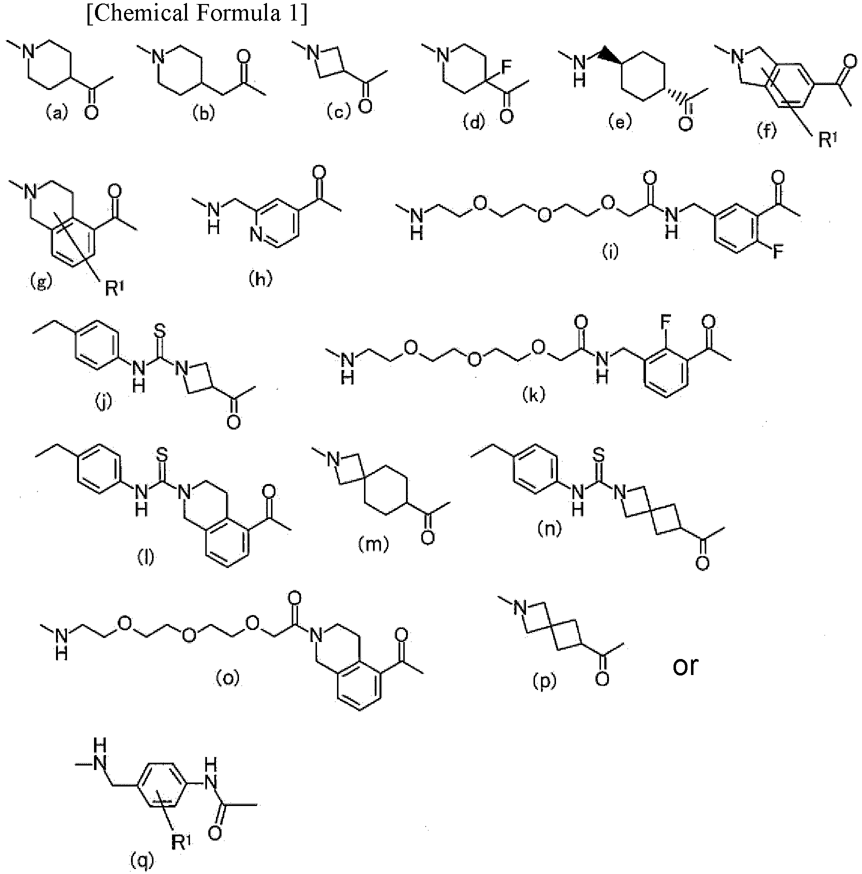 wherein R¹ means a hydrogen atom, a halogen, or C₁₋₆ alkyl, and the same applies below; and
   X is a peptide linker selected from the group consisting of
   (1) -Met-Ile-NH-(CH₂)₂-Z₁-,
   (2) -Gly-Lys^{∗}-Z₂-,
   (3) -Gly-Phe-Lys^{∗}-Z₂-,
   (4) -Met-Val-Lys^{∗}-Z₂-,
   (5) -Gly-Tyr^{∗}-CH₂-C(=O)-NH-(CH₂)₂-Z₁-,
   (6) -Gly-Lys-Lys^{∗}-Z₂-,
   (7) -Gly-Arg-Lys^{∗}-Z₂-,
   (8) -Gly-Lys^{∗}-C(=S)-NH-(1,4-phenylene)-NH-C(=S)-,
   (9)-Met-Ile-NH-(CH₂)₂-NH-C(=S)-NH-(1,4-phenylene)-NH-C(=S)-,
   (10) -Asp-Gly-Lys^{∗}-Z₂-,
   (11) -Met-Gly-Lys^{∗}-Z₂-,
   (12) -Met-Ile-Lys^{∗}-Z₂-,
   (13) -Gly-Tyr^{∗}-CH₂-C(=O)-Lys^{∗}-Z₂-,
   (14) -Val-NH-(CH₂)₂-Z₁-,
   (15) -Ile-NH-(CH₂)₂-Z₁-,
   (16) -Gly-Val-NH-(CH₂)₂-Z₁-,
   (17) -Gly-Ile-NH-(CH₂)₂-Z₁-,
   (18) -Met-Phe-Lys^{∗}-Z₂-,
   (19) -Gly-Tyr-Lys^{∗}-Z₂-,
   (20) -Gly-Tyr^{∗}-CH₂-C(=O)-NH-(CH₂O)₃-CH₂-C(=O)-NH-(CH₂)₂-Z₁-,
   (21) -Gly-(3-(2-naphthyl)alanine)-Lys^{∗}-Z₂-,
   (22) -Gly-diphenylalanine-Lys^{∗}-Z₂-,
   (23) -Gly-Tyr-NH-(CH₂)₅-Z₁-,
   (24) -Met-Ile-NH-(CH₂)₂-(A-4)-,
   (25) -Met-Ile-NH-(CH₂)₂-(A-5)-,
   (26) -Met-Ile-NH-(CH₂)₂-(II-II)-,
   (27) -Met-Ile-NH-(CH₂)₂-(A-3)-, and
   (28) -Met-Gly-Lys^{∗}-Z₃-, or
   (29) a bond,
   wherein Met represents methionine, Ile represents isoleucine, Gly represents glycine, Lys represents lysine, Phe represents phenylalanine, Val represents valine, Tyr represents tyrosine, Arg represents arginine, Asp represents aspartic acid, Z₁ represents a group represented by the following formula (II-I) or (II-II), and -Lys^{∗}-Z₂- represents a group represented by the following formula (III-I) or (III-II), Tyr^{∗}-CH₂- represents a group represented by the following formula (IV), -Lys^{∗}-C(=S)- represents a group represented by the following formula (V), -Lys^{∗}-Z₃- represents a group represented by the following formula (III-III), and
   group (A-3), (A-4), or (A-5) is as represented by the following formulas. 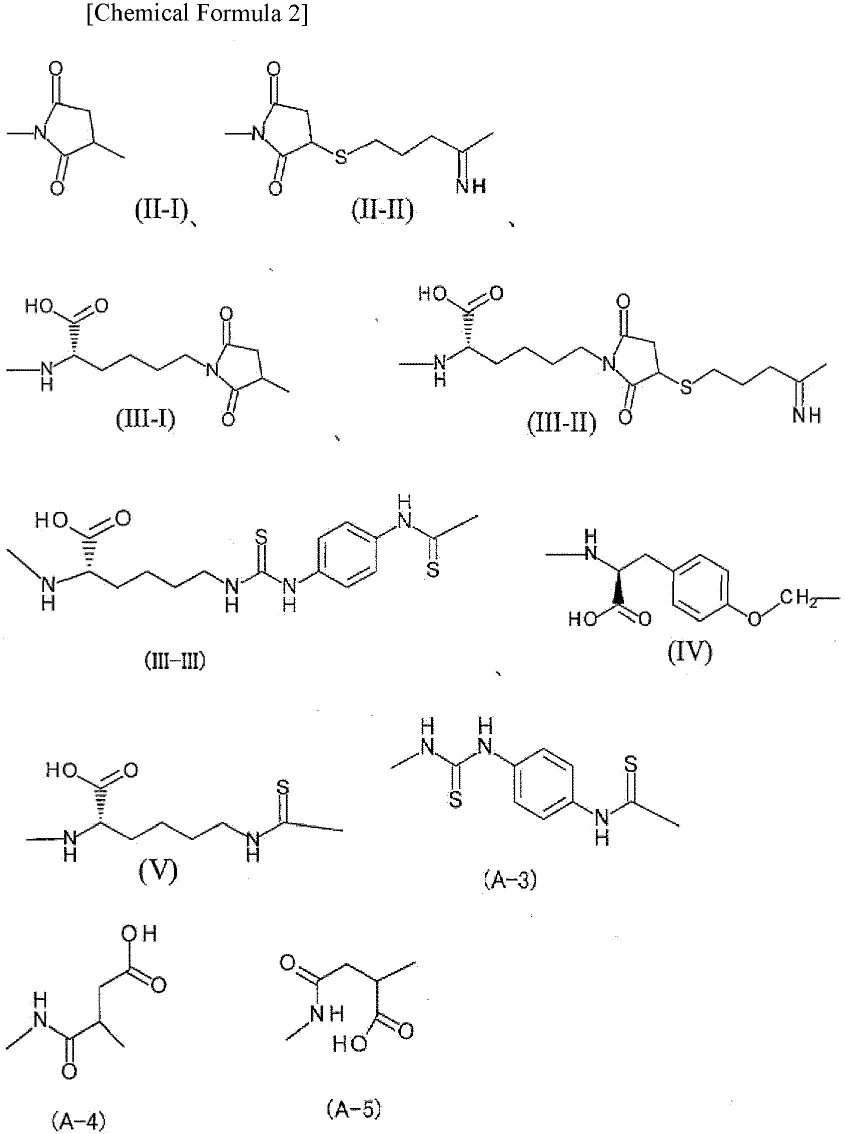
[6] The conjugate according to any of [1] to [5], wherein S₁ is -C(=O)-CH₂O-(1,3-phenylene)-C(=O)-, -C(=O)-(CH₂CH₂O)₄-(1,3-phenylene)-C(=O)-, or -C(=O)-(1,3-phenylene)-C(=O)-, and
   X is a peptide linker selected from the group consisting of
   (1) -Met-Ile-NH-(CH₂)₂-Z₁-,
   (2) -Gly-Lys^{*}-Z₂-,
   (3) -Gly-Phe-Lys^{*}-Z₂-,
   (5) -Gly-Tyr^{*}-CH₂-C(=O)-NH-(CH₂)₂-Z₁-,
   (6) -Gly-Lys-Lys^{∗}-Z₂-, and
   (8) -Gly-Lys^{∗}-C(=S)-NH-(1,4-phenylene)-NH-C(=S)-.
[7-1] The conjugate according to any of [1] to [5], wherein S₁ is -C(=O)-CH₂O-(1,3-phenylene)-C(=O)-, -C(=O)-(CH₂CH₂O)₄-(1,3-phenylene)-C(=O)-, -NH-CH₂-(1,3-phenylene)-C(=O)-, -NH-CH₂-(1,4-phenylene)-NH-C(=O)-, -NH-CH₂-(1,4-phenylene)-C(=O)-, -C(=O)-(1,3-phenylene)-C(=O)-, or the following formula 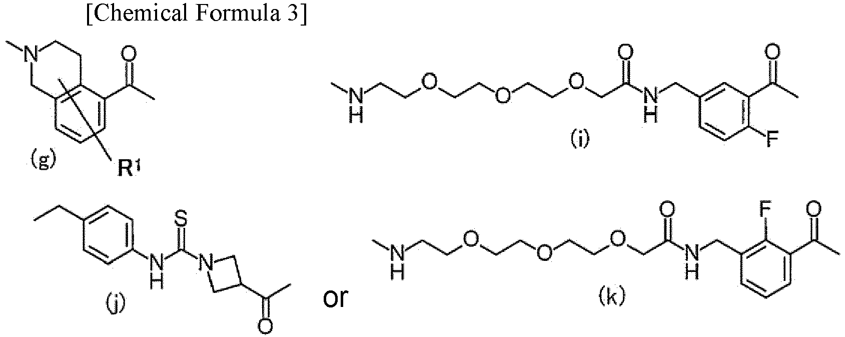 , and
   X is a peptide linker selected from the group consisting of
   (1) -Met-Ile-NH-(CH₂)₂-Z₁-,
   (2) -Gly-Lys^{*}-Z₂-,
   (3) -Gly-Phe-Lys^{*}-Z₂-,
   (5) -Gly-Tyr^{*}-CH₂-C(=O)-NH-(CH₂)₂-Z₁-,
   (6)-Gly-Lys-Lys^{∗}-Z₂-,
   (8) -Gly-Lys^{*}-C(=S)-NH-(1,4-phenylene)-NH-C(=S)-,
   (24) -Met-Ile-NH-(CH₂)₂-(A-4)-,
   (25) -Met-Ile-NH-(CH₂)₂-(A-5)-,
   (26) -Met-Ile-NH-(CH₂)₂-(II-II)-,
   (27) -Met-Ile-NH-(CH₂)₂-(A-3)-, and
   (28) -Met-Gly-Lys^{∗}-Z₃-.
[7-2] The conjugate according to [7-1], wherein S₁ is -NH-CH₂-(1,4-phenylene)-NH-C(=O)-, or the following formula 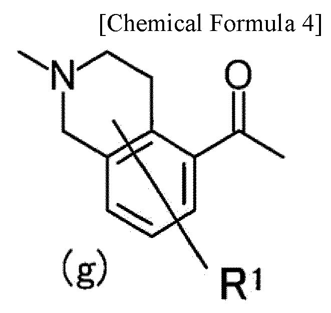 , and
   X is a peptide linker selected from the group consisting of
   (1) -Met-Ile-NH-(CH₂)₂-Z₁-,
   (24) -Met-Ile-NH-(CH₂)₂-(A-4)-,
   (25) -Met-Ile-NH-(CH₂)₂-(A-5)-,
   (26) -Met-Ile-NH-(CH₂)₂-(II-II)-,
   (27) -Met-Ile-NH-(CH₂)₂-(A-3)-, and
   (28) -Met-Gly-Lys^{∗}-Z₃-.
[8] The conjugate according to any of [1] to [5], wherein S₁ is -NH-CH₂-(1,3-phenylene)-C(=O)-, -NH-(CH₂)₂-C(=O)-, -CH₂-(1,4-phenylene)-NH-C(=S)-, -NH-(CH₂CH₂O)₃-CH₂-C(=O)-NH-CH₂-(1,3-phenylene)-C(=O)-, -NH-CH₂-(1,4-phenylene)-NH-C(=O)-, -NH-(CH₂)₃-C(=O)-, -NH-(CH₂CH₂O)₃-CH₂-C(=O)-, -NH-CH₂-(1,4-phenylene)-C(=O)-, -CH₂-(1,4-phenylene)-NH-C(=S)-NH-CH₂-(1,3-phenylene)-C(=O)-, -CH₂-(1,4-phenylene)-NH-C(=S)-NH-(CH₂CH₂O)₃-CH₂-C(=O)-NH-CH₂-(1,3-phenylene)-C(=O)-, a spacer represented by any of the following formulas (a) to (q), or a bond 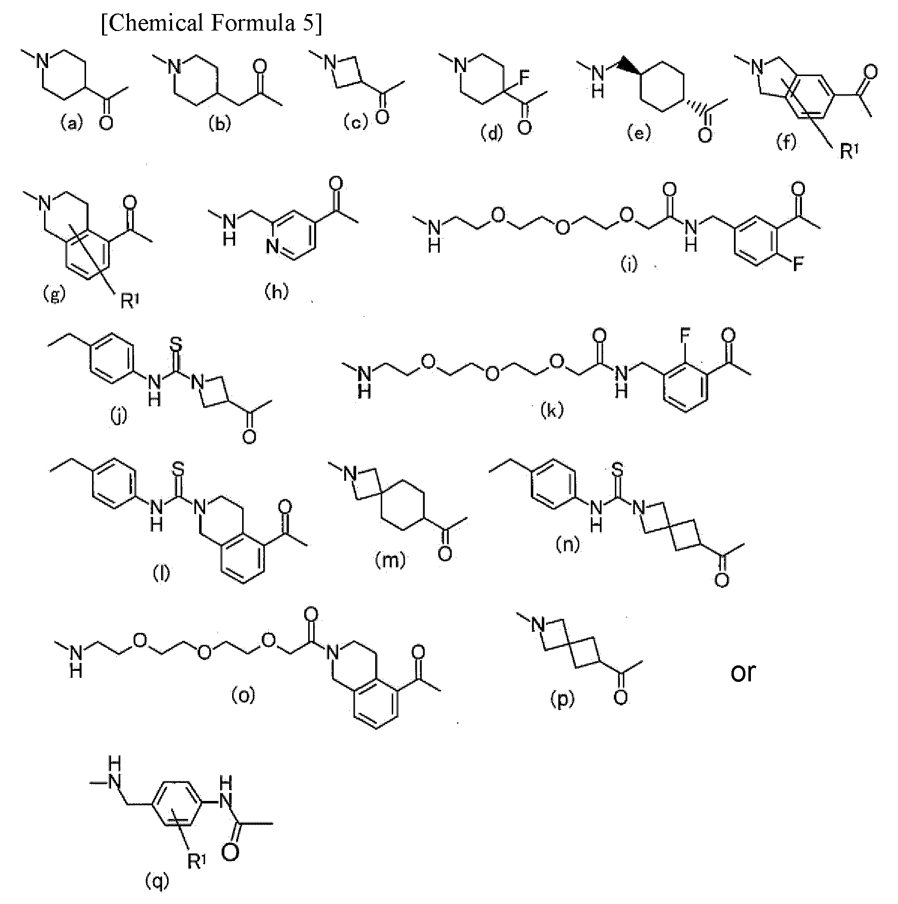 , and
   X is a peptide linker selected from the group consisting of
   (1) -Met-Ile-NH-(CH₂)₂-Z₁-,
   (2) -Gly-Lys^{*}-Z₂-,
   (3) -Gly-Phe-Lys^{*}-Z₂-,
   (4) -Met-Val-Lys^{*}-Z₂-,
   (5) -Gly-Tyr^{*}-CH₂-C(=O)-NH-(CH₂)₂-Z₁-,
   (7) -Gly-Arg-Lys^{∗}-Z₂-,
   (8) -Gly-Lys^{*}-C(=S)-NH-(1,4-phenylene)-NH-C(=S)-,
   (9)-Met-Ile-NH-(CH₂)₂-NH-C(=S)-NH-(1,4-phenylene)-NH-C(=S)-,
   (10) -Asp-Gly-Lys^{*}-Z₂-,
   (11) -Met-Gly-Lys^{*}-Z₂-,
   (12) -Met-Ile-Lys^{*}-Z₂-,
   (13) -Gly-Tyr^{∗}-CH₂-C(=O)-Lys^{∗}-Z₂-,
   (14) -Val-NH-(CH₂)₂-Z₁-,
   (15) -Ile-NH-(CH₂)₂-Z₁-,
   (16) -Gly-Val-NH-(CH₂)₂-Z₁-,
   (17) -Gly-Ile-NH-(CH₂)₂-Z₁-,
   (18) -Met-Phe-Lys^{∗}-Z₂-,
   (19) -Gly-Tyr-Lys^{∗}-Z₂-,
   (20) -Gly-Tyr^{∗}-CH₂-C(=O)-NH-(CH₂O)₃-CH₂-C(=O)-NH-(CH₂)₂-Z₁-,
   (21) -Gly-(3-(2-naphthyl)alanine)-Lys^{*}-Z₂-,
   (22) -Gly-diphenylalanine-Lys^{∗}-Z₂-,
   (23) -Gly-Tyr-NH-(CH₂)₅-Z₁-
   (24) -Met-Ile-NH-(CH₂)₂-(A-4)-,
   (25) -Met-Ile-NH-(CH₂)₂-(A-5)-,
   (26) -Met-Ile-NH-(CH₂)₂-(II-II)-,
   (27) -Met-Ile-NH-(CH₂)₂-(A-3)-, and
   (28) -Met-Gly-Lys^{∗}-Z₃-.
[9] The conjugate according to any of [1] to [5], wherein S₁ is -NH-CH₂-(1,3-phenylene)-C(=O)-, -CH₂-(1,4-phenylene)-NH-C(=S)-, -NH-(CH₂CH₂O)₃-CH₂-C(=O)-NH-CH₂-(1,3-phenylene)-C(=O)-, -CH₂-(1,4-phenylene)-NH-C(=S)-NH-CH₂-(1,3-phenylene)-C(=O)-, -NH-CH₂-(1,4-phenylene)-NH-C(=O)-, a spacer represented by any of the following formulas (e) to (i) or (k), or a bond 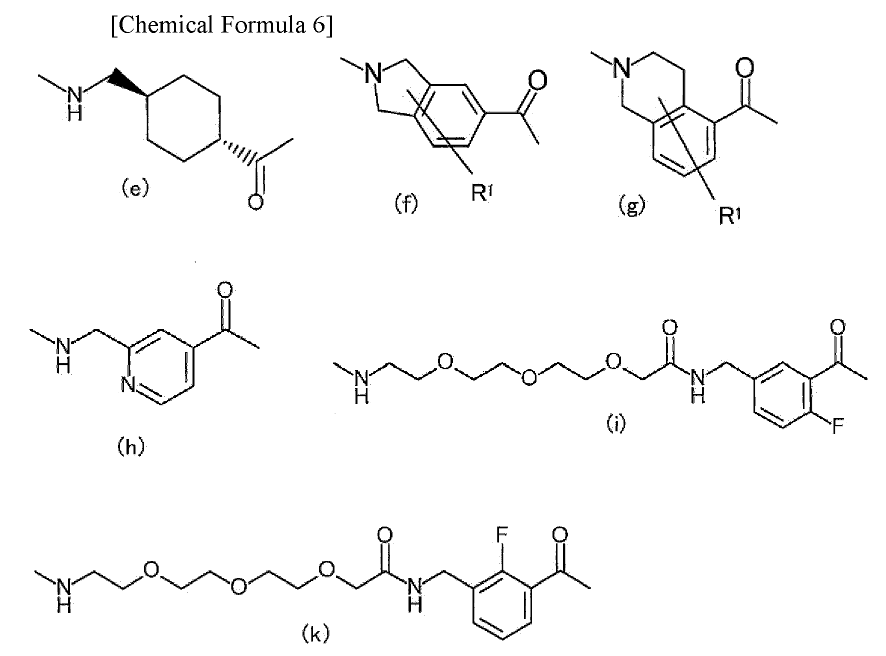 , and
   X is a peptide linker selected from the group consisting of
   (1) -Met-Ile-NH-(CH₂)₂-Z₁-,
   (2) -Gly-Lys^{*}-Z₂-,
   (3) -Gly-Phe-Lys^{*}-Z₂-,
   (9)-Met-Ile-NH-(CH₂)₂-NH-C(=S)-NH-(1,4-phenylene)-NH-C(=S)-,
   (10) -Asp-Gly-Lys^{*}-Z₂-,
   (11) -Met-Gly-Lys^{*}-Z₂-,
   (12) -Met-Ile-Lys^{*}-Z₂-,
   (21) -Gly-(3-(2-naphthyl)alanine)-Lys^{*}-Z₂-,
   (24) -Met-Ile-NH-(CH₂)₂-(A-4)-,
   (25) -Met-Ile-NH-(CH₂)₂-(A-5)-,
   (26) -Met-Ile-NH-(CH₂)₂-(II-II)-,
   (27) -Met-Ile-NH-(CH₂)₂-(A-3)-, and
   (28) -Met-Gly-Lys^{∗}-Z₃-.
[10] The conjugate according to any of [1] to [5], wherein S₁ is a group selected from the group consisting of -NH-CH₂-(1,3-phenylene)-C(=O)-, -CH₂-(1,4-phenylene)-NH-C(=S)-, - NH-(CH₂CH₂O)₃-CH₂-C(=O)-NH-CH₂-(1,3-phenylene)-C(=O)-, -CH₂-(1,4-phenylene)-NH-C(=S)-NH-CH₂-(1,3-phenylene)-C(=O)-, -NH-CH₂-(1,4-phenylene)-NH-C(=O)-, a spacer represented by the following formula (f) or (g), or a bond 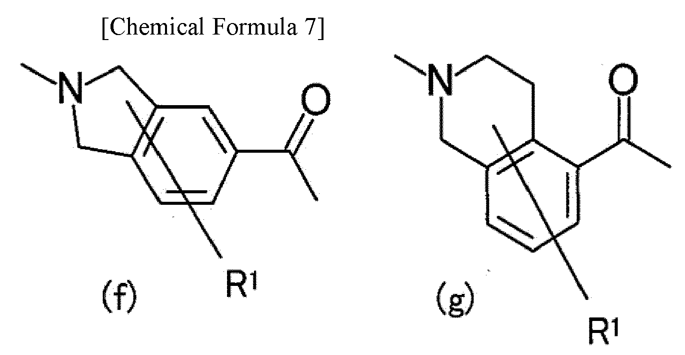 , and
   X is a peptide linker selected from the group consisting of
   (1) -Met-Ile-NH-(CH₂)₂-Z₁-,
   (4) -Met-Val-Lys^{*}-Z₂-,
   (9)-Met-Ile-NH-(CH₂)₂-NH-C(=S)-NH-(1,4-phenylene)-NH-C(=S)-,
   (11) -Met-Gly-Lys^{*}-Z₂-,
   (12) -Met-Ile-Lys^{*}-Z₂-,
   (18) -Met-Phe-Lys^{∗}-Z₂-,
   (24) -Met-Ile-NH-(CH₂)₂-(A-4)-,
   (25) -Met-Ile-NH-(CH₂)₂-(A-5)-,
   (26) -Met-Ile-NH-(CH₂)₂-(II-II)-,
   (27) -Met-Ile-NH-(CH₂)₂-(A-3)-, and
   (28) -Met-Gly-Lys^{∗}-Z₃-.
[11] The conjugate according to any of [1] to [5], wherein X is a peptide linker selected from the group consisting of
   (1) -Met-Ile-NH-(CH₂)₂-Z₁-,
   (9)-Met-Ile-NH-(CH₂)₂-NH-C(=S)-NH-(1,4-phenylene)-NH-C(=S)-,
   (12) -Met-Ile-Lys^{*}-Z₂-,
   (24) -Met-Ile-NH-(CH₂)₂-(A-4)-,
   (25) -Met-Ile-NH-(CH₂)₂-(A-5)-,
   (26) -Met-Ile-NH-(CH₂)₂-(II-II)-, and
   (27) -Met-Ile-NH-(CH₂)₂-(A-3)-.
[12] The conjugate according to any of [1] to [5], wherein X is a peptide linker selected from the group consisting of
   (4) -Met-Val-Lys^{*}-Z₂-,
   (11) -Met-Gly-Lys^{*}-Z₂-,
   (18) -Met-Phe-Lys^{∗}-Z₂-, and
   (28) -Met-Gly-Lys^{∗}-Z₃-.
[13] The conjugate according to any of [1] to [5], wherein X is a peptide linker selected from the group consisting of
   (11) -Met-Gly-Lys^{*}-Z₂-, and
   (28) -Met-Gly-Lys^{∗}-Z₃-.
[14] The conjugate according to any of [1] to [5], wherein the conjugate is a conjugate selected from the group consisting of the compounds represented by the following formulas, and Fab¹ is bound to an adjacent carbon atom via p amino groups or thiol groups in the Fab¹. 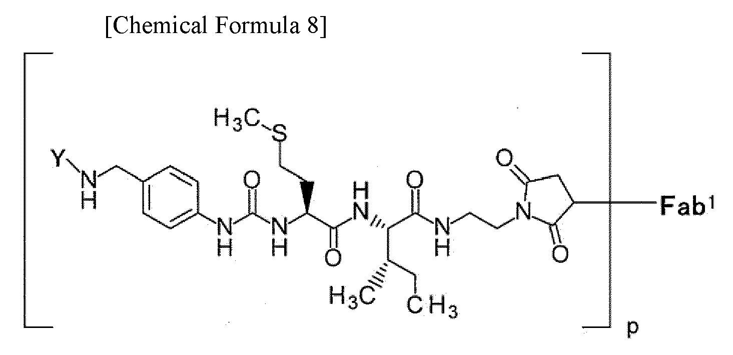 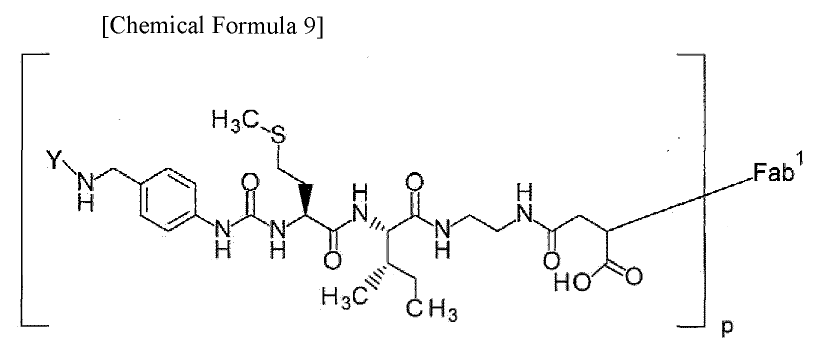 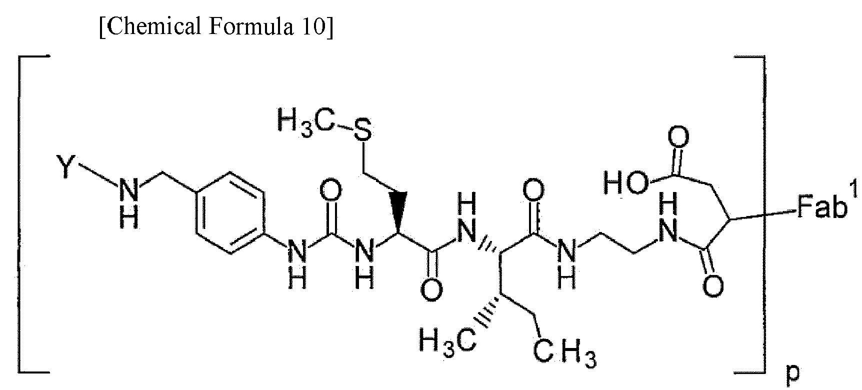 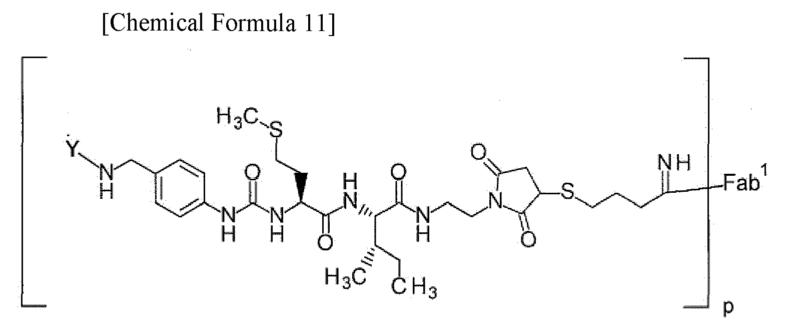 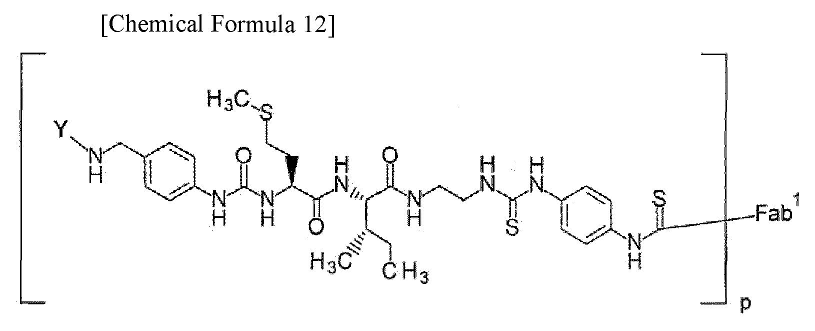 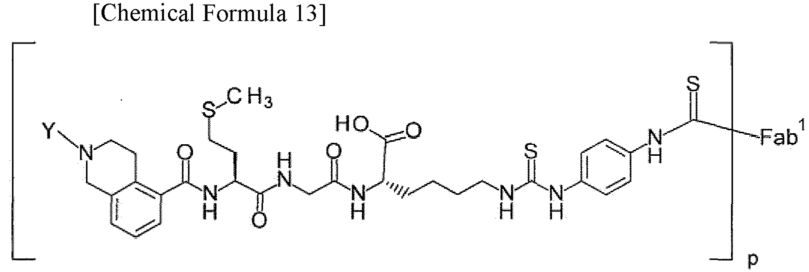
[15] The conjugate according to any of [1] to [14], wherein Y is deferoxamine (hereinafter, sometimes abbreviated as DFO) or 1,4,7,10-tetraazacyclododecane-1,4,7,10-tetraacetic acid (hereinafter, sometimes abbreviated as DOTA).
[16] The conjugate according to [15], wherein Y is DFO.
[17] The conjugate according to [15], wherein Y is DOTA.
[18] The conjugate according to [17], wherein Y is 3arm DOTA or 4arm DOTA.
[19] The conjugate according to [18], wherein Y is 3arm DOTA.
[20] The conjugate according to [18], wherein Y is 4arm DOTA.
[21] The conjugate according to any of [15] or [17] to [20], wherein the conjugate is a conjugate selected from the group consisting of the compounds represented by the following formulas, and Fab¹ is bound to an adjacent carbon atom via p amino groups or thiol groups in the Fab¹. 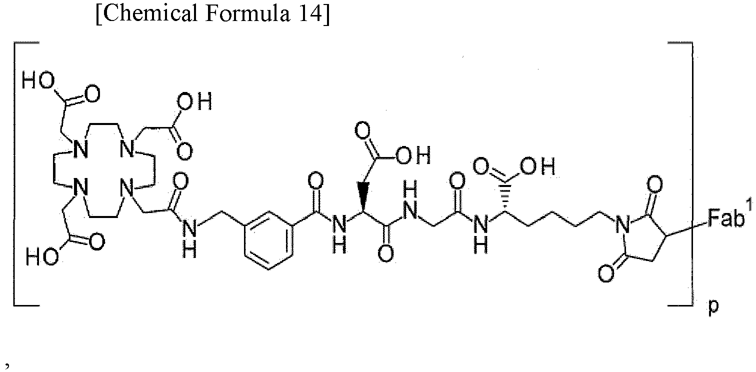 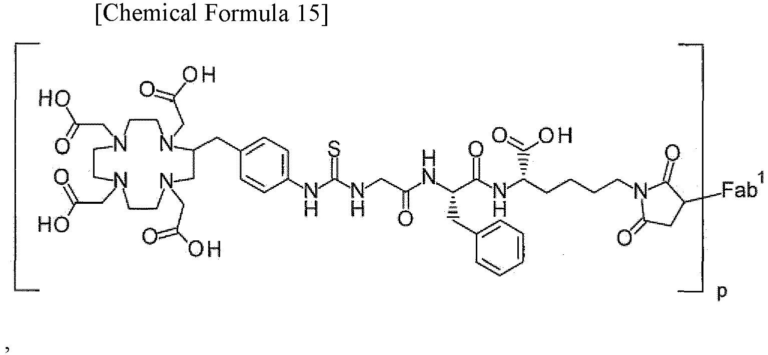 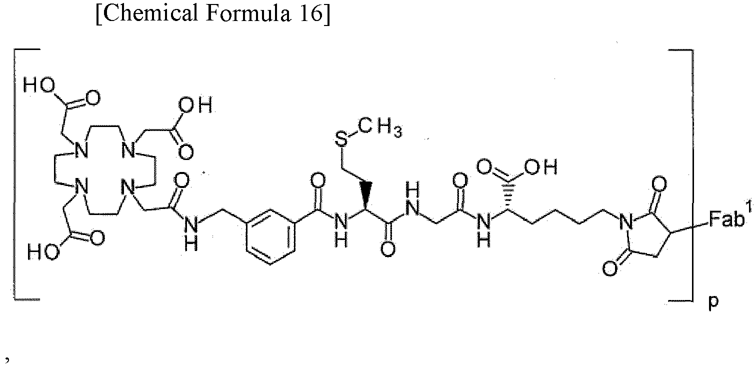 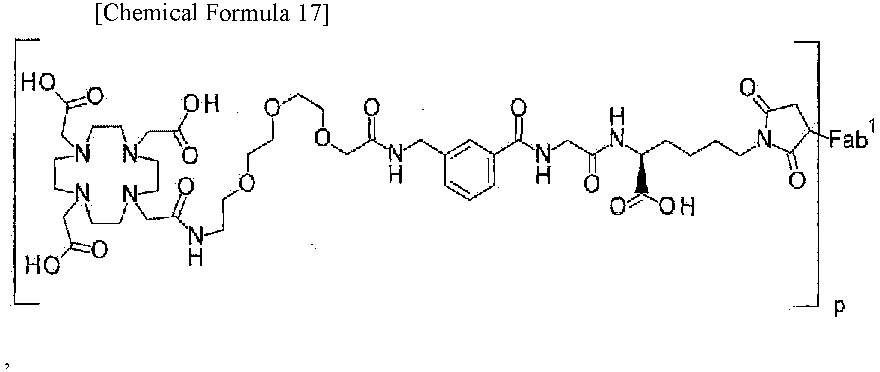 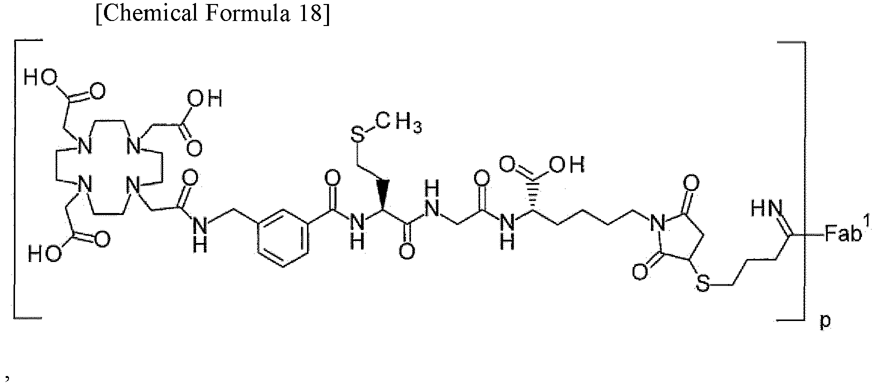 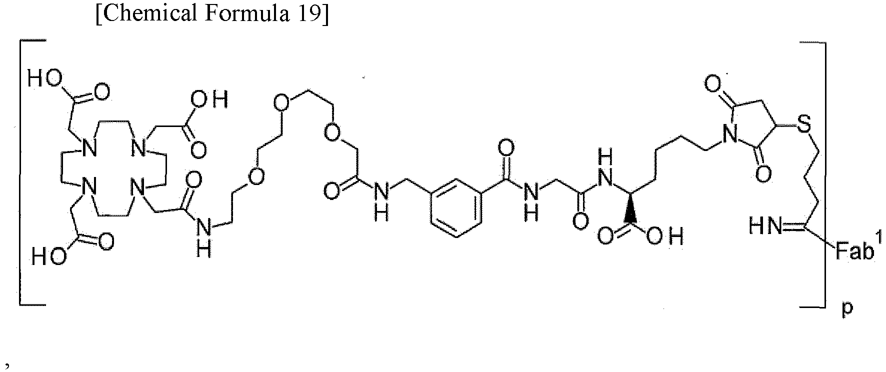 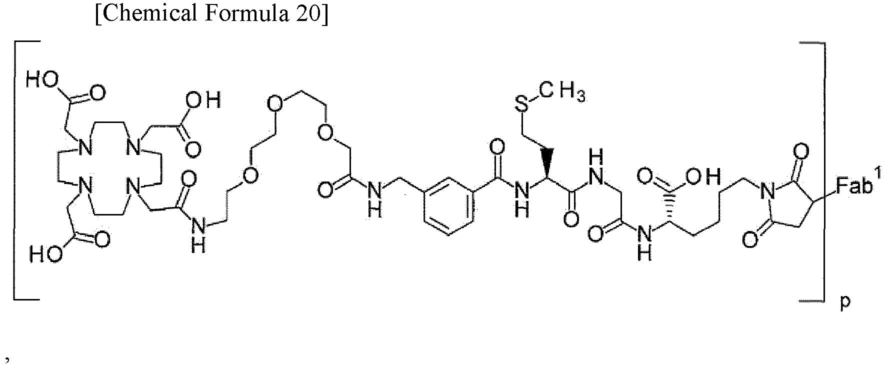 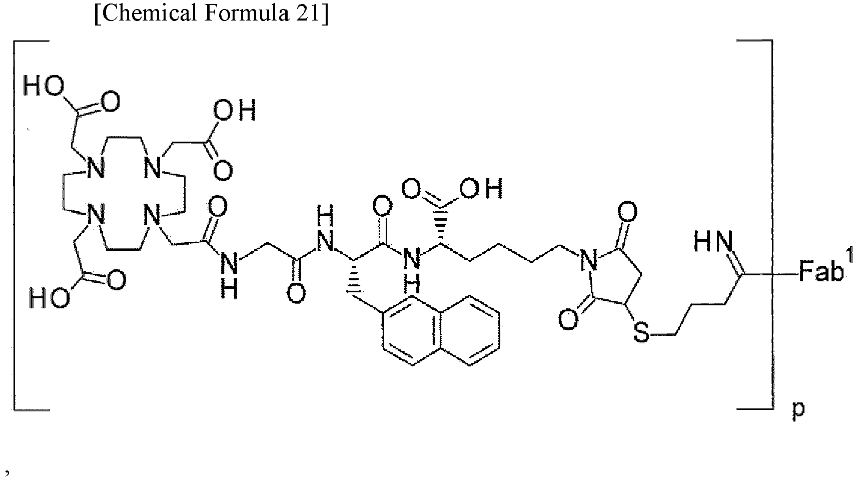 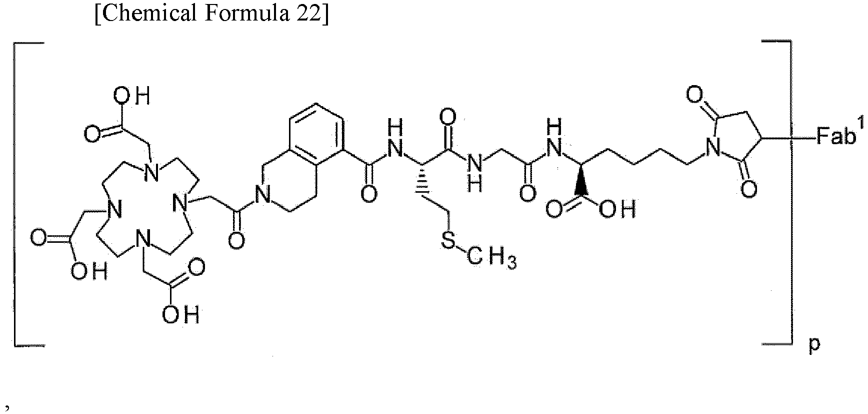 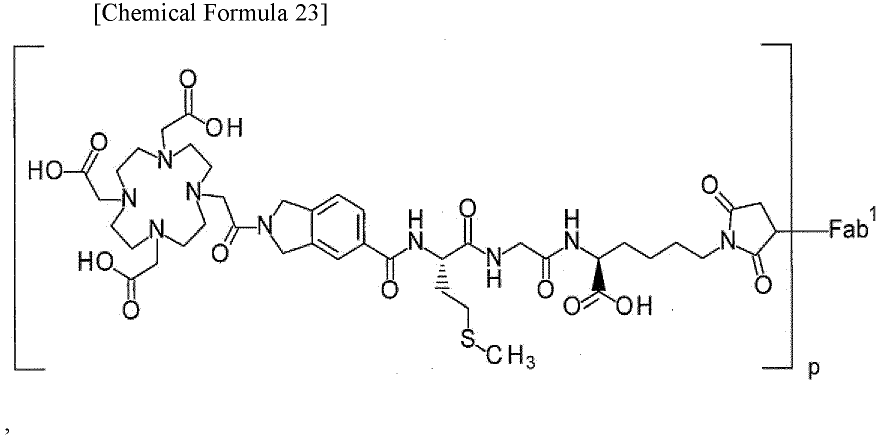 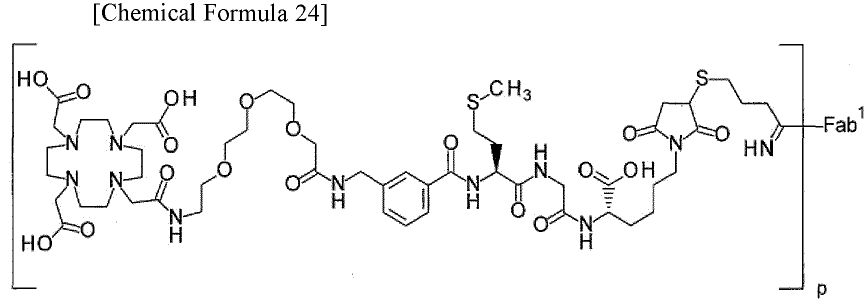 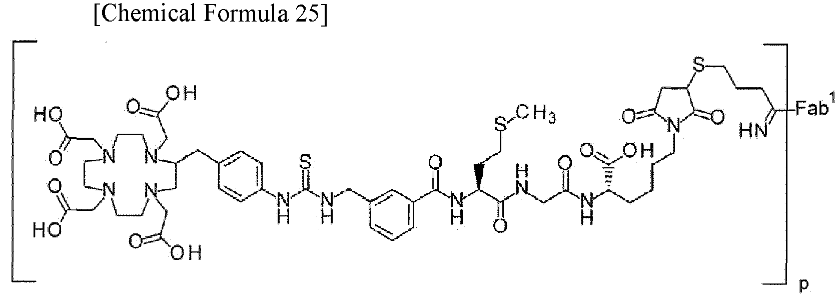 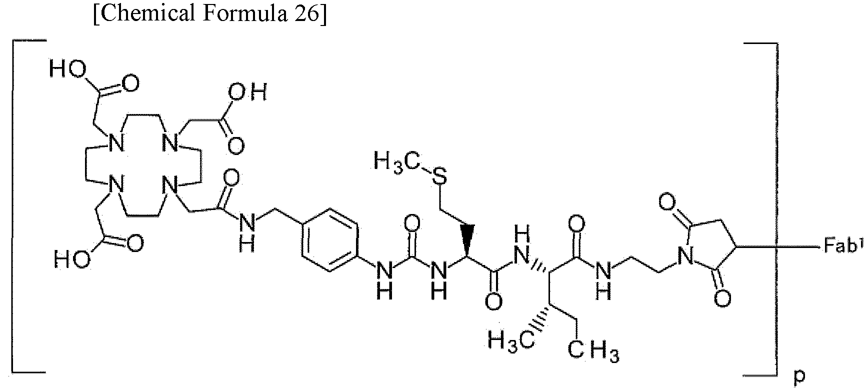 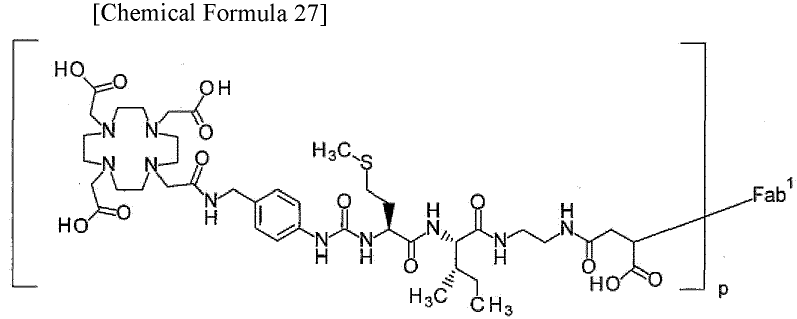 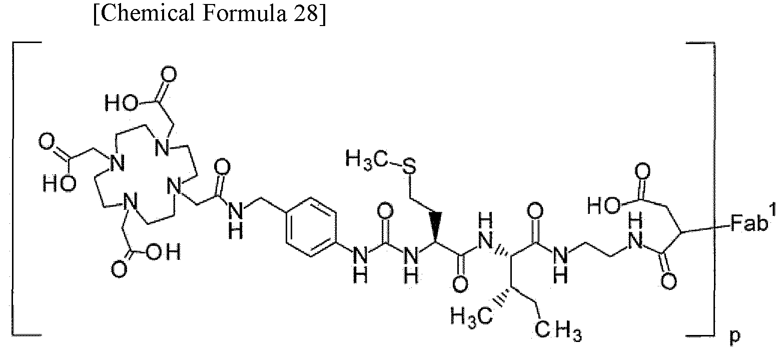 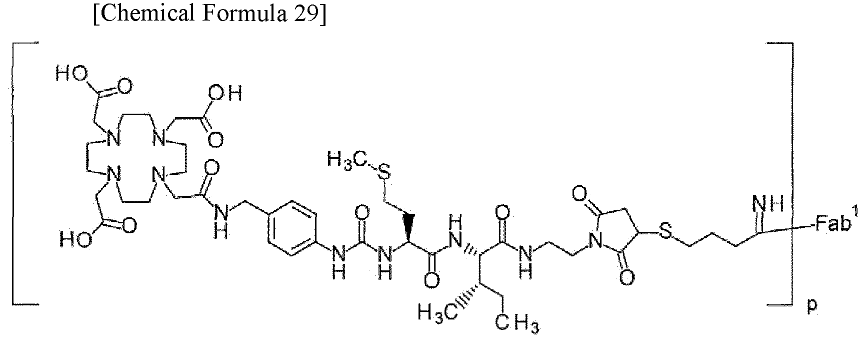 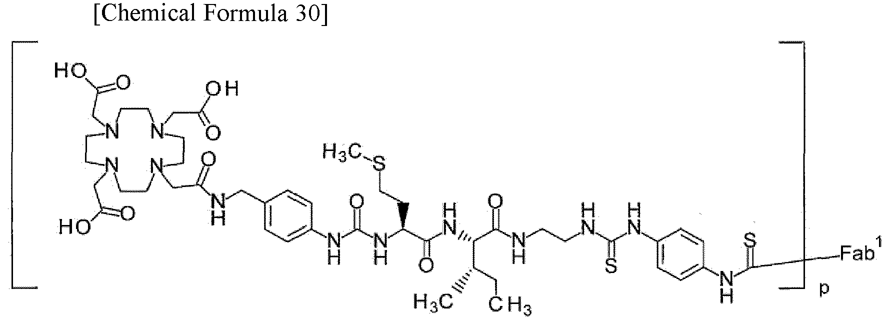 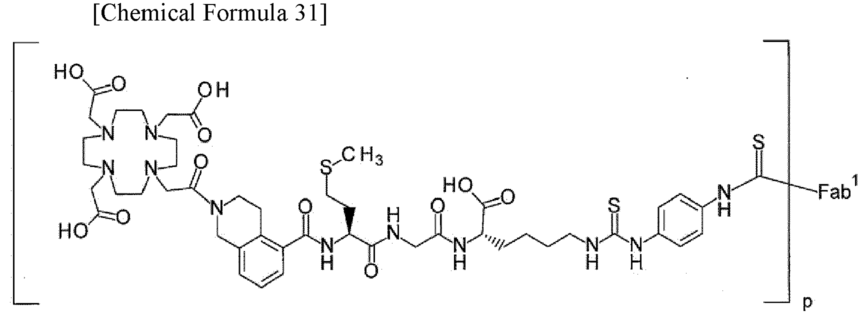 In addition, the conjugate of the present invention may be a mixture of the following two conjugates. 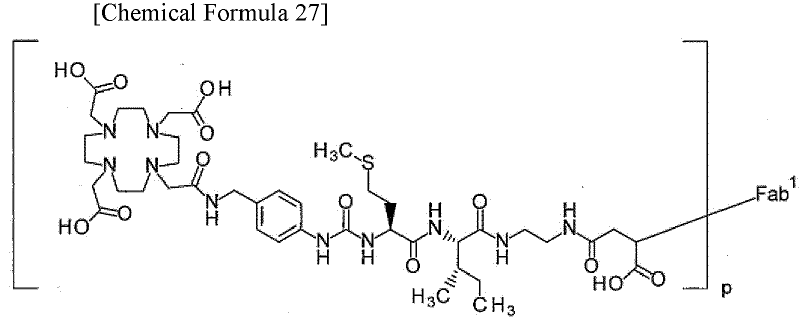 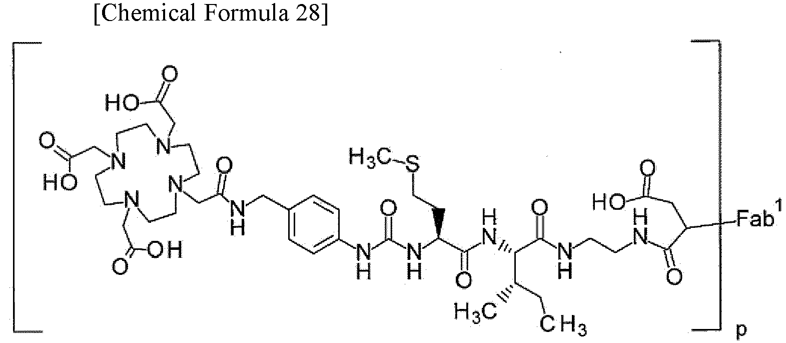
[22] The conjugate according to any of [15] or [17] to [20], wherein the conjugate is a conjugate selected from the group consisting of the compounds represented by the following formulas, and Fab¹ is bound to an adjacent carbon atom via p amino groups or thiol groups in the Fab¹. 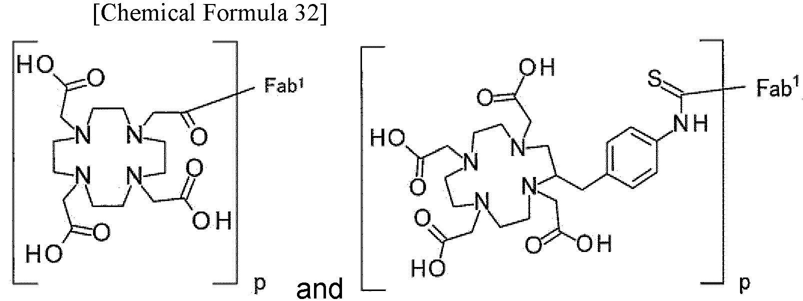
[23] The conjugate according to any of [1] to [22], wherein p is a natural number of 1 to 5.
[24] The conjugate according to any of [1] to [20], wherein a metal is coordinated to Y.
[25] The conjugate according to any one of [21] to [22], wherein a metal is coordinated.
[26] The conjugate according to [24] or [25], wherein the metal is a metal radioisotope.
[27] The conjugate according to [26], wherein the metal is ⁸⁹Zr.
[28] The conjugate according to [24] or [25], wherein the metal is a paramagnetic metal ion.
[29] The conjugate according to [28], wherein the metal is Gd³⁺.
[30] The conjugate according to any of [24] to [29], wherein the conjugate is a PET tracer.
[31] A diagnostic composition comprising one or more conjugates according to any of [24] to [30] and a pharmaceutically acceptable carrier.
[32] The diagnostic composition according to [31], wherein the diagnostic composition is used as an early diagnostic drug or a staging drug.
[33] The diagnostic composition according to any of [31] or [32], wherein the diagnostic composition is used for diagnosing a cancer expressing human CEACAM5.
[34] The diagnostic composition according to [33], wherein the cancer is colorectal cancer, breast cancer, lung cancer, thyroid cancer, or a cancer resulting from metastasis thereof.
[35] A pharmaceutical composition comprising one or more conjugates according to any of [24] to [29] and a pharmaceutically acceptable carrier.
[36] The pharmaceutical composition according to [35], wherein the pharmaceutical composition is a pharmaceutical composition for treating a cancer expressing human CEACAM5.
[37] The pharmaceutical composition according to [36], wherein the cancer is colorectal cancer, breast cancer, lung cancer, thyroid cancer, or a cancer resulting from metastasis thereof.
[38] Use of one or more according to any of [24] to [29] for producing a diagnostic composition for a cancer and/or a pharmaceutical composition for treating a cancer.
[39] The conjugate according to any of [24] to [30], wherein the conjugate is used for diagnosing a cancer and/or treating a cancer.
[40] A method for diagnosing a cancer, comprising administering one or more conjugates according to any of [24] to [30] to a subject.
[41] A method for treating a cancer, comprising administering a therapeutically effective amount of the conjugate according to any of [24] to [30] to a subject.
[42] A conjugate represented by the following formula (Ia) 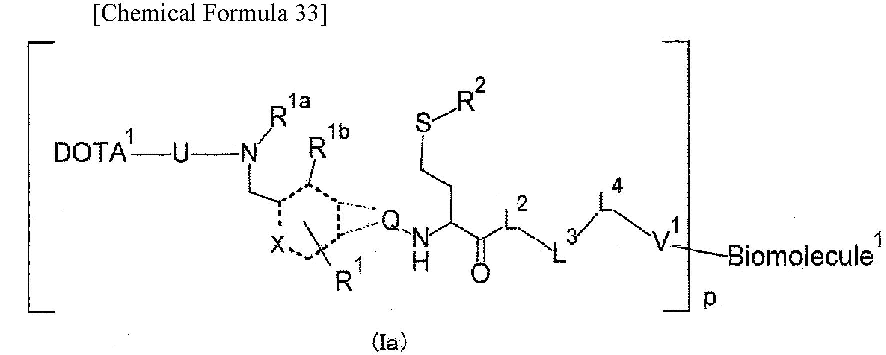 wherein
   DOTA¹: 3arm DOTA,
   U: a bond or -NH(CH₂)₂O(CH₂)₂O(CH₂)₂OCH₂C(=O)-
   Q: -C(=O)-, -NH-C(=O)-, or -NH-C(=S)-
   X: CorN
   R^{1a}, R^{1b}: identical or different, a hydrogen atom, or C₁₋₆ alkyl, provided that R^{1a} and R^{1b} together can form C₁₋₆ alkylene;
   p is a natural number of 1 to 25 and is bound to an adjacent carbon atom via p amino groups or thiol groups in Biomolecule¹;
   R¹: H, a halogen, C₁₋₆ alkyl, or halo C₁₋₆ alkyl,
   R²: C₁₋₆ alkyl or halo C₁₋₆ alkyl,
   L²: Ile, Gly, Ala, Val, Phe, -NHCH(CHCH₃NR³R⁴)C(=O)-, -NHCH(CHCH₃N₃)C(=O)-, or - NHCH(CHCH₃CH₂CH₂CH₃)C(=O)-,
   R³: H, C₁₋₆ alkyl,
   R⁴: H, C₁₋₆ alkyl,
   L³: a bond, Arg, or His,
   L⁴: -NH-(CH₂)₂-, -NHCH(C(=O)OH)(CH₂)₄-, or a bond,
   V¹: a group represented by any of the following formulas (A-1) to (A-5), 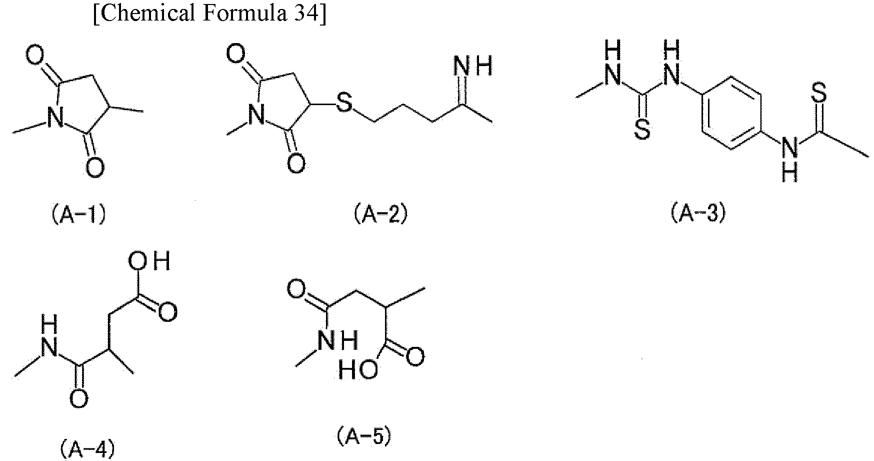 or, groups -L³-L⁴-V¹- together form the following formula 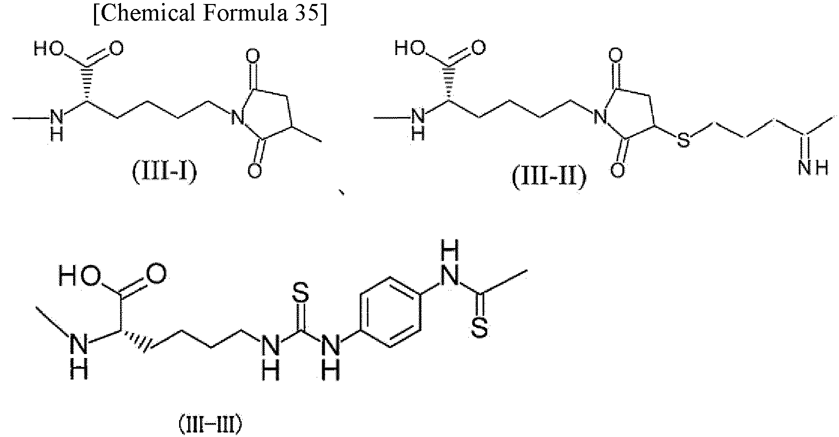 Biomolecule¹: a biomolecule [Chemical Formula 36]
      dotted line 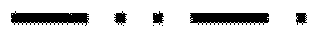 : Q is bound to any one of the two carbon atoms on the ring;
      dotted line 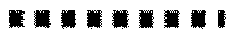 ; a single bond or a double bond.
   [43] The conjugate according to [42], wherein L³, L⁴, and V¹ are the following groups.
      L³: a bond, Arg, or His,
      L⁴: -NH-(CH₂)₂-, -NHCH(C(=O)OH)(CH₂)₄-, or a bond,
      V¹: a group represented by any of the following formulas (A-1) to (A-5), 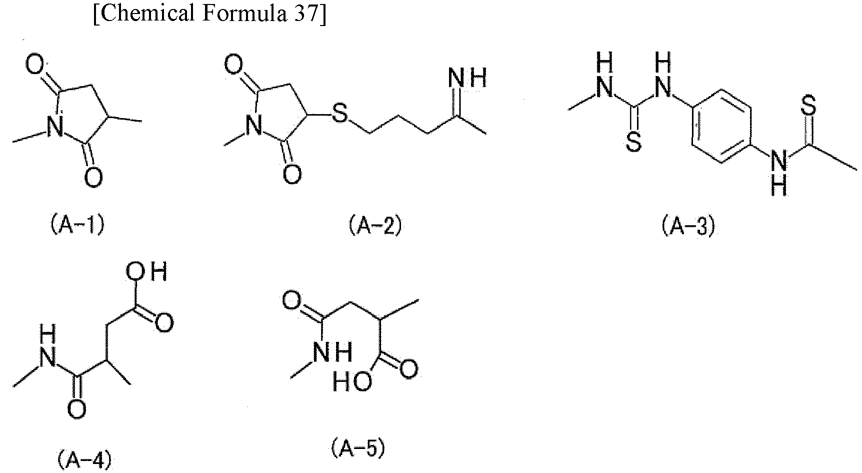
   [44] The conjugate according to [43], wherein
      the groups -L³-L⁴-V¹- together form the following formula. 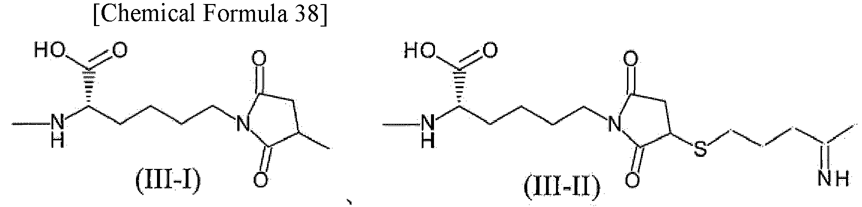
   [45]
      A conjugate represented by the following formula (Ib) 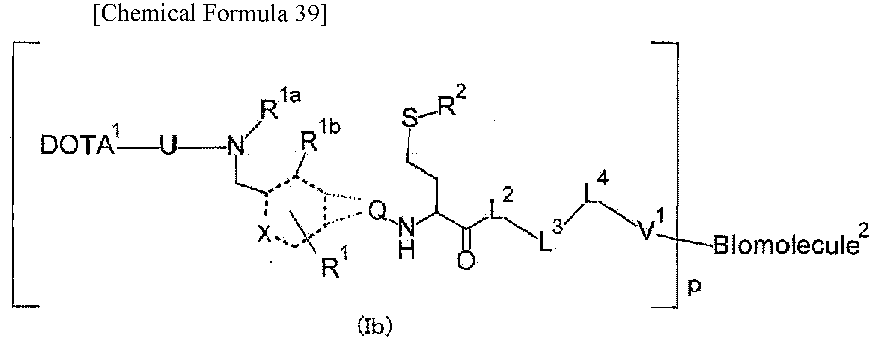 wherein
      DOTA¹: 3arm DOTA,
      U: a bond or -NH(CH₂)₂O(CH₂)₂O(CH₂)₂OCH₂C(=O)-
      Q: -C(=O)-, -NH-C(=O)-, or -NH-C(=S)-
      X: CorN
      R^{1a}, R^{1b}: identical or different, a hydrogen atom, or C₁₋₆ alkyl,
      provided that R^{1a} and R^{1b} together can form C₁₋₆ alkylene;
      p is a natural number of 1 to 25 and is bound to an adjacent carbon atom via p amino groups or thiol groups in Biomolecule²;
      R¹: H, a halogen, C₁₋₆ alkyl, or halo C₁₋₆ alkyl,
      R²: C₁₋₆ alkyl or halo C₁₋₆ alkyl,
      L²: Ile, Gly, Ala, Val, Phe, -NHCH(CHCH₃NR³R⁴)C(=O)-, -NHCH(CHCH₃N₃)C(=O)-, or - NHCH(CHCH₃CH₂CH₂CH₃)C(=O)-,
      R³: H, C₁₋₆ alkyl,
      R⁴: H, C₁₋₆ alkyl,
      L³: a bond, Arg, or His,
      L⁴: -NH-(CH₂)₂-, -NHCH(C(=O)OH)(CH₂)₄-, or a bond,
      V¹: a group represented by any of the following formulas (A-1) to (A-5), 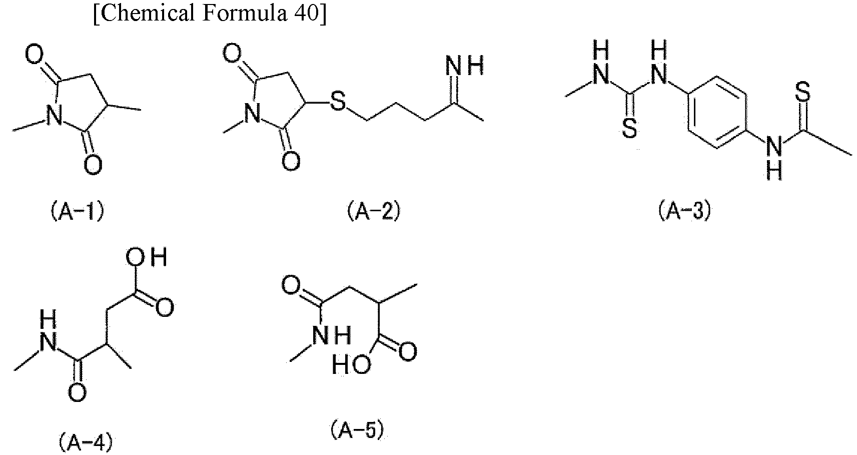 or, groups -L³-L⁴-V¹- together form the following formula 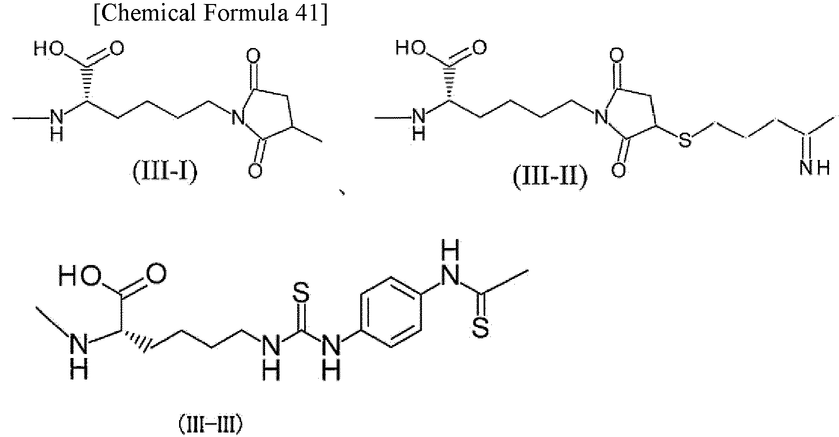
      Biomolecule²: an antibody Fab fragment
      [Chemical Formula 42]
      dotted line 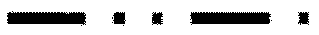 : Q is bound to any one of the two carbon atoms on the ring;
      dotted line 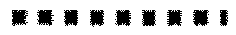 : a single bond or a double bond.
   [46-1] The conjugate according to any of [42] to [45], wherein the conjugate has the following formula (Ic): 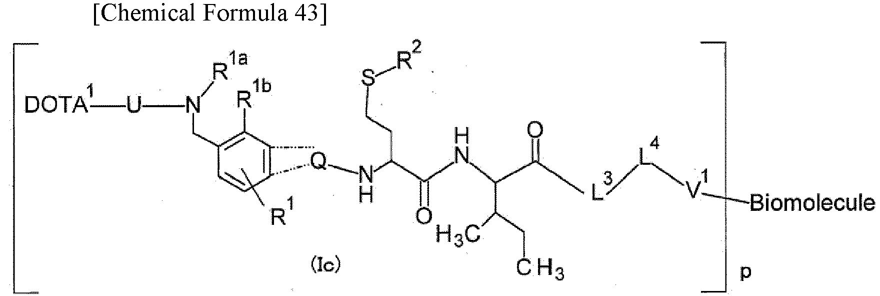 wherein
      L³: a bond,
      L⁴: -NH-(CH₂)₂- or -NHCH(C(=O)OH)(CH₂)₄-,
      Biomolecule: Biomolecule¹ or Biomolecule².
[46-2] The conjugate according to [46-1], wherein in formula (Ic),
   L³ is a bond, and
   L⁴ is -NH-(CH₂)₂-.
[47-1] The conjugate according to any of [42] to [46-2], wherein the conjugate is represented by the following formula (Id): 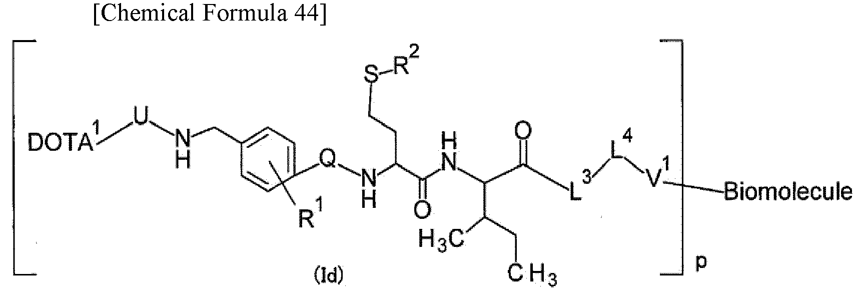 wherein
   L³: a bond,
   L⁴: -NH-(CH₂)₂- or -NHCH(C(=O)OH)(CH₂)₄-,
   Biomolecule: Biomolecule¹ or Biomolecule²
   the conjugate according to any of [42] to [45].
[47-2] The conjugate according to [47-1], wherein in formula (Id),
   L³ is a bond, and
   L⁴ is -NH-(CH₂)₂-.
[48]
   The conjugate according to any of [42] to [47-2], wherein V¹ is any of the following formulas (A-3) to (A-5). 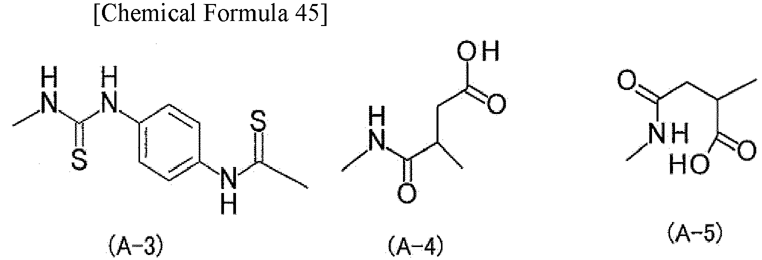
[49] The conjugate according to any of [42] to [48], wherein group (B) in formula (Ia) or (Ib) 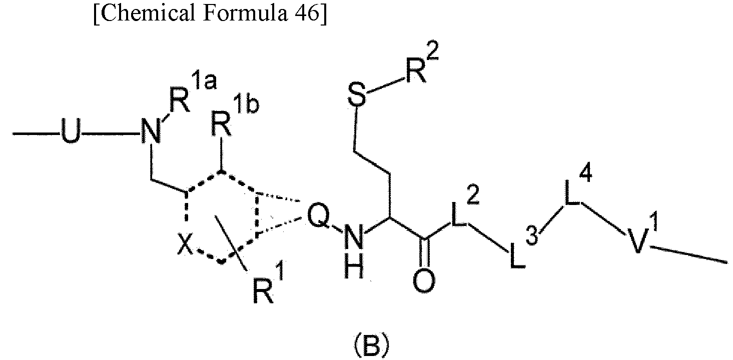 is a group selected from the group consisting of the following formulas (B-1) to (B-7). 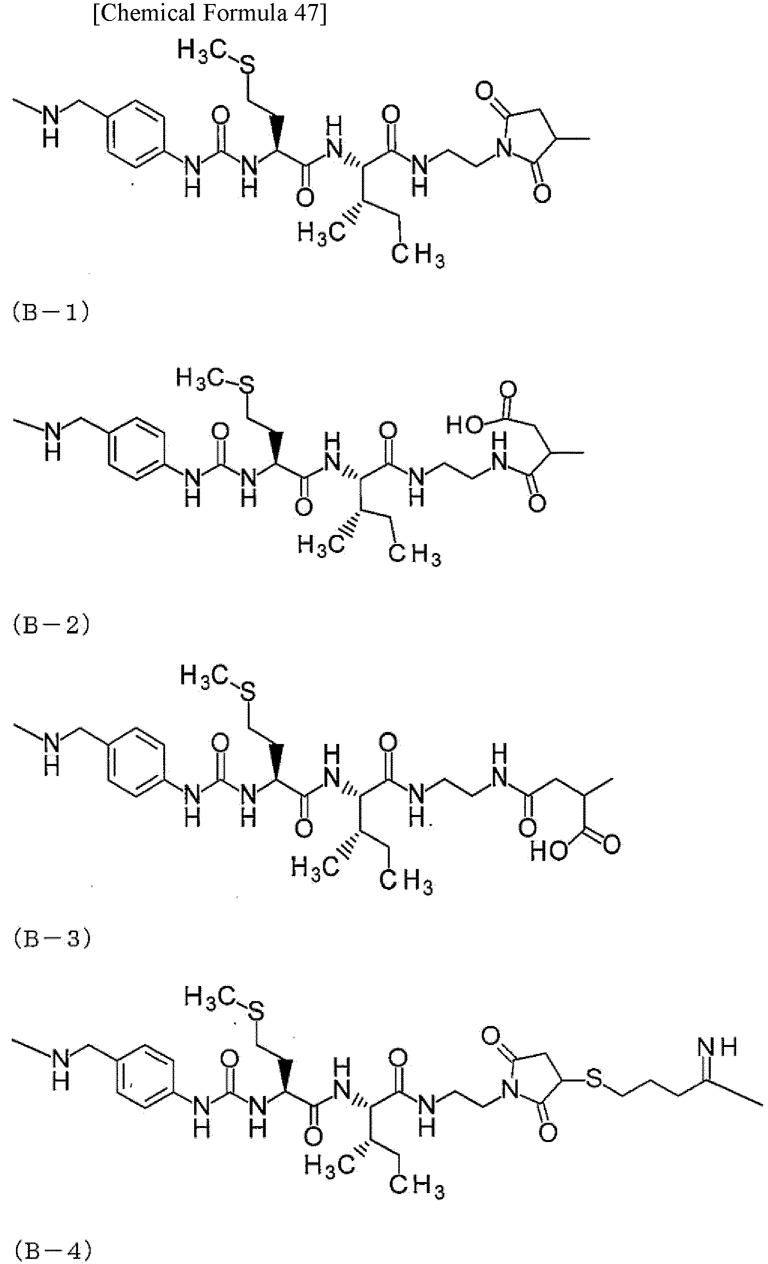 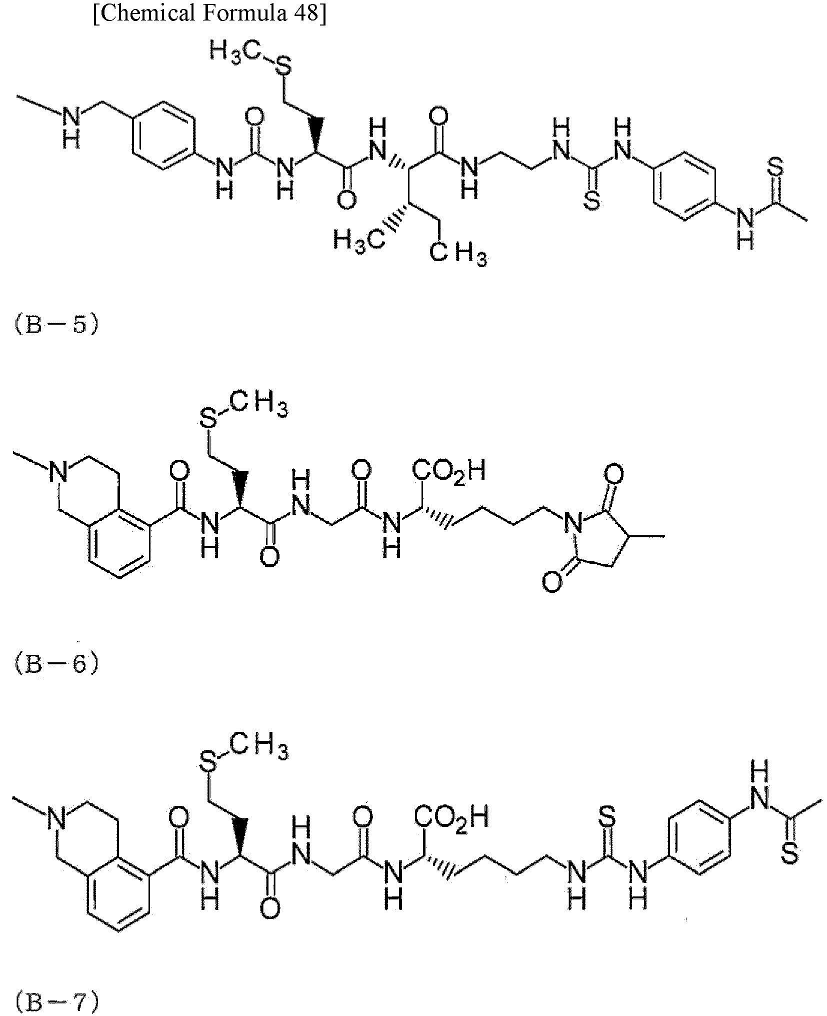
[50] The conjugate according to any of [42] to [49], wherein the conjugate is selected from the group consisting of the compounds represented by the following formulas. 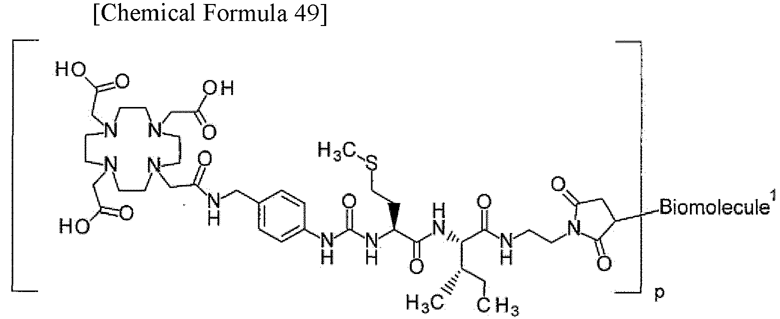 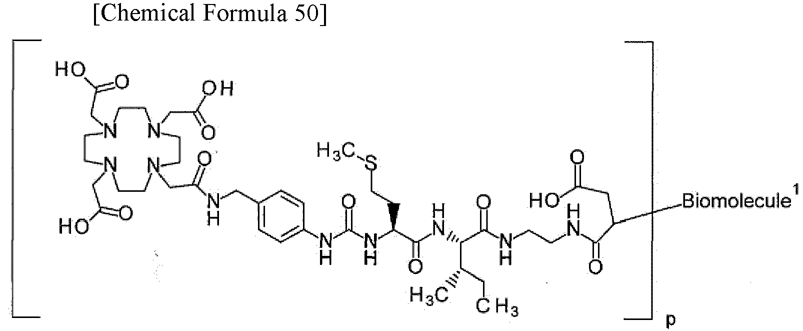 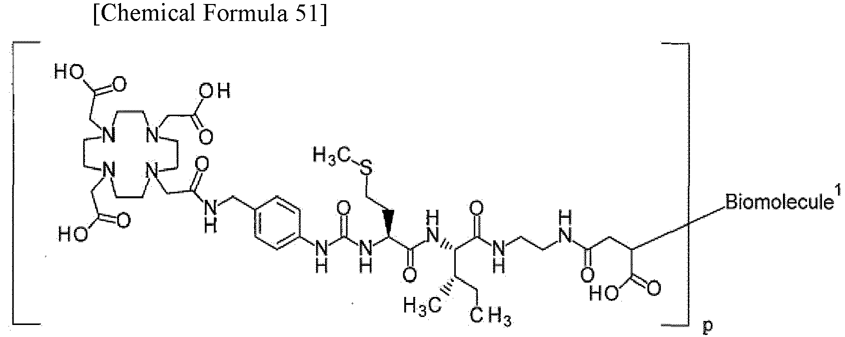 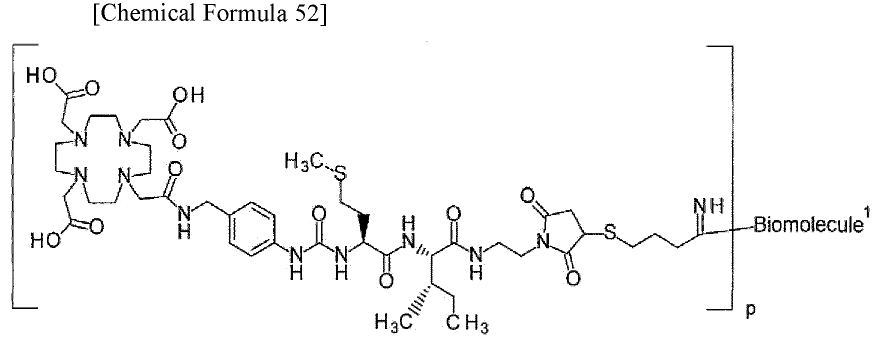 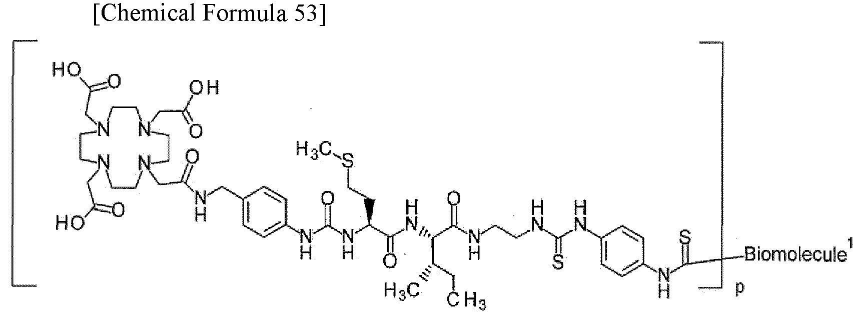 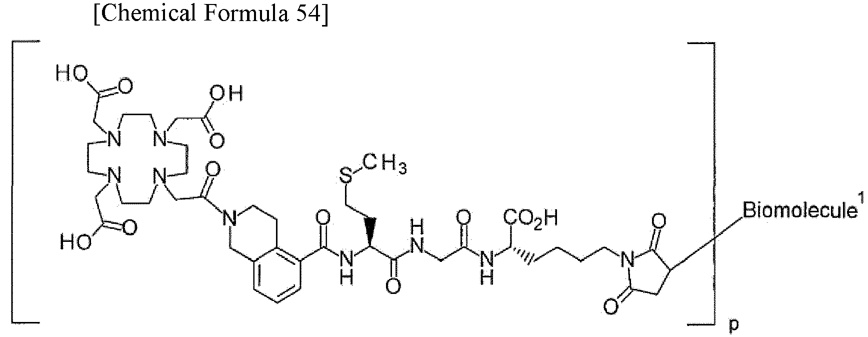 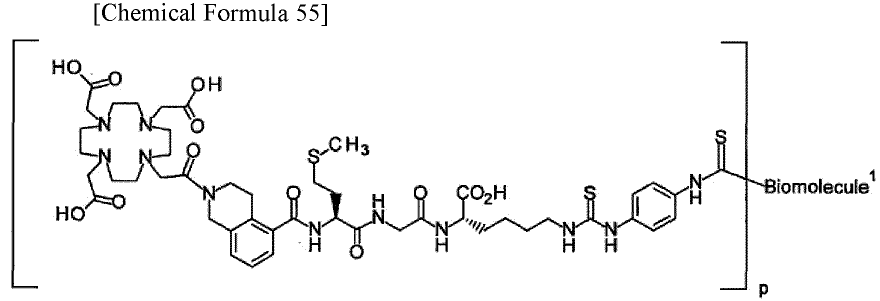
[51-1] The conjugate according to any of [42] to [50], wherein Biomolecule¹ and Biomolecule² are each a biomolecule or an antibody Fab fragment other than the following antibody Fab fragments:
   (a) an anti-human CEACAM5 antibody Fab fragment comprising a heavy chain fragment including a heavy chain variable region consisting of the amino acid sequence of amino acids 1 to 121 of SEQ ID NO: 2 and a light chain including a light chain variable region consisting of the amino acid sequence of amino acids 1 to 112 of SEQ ID NO: 4, and
   (b) an anti-human CEACAM5 antibody Fab fragment selected from the group consisting of an anti-human CEACAM5 antibody Fab fragment comprising a heavy chain fragment including a heavy chain variable region which consists of the amino acid sequence of amino acids 1 to 121 of SEQ ID NO: 2 and in which glutamic acid of amino acid 1 of SEQ ID NO: 2 is modified to pyroglutamic acid, and a light chain including a light chain variable region consisting of the amino acid sequence of amino acids 1 to 112 of SEQ ID NO: 4.
[51-2] The conjugate according to [51-1], wherein Biomolecule¹ and Biomolecule² are each a biomolecule or an antibody Fab fragment other than the following antibody Fab fragments:
   (a) an anti-human CEACAM5 antibody Fab fragment comprising a heavy chain fragment including a heavy chain variable region consisting of the amino acid sequence of amino acids 1 to 121 of SEQ ID NO: 2 and a light chain including a light chain variable region consisting of the amino acid sequence of amino acids 1 to 112 of SEQ ID NO: 4, and
   (b) an anti-human CEACAM5 antibody Fab fragment comprising a heavy chain fragment including a heavy chain variable region which consists of the amino acid sequence of amino acids 1 to 121 of SEQ ID NO: 2 and in which glutamic acid of amino acid 1 of SEQ ID NO: 2 is modified to pyroglutamic acid, and a light chain including a light chain variable region consisting of the amino acid sequence of amino acids 1 to 112 of SEQ ID NO: 4,
   (c) an anti-human CEACAM5 antibody Fab fragment comprising a heavy chain fragment consisting of the amino acid sequence shown in SEQ ID NO: 2 and a light chain consisting of the amino acid sequence shown in SEQ ID NO: 4, or
   (b) an anti-human CEACAM5 antibody Fab fragment comprising a heavy chain fragment which consists of the amino acid sequence shown in SEQ ID NO: 2 and in which glutamic acid of amino acid 1 of SEQ ID NO: 2 is modified to pyroglutamic acid, and a light chain including the light chain shown in SEQ ID NO: 4.
[52] The conjugate according to any of [42] to [51-2], wherein Biomolecule¹ or Biomolecule² is an anti-human MUC1 antibody Fab fragment selected from the group consisting of the following (a) and (b):
   (a) an anti-human MUC1 antibody Fab fragment comprising a heavy chain fragment including a heavy chain variable region consisting of the amino acid sequence shown in SEQ ID NO: 12 or SEQ ID NO: 14 and a light chain including a light chain variable region consisting of the amino acid sequence shown in SEQ ID NO: 16,
      and
   (b) an anti-human MUC1 antibody Fab fragment selected from the group consisting of an anti-human MUC1 antibody Fab fragment comprising a heavy chain fragment including a heavy chain variable region which consists of the amino acid sequence shown in SEQ ID NO: 12 or SEQ ID NO: 14 and in which glutamine of amino acid 1 of SEQ ID NO: 12 or SEQ ID NO: 14 is modified to pyroglutamic acid, and a light chain including a light chain variable region consisting of the amino acid sequence shown in SEQ ID NO: 16.
[53] The conjugate according to [52], wherein Biomolecule¹ or Biomolecule² is an anti-human MUC1 antibody Fab fragment selected from the group consisting of the following (a) and (b):
   (a) an anti-human MUC1 antibody Fab fragment comprising a heavy chain fragment consisting of the amino acid sequence shown in SEQ ID NO: 8 and a light chain consisting of the amino acid sequence shown in SEQ ID NO: 10; and
   (b) an anti-human MUC1 antibody Fab fragment comprising a heavy chain fragment which consists of the amino acid sequence shown in SEQ ID NO: 8 and in which glutamine of amino acid 1 of SEQ ID NO: 8 is modified to pyroglutamic acid, and a light chain consisting of the amino acid sequence shown in SEQ ID NO: 10.
[54] The conjugate according to [53], wherein Biomolecule¹ or Biomolecule² is the following anti-human MUC1 antibody Fab fragment:
   an anti-human MUC1 antibody Fab fragment comprising a heavy chain fragment which consists of the amino acid sequence shown in SEQ ID NO: 8 and in which glutamine of amino acid 1 of SEQ ID NO: 8 is modified to pyroglutamic acid, and a light chain consisting of the amino acid sequence shown in SEQ ID NO: 10.
[55] The conjugate according to any of [42] to [54], wherein p is a natural number of 1 to 4.
[56] The conjugate according to any of [42] to [55], wherein a metal is coordinated to DOTA¹.
[57] The conjugate according to [56], wherein the metal is a metal radioisotope.
[58] The conjugate according to [57], wherein the metal is ⁸⁹Zr.
[59] The conjugate according to [56], wherein the metal is a paramagnetic metal ion.
[60] The conjugate according to [59], wherein the metal is Gd³⁺.
[61] The conjugate according to any of [56] to [60], wherein the conjugate is a PET tracer.
[62] A diagnostic composition comprising one or more conjugates according to any of [56] to
[61] and a pharmaceutically acceptable carrier.
[63] The diagnostic composition according to [62], wherein the diagnostic composition is used as an early diagnostic drug or a staging drug.
[64] The diagnostic composition according to any of [62] or [63], wherein the diagnostic composition is used for diagnosing a disease associated with Biomolecule¹ or Biomolecule².
[65] The diagnostic composition according to [64], wherein the disease associated with Biomolecule¹ or Biomolecule² is a disease associated with MUC1.
[66] The diagnostic composition according to [65], wherein the diagnostic composition is used for diagnosing a cancer expressing MUC1.
[67] The diagnostic composition according to [66], wherein the cancer is breast cancer, lung cancer, colorectal cancer, bladder cancer, skin cancer, thyroid cancer, gastric cancer, pancreatic cancer, kidney cancer, ovarian cancer, or cervical cancer.
[68] A pharmaceutical composition comprising one or more conjugates according to any of [56] to [60] and a pharmaceutically acceptable carrier.
[69] The pharmaceutical composition according to [68], wherein the pharmaceutical composition is used for diagnosing a disease associated with Biomolecule¹ or Biomolecule².
[70] The pharmaceutical composition according to [69], wherein the disease associated with Biomolecule¹ or Biomolecule² is a disease associated with MUC1.
[71] The pharmaceutical composition according to [70], wherein the pharmaceutical composition is a pharmaceutical composition for treating a cancer expressing MUC1.
[72] The pharmaceutical composition according to [71], wherein the cancer is breast cancer, lung cancer, colorectal cancer, bladder cancer, skin cancer, thyroid cancer, gastric cancer, pancreatic cancer, kidney cancer, ovarian cancer, or cervical cancer.
[73] Use of one or more according to any of [56] to [60] for producing a diagnostic composition for a cancer and/or a pharmaceutical composition for treating a cancer.
[74] The conjugate according to any of [56] to [60], wherein the conjugate is used for diagnosing a cancer and/or treating a cancer.
[75] A method for diagnosing a cancer, comprising administering one or more conjugates according to any of [56] to [60] to a subject.
[76] A method for treating a cancer, comprising administering a therapeutically effective amount of the conjugate according to any of [56] to [60] to a subject.

### ADVANTAGEOUS EFFECTS OF INVENTION

The conjugate including an anti-human CEACAM5 antibody Fab fragment, a peptide linker, and a ligand, and the conjugate including an anti-human CEACAM5 antibody Fab fragment and a specific ligand described in the present invention have excellent binding activity to human CEACAM5. Because of this, the conjugate of the present invention further including a metal is expected to be useful for diagnosis and/or treatment of a cancer. In addition, the conjugate including a human MUC1 antibody Fab fragment, a peptide linker, and a ligand described in the present invention has excellent binding activity to human MUC1. The conjugate consisting of 3arm DOTA, a spacer, a peptide linker, and a biomolecule described in the present invention is excreted by the kidneys more rapidly. Because of this, the conjugate of the present invention further including a metal is expected to be useful for the diagnosis and/or treatment of a cancer.

### BRIEF DESCRIPTION OF DRAWINGS

[Fig. 1] Fig. 1 shows a PET/CT image obtained about 3 hours after the administration of a PBS solution containing ⁶⁴Cu-protein conjugate solution (A).
[Fig. 2] Fig. 2 shows a PET/CT image obtained about 3 hours after the administration of a PBS solution containing ⁶⁴Cu-protein conjugate solution (B).
[Fig. 3] Fig. 3 shows SUV.

### DESCRIPTION OF EMBODIMENTS

The present invention will be described in detail below, but the present invention is not limited thereto. Unless otherwise defined herein, scientific and technical terms used in the context of the present invention shall have meanings commonly understood by those skilled in the art.
"Alkyl" means a linear or branched saturated hydrocarbon chain and means a monovalent group.
"C₁₋₆ alkyl" refers to alkyl having 1 to 6 carbon atoms, such as methyl, ethyl, n-propyl, isopropyl, n-butyl, isobutyl, sec-butyl, tert-butyl, n-pentyl, or n-hexyl. In an embodiment C₁₋₆ alkyl is C₁₋₄ alkyl, in an embodiment C₁₋₆ alkyl is methyl or ethyl, and in an embodiment C₁₋₆ alkyl is methyl.
"C₁₋₆ alkylene" is a divalent group obtained by removing hydrogen from the above C₁₋₆ alkyl. In an embodiment, C₁₋₆ alkylene is methylene, ethylene, propylene, methylmethylene, or the like.
"Halogen" means F, Cl, Br, or I.
"Halo C₁₋₆ alkyl" is C₁₋₆ alkyl substituted with one or more halogens. In an embodiment halo C₁₋₆ alkyl is C₁₋₆ alkyl substituted with 1 to 5 halogens, and in an embodiment halo C₁₋₆ alkyl is CF₃.

### 1-1. Conjugate of the present invention

The conjugate of the present invention is a conjugate represented by the following formula (I):

(Y-S₁-X)ₚ-Fab¹ (I)

wherein
Fab¹ is an anti-human CEACAM5 antibody Fab fragment, and the Fab¹ is bound to X via p amino groups or thiol groups in the Fab¹,
X is a peptide linker or a bond,
S₁ is a spacer or a bond,
Y is a ligand, and
p is a natural number of 1 to 25 and represents the number of (Y-S₁-X) bound to Fab¹, provided that when X is a bond, S₁ is -CH₂-(1,4-phenylene)-NH-C(=S)- or a bond, and Y is a group represented by the following formula

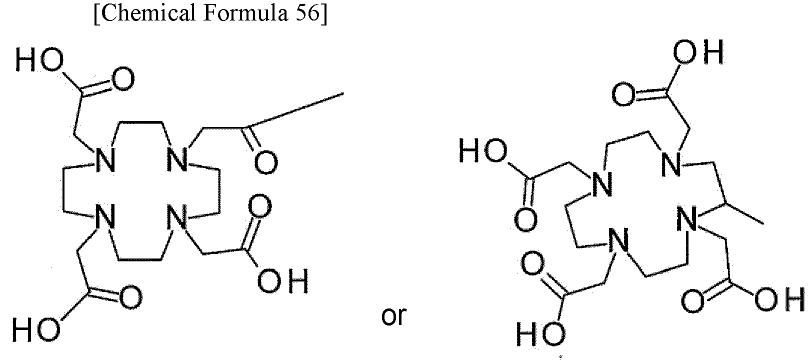

### Anti-human CEACAM5 antibody Fab fragment (Fab¹)

The anti-human CEACAM5 antibody Fab fragment represented by "Fab¹" in formula (I) will be described.

The basic structure of an antibody molecule is common to each class and is composed of a heavy chain having a molecular weight of 50,000 to 70,000 and a light chain having a molecular weight of 20,000 to 30,000. The heavy chain usually consists of a polypeptide chain including about 440 amino acids, has a characteristic structure for each class, and is called a γ, µ, α, δ, or ε chain, corresponding to IgG, IgM, IgA, IgD, or IgE, respectively. Further, IgG includes IgG1, IgG2, IgG3, and IgG4, which are called γ1, y2, γ3, and y4, respectively. The light chain usually consists of a polypeptide chain including about 220 amino acids, and two types, L-type and K-type, are known and are called λ and κ chains, respectively. The peptide configuration of the basic structure of an antibody molecule is such that two homologous heavy chains and two homologous light chains are bound by disulfide bonds (S-S bonds) and non-covalent bonds, and the molecular weight is 150,000 to 190,000. The two light chains can be paired with any heavy chain. Each antibody molecule is always composed of two identical light chains and two identical heavy chains.

There are four intrachain S-S bonds in a heavy chain (five for µ and ε chains) and two intrachain S-S bonds in a light chain; one loop is formed for every 100 to 110 amino acid residues, and this steric structure is alike among the loops and is called a structural unit or a domain. The domains located at the N termini of both a heavy chain and a light chain are called variable regions because the amino acid sequences thereof are not constant, even in an authentic sample from the same class (subclass) of the same animal species, and their respective domains are called a heavy chain variable region (V_{H}) and a light chain variable region (V_{L}). The amino acid sequence located closer to the C-terminal side than the N terminus is nearly constant for each class or subclass and is called a constant region, and each of the domains is represented by C_{H}1, C_{H}2, C_{H}3, or C_{L}.

The specificity of antibody-antigen binding depends on the amino acid sequence of the portion composed of V_{H} and V_{L}. On the other hand, biological activities such as binding to complements and various cells reflect the differences in the structure of the constant region among classes of Ig. The variability in the variable regions of a heavy chain and a light chain has been found to be mostly limited to the three small hypervariable regions present in both chains, and these regions are called complementarity determining regions (CDRs; CDR1, CDR2, and CDR3 starting from the N terminus side). The remaining part of the variable region is called a framework region (FR) and is relatively constant.

A region between the C_{H}1 domain and the C_{H}2 domain of the heavy chain constant region of an antibody is called a hinge region, and this region includes many proline residues and includes a plurality of interchain S-S bonds connecting two heavy chains. For example, the hinge regions of human IgG1, IgG2, IgG3, and IgG4 include 2, 4, 11, and 2 cysteine residues, respectively, which constitute the inter-heavy chain S-S bonds. The hinge region is a region highly sensitive to a proteolytic enzyme such as papain or pepsin. When an antibody is digested with papain, its heavy chain is cleaved at a position closer to the N terminus side than to the inter-heavy chain S-S bond of the hinge region, and the antibody is broken down into two Fab fragments and one Fc fragment. The Fab fragment is composed of a light chain and a heavy chain fragment including a heavy chain variable region (V_{H}), the C_{H}1 domain, and a portion of the hinge region. The Fab fragment includes the variable region and has antigen-binding activity.

In one embodiment, the anti-human CEACAM5 antibody Fab fragment included in the conjugate of the present invention is a Fab fragment having the following characteristic: an anti-human CEACAM5 antibody Fab fragment comprising a heavy chain fragment including a heavy chain variable region consisting of the amino acid sequence of amino acids 1 to 121 of SEQ ID NO: 2 and a light chain including a light chain variable region consisting of the amino acid sequence of amino acids 1 to 112 of SEQ ID NO: 4.

As the heavy chain constant region of the anti-human CEACAM5 antibody Fab fragment included in the conjugate of the present invention, any constant region such as Igγ1, Igy2, Igy3, or Igy4 can be selected. In one embodiment, the heavy chain constant region of the anti-human CEACAM5 antibody Fab fragment included in the conjugate of the present invention is the human Igγ1 constant region.

As the light chain constant region of the anti-human CEACAM5 antibody Fab fragment included in the conjugate of the present invention, either constant region of Igλ, or Igκ can be selected. In one embodiment, the light chain constant region of the anti-human CEACAM5 antibody Fab fragment included in the conjugate of the present invention is the human Igκ1 constant region.

In one embodiment, the anti-human CEACAM5 antibody Fab fragment included in the conjugate of the present invention is the following Fab² fragment:
an anti-human CEACAM5 antibody Fab fragment including a heavy chain fragment consisting of the amino acid sequence shown in SEQ ID NO: 2 and a light chain consisting of the amino acid sequence shown in SEQ ID NO: 4 (referred to as Fab²).

It is known that when an antibody, including a Fab fragment, is expressed in a cell, the antibody undergoes a post-translational modification. Examples of the post-translational modification include cleavage of lysine at the heavy chain C terminus by a carboxypeptidase, modification of glutamine or glutamic acid at the heavy chain and light chain N termini to pyroglutamic acid by pyroglutamylation, glycosylation, oxidation, deamidation, and glycosylation, and it is known that such a post-translational modification occurs in various antibodies (J. Pharm. Sci.;2008;97:2426-2447).

The anti-CEACAM5 antibody Fab fragment included in the conjugate of the present invention can also include a Fab fragment produced by a post-translational modification. Examples of the anti-human CEACAM5 antibody Fab fragment of the present invention that can be produced by a post-translational modification include a pyroglutamylated anti-human CEACAM5 antibody Fab fragment at the heavy chain N terminus. It is known in the art that such a post-translational modification by N-terminal polyglutamylation does not affect the activity of the antibody (Anal. Biochem.;2006;348:24-39).

In one embodiment, the anti-human CEACAM5 antibody Fab fragment included in the conjugate of the present invention is an anti-human CEACAM5 antibody Fab fragment having the following characteristic:
an anti-human CEACAM5 antibody Fab fragment comprising a heavy chain fragment including a heavy chain variable region which consists of the amino acid sequence of amino acids 1 to 121 of SEQ ID NO: 2 and in which glutamic acid of amino acid 1 of SEQ ID NO: 2 is modified to pyroglutamic acid, and a light chain including a light chain variable region consisting of the amino acid sequence of amino acids 1 to 112 of SEQ ID NO: 4.

In an embodiment, the anti-human CEACAM5 antibody Fab fragment included in the conjugate of the present invention is an anti-human CEACAM5 antibody Fab fragment having the following characteristic:
an anti-human CEACAM5 antibody Fab fragment comprising a heavy chain fragment which consists of the amino acid sequence shown in SEQ ID NO: 2 and in which glutamic acid of amino acid 1 of SEQ ID NO: 2 is modified to pyroglutamic acid, and a light chain consisting of the amino acid sequence shown in SEQ ID NO: 4.

In another embodiment, the anti-CEACAM5 antibody Fab fragment included in the conjugate of the present invention is an anti-human CEACAM5 antibody Fab fragment having the following characteristic:
an anti-human CEACAM5 antibody Fab fragment comprising a heavy chain fragment including a heavy chain variable region including CDR1 consisting of the amino acid sequence of amino acids 31 to 35 of SEQ ID NO: 2, CDR2 consisting of the amino acid sequence of amino acids 50 to 66 of SEQ ID NO: 2, and CDR3 consisting of the amino acid sequence of amino acids 99 to 110 of SEQ ID NO: 2, and a light chain including a light chain variable region including CDR1 consisting of the amino acid sequence of amino acids 24 to 38 of SEQ ID NO: 4, CDR2 consisting of the amino acid sequence of amino acids 54 to 60 of SEQ ID NO: 4, and CDR3 consisting of the amino acid sequence of amino acids 93 to 101 of SEQ ID NO: 4.

The anti-human CEACAM5 antibody Fab fragment included in the conjugate of the present invention binds to human CEACAM5. Examples of a method for measuring the binding activity of the obtained anti-human CEACAM5 antibody Fab fragment to human CEACAM5 include methods such as analysis by a surface plasmon resonance (SPR) method and ELISA. For example, when analysis by the SPR method is used, the binding rate constant (ka), the dissociation rate constant (kd), and the dissociation constant (K_{D}) can be measured by immobilizing Biotin CAPture Kit (GE Healthcare Japan Corporation) and biotinylated human CEACAM5 on a sensor chip using Biacore T200 (GE Healthcare Japan Corporation) and adding a serially diluted Fab fragment.

The anti-human CEACAM5 antibody Fab fragment included in the conjugate of the present invention can be easily prepared by those skilled in the art using a known method in the art based on the sequence information of the heavy chain fragment and the light chain of the anti-human CEACAM5 antibody Fab fragment of the present invention disclosed herein. The anti-human CEACAM5 antibody Fab fragment of the present invention is not particularly limited, and can be produced, for example, according to the method described in <Method for producing the anti-human CEACAM5 antibody Fab fragment included in the conjugate of the present invention> described later.

### 1-2. Ligand

The "ligand" is a moiety of a conjugate that can form a chelate complex with a metal and means a group composed of a chelating agent. The group composed refers to a group that has a bond due to removal of a proton from the chelating agent. The group composed of a chelating agent binds to the anti-human CEACAM5 antibody Fab fragment directly or via a spacer and/or a peptide linker.

The "chelating agent" refers to a compound that can coordinate with a metal. Examples of the "chelating agent" as used herein include a siderophore and a non-siderophore. Examples of the siderophore include a hydroxamic acid type, a catechol type, and a mixed ligand type. Examples of the hydroxamic acid type siderophore include ferrichrome, deferoxamine (DFO) represented by the following formula: 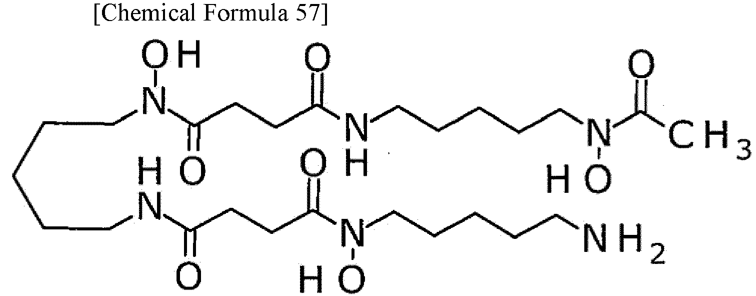 , fusarinin C, ornibactin, and rhodotorulic acid. Examples of the catechol type siderophore include enterobactin, bacillibactin, and vibriobactin. Examples of the mixed ligand type siderophore include azotobactin, pyoverdine, and yersiniabactin. In the case of the siderophores described above, DFO can be reacted with a spacer or a peptide linker via -NH₂, which is a reactive functional group thereof, and in the case of the siderophores other than DFO, they can also be reacted with a spacer or a peptide linker via a reactive functional group such as a carboxyl group, a hydroxyl group, or an amino group by a method usually used by those skilled in the art.

### Examples of the non-siderophore include

DOTA (1,4,7,10-tetraazacyclododecane-1,4,7,10-tetraacetic acid, CAS number: 60239-18-1) represented by the following formula: 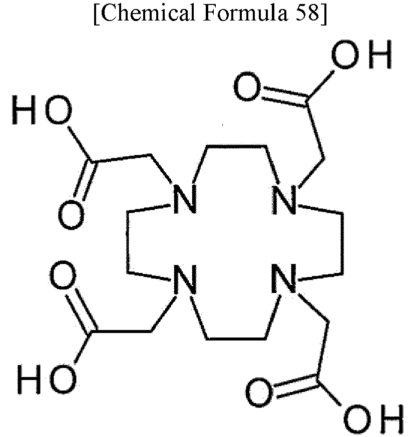 DTPA (diethylenetriamine pentaacetic acid, CAS number: 67-43-6), DTPA-BMA (1,7-bis(methylcarbamoylmethyl)-1,4,7-triazaheptane-1,4,7-triacetic acid, CAS number: 119895-95-3), EOB-DTPA (N-[(2S)-2-[bis(carboxymethyl)amino]-3-(4-ethoxyphenyl)propyl]-N-[2-[bis(carboxymethyl)amino]ethyl]glycine, CAS number: 158599-72-5), TTHA (triethylenetetramine hexaacetic acid, CAS number: 869-52-3), DO3A (1,4,7,10-tetraazacyclododecane-1,4,7-triacetic acid, CAS number: 217973-03-0), HP-DO3A (10-(2-hydroxypropyl)-1,4,7,10-tetraazacyclododecane-1,4,7-triacetic acid, CAS number: 120041-08-9), and known reactive derivatives thereof.

DOTA can be reacted with a spacer or a peptide linker via one of carboxylic acids which are reactive functional groups thereof (hereinafter, DOTA having three carboxylic acids thus bound is sometimes written as 3arm DOTA (PLoS One. 2019 Mar 22;14(3):e0213397)).

Among DOTA, examples of 3arm DOTA include the following.

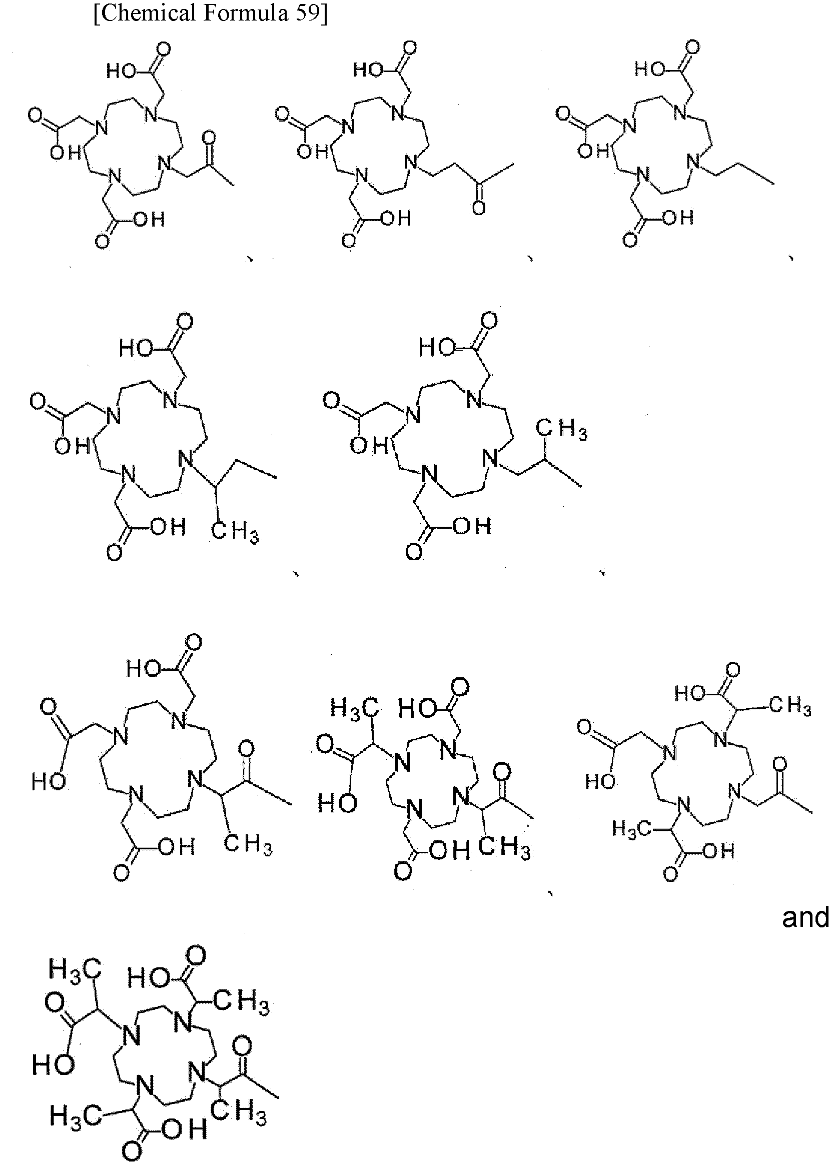

Alternatively, DOTA in which four carboxylic acids are maintained (hereinafter, sometimes written as 4arm-DOTA) using the reagent p-SCN-Bn-DOTA of the following formula can also be reacted with a spacer or a peptide linker.

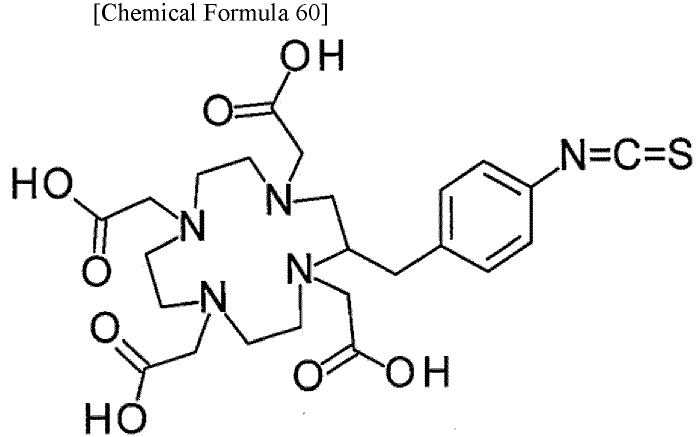

Examples of an embodiment of the "chelating agent" that forms the ligand included in the conjugate of the present invention include DFO, DOTA, DTPA, DTPA-BMA, EOB-DTPA, D03A, and HP-DO3A. In an embodiment, the chelating agent is DFO or DOTA.

The compounds and conjugates herein also include free forms and salts thereof, unless otherwise stated. Here, the "salts thereof" are salts that, when an acid addition salt or a salt with a base may be formed depending on the type of a substituent of a compound and a conjugate thereof, can be formed by the compound and the conjugate. Specific examples thereof include acid addition salts with inorganic acids such as hydrochloric acid, hydrobromic acid, hydroiodic acid, sulfuric acid, nitric acid, and phosphoric acid, and with organic acids such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditoluoyltartaric acid, citric acid, methanesulfonic acid, ethanesulfonic acid, benzenesulfonic acid, p-toluenesulfonic acid, aspartic acid, and glutamic acid, salts with inorganic bases such as sodium, potassium, magnesium, calcium, and aluminum, and with organic bases such as methylamine, ethylamine, ethanolamine, lysine, and ornithine, salts with various amino acids and amino acid derivatives such as acetylleucine, and ammonium salts. For example, DFO also exists as deferoxamine methanesulfonate and as another salt. DTPA exists as a DTPA sodium salt as well as a free form.

The conjugate of the present invention including a metal can be used for various contrast agents and/or therapeutic agents for cancers, and is used, for example, for an MRI contrast agent and an agent used for a PET tracer.

Examples of an embodiment of the "chelating agent" when used for an MRI contrast agent include the siderophore and non-siderophore chelating agents described above.

Examples of an embodiment of the "chelating agent" when used for a PET tracer include the siderophore and non-siderophore chelating agents described above, and in an embodiment, the chelating agent is DFO or DOTA.

In the conjugate of the present invention, the chelating agent may include a metal. As used herein, the "metal" means a paramagnetic metal ion or a metal radioisotope. The metal is not particularly limited as long as it is a metal that is coordinated to each chelating agent. An appropriate combination of a chelating agent and a metal is selected according to the intended use of the conjugate.

A paramagnetic metal ion is preferably used for an MRI contrast agent Examples of an embodiment of the paramagnetic metal ion include, but are not limited to, Fe²⁺, Fe³⁺, Cu²⁺, Ni²⁺, Rh²⁺, Co²⁺, Gd³⁺, Eu³⁺, Dy³⁺, Tb³⁺, Pm³⁺, Nd³⁺, Tm³⁺, Ce³⁺, Y³⁺, Ho³⁺, Er³⁺, La³⁺, Yb³⁺, Mn³⁺, or Mn²⁺. In an embodiment, the paramagnetic metal ion is Gd³⁺, Mn³⁺, Mn²⁺, Fe²⁺, or Fe³⁺. In an embodiment, the paramagnetic metal ion is Gd³⁺. In an embodiment, the paramagnetic metal ion is Mn³⁺ or Mn²⁺. In this case, a halogen or the like can be used as a counter anion in the conjugate. In addition, the counter anion may be the ligand C(=O)O⁻, and further, the conjugate may have a counter cation such as Na⁺.

A metal radioisotope is used for a PET tracer and the like. Examples of an embodiment of the metal radioisotope include, but are not limited to, ⁸⁹Zr, ⁵²Mn, ⁵²Fe, ⁶⁴Cu, ⁶⁷Ga, ⁶⁸Ga, ⁷²As, ⁹⁰Y, ^{99m}Tc, ¹¹¹In, or ¹⁷⁷Lu. Examples of an embodiment of the metal radioisotope used for a PET tracer, a SPECT tracer, and the like include ⁸⁹Zr, ⁶⁴Cu, ⁶⁷Ga, ⁶⁸Ga, ^{99m}Tc, or ¹¹¹In. In an embodiment, the metal radioisotope is a radioisotope of zirconium (Zr). In an embodiment, the metal radioisotope is ⁸⁹Zr. Examples of an embodiment of the metal radioisotope used for treatment of a cancer include ⁹⁰Y or ¹⁷⁷Lu.

An embodiment of the conjugate of the present invention is a conjugate in which Y is DFO to which ⁸⁹Zr is coordinated. Another embodiment is a conjugate in which Y is DOTA to which a metal radioisotope consisting of ⁹⁰Y, ⁶⁷Ga, ⁶⁸Ga, and ¹⁷⁷Lu is coordinated. Yet another embodiment is a conjugate in which Y is DOTA to which a paramagnetic metal ion consisting of Gd³⁺ and Y³⁺ is coordinated. Yet another embodiment is a conjugate in which Y is DOTA to which Gd³⁺ is coordinated.

### 1-3. Peptide linker or bond

In the conjugate of the present invention, a ligand (Y) or a spacer (S₁) and Fab¹ may be directly bound (that is, X is a bond), or may be bound via a peptide linker (that is, X is a peptide linker).

As used herein, the "peptide linker" is a linker including a peptide consisting of 2 to 4 amino acids, and, if desired, has attachments Z₁ to Z₃ or the like suitable for binding to an anti-human CEACAM5 antibody Fab fragment. Here, the peptide included in the peptide linker is not particularly limited, and is preferably a peptide consisting of 2 to 4 amino acids, each selected from the group consisting of glycine (Gly), lysine (Lys), methionine (Met), isoleucine (Ile), phenylalanine (Phe), valine (Val), tyrosine (Tyr), arginine (Arg), alanine (Ala), glutamine (Gln), glutamic acid (Glu), asparagine (Asn), aspartic acid (Asp), histidine (His) and leucine (Leu), 3-(2-naphthyl)alanine, and diphenylalanine, and more preferably a peptide consisting of 2 to 4 amino acids, each selected from the group consisting of glycine, lysine, methionine, isoleucine, phenylalanine, valine, tyrosine, aspartic acid, arginine, 3-(2-naphthyl)alanine, and diphenylalanine. Unless otherwise specified, the configuration of the amino acid residues other than glycine are the L-form.

An embodiment of the peptide linker is a peptide linker that includes a peptide consisting of 2 to 4 amino acids having an amino acid sequence cleaved by a renal brush border membrane enzyme or a lysosomal enzyme and further may have an attachment intervening between the peptide linker and a biomolecule or an antibody Fab fragment. It has been reported that the peptide linker having an amino acid sequence cleaved by a renal brush border membrane enzyme or a lysosomal enzyme is specifically cleaved by these enzymes present in the kidneys, thus reducing the accumulation of a labeling portion in the kidneys and that reduction in risk of exposure of the kidneys and renal disorder can be expected. For example, Adv Drug Deliv Rev. 2008 Sep;60(12): 1319-28., Bioconjug Chem. 2005 Nov-Dec;16(6): 1610-6., and Cancer Res. 1999 Jan 1;59(1):128-34. disclose that a glycine-lysine linker is specifically cleaved by a renal brush border membrane enzyme present in the kidneys. Japanese Patent No. 6164556 discloses that a glycine-phenylalanine-lysine linker is specifically cleaved in the kidneys by a renal brush border membrane enzyme; in addition, Bioconjug Chem. 2002 Sep-Oct;13(5):985-95. discloses that a linker including a glycine-leucine-glycine-lysine sequence is specifically cleaved in the kidneys by a renal brush border membrane enzyme; and in addition, Bioconjug Chem. 2013 Feb 20;24(2):291-9. discloses that a glycine-tyrosine linker is specifically cleaved by a renal brush border membrane enzyme. Further, Bioconjug Chem. 2014 Nov 19;25(11):2038-45. discloses that a linker including a methionine-isoleucine sequence is specifically cleaved by a lysosomal enzyme present in the kidneys. An embodiment of the peptide linker is a peptide linker including an amino acid sequence selected from the group consisting of methionine-isoleucine, glycine-lysine, glycine-phenylalanine-lysine, methionine-valine-lysine, glycine-tyrosine, glycine-lysine-lysine, and glycine-arginine-lysine, aspartic acid-glycine-lysine, methionine-glycine-lysine, methionine-isoleucine-lysine, glycine-tyrosine-lysine, glycine-valine, glycine-isoleucine, methionine-phenylalanine-lysine, glycine-(3-(2-naphthyl)alanine)-lysine, and glycine-diphenylalanine-lysine. An embodiment is a peptide linker including an amino acid sequence selected from the group consisting of methionine-isoleucine aspartic acid-glycine-lysine, glycine-phenylalanine-lysine, methionine-glycine-lysine, glycine-lysine, and glycine- (3-(2-naphthyl)alanine)-lysine.

The "peptide linker" may optionally have an attachment suitable for binding to an anti-human CEACAM5 antibody Fab fragment, wherein the attachment suitable for binding to an anti-human CEACAM5 antibody Fab fragment is a group that organic chemically forms a bond between the peptide linker portion and an amino group or a thiol group of the anti-human CEACAM5 antibody Fab fragment, and an embodiment thereof is a group including a maleimide-derived group (for example, a group represented by the following formula (II-I) or (II-II)) or an isothiocyanate-derived group (-NH-C(=S)-) at an end. An embodiment is -NH-(CH₂)₂-Z₁-, -CH₂-C(=O)-NH-(CH₂)₂-Z₁-, -C(=S)-NH-(1,4-phenylene)-NH-C(=S)-, or -NH-(CH₂)₂-NH-C(=S)-NH-(1,4-phenylene)-NH-C(=S)-, wherein Z₁ is represented by the following formula (II-I) or (II-II). Further, the following formula (II-I) may be written as -Zi(#N)-, and the following formula (II-II) may be written as -Zi(#S)-.

The attachment forms a peptide linker by binding to an amino group or a carboxyl group of the amino acid at an end of the peptide, or to an amino group (for example, lysine) or a hydroxyl group (for example, tyrosine) in a side chain of the amino acid. Examples of the attachment which forms a peptide linker by binding to a functional group in a side chain of the amino acid at an end of the peptide include a group represented by the following formula (III-I) or (III-II) as an attachment integrated with Lys, and herein, these two are collectively written as -Lys^{∗}-Z₂-. The following formula (III-I) may be written as -Lys^{∗}-Z₂(#N)-, and the following formula (III-II) may be written as -Lys^{∗}-Z₂(#S)-. The following formula (III-III) may be written as -Lys^{∗}-Z₃-.

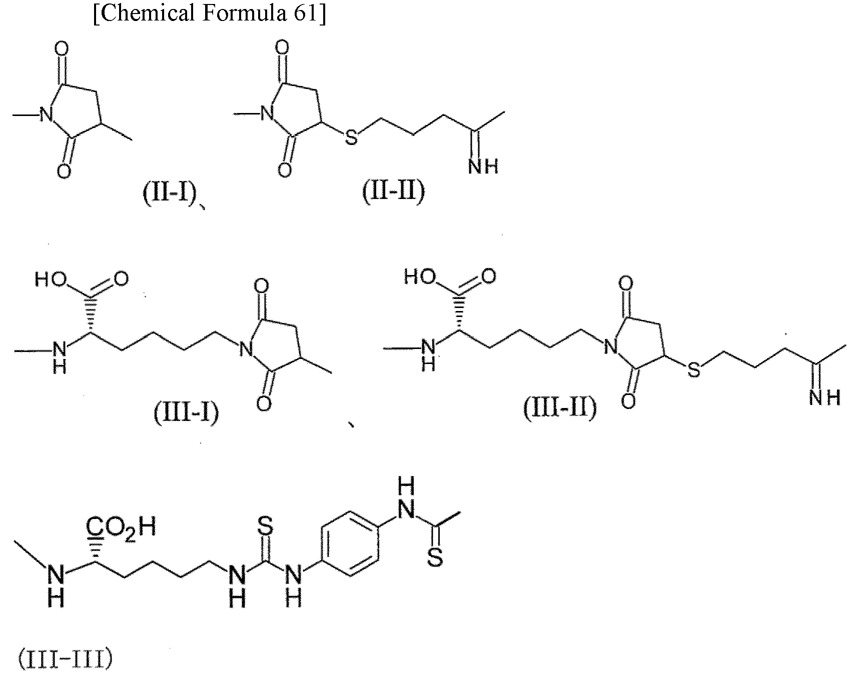

Further, herein, similarly, the group represented by the following formula (IV) and the group represented by the following formula (V) which have a structure in which a functional group in a side chain of the terminal amino acid and an attachment are bound are written as -Tyr^{∗}-CH₂- and -Lys^{∗}-C(=S)-, respectively.

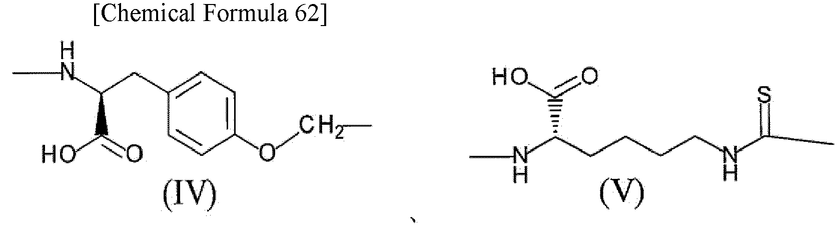

An embodiment of the "X" peptide linker is a peptide linker that includes an attachment. An embodiment is a peptide linker selected from the group consisting of
(1) -Met-Ile-NH-(CH₂)₂-Z₁-,
(2) -Gly-Lys^{∗}-Z₂-,
(3) -Gly-Phe-Lys^{∗}-Z₂-,
(4) -Met-Val-Lys^{∗}-Z₂-,
(5) -Gly-Tyr^{∗}-CH₂-C(=O)-NH-(CH₂)₂-Z₁-,
(6) -Gly-Lys-Lys^{∗}-Z₂-,
(7) -Gly-Arg-Lys^{∗}-Z₂-,
(8) -Gly-Lys^{∗}-C(=S)-NH-(1,4-phenylene)-NH-C(=S)-,
(9)-Met-Ile-NH-(CH₂)₂-NH-C(=S)-NH-(1,4-phenylene)-NH-C(=S)-,
(10) -Asp-Gly-Lys^{∗}-Z₂-,
(11) -Met-Gly-Lys^{∗}-Z₂-,
(12) -Met-Ile-Lys^{∗}-Z₂-,
(13) -Gly-Tyr^{∗}-CH₂-C(=O)-Lys^{∗}-Z₂-,
(14) -Val-NH-(CH₂)₂-Z₁-,
(15) -Ile-NH-(CH₂)₂-Z₁-,
(16) -Gly-Val-NH-(CH₂)₂-Z₁-,
(17) -Gly-Ile-NH-(CH₂)₂-Z₁-,
(18) -Met-Phe-Lys^{∗}-Z₂-,
(19) -Gly-Tyr-Lys^{∗}-Z₂-,
(20) -Gly-Tyr^{∗}-CH₂-C(=O)-NH-(CH₂O)₃-CH₂-C(=O)-NH-(CH₂)₂-Z₁-,
(21) -Gly-(3-(2-naphthyl)alanine)-Lys^{∗}-Z₂-,
(22) -Gly-diphenylalanine-Lys^{∗}-Z₂-,
(23) -Gly-Tyr-NH-(CH₂)₅-Z₁-,
(24) -Met-Ile-NH-(CH₂)₂-(A-4)-,
(25) -Met-Ile-NH-(CH₂)₂-(A-5)-,
(26) -Met-Ile-NH-(CH₂)₂-(II-II)-,
(27) -Met-Ile-NH-(CH₂)₂-(A-3)-, and
(28) -Met-Gly-Lys^{∗}-Z₃-.

Further, an embodiment of the "X" peptide linker is a peptide linker selected from the group consisting of
(1) -Met-Ile-NH-(CH₂)₂-Z₁-,
(2) -Gly-Lys^{∗}-Z₂-,
(3) -Gly-Phe-Lys^{∗}-Z₂-,
(4) -Met-Val-Lys^{∗}-Z₂-,
(5) -Gly-Tyr^{∗}-CH₂-C(=O)-NH-(CH₂)₂-Z₁-,
(6) -Gly-Lys-Lys^{∗}-Z₂-,
(7) -Gly-Arg-Lys^{∗}-Z₂-,
(8) -Gly-Lys^{∗}-C(=S)-NH-(1,4-phenylene)-NH-C(=S)-, and
(9)-Met-Ile-NH-(CH₂)₂-NH-C(=S)-NH-(1,4-phenylene)-NH-C(=S)-. An embodiment is a peptide linker selected from the group consisting of
   (1) -Met-Ile-NH-(CH₂)₂-Z₁-,
   (2) -Gly-Lys^{∗}-Z₂-,
   (3) -Gly-Phe-Lys^{∗}-Z₂-,
   (10) -Asp-Gly-Lys^{∗}-Z₂-,
   (11) -Met-Gly-Lys^{∗}-Z₂-,
   (21) -Gly-(3-(2-naphthyl)alanine)-Lys^{∗}-Z₂-,
   (24) -Met-Ile-NH-(CH₂)₂-(A-4)-,
   (25) -Met-Ile-NH-(CH₂)₂-(A-5)-,
   (26) -Met-Ile-NH-(CH₂)₂-(II-II)-,
   (27) -Met-Ile-NH-(CH₂)₂-(A-3)-, and
   (28) -Met-Gly-Lys^{∗}-Z₃-.

An embodiment of the "X" peptide linker is a peptide linker selected from the group consisting of
(1) -Met-Ile-NH-(CH₂)₂-Z₁-,
(2) -Gly-Lys^{∗}-Z₂-,
(3) -Gly-Phe-Lys^{∗}-Z₂-,
(4) -Met-Val-Lys^{∗}-Z₂-,
(5) -Gly-Tyr^{∗}-CH₂-C(=O)-NH-(CH₂)₂-Z₁-,
(7) -Gly-Arg-Lys^{∗}-Z₂-,
(8) -Gly-Lys^{∗}-C(=S)-NH-(1,4-phenylene)-NH-C(=S)-,
(9)-Met-Ile-NH-(CH₂)₂-NH-C(=S)-NH-(1,4-phenylene)-NH-C(=S)-,
(10) -Asp-Gly-Lys^{∗}-Z₂-,
(11) -Met-Gly-Lys^{∗}-Z₂-,
(12) -Met-Ile-Lys^{∗}-Z₂-,
(13) -Gly-Tyr^{∗}-CH₂-C(=O)-Lys^{∗}-Z₂-,
(14) -Val-NH-(CH₂)₂-Z₁-,
(15) -Ile-NH-(CH₂)₂-Z₁-,
(16) -Gly-Val-NH-(CH₂)₂-Z₁-,
(17) -Gly-Ile-NH-(CH₂)₂-Z₁-,
(18) -Met-Phe-Lys^{∗}-Z₂-,
(19) -Gly-Tyr-Lys^{∗}-Z₂-,
(20) -Gly-Tyr^{∗}-CH₂-C(=O)-NH-(CH₂O)₃-CH₂-C(=O)-NH-(CH₂)₂-Z₁-,
(21) -Gly-(3-(2-naphthyl)alanine)-Lys^{∗}-Z₂-,
(22) -Gly-diphenylalanine-Lys^{∗}-Z₂-,
(23) -Gly-Tyr-NH-(CH₂)₅-Z₁-,
(24) -Met-Ile-NH-(CH₂)₂-(A-4)-,
(25) -Met-Ile-NH-(CH₂)₂-(A-5)-,
(26) -Met-Ile-NH-(CH₂)₂-(II-II)-,
(27) -Met-Ile-NH-(CH₂)₂-(A-3)-, and
(28) -Met-Gly-Lys^{∗}-Z₃-.

An embodiment of the "X" peptide linker is a peptide linker selected from the group consisting of
(1) -Met-Ile-NH-(CH₂)₂-Z₁-,
(2) -Gly-Lys^{∗}-Z₂-,
(3) -Gly-Phe-Lys^{∗}-Z₂-,
(5) -Gly-Tyr^{∗}-CH₂-C(=O)-NH-(CH₂)₂-Z₁-,
(6) -Gly-Lys-Lys^{∗}-Z₂-,
(7) -Gly-Arg-Lys^{∗}-Z₂-, and
(8) -Gly-Lys^{∗}-C(=S)-NH-(1,4-phenylene)-NH-C(=S)-.

An embodiment of the "X" peptide linker is a peptide linker selected from the group consisting of
(2) -Gly-Lys^{∗}-Z₂-,
(3) -Gly-Phe-Lys^{∗}-Z₂-,
(10) -Asp-Gly-Lys^{∗}-Z₂-,
(11) -Met-Gly-Lys^{∗}-Z₂-, and
(21) -Gly-(3-(2-naphthyl)alanine)-Lys^{∗}-Z₂-.

An embodiment of the "X" peptide linker is a peptide linker selected from the group consisting of
(1) -Met-Ile-NH-(CH₂)₂-Z₁-,
(2) -Gly-Lys^{∗}-Z₂-,
(3) -Gly-Phe-Lys^{∗}-Z₂-,
(5) -Gly-Tyr^{∗}-CH₂-C(=O)-NH-(CH₂)₂-Z₁-,
(6) -Gly-Lys-Lys^{∗}-Z₂-, and
(8) -Gly-Lys^{∗}-C(=S)-NH-(1,4-phenylene)-NH-C(=S)-.

An embodiment of the "X" peptide linker is a peptide linker selected from the group consisting of
(1) -Met-Ile-NH-(CH₂)₂-Z₁-,
(2) -Gly-Lys^{∗}-Z₂-,
(3) -Gly-Phe-Lys^{∗}-Z₂-,
(5) -Gly-Tyr^{∗}-CH₂-C(=O)-NH-(CH₂)₂-Z₁-,
(6) -Gly-Lys-Lys^{∗}-Z₂-,
(8) -Gly-Lys^{∗}-C(=S)-NH-(1,4-phenylene)-NH-C(=S)-,
(24) -Met-Ile-NH-(CH₂)₂-(A-4)-,
(25) -Met-Ile-NH-(CH₂)₂-(A-5)-,
(26) -Met-Ile-NH-(CH₂)₂-(II-II)-,
(27) -Met-Ile-NH-(CH₂)₂-(A-3)-, and
(28) -Met-Gly-Lys^{∗}-Z₃-.

An embodiment of the "X" peptide linker is a peptide linker selected from the group consisting of
(1) -Met-Ile-NH-(CH₂)₂-Z₁-,
(2) -Gly-Lys^{∗}-Z₂-,
(3) -Gly-Phe-Lys^{∗}-Z₂-,
(9)-Met-Ile-NH-(CH₂)₂-NH-C(=S)-NH-(1,4-phenylene)-NH-C(=S)-,
(10) -Asp-Gly-Lys^{∗}-Z₂-,
(11) -Met-Gly-Lys^{∗}-Z₂-,
(12) -Met-Ile-Lys^{∗}-Z₂-,
(21) -Gly-(3-(2-naphthyl)alanine)-Lys^{∗}-Z₂-,
(24) -Met-Ile-NH-(CH₂)₂-(A-4)-,
(25) -Met-Ile-NH-(CH₂)₂-(A-5)-,
(26) -Met-Ile-NH-(CH₂)₂-(II-II)-,
(27) -Met-Ile-NH-(CH₂)₂-(A-3)-, and
(28) -Met-Gly-Lys^{∗}-Z₃-.

An embodiment of the "X" peptide linker is a peptide linker selected from the group consisting of
(1) -Met-Ile-NH-(CH₂)₂-Z₁-,
(4) -Met-Val-Lys^{∗}-Z₂-,
(9) -Met-Ile-NH-(CH₂)₂-NH-C(=S)-NH-(1,4-phenylene)-NH-C(=S)-,
(11) -Met-Gly-Lys^{∗}-Z₂-,
(12) -Met-Ile-Lys^{∗}-Z₂-,
(18) -Met-Phe-Lys^{∗}-Z₂-,
(24) -Met-Ile-NH-(CH₂)₂-(A-4)-,
(25) -Met-Ile-NH-(CH₂)₂-(A-5)-,
(26) -Met-Ile-NH-(CH₂)₂-(II-II)-,
(27) -Met-Ile-NH-(CH₂)₂-(A-3)-, and
(28) -Met-Gly-Lys^{∗}-Z₃-.

An embodiment of the "X" peptide linker is a peptide linker selected from the group consisting of
(1) -Met-Ile-NH-(CH₂)₂-Z₁-,
(9)-Met-Ile-NH-(CH₂)₂-NH-C(=S)-NH-(1,4-phenylene)-NH-C(=S)-,
(12) -Met-Ile-Lys^{∗}-Z₂-,
(24) -Met-Ile-NH-(CH₂)₂-(A-4)-,
(25) -Met-Ile-NH-(CH₂)₂-(A-5)-,
(26) -Met-Ile-NH-(CH₂)₂-(II-II)-, and
(27) -Met-Ile-NH-(CH₂)₂-(A-3)-.

An embodiment of the "X" peptide linker is a peptide linker selected from the group consisting of
(4) -Met-Val-Lys^{∗}-Z₂-,
(11) -Met-Gly-Lys^{∗}-Z₂-,
(18) -Met-Phe-Lys^{∗}-Z₂-, and
(28) -Met-Gly-Lys^{∗}-Z₃-.

An embodiment of the "X" peptide linker is a peptide linker selected from the group consisting of
(11) -Met-Gly-Lys^{∗}-Z₂-, and
(28) -Met-Gly-Lys^{∗}-Z₃-.
1-4. Spacer or bond (S₁)

In the conjugate of the present invention, a ligand (Y) and a peptide linker (X) are directly bound (that is, S₁ is a bond) or are bound via a spacer (that is, S₁ is a spacer).

As used herein, the S₁ "spacer" is a group introduced to create a certain distance between the ligand and the peptide linker or Fab¹ or for binding between the ligand and the peptide linker, and examples of an embodiment thereof include -C(=O)-CH₂O-(1,3-phenylene)-C(=O)-, -C(=O)-(CH₂CH₂O)₄-(1,3-phenylene)-C(=O-, -C(=O)-(1,3-phenylene)-C(=O)-, -NH-CH₂-(1,3-phenylene)-C(=O)-, -NH-(CH₂)₂-C(=O)-, -CH₂-(1,4-phenylene)-NH-C(=S)-, -NH-(CH₂CH₂O)₃-CH₂-C(=O)-NH-CH₂-(1,3-phenylene)-C(=O)-, -NH-CH₂-(1,4-phenylene)-NH-C(=O)-, -NH-(CH₂)₃-C(=O)-, -NH-(CH₂CH₂O)₃-CH₂-C(=O)-, -NH-CH₂-(1,4-phenylene)-C(=O)-, -CH₂-(1,4-phenylene)-NH-C(=S)-NH-CH₂-(1,3-phenylene)-C(=O)-, -CH₂-(1,4-phenylene)-NH-C(=S)-NH-(CH₂CH₂O)₃-CH₂-C(=O)-NH-CH₂-(1,3-phenylene)-C(=O)-, and spacers represented by the following formulas (a) to (q).

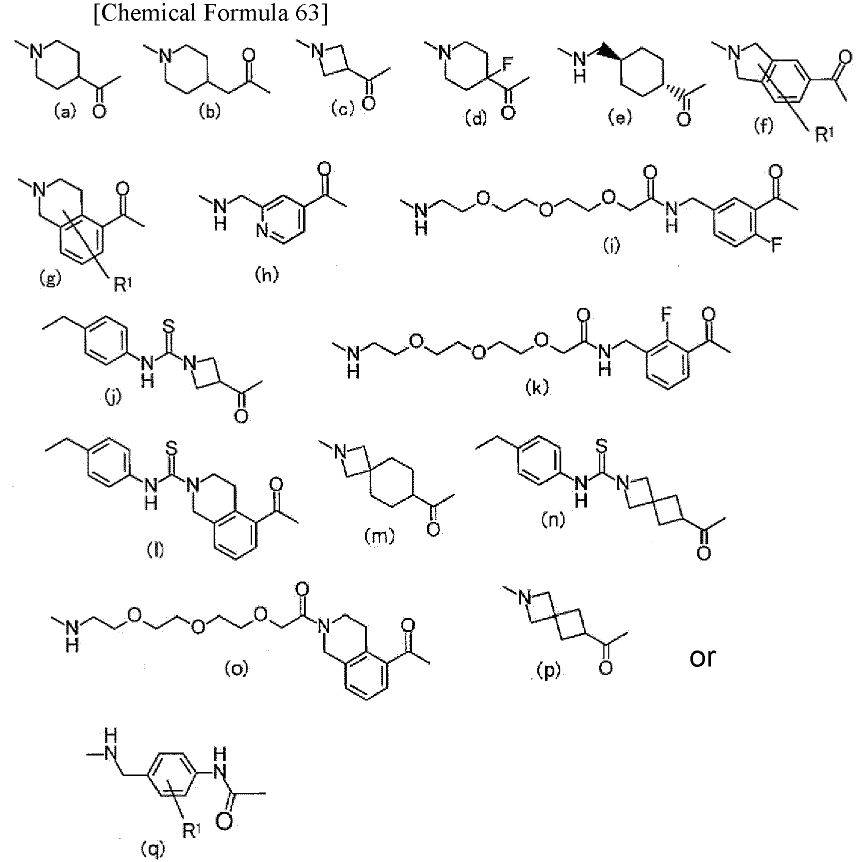

In an embodiment, S₁ is -C(=O)-CH₂O-(1,3-phenylene)-C(=O)-, -C(=O)-(CH₂CH₂O)₄-(1,3-phenylene)-C(=O)-, or -C(=O)-(1,3-phenylene)-C(=O)-. In an embodiment, S₁ is -NH-CH₂-(1,3-phenylene)-C(=O)-, -NH-(CH₂)₂-C(=O)-, -CH₂-(1,4-phenylene)-NH-C(=S)-, -NH-(CH₂CH₂O)₃-CH₂-C(=O)-NH-CH₂-(1,3-phenylene)-C(=O)-, - NH-CH₂-(1,4-phenylene)-NH-C(=O)-, -NH-(CH₂)₃-C(=O)-, -NH-(CH₂CH₂O)₃-CH₂-C(=O)-, -NH-CH₂-(1,4-phenylene)-C(=O)-, -CH₂-(1,4-phenylene)-NH-C(=S)-NH-CH₂-(1,3-phenylene)-C(=O)-, -CH₂-(1,4-phenylene)-NH-C(=S)-NH-(CH₂CH₂O)₃-CH₂-C(=O)-NH-CH₂-(1,3-phenylene)-C(=O)-, or a spacer represented by any of the above formulas (a) to (q). In an embodiment, S₁ is -NH-CH₂-(1,4-phenylene)-NH-C(=O)- or a spacer represented by the above formula (g), (i), or (k).

In an embodiment, S₁ is -C(=O)-CH₂O-(1,3-phenylene)-C(=O)-, -C(=O)-(CH₂CH₂O)₄-(1,3-phenylene)-C(=O)-, -NH-CH₂-(1,3-phenylene)-C(=O)-, -NH-CH₂-(1,4-phenylene)-NH-C(=O)-, -NH-CH₂-(1,4-phenylene)-C(=O)-, or -C(=O)-(1,3-phenylene)-C(=O)-, or is

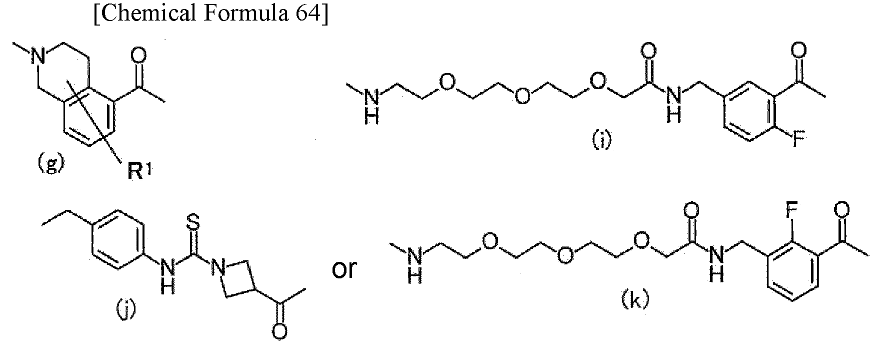

In an embodiment, S₁ is a bond.

In addition, in the conjugate in which Y is DOTA according to the present invention, DOTA and the anti-human CEACAM5 antibody Fab fragment (Fab¹) may be directly bound or bound via a spacer (-CH₂-(1,4-phenylene)-NH-C(=S)-). However, the conjugate in which DOTA and the anti-human CEACAM5 antibody Fab fragment (Fab¹) are bound via - CH₂-(1,4-phenylene)-NH-C(=S)-, which is a spacer, is a conjugate represented by the following formula (VI). 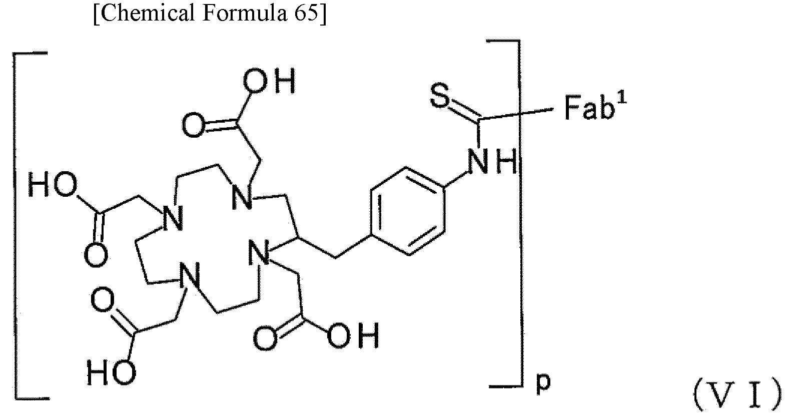 (VI)

In addition, the S₁ spacer described herein includes a novel spacer and spacers represented by formulas (g) and (1), which are particularly useful as a spacer when DOTA is bound to a peptide linker including a peptide consisting of 2 to 4 amino acids having an amino acid sequence cleaved by a renal brush border membrane enzyme or a lysosomal enzyme. As shown in the kidney accumulation evaluation test of Example 4 described later, in a conjugate in which a peptide linker cleaved by an enzyme is combined with DOTA and a spacer represented by formula (g), it has been confirmed that the accumulation of the labeling portion in the kidneys is reduced. In an embodiment, S₁ is a spacer represented by formula (g) or (1).

An embodiment of the conjugate of the present invention is a conjugate in which Y is DOTA, S₁ is a spacer represented by formula (g) or (1), and X is a peptide linker including a peptide consisting of 2 to 4 amino acids having an amino acid sequence cleaved by a renal brush border enzyme or a lysosomal enzyme.

An embodiment thereof is a conjugate in which Y is DOTA, S₁ is a spacer represented by formula (g) or (1), and X is a peptide linker selected from the group consisting of
(2) -Gly-Lys^{∗}-Z₂-,
(3) -Gly-Phe-Lys^{∗}-Z₂-,
(10) -Asp-Gly-Lys^{∗}-Z₂-,
(11) -Met-Gly-Lys^{∗}-Z₂-, and
(21) -Gly-(3-(2-naphthyl)alanine)-Lys^{∗}-Z₂-.

An embodiment is a conjugate in which Y is DOTA, S₁ is -NH-CH₂-(1,4-phenylene)-NH-C(=O)- or a spacer represented by any of formula (g), (i), or (k), and X is a peptide linker selected from the group consisting of
(1)-Met-Ile-NH-(CH₂)₂-Z₁-,
(11) -Met-Gly-Lys^{∗}-Z₂-,
(12) -Met-Ile-Lys^{∗}-Z₂-,
(24) -Met-Ile-NH-(CH₂)₂-(A-4)-,
(25) -Met-Ile-NH-(CH₂)₂-(A-5)-,
(26)-Met-Ile-NH-(CH₂)₂-(II-II)-,
(27)-Met-Ile-NH-(CH₂)₂-(A-3)-, and
(28)-Met-Gly-Lys^{∗}-Z₃-.

An embodiment is a conjugate in which Y is DOTA, S₁ is a spacer represented by formula (g), and X is a peptide linker selected from the group consisting of
(11) -Met-Gly-Lys^{∗}-Z₂-, and
(28) -Met-Gly-Lys^{∗}-Z₃-.

The conjugate of the present invention consists of a combination of the above embodiments.
Specific embodiments of the conjugate of the present invention are as follows. In the formulas, Fab² is a Fab fragment comprising a heavy chain fragment consisting of the amino acid sequence shown in SEQ ID NO: 2 and a light chain consisting of the amino acid sequence shown in SEQ ID NO: 4.
p is a natural number of 1 to 25. Fab² is bound to an adjacent carbon atom via p amino groups or thiol groups in the Fab².

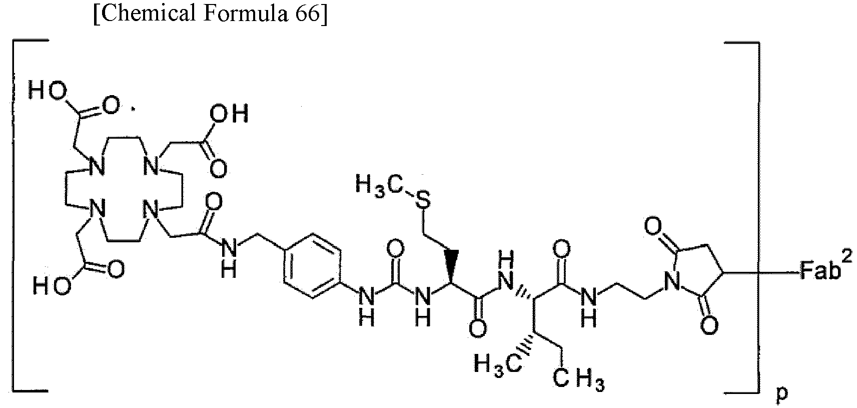

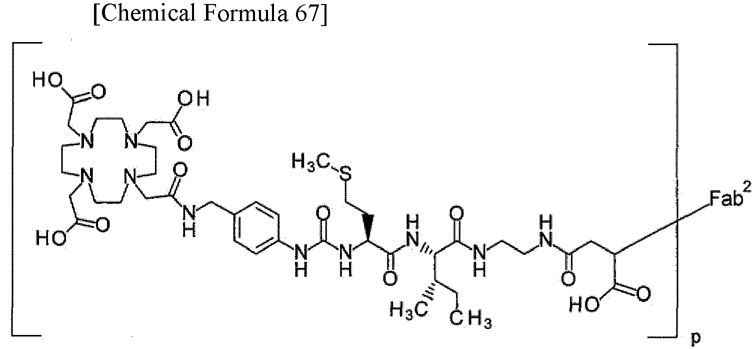

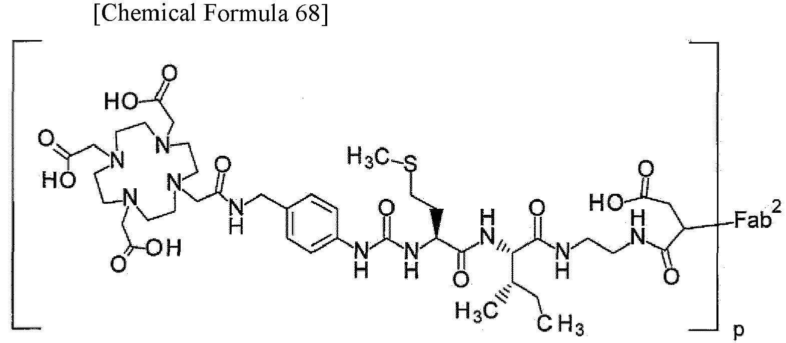

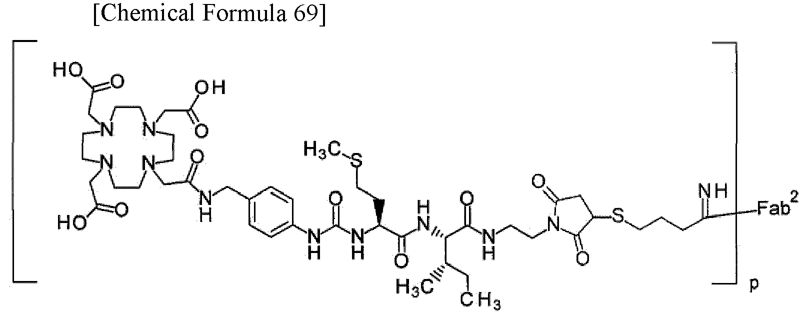

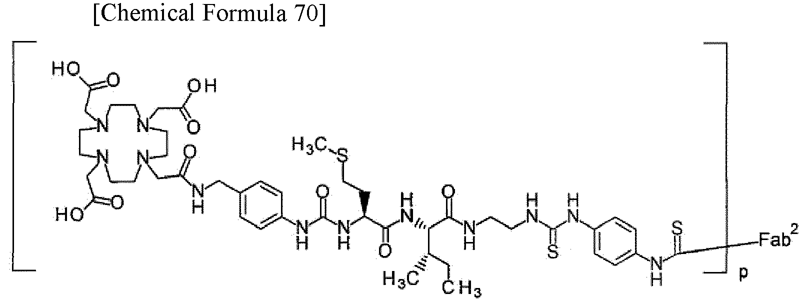

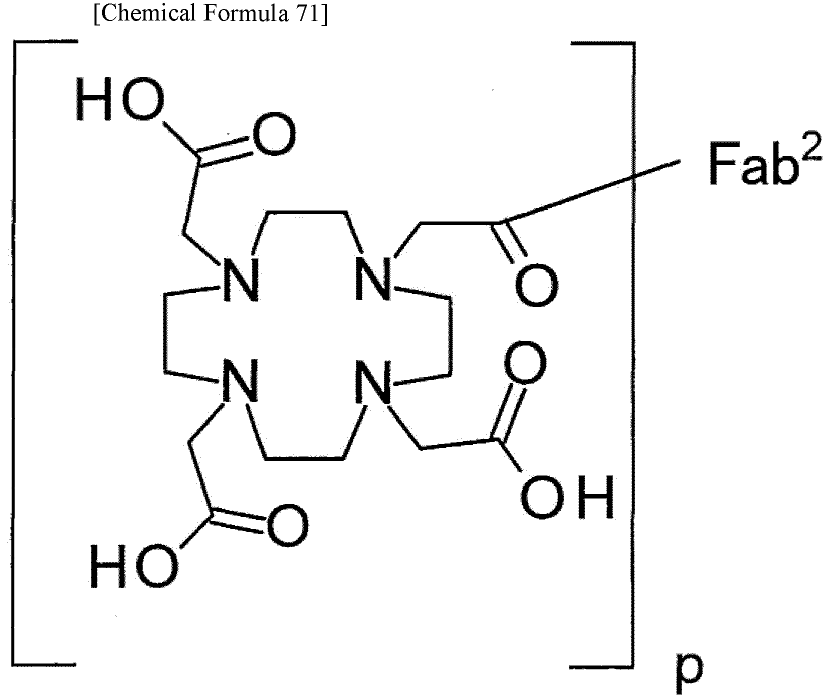

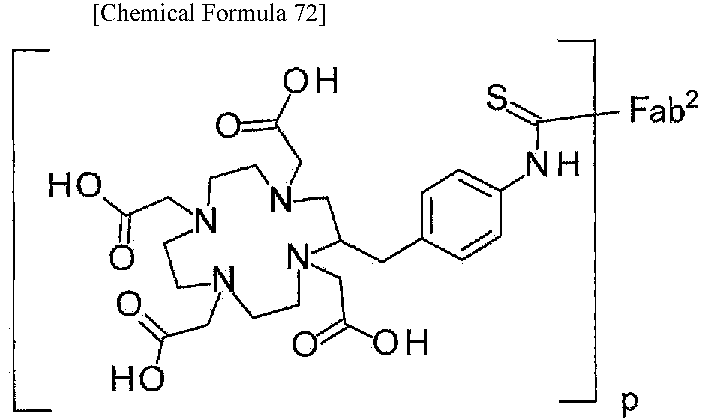

The labeling portion of a combination in which Y is DOTA, S₁ is a spacer represented by formula (g) or (1), and X is a peptide linker including a peptide consisting of 2 to 4 amino acids having an amino acid sequence cleaved by a renal brush border membrane enzyme or a lysosomal enzyme is useful as a labeling portion which is expected to reduce nephrotoxicity and the like in similar conjugates using various Fab antibodies without being limited to the combination with Fab¹ of the present invention, and the present invention also includes the invention of the labeling portion itself.

In the production of the conjugate of the present invention, the binding of the anti-CEACAM5 antibody Fab fragment to the ligand, spacer and/or peptide linker, and the binding of the ligand to the spacer and/or peptide linker can be appropriately carried out by those skilled in the art by a known method.

As used herein, the "labeling portion" means, for example, a portion other than Fab¹ in formula (I) and a portion other than Biomolecule¹ or Biomolecule² in formula (Ia) or (Ib). The labeling portion is (i) a ligand and a peptide linker (Y-Si-X: wherein S₁ is a bond and X is a peptide linker), (ii) a ligand (Y-Si-X: wherein S₁ and X are each a bond), or (iii) a ligand, a spacer, and a peptide linker (Y-Si-X: wherein S₁ is a spacer and X is a peptide linker). In an embodiment, the labeling portion is (ii) a ligand. In an embodiment, the labeling portion is (i) a ligand and a peptide linker or (iii) a ligand, a spacer, and a peptide linker. Here, the ligand of the "labeling portion" may further include a metal, and some embodiments are (i) a ligand including a metal and a peptide linker, (ii) a ligand, or (iii) a ligand, a spacer, and a peptide linker, and also are (i) a ligand forming a chelate complex with a metal and a peptide linker, (ii) a ligand forming a chelate complex with a metal, or (iii) a ligand forming a chelate complex with a metal, a spacer, and a peptide linker. Further, there is the case of (iv) a peptide linker in which a spacer is further included in the peptide linker. In addition, in formula (Ia) or (Ib), the labeling portion means a portion other than Biomolecule¹ or Biomolecule^{2.}

### 1-5. The number (p) of the labeling portion (Y-S₁-X) bound to the anti-human CEACAM5 antibody Fab fragment (Fab¹) and the number (p) of the labeling portion bound to Biomolecule¹ and Biomolecule² of formula (Ia) or (Ib)

The conjugate of the present invention is a conjugate in which one or more labeling portions (Y-S₁-X) are bound via one or more amino groups or thiol groups in the anti-human CEACAM5 antibody Fab fragment (Fab¹). In addition, the conjugate of the present invention is a conjugate in which one or more labeling portions are bound via one or more amino groups or thiol groups in Biomolecule¹ and biomolecule² of formula (Ia) or (Ib). The conjugate of the present invention may be a mixture of conjugates in which the number of labeling portions bound is different from each other, and this shows that the conjugate is any of conjugates in which 1 to 25 labeling portions (Y-S₁-X) are bound to Fab¹ in formula (I) or a mixture thereof. For one Fab¹, the conjugate of the present invention includes 1 to 25 labeling portions (Y-S₁-X) in an embodiment, includes 1 to 23 labeling portions (Y-S₁-X) in an embodiment, includes 1 to 15 labeling portions (Y-S₁-X) in an embodiment, includes 1 to 11 labeling portions (Y-S₁-X) in an embodiment, includes 1 to 9 labeling portions (Y-S₁-X) in an embodiment, includes 1 to 7 labeling portions (Y-S₁-X) in an embodiment, includes 1 to 5 labeling portions (Y-S₁-X) in an embodiment, and further, includes 1 to 4 labeling portions (Y-S₁-X) in an embodiment.
It is shown that in formula (Ia) or (Ib), Biomolecule¹ and Biomolecule² are any of conjugates in which the 1 to 25 labeling portions are bound or a mixture thereof. For one Biomolecule¹ and Biomolecule², the conjugate of the present invention includes the 1 to 25 labeling portions in an embodiment, includes the 1 to 23 labeling portions in an embodiment, includes the 1 to 15 labeling portions in an embodiment, includes the 1 to 11 labeling portions in an embodiment, includes the 1 to 9 labeling portions in an embodiment, includes the 1 to 7 labeling portions in an embodiment, includes the 1 to 5 labeling portions in an embodiment, and further, includes the 1 to 4 labeling portions in an embodiment.

That is, "p" that represents the number of a labeling portion (Y-S₁-X) bound to one Fab¹ and "p" that represents the number of the labeling portion bound to one Biomolecule¹ and Biomolecule² are identical or different and each are a natural number of 1 to 25 in an embodiment, are a natural number of 1 to 23 in an embodiment, are a natural number of 1 to 15 in an embodiment, are a natural number of 1 to 11 in an embodiment, are a natural number of 1 to 9 in an embodiment, are a natural number of 1 to 7 in an embodiment, are a natural number of 1 to 6 in an embodiment, are a natural number of 1 to 5 in an embodiment, are a natural number of 1 to 4 in an embodiment, and further, are a natural number of 1 to 3 in an embodiment.

### 2. Polynucleotide encoding the anti-human CEACAM5 antibody Fab fragment included in the conjugate of the present invention

In an embodiment, the anti-human CEACAM5 antibody Fab fragment included in the conjugate of the present invention is encoded by a polynucleotide including a nucleotide sequence encoding the heavy chain fragment of the anti-human CEACAM5 antibody Fab fragment, and a polynucleotide including a nucleotide sequence encoding the light chain of the anti-human CEACAM5 antibody Fab fragment.

In an embodiment, the polynucleotide encoding the anti-human CEACAM5 antibody Fab fragment included in the conjugate of the present invention is a polynucleotide including a nucleotide sequence encoding a heavy chain fragment including a heavy chain variable region consisting of the amino acid sequence shown by amino acids 1 to 121 of SEQ ID NO: 2, or a polynucleotide including a nucleotide sequence encoding a light chain including a light chain variable region consisting of the amino acid sequence shown by amino acids 1 to 112 of SEQ ID NO: 4.

Examples of the polynucleotide including a nucleotide sequence encoding a heavy chain fragment including a heavy chain variable region consisting of the amino acid sequence shown by amino acids 1 to 121 of SEQ ID NO: 2 include a polynucleotide including the nucleotide sequence of nucleotides 1 to 363 of SEQ ID NO: 1. Examples of the polynucleotide including a nucleotide sequence encoding a light chain including a light chain variable region consisting of the amino acid sequence of amino acids 1 to 112 of SEQ ID NO: 4 include a polynucleotide including the nucleotide sequence of nucleotides 1 to 336 of SEQ ID NO: 3.

In one embodiment, the polynucleotide encoding the anti-human CEACAM5 antibody Fab fragment included in the conjugate of the present invention is a polynucleotide including a nucleotide sequence encoding a heavy chain fragment consisting of the amino acid sequence shown in SEQ ID NO: 2, or a polynucleotide including a nucleotide sequence encoding a light chain consisting of the amino acid sequence shown in SEQ ID NO: 4.

Examples of the polynucleotide including a nucleotide sequence encoding a heavy chain fragment consisting of the amino acid sequence shown in SEQ ID NO: 2 include a polynucleotide including the nucleotide sequence shown in SEQ ID NO: 1. Examples of the polynucleotide including a nucleotide sequence encoding a light chain consisting of the amino acid sequence shown in SEQ ID NO: 4 include a polynucleotide including the nucleotide sequence shown in SEQ ID NO: 3.

The polynucleotide encoding the anti-human CEACAM5 antibody Fab fragment included in the conjugate of the present invention can be synthesized by using a gene synthesis method known in the art based on the nucleotide sequence designed based on the amino acid sequences of the heavy chain fragment and the light chain of the anti-human CEACAM5 antibody Fab fragment. As such a gene synthesis method, various methods known to those skilled in the art such as the method for synthesizing an antibody gene disclosed in International Publication No. 90/07861 can be used.

### 3. Expression vector of a polynucleotide encoding the anti-human CEACAM5 antibody Fab fragment included in the conjugate of the present invention

The expression vector of a polynucleotide encoding the anti-human CEACAM5 antibody Fab fragment included in the conjugate of the present invention includes an expression vector including a polynucleotide including a nucleotide sequence encoding the heavy chain fragment of the anti-human CEACAM5 antibody Fab fragment, an expression vector including a polynucleotide including a nucleotide sequence encoding the light chain of the anti-human CEACAM5 antibody Fab fragment, and an expression vector including a polynucleotide including a nucleotide sequence encoding the heavy chain fragment of the anti-human CEACAM5 antibody Fab fragment and a polynucleotide including a nucleotide sequence encoding the light chain of the anti-human CEACAM5 antibody Fab fragment.

Examples of preferable expression vectors include an expression vector including a polynucleotide including a nucleotide sequence encoding a heavy chain fragment including a heavy chain variable region consisting of the amino acid sequence shown by amino acids 1 to 121 of SEQ ID NO: 2, an expression vector including a polynucleotide including a nucleotide sequence encoding a light chain including a light chain variable region consisting of the amino acid sequence shown by amino acids 1 to 112 of SEQ ID NO: 4, and an expression vector including a polynucleotide including a nucleotide sequence encoding a heavy chain fragment including a heavy chain variable region consisting of the amino acid sequence shown by amino acids 1 to 121 of SEQ ID NO: 2 and a polynucleotide including a nucleotide sequence encoding a light chain including a light chain variable region consisting of the amino acid sequence shown by amino acids 1 to 112 of SEQ ID NO: 4.

Preferable expression vectors include an expression vector including a polynucleotide including a nucleotide sequence encoding a heavy chain fragment consisting of the amino acid sequence shown in SEQ ID NO: 2, an expression vector including a polynucleotide including a nucleotide sequence encoding a light chain consisting of the amino acid sequence shown in SEQ ID NO: 4, and an expression vector including a polynucleotide including a nucleotide sequence encoding a heavy chain fragment consisting of the amino acid sequence shown in SEQ ID NO: 2 and a polynucleotide including a nucleotide sequence encoding a light chain consisting of the amino acid sequence shown in SEQ ID NO: 4.

These expression vectors are not particularly limited as long as they can produce a polypeptide encoded by the polynucleotide of the present invention in various host cells of prokaryotic cells and/or eukaryotic cells. Examples of such expression vectors include a plasmid vector and a viral vector (for example, an adenovirus or a retrovirus), and preferably pEE6.4 or pEE12.4 (Lonza) can be used.

In addition, these expression vectors can include a promoter operably linked to a gene encoding a heavy chain fragment and/or a light chain of a polynucleotide encoding the anti-human CEACAM5 antibody Fab fragment included in the conjugate of the present invention. Examples of the promoter for expressing a Fab fragment in a host cell include, when the host cell is a bacterium of the genus Escherichia, a Trp promoter, a lac promoter, a recA promoter, a λPL promoter, a lpp promoter, and a tac promoter. Examples of a promoter for expression in a yeast include a PH05 promoter, a PGK promoter, a GAP promoter, and an ADH promoter, and examples of a promoter for expression in a bacterium of the genus Bacillus include an SL01 promoter, an SP02 promoter, and a penP promoter.
In addition, examples thereof include, when the host is a eukaryotic cell such as a mammalian cell, a promoter derived from a virus such as CMV, RSV, or SV40, a retrovirus promoter, an actin promoter, an EF (elongation factor) 1α promoter, and a heat shock promoter.

These expression vectors can further include, when a bacterium, particularly E. coli, is used as a host cell, a start codon, a stop codon, a terminator region, and a replicable unit. On the other hand, when a yeast, an animal cell, or an insect cell is used as the host, the expression vectors can include a start codon and a stop codon. In addition, in this case, the expression vectors may include an enhancer sequence, 5' and 3' untranslated regions of a gene encoding the heavy chain fragment and/or the light chain of the invention, a secretory signal sequence, a splicing junction, a polyadenylation site, a replicable unit, or the like. In addition, the expression vectors may include a selection marker commonly used depending on the intended purpose (for example, a tetracycline resistance gene, an ampicillin resistance gene, a kanamycin resistance gene, a neomycin resistance gene, or a dihydrofolate reductase gene).

### 4. Host cell transformed with an expression vector

Host cells transformed with an expression vector include a host cell selected from the group consisting of the following (a) to (d):
(a) a host cell transformed with an expression vector including a polynucleotide including a nucleotide sequence encoding the heavy chain fragment of the anti-human CEACAM5 antibody Fab fragment;
(b) a host cell transformed with an expression vector including a polynucleotide including a nucleotide sequence encoding the light chain of the anti-human CEACAM5 antibody Fab fragment;
(c) a host cell transformed with an expression vector including a polynucleotide including a nucleotide sequence encoding the heavy chain fragment of the anti-human CEACAM5 antibody Fab fragment and a polynucleotide including a nucleotide sequence encoding the light chain of the anti-human CEACAM5 antibody Fab fragment; and
(d) a host cell transformed with an expression vector including a polynucleotide including a nucleotide sequence encoding the heavy chain fragment of the anti-human CEACAM5 antibody Fab fragment and an expression vector including a polynucleotide including a nucleotide sequence encoding the light chain of the anti-human CEACAM5 antibody Fab fragment.

In one embodiment, the host cell transformed with an expression vector is a host cell transformed with an expression vector selected from the group consisting of the following (a) to (d):
(a) a host cell transformed with an expression vector including a polynucleotide including a nucleotide sequence encoding a heavy chain fragment including a heavy chain variable region consisting of the amino acid sequence shown by amino acids 1 to 121 of SEQ ID NO: 2;
(b) a host cell transformed with an expression vector including a polynucleotide including a nucleotide sequence encoding a light chain including a light chain variable region consisting of the amino acid sequence shown in amino acids 1 to 112 of SEQ ID NO: 4;
(c) a host cell transformed with an expression vector including a polynucleotide including a nucleotide sequence encoding a heavy chain fragment including a heavy chain variable region consisting of the amino acid sequence shown by amino acids 1 to 121 of SEQ ID NO: 2 and a polynucleotide including a nucleotide sequence encoding a light chain including a light chain variable region consisting of the amino acid sequence shown by amino acids 1 to 112 of SEQ ID NO: 4; and
(d) a host cell transformed with an expression vector including a polynucleotide including a nucleotide sequence encoding a heavy chain fragment including a heavy chain variable region consisting of the amino acid sequence shown by amino acids 1 to 121 of SEQ ID NO: 2 and an expression vector including a polynucleotide including a nucleotide sequence encoding a light chain including a light chain variable region consisting of the amino acid sequence shown by amino acids 1 to 112 of SEQ ID NO: 4.

In one embodiment, the host cell transformed with an expression vector is a host cell transformed with an expression vector selected from the group consisting of the following (a) to (d):
(a) a host cell transformed with an expression vector including a polynucleotide including a nucleotide sequence encoding a heavy chain fragment consisting of the amino acid sequence shown in SEQ ID NO: 2;
(b) a host cell transformed with an expression vector including a polynucleotide including a nucleotide sequence encoding a light chain consisting of the amino acid sequence shown in SEQ ID NO: 4;
(c) a host cell transformed with an expression vector including a polynucleotide including a nucleotide sequence encoding a heavy chain fragment consisting of the amino acid sequence shown in SEQ ID NO: 2 and a polynucleotide including a nucleotide sequence encoding a light chain consisting of the amino acid sequence shown in SEQ ID NO: 4; and
(d) a host cell transformed with an expression vector including a polynucleotide including a nucleotide sequence encoding a heavy chain fragment consisting of the amino acid sequence shown in SEQ ID NO: 2 and an expression vector including a polynucleotide including a nucleotide sequence encoding a light chain consisting of the amino acid sequence shown in SEQ ID NO: 4.

Examples of a host cell transformed with a preferable expression vector include a host cell transformed with an expression vector including a polynucleotide including a nucleotide sequence encoding the heavy chain fragment of the anti-human CEACAM5 antibody Fab fragment included in the conjugate of the present invention and a polynucleotide including a nucleotide sequence encoding the light chain of the anti-human CEACAM5 antibody Fab fragment included in the conjugate of the present invention, and a host cell transformed with an expression vector including a polynucleotide including a nucleotide sequence encoding the heavy chain fragment of the anti-human CEACAM5 antibody Fab fragment included in the conjugate of the present invention and an expression vector including a polynucleotide including a nucleotide sequence encoding the light chain of the anti-human CEACAM5 antibody Fab fragment included in the conjugate of the present invention.

The host cell transformed with an expression vector is not particularly limited as long as it is compatible with the expression vector used and can be transformed with the expression vector to express the Fab fragment, and examples thereof include various cells such as a natural cell or an artificially established cell (for example, a bacterium (a bacterium of the genus Escherichia or a bacterium of the genus Bacillus), a yeast (of the genus Saccharomyces, the genus Pichia, or the like), and an animal cell or an insect cell (for example, Sf9)), a mammalian cell line (for example, a cultured cell such as a CHO-K1SV cell, a CHO-DG44 cell, or a 293 cell) commonly used in the technical field of the present invention. The transformation itself can be carried out by a known method such as a calcium phosphate method or an electroporation method.

### 5. Method for producing the anti-human CEACAM5 antibody Fab fragment included in the conjugate of the present invention

Production of the anti-human CEACAM5 antibody Fab fragment included in the conjugate of the present invention includes the step of culturing the transformed host cell described above to express the anti-human CEACAM5 antibody Fab fragment.

In one embodiment, the transformed host cell cultured in the production of the anti-human CEACAM5 antibody Fab fragment included in the conjugate of the present invention is selected from the group consisting of the following (a) to (c):
(a) a host cell transformed with an expression vector including a polynucleotide including a nucleotide sequence encoding the heavy chain fragment of the anti-human CEACAM5 antibody Fab fragment included in the conjugate of the present invention and a polynucleotide including a nucleotide sequence encoding the light chain of the anti-human CEACAM5 antibody Fab fragment included in the conjugate of the present invention;
(b) a host cell transformed with an expression vector including a polynucleotide including a nucleotide sequence encoding the heavy chain fragment of the anti-human CEACAM5 antibody Fab fragment included in the conjugate of the present invention and an expression vector including a polynucleotide including a nucleotide sequence encoding the light chain of the anti-human CEACAM5 antibody Fab fragment included in the conjugate of the present invention; and
(c) a host cell transformed with an expression vector including a polynucleotide containing a nucleotide sequence encoding a heavy chain fragment of the anti-human CEACAM5 antibody Fab fragment included in the conjugate of the present invention, and a host cell transformed with an expression vector including a polynucleotide including a nucleotide sequence encoding the light chain of the anti-human CEACAM5 antibody Fab flagmen included in the conjugate of the present invention.

In an embodiment, the transformed host cell cultured in the production of the anti-human CEACAM5 antibody Fab fragment included in the conjugate of the present invention is selected from the group consisting of the following (a) to (c):
(a) a host cell transformed with an expression vector including a polynucleotide including a nucleotide sequence encoding a heavy chain fragment including a heavy chain variable region consisting of the amino acid sequences shown by amino acids 1 to 121 of SEQ ID NO: 2 and a polynucleotide including a nucleotide sequence encoding a light chain including a light chain variable region consisting of the amino acid sequence shown by amino acids 1 to 112 of SEQ ID NO: 4;
(b) a host cell transformed with an expression vector including a polynucleotide including a nucleotide sequence encoding a heavy chain fragment including a heavy chain variable region consisting of the amino acid sequence shown by amino acids 1 to 121 of SEQ ID NO: 2, and an expression vector including a polynucleotide including a nucleotide sequence encoding a light chain including a light chain variable region consisting of the amino acid sequence shown by amino acids 1 to 112 of SEQ ID NO: 4; and
(c) a host cell transformed with an expression vector including a polynucleotide including a nucleotide sequence encoding a heavy chain fragment including a heavy chain variable region consisting of the amino acid sequence shown by amino acids 1 to 121 of SEQ ID NO: 2, and a host cell transformed with an expression vector including a polynucleotide including a nucleotide sequence encoding a light chain including a light chain variable region consisting of the amino acid sequence shown by amino acids 1 to 112 of SEQ ID NO: 4.

In an embodiment, the transformed host cell cultured in the production of the anti-human CEACAM5 antibody Fab fragment included in the conjugate of the present invention is selected from the group consisting of the following (a) to (c):
(a) a host cell transformed with an expression vector including a polynucleotide including a nucleotide sequence encoding a heavy chain fragment consisting of the amino acid sequence shown in SEQ ID NO: 2 and a polynucleotide including a nucleotide sequence encoding a light chain consisting of the amino acid sequence shown in SEQ ID NO: 4;
(b) a host cell transformed with an expression vector including a polynucleotide including a nucleotide sequence encoding a heavy chain fragment consisting of the amino acid sequence shown in SEQ ID NO: 2 and an expression vector including a polynucleotide including a nucleotide sequence encoding a light chain consisting of the amino acid sequence shown in SEQ ID NO: 4; and
(c) a host cell transformed with an expression vector including a polynucleotide including a nucleotide sequence encoding a heavy chain fragment consisting of the amino acid sequence shown in SEQ ID NO: 2, and a host cell transformed with an expression vector including a polynucleotide including a nucleotide sequence encoding a light chain consisting of the amino acid sequence shown in SEQ ID NO: 4.

The transformed host cell used is preferably a host cell transformed with an expression vector including a polynucleotide including a nucleotide sequence encoding the heavy chain fragment of the anti-human CEACAM5 antibody Fab fragment included in the conjugate of the present invention and a polynucleotide including a nucleotide sequence encoding the light chain of the anti-human CEACAM5 antibody Fab fragment included in the conjugate of the present invention, or a host cell transformed with an expression vector including a polynucleotide including a nucleotide sequence encoding the heavy chain fragment of the anti-human CEACAM5 antibody Fab fragment included in the conjugate of the present invention and an expression vector including a polynucleotide including a nucleotide sequence encoding the light chain of the anti-human CEACAM5 antibody Fab fragment included in the conjugate of the present invention.

In the production of the anti-human CEACAM5 antibody Fab fragment included in the conjugate of the present invention, a transformed host cell can be cultured in a nutrient medium. The nutrient medium preferably includes a carbon source, an inorganic nitrogen source, or an organic nitrogen source necessary for the growth of the transformed host cell. Examples of the carbon source include glucose, dextran, soluble starch, and sucrose, and examples of the inorganic nitrogen source or the organic nitrogen source include an ammonium salt, a nitrate, an amino acid, corn steep liquor, peptone, casein, meat extract, soybean starch, and potato extract. In addition, the nutrient medium may, if desired, include another nutrient (for example, an inorganic salt (for example, calcium chloride, sodium dihydrogen phosphate, or magnesium chloride), a vitamin, and an antibiotic (for example, tetracycline, neomycin, ampicillin, or kanamycin).

The culture itself of a transformed host cell is carried out by a known method. Culture conditions, such as temperature, pH of the medium, and culture time, are appropriately selected. For example, when the host is an animal cell, as the medium, MEM medium (Science;1955;122:501.) containing about 5 to 20% of fetal bovine serum, DMEM medium (Virology; 1959;8:396-97.), RPMI 1640 medium (J. Am. Med. Assoc.;1967;199:519-24.), 199 medium (Proc. Soc. Exp. Biol. Med.;1950;73:1-8.), or the like can be used. The pH of the medium is preferably about 6 to 8, and the culture is usually carried out at about 30 to 40°C for about 15 to 336 hours, and if necessary, aeration and stirring can also be carried out. When the host is an insect cell, examples of the medium include Grace's medium (PNAS;1985;82:8404-8.) containing fetal bovine serum, and the pH thereof is preferably about 5 to 8. Culture is usually carried out at about 20 to 40°C for 15 to 100 hours, and if necessary, aeration and stirring can also be carried out. When the host is a bacterium, an actinomycete, a yeast, or a filamentous fungus, for example, a liquid medium containing the above nutrient source is suitable. A medium having a pH of 5 to 8 is preferable. When the host is E. coli, examples of a preferable medium include LB medium and M9 medium (Miller et al., Exp. Mol. Genet, Cold Spring Harbor Laboratory; 1972:431.). In such a case, the culture can be usually carried out at 14 to 43°C for about 3 to 24 hours, if necessary under aeration and stirring. When the host is a bacterium of the genus Bacillus, the culture can be carried out at 30 to 40°C for about 16 to 96 hours, if necessary under aeration and stirring. When the host is a yeast, examples of the medium include Burkholder minimal medium (PNAS;1980;77:4505-4508.), and the pH thereof is desirably 5 to 8. The culture is usually carried out at about 20 to 35°C for 14 to 144 hours, and if necessary, aeration and stirring can also be carried out.

The production of the anti-human CEACAM5 antibody Fab fragment included in the conjugate of the present invention may include, in addition to the step of culturing the transformed host cell described above to express the anti-human CEACAM5 antibody Fab fragment, the step of recovering, and preferably isolating and purifying the anti-human CEACAM5 antibody Fab fragment expressed. Examples of the isolation and purification methods include a method using solubility such as salting out or a solvent precipitation method, a method using difference in molecular weight such as dialysis, ultrafiltration, gel filtration, or sodium dodecyl sulfate-polyacrylamide gel electrophoresis, a method using charge such as ion exchange chromatography or hydroxylapatite chromatography, a method using specific affinity such as affinity chromatography, a method using difference in hydrophobicity such as reverse phase high performance liquid chromatography, and a method using difference in isoelectric point such as isoelectric focusing.

### 6. Method for producing the conjugate of the present invention

The method for producing the conjugate of the present invention can include the step of covalently binding the anti-human CEACAM5 antibody Fab fragment to a labeling portion (Y-S₁-X). Those skilled in the art can appropriately carry out the binding between the components in the labeling portion (Y-S₁-X) by a known method. As a reaction example, after a ligand (Y) is bound to a peptide linker (X) directly or via a spacer (S₁), the peptide linker can be bound to the anti-human CEACAM5 antibody Fab fragment. In addition, after the anti-human CEACAM5 antibody Fab fragment is bound to a peptide linker (X), the peptide linker can also be bound to a ligand (Y) directly or via a spacer (S₁). As a starting material, a compound in which a ligand and a spacer (S₁) are bound in advance can also be used.

The method for producing the conjugate of the present invention may also include the step of culturing the transformed host cell described above to express the anti-human CEACAM5 antibody Fab fragment, and the step of covalently binding the Fab fragment and a labeling portion (Y-S₁-X). The method for producing the conjugate of the present invention may also include the step of culturing the transformed host cell described above to express the anti-human CEACAM5 antibody Fab fragment, the step of recovering the Fab fragment expressed, and the step of covalently binding the Fab fragment and a labeling portion (Y-S₁-X). The method for producing the conjugate of the present invention may further include the step of adding a metal. As the chelating agent, the peptide linker, the spacer, the number of labeling portions, the metal, and the like used, those described herein can be used.

The method for producing the conjugate of the present invention can be carried out as a method including two or more steps specified above as a series of steps, or can be carried out as a method including at least one step specified above. For example, a method including the step of binding the anti-human CEACAM5 antibody Fab fragment to a labeling portion (Y-S₁-X) and a method including the step of coordinating a metal to the anti-human CEACAM5 antibody Fab fragment to which a labeling portion (Y-S₁-X) is bound are also included in the method of producing the conjugate of the present invention. In addition, the method for producing the conjugate of the present invention also includes a method in which the order of steps is different. For example, a method for covalently binding the anti-human CEACAM5 antibody Fab fragment to a labeling portion (Y-S₁-X) in which a metal is coordinated to a ligand is also included in the method for producing the conjugate of the present invention.
In addition, the conjugate of the present invention can be produced in the same manner, also with the biomolecule described in the present invention instead of the anti-human CEACAM5 antibody Fab fragment described above.

### 7. Conjugate consisting of a ligand, a spacer, a peptide linker, and a biomolecule

The conjugate consisting of a ligand, a spacer, a peptide linker, and a biomolecule according to the present invention represented by the following formula will be described.

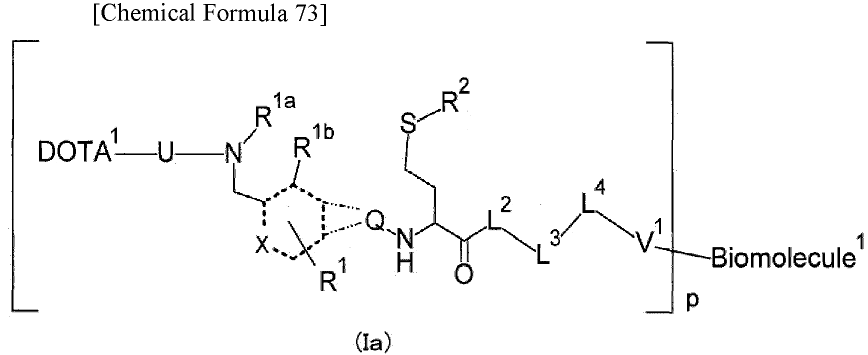

An embodiment of
the following formula (S2) corresponding to a spacer 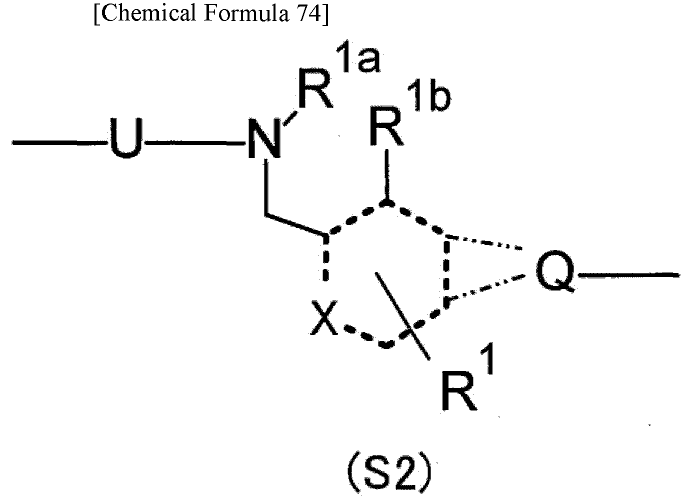 is

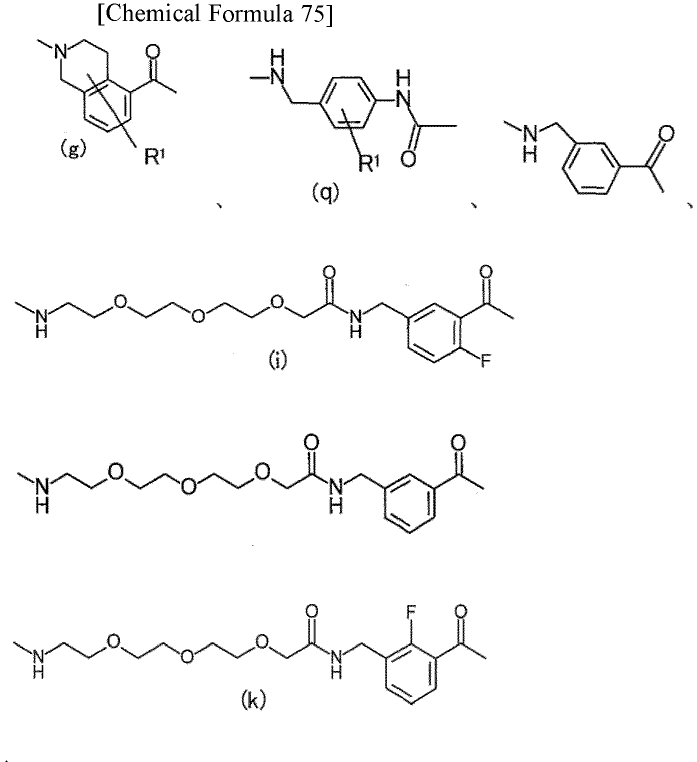

Another embodiment
is a group wherein formula (S2) is

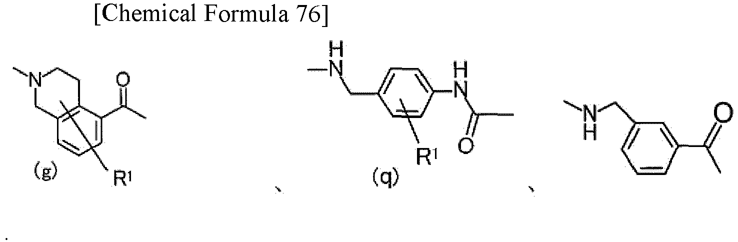

Another embodiment
is a group wherein formula (S2) is

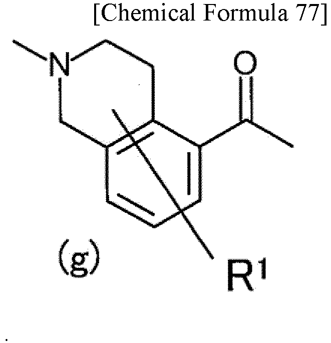

Another embodiment
is a group wherein formula (S2) is

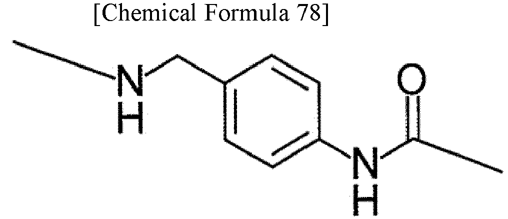

An embodiment of group Q in formula (S2) is -C(=O)-, -NH-C(=O)-, or -NH-C(=S)-.

An embodiment of group Q in formula (S2) is -C(=O)- or -NH-C(=O)-.

An embodiment of group Q in formula (S2) is -NH-C(=O)-.

An embodiment of group L² in formula (Ia) or (Ib) is Ile, Phe, or Gly.

An embodiment of group L² in formula (Ia) or (Ib) is Ile.

An embodiment of group L² in formula (Ia) or (Ib) is Phe.

An embodiment of group L² in formula (Ia) or (Ib) is Gly.

An embodiment of group L³ in formula (Ia) or (Ib) is a bond, Arg, or His.

An embodiment of group L³ in formula (Ia) or (Ib) is a bond.

An embodiment of group L³ in formula (Ia) or (Ib) is Arg.

An embodiment of group L³ in formula (Ia) or (Ib) is His.

An embodiment of group L⁴ in formula (Ia) or (Ib) is -NH-(CH₂)₂-, - NHCH(C(=O)OH)(CH₂)₄-, or a bond.

An embodiment of group L⁴ in formula (Ia) or (Ib) is -NH-(CH₂)₂-.

An embodiment of group L⁴ in formula (Ia) or (Ib) is -NHCH(C(=O)OH)(CH₂)₄-.

An embodiment of group L⁴ in formula (Ia) or (Ib) is a bond.

An embodiment of group V¹ in formula (Ia) or (Ib) is a group represented by any of the following formulas (A-1) to (A-5)

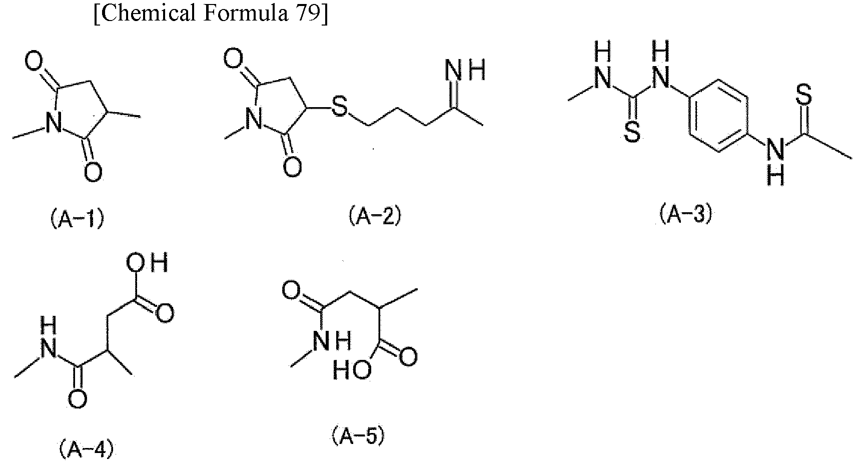

An embodiment of group V¹ in formula (Ia) or (Ib) is
a group represented by the following formula (A-1)

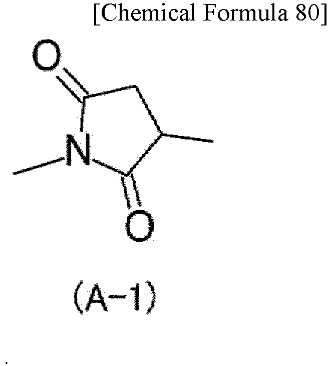

An embodiment of group V¹ in formula (Ia) or (Ib) is
a group represented by the following formula (A-2)

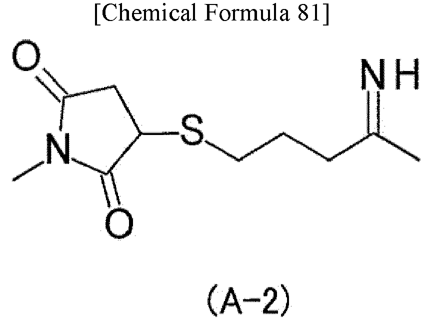

An embodiment of group V¹ in formula (Ia) or (Ib) is
a group represented by the following formula (A-3)

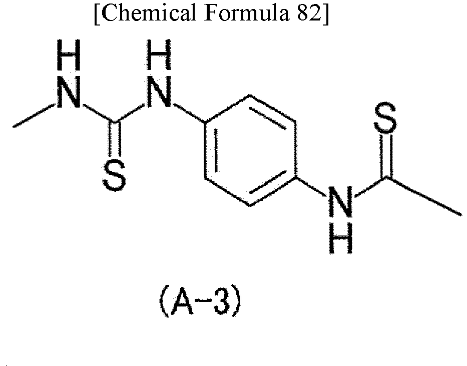

An embodiment of group V¹ in formula (Ia) or (Ib) is
a group represented by the following formula (A-4)

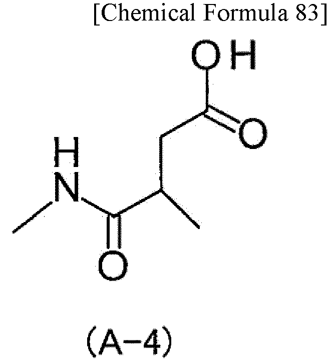

An embodiment of group V¹ in formula (Ia) or (Ib) is
a group represented by the following formula (A-5)

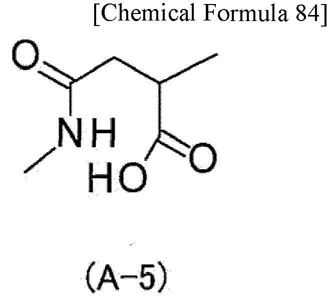

The following formula (B) in formula (Ia) or (Ib) is a group consisting of a spacer and a peptide linker.

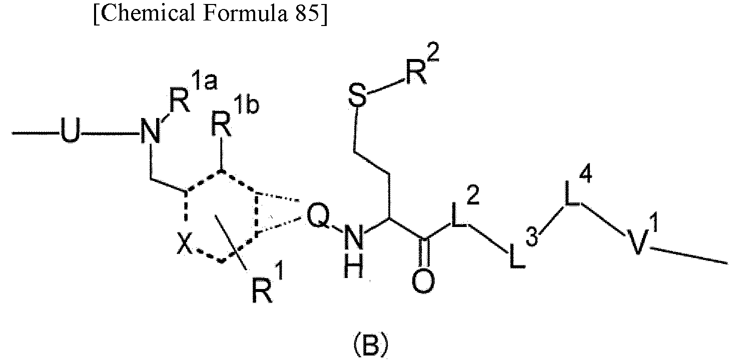

Examples thereof include a group selected from the group consisting of the following formulas (B-1) to (B-7).

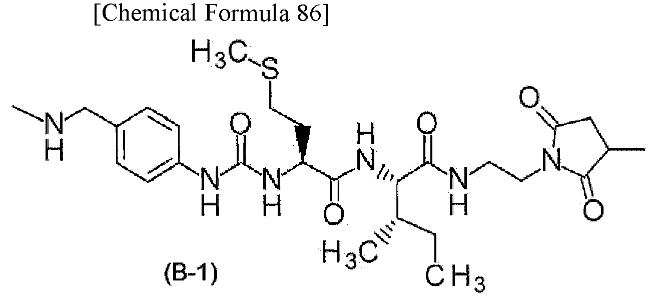

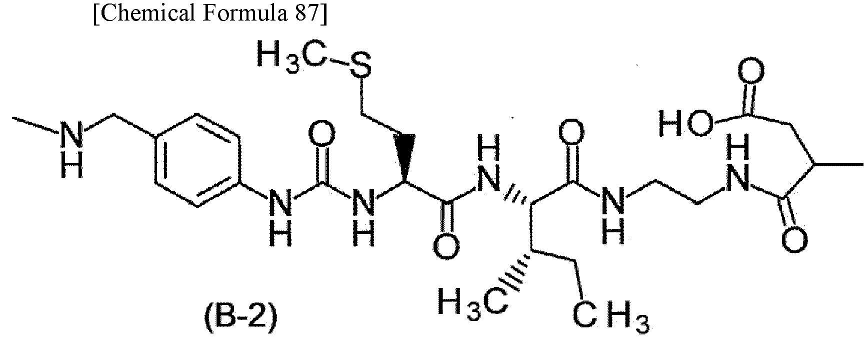

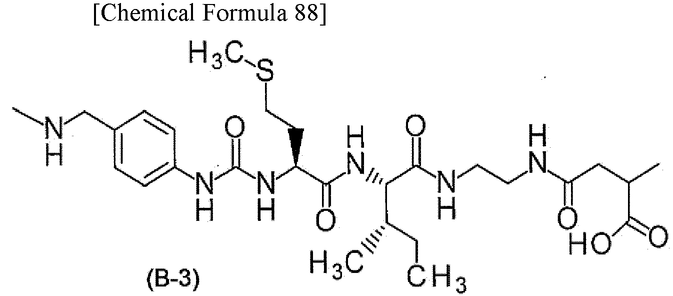

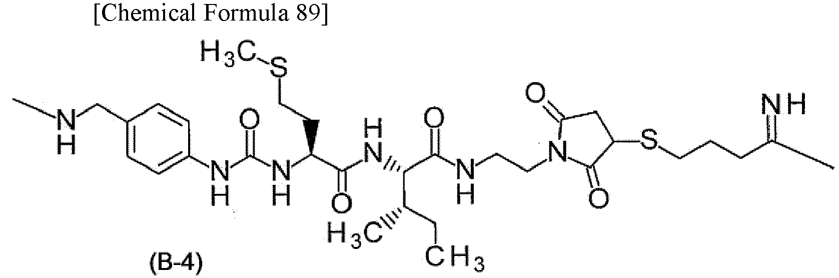

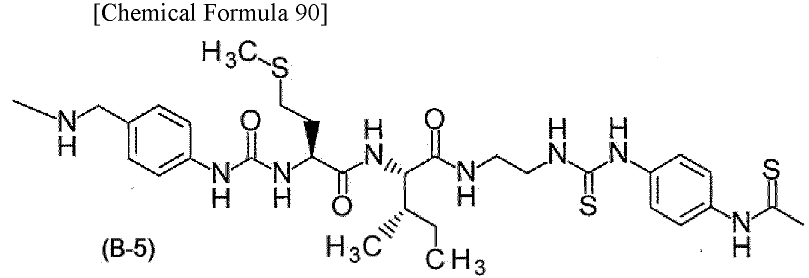

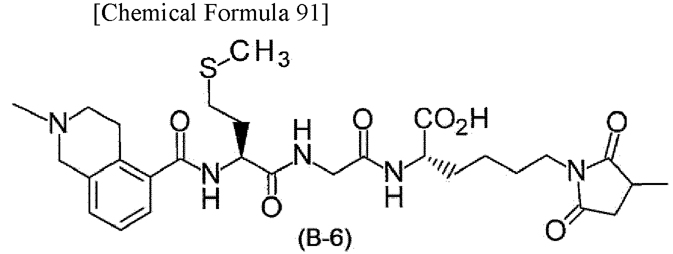

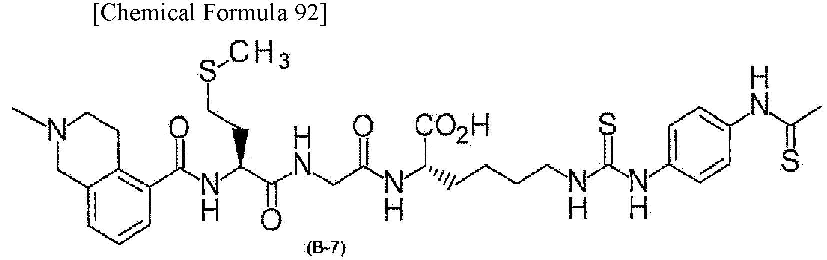

An embodiment is the conjugate represented by formula (Ia) wherein the conjugate is a conjugate selected from the group consisting of the compounds of the following formulas, p is a natural number of 1 to 25, and Biomolecule¹ is bound to an adjacent carbon atom via p amino groups or thiol groups in Biomolecule¹.

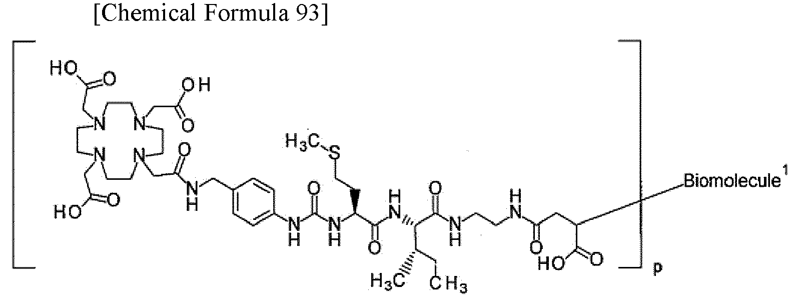

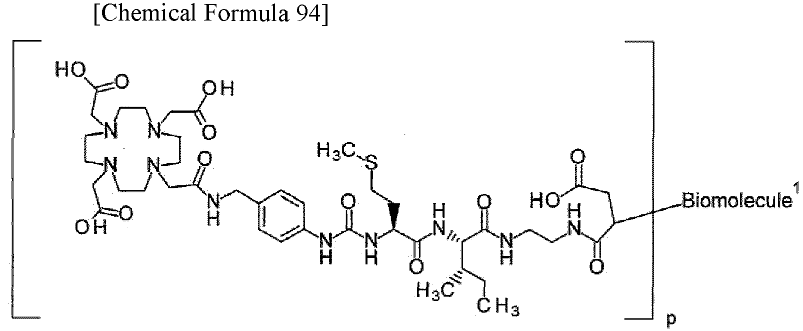

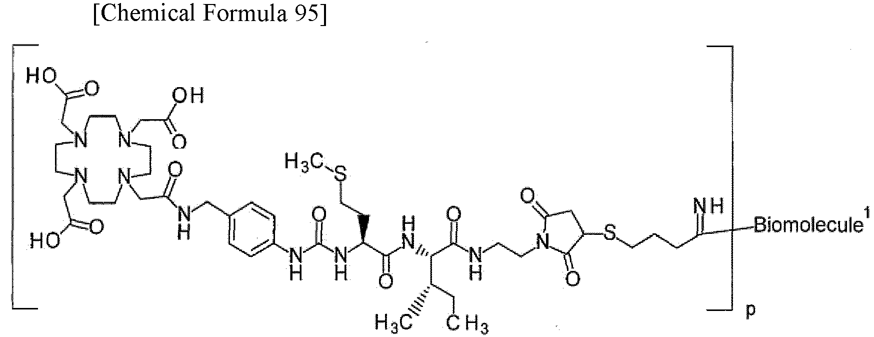

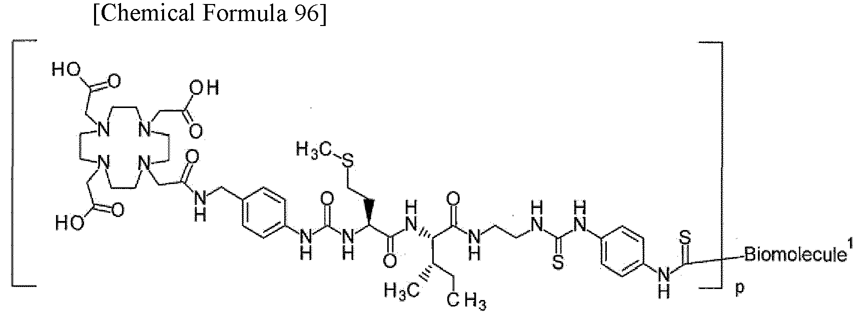

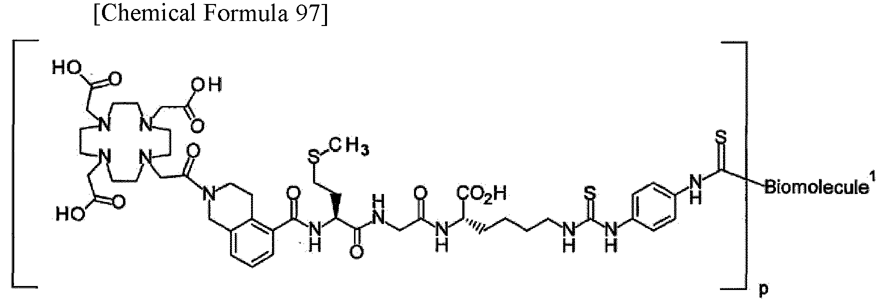

An embodiment of the present invention is a conjugate represented by the following formula.

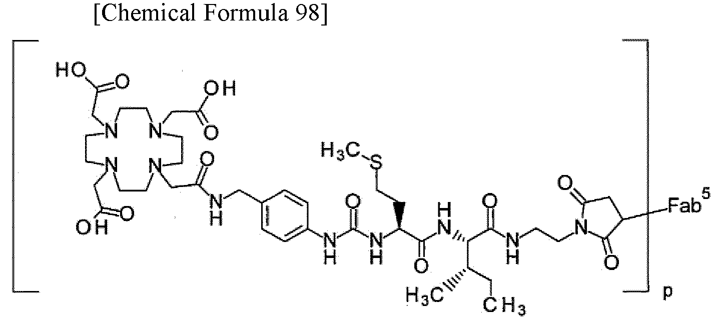

wherein Fab⁵ is the following antibody Fab fragment:
an anti-human MUC1 antibody Fab fragment comprising a heavy chain fragment which consists of the amino acid sequence shown in SEQ ID NO: 8 and in which glutamine of amino acid 1 of SEQ ID NO: 8 is modified to pyroglutamic acid, and a light chain consisting of the amino acid sequence shown in SEQ ID NO: 10.

An embodiment of the present invention is a conjugate represented by the following formula.

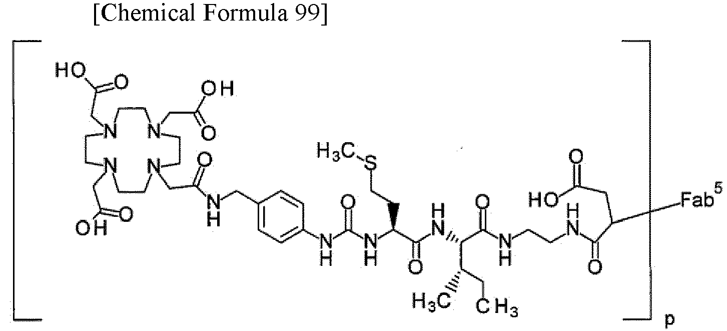

wherein Fab⁵ is the following antibody Fab fragment:
an anti-human MUC1 antibody Fab fragment comprising a heavy chain fragment which consists of the amino acid sequence shown in SEQ ID NO: 8 and in which glutamine of amino acid 1 of SEQ ID NO: 8 is modified to pyroglutamic acid, and a light chain consisting of the amino acid sequence shown in SEQ ID NO: 10.

An embodiment of the present invention is a conjugate represented by the following formula. 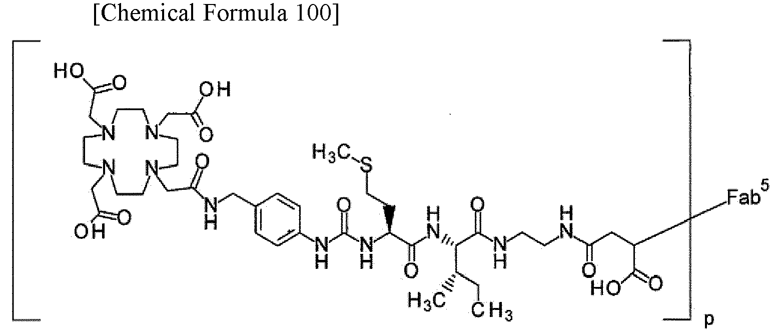 wherein Fab⁵ is the following antibody Fab fragment:
an anti-human MUC1 antibody Fab fragment comprising a heavy chain fragment which consists of the amino acid sequence shown in SEQ ID NO: 8 and in which glutamine of amino acid 1 of SEQ ID NO: 8 is modified to pyroglutamic acid, and a light chain consisting of the amino acid sequence shown in SEQ ID NO: 10.

An embodiment of the present invention is a conjugate represented by the following formula. 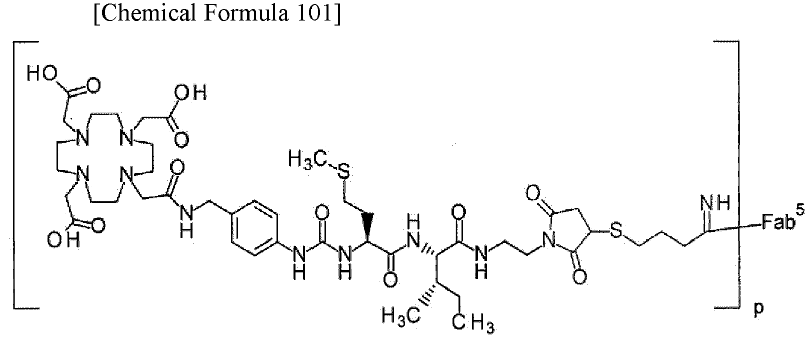 wherein Fab⁵ is the following antibody Fab fragment:
an anti-human MUC1 antibody Fab fragment comprising a heavy chain fragment which consists of the amino acid sequence shown in SEQ ID NO: 8 and in which glutamine of amino acid 1 of SEQ ID NO: 8 is modified to pyroglutamic acid, and a light chain consisting of the amino acid sequence shown in SEQ ID NO: 10.

An embodiment of the present invention is a conjugate represented by the following formula.

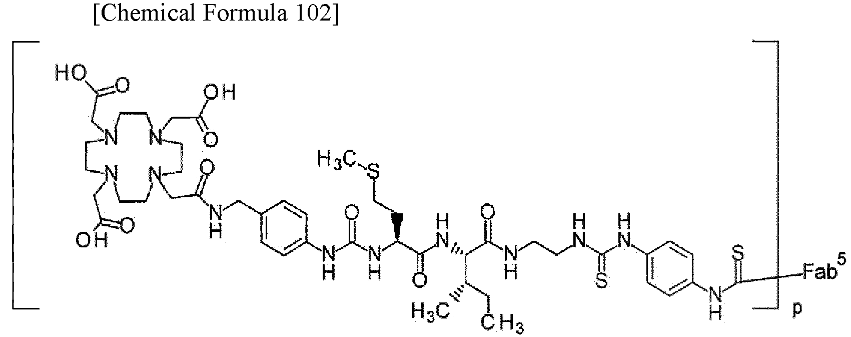

wherein Fab⁵ is the following antibody Fab fragment:
an anti-human MUC1 antibody Fab fragment comprising a heavy chain fragment which consists of the amino acid sequence shown in SEQ ID NO: 8 and in which glutamine of amino acid 1 of SEQ ID NO: 8 is modified to pyroglutamic acid, and a light chain consisting of the amino acid sequence shown in SEQ ID NO: 10.

An embodiment of the present invention is a conjugate represented by the following formula. 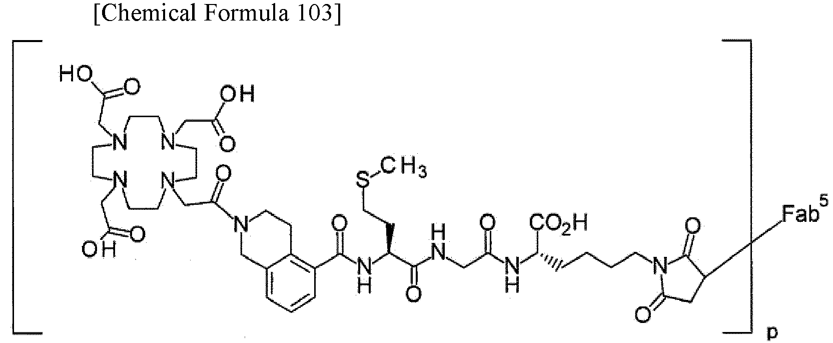 wherein Fab⁵ is the following antibody Fab fragment:
an anti-human MUC1 antibody Fab fragment comprising a heavy chain fragment which consists of the amino acid sequence shown in SEQ ID NO: 8 and in which glutamine of amino acid 1 of SEQ ID NO: 8 is modified to pyroglutamic acid, and a light chain consisting of the amino acid sequence shown in SEQ ID NO: 10.

An embodiment of the present invention is a conjugate represented by the following formula. 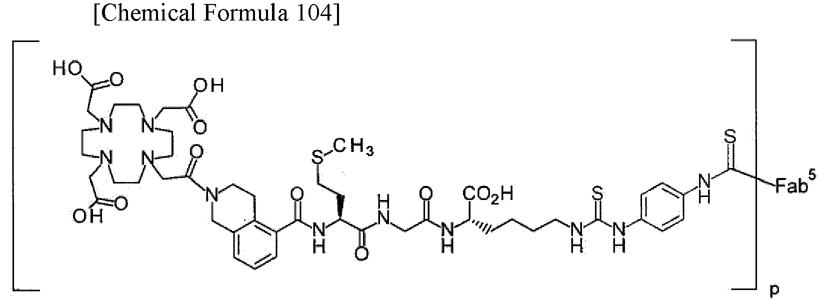 wherein Fab⁵ is the following antibody Fab fragment:
an anti-human MUC1 antibody Fab fragment comprising a heavy chain fragment which consists of the amino acid sequence shown in SEQ ID NO: 8 and in which glutamine of amino acid 1 of SEQ ID NO: 8 is modified to pyroglutamic acid, and a light chain consisting of the amino acid sequence shown in SEQ ID NO: 10.
In addition, examples of an embodiment of the present invention include the following formulas (Ie) and (If).

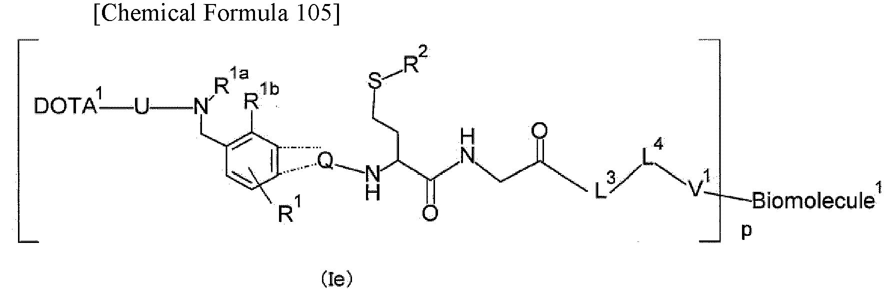

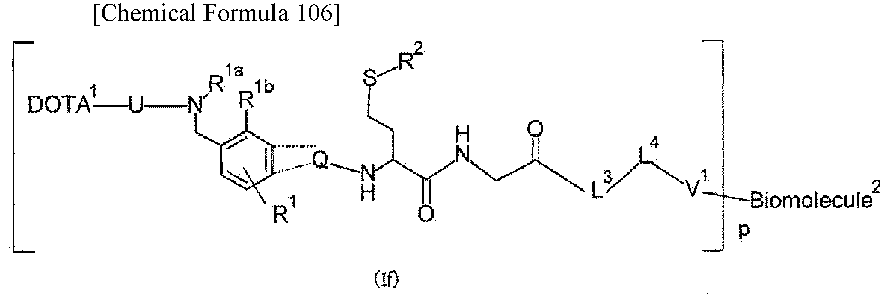

The conjugate of the present invention may have a geometric isomer and a tautomer. In addition, the compound of the present invention may have an asymmetric carbon. Separated versions of these isomers or mixtures thereof are included in the present invention. In addition, a labeled form, that is, a compound in which one or more atoms of the compound of the present invention are replaced with a radioisotope or a non-radioactive isotope is also included in the present invention.

The embodiment of the biomolecule may be any biomolecule as long as it has physiological activity, and examples thereof include a peptide, a protein, a hormone, a drug, a nucleotide, an oligonucleotide, a nucleic acid, and a polysaccharide. In the case of a protein, an antibody, an enzyme, a receptor, and a fragment thereof are included.

Another embodiment of the biomolecule is a peptide and a protein.

Another embodiment of the biomolecule is a protein.

Another embodiment of the biomolecule is a Fab fragment, an enzyme, a receptor, or a fragment thereof of proteins. For example, somatostatin, a PSMA ligand, an RGD (Arg-Gly-Asp) Peptide, ATSM (Diacetyl-bis(N4-methylthiosemicarbazone)), or ²¹¹At-AITM (4-²¹¹At-astato-N-[4-(6-(isopropylamino)pyridine-4-yl)-1,3-thiazol-2-yl]-N-methylbenzamide) corresponds to the above.

Another embodiment of the biomolecule is a marketed antibody or Fab fragment thereof.

Examples thereof include nivolumab, pembrolizumab, bevacizumab, rituximab, pertuzumab, daratumumab, denosumab, cetuximab, ipilimumab, panitumumab, brentuximab, ramucirumab, atezolizumab, obinutuzumab, elotuzumab, avelumab, ibritumomab, alemtuzumab, gemtuzumab, and necitumumab.

In addition, another embodiment of the biomolecule is one that can be expected for alpha particle therapy or beta particle therapy.

Examples thereof include Octreoscan, ¹³¹I-MIBG (I-131 metaiodobenzylguanidine), ²¹¹ At-MABG (²¹¹ At-astato-benzylguanidine), Trastuzumab , Humanized antibody A33 (Cancer Biotherapy & Radiopharmaceuticals 2005 Oct; 20(5): 514-23.), Omburtamab, Tenatumomab, CD45 antibody (ClinicalTrials. gov Identifier: NCT03128034; 9595; NCI-2017-00452), Ibritumomab, and Actimab.

In addition, another embodiment of the biomolecule is HuM195, MX35-F(ab')₂ monoclonal antibodies, Murine 9.2.27, Proteoglycan (MCSP) antigen, CD20 antigen, CD30 antigen, or the like disclosed in Cancer studies and molecular medicine (Cancer studies and molecular medicine (2004), 1, 1, 1-7).

In addition, another embodiment of the biomolecule is CD19, GD2, VE cadherin, or the like.

An embodiment of the biomolecule is an MUC1 antibody Fab fragment (referred to as Fab³, Fab⁴, or Fab⁵), and examples include what is disclosed in International Publication WO2018/092885.
[1M] Specifically, the Fab³ or Fab⁴ is an anti-human MUC1 antibody Fab fragment selected from the group consisting of the following (a) and (b), and a Fab fragment having a heavy chain fragment including a heavy chain variable region consisting of the amino acid sequence shown in SEQ ID NO: 12 is referred to as Fab³, and a Fab fragment having a heavy chain fragment including a heavy chain variable region consisting of the amino acid sequence shown in SEQ ID NO: 14 is referred to as Fab⁴:
   (a) an anti-human MUC1 antibody Fab fragment comprising a heavy chain fragment including a heavy chain variable region consisting of the amino acid sequence shown in SEQ ID NO: 12 or SEQ ID NO: 14 and a light chain including a light chain variable region consisting of the amino acid sequence shown in SEQ ID NO: 16; and
   (b) an anti-human MUC1 antibody Fab fragment comprising a heavy chain fragment including a heavy chain variable region which consists of the amino acid sequence shown in SEQ ID NO: 12 or SEQ ID NO: 14 and in which glutamine of amino acid 1 of SEQ ID NO: 12 or SEQ ID NO: 14 is modified to pyroglutamic acid, and a light chain including a light chain variable region consisting of the amino acid sequence shown in SEQ ID NO: 16.
[2M] An embodiment is the anti-human MUC1 antibody Fab fragment according to [1M], which is selected from the group consisting of the following (a) and (b):
   (a) an anti-human MUC1 antibody Fab fragment comprising a heavy chain fragment consisting of the amino acid sequence shown in SEQ ID NO: 6 or SEQ ID NO: 8 and a light chain consisting of the amino acid sequence shown in SEQ ID NO: 10; and
   (b) an anti-human MUC1 antibody Fab fragment comprising a heavy chain fragment which consists of the amino acid sequence shown in SEQ ID NO: 6 or SEQ ID NO: 8 and in which glutamine of amino acid 1 of SEQ ID NO: 6 or SEQ ID NO: 8 is modified to pyroglutamic acid, and a light chain consisting of the amino acid sequence shown in SEQ ID NO: 10.
[3M] An embodiment is the anti-human MUC1 antibody Fab fragment according to [1M], which is selected from the group consisting of the following (a) and (b):
   (a) an anti-human MUC1 antibody Fab fragment comprising a heavy chain fragment including a heavy chain variable region consisting of the amino acid sequence shown in SEQ ID NO: 14 and a light chain including a light chain variable region consisting of the amino acid sequence shown in SEQ ID NO: 16; and
   (b) an anti-human MUC1 antibody Fab fragment comprising a heavy chain fragment including a heavy chain variable region which consists of the amino acid sequence shown in SEQ ID NO: 14 and in which glutamine of amino acid 1 of SEQ ID NO: 10 is modified to pyroglutamic acid, and a light chain including a light chain variable region consisting of the amino acid sequence shown in SEQ ID NO: 16.
[4M] An embodiment is the anti-human MUC1 antibody Fab fragment according to [3M], which is selected from the group consisting of the following (a) and (b):
   (a) an anti-human MUC1 antibody Fab fragment comprising a heavy chain fragment consisting of the amino acid sequence shown in SEQ ID NO: 8 and a light chain consisting of the amino acid sequence shown in SEQ ID NO: 10; and
   (b) an anti-human MUC1 antibody Fab fragment comprising a heavy chain fragment which consists of the amino acid sequence shown in SEQ ID NO: 8 and in which glutamine of amino acid 1 of SEQ ID NO: 8 is modified to pyroglutamic acid, and a light chain consisting of the amino acid sequence shown in SEQ ID NO: 10.
[5M] An embodiment is the anti-human MUC1 antibody Fab fragment according to [4M], which is an anti-human MUC1 antibody Fab fragment comprising a heavy chain fragment consisting of the amino acid sequence shown in SEQ ID NO: 6 and a light chain consisting of the amino acid sequence shown in SEQ ID NO: 10.
[6M] An embodiment is the anti-human MUC1 antibody Fab fragment according to [4M], which is an anti-human MUC1 antibody Fab fragment comprising a heavy chain fragment which consists of the amino acid sequence shown in SEQ ID NO: 8 and in which glutamine of amino acid 1 of SEQ ID NO: 8 is modified to pyroglutamic acid, and a light chain consisting of the amino acid sequence shown in SEQ ID NO: 10.
[7M] P10-1 or P10-2 (referred to as Fab⁵), which is the anti-MUCl antibody Fab fragment disclosed in International Publication WO2018/092885.
   In addition, the conjugate of the present invention can be composed of a combination of the individual embodiments described above.

### 7. Diagnostic composition and method for diagnosis

The present invention relates to a diagnostic composition comprising a conjugate of the present invention including a metal (hereinafter, referred to as a detectable conjugate of the present invention). The diagnostic composition of the present invention may include one or more conjugates of the present invention. That is, the diagnostic composition of the present invention may include one conjugate of the present invention, or may include a combination of two or more conjugates of the present invention. The detectable conjugate of the present invention can be formulated according to a conventional method and used as an early diagnostic drug or a staging drug (particularly a diagnostic drug for a cancer).

The early diagnostic drug means a diagnostic drug whose purpose is to make a diagnosis at a stage where no disease condition is observed or at an early disease stage. For example, in the case of a cancer, the early diagnostic drug means a diagnostic drug used at a stage where no disease condition is observed or at stage 0 or stage 1.

The staging drug means a diagnostic drug which can examine how far the disease condition has progressed. For example, in the case of a cancer, the staging drug means a diagnostic drug which can examine the stage thereof.

The cancer expected to be able to be diagnosed by the diagnostic composition of the present invention is a cancer expressing human CEACAM5. In an embodiment, examples thereof include colorectal cancer, breast cancer, lung cancer, thyroid cancer, and a cancer resulting from metastasis thereof. In an embodiment, the cancer is colorectal cancer or a cancer resulting from metastasis of colorectal cancer. More preferably, the cancer is a cancer resulting from metastasis of colorectal cancer, and such a cancer includes metastatic liver cancer. In addition, the cancer is a cancer expressing human MUC1. In an embodiment, examples of the cancer include breast cancer, lung cancer, colorectal cancer, bladder cancer, skin cancer, thyroid cancer, gastric cancer, pancreatic cancer, kidney cancer, ovarian cancer, or cervical cancer. In an embodiment, the cancer is breast cancer or bladder cancer.

The amount of the conjugate of the present invention added in the formulation of the diagnostic composition of the present invention varies depending on the degree of a symptom and the age of the patient, the dosage form of the formulation used, the binding potency of the Fab fragment or the biomolecule, and the like, and, for example, about 0.001 mg/kg to 100 mg/kg may be used based on the mass of the Fab fragment or biomolecule per unit body weight of the patient.

Examples of the dosage form of the diagnostic composition of the present invention include a parenteral preparation such as an injection or an infusion, and can be administered by intravenous injection, intramuscular injection to a local target tissue, subcutaneous injection, intravesical administration, or the like. In addition, in the formulation, a carrier and an additive according to these dosage forms can be used within a pharmaceutically acceptable range. The types of a pharmaceutically acceptable carrier and additive are not particularly limited, and a carrier and an additive well known to those skilled in the art can be used.

The present invention also relates to use of a detectable conjugate of the present invention for the production of a composition for early diagnosis or a composition for staging of a cancer. The present invention also relates to a detectable conjugate of the present invention for use in early diagnosis and staging of a cancer.

Further, the present invention also relates to a method for diagnosing a cancer, comprising administering a detectable conjugate of the present invention to a subject. Here, the "subject" refers to a human or another mammal animal that needs to be diagnosed. In an embodiment, the subject is a human who needs to be diagnosed. The effective amount of the detectable conjugate of the present invention in the method for diagnosis of the present invention may be the same amount as the effective amount of the conjugate of the present invention in the above formulation. In the method for diagnosis of the present invention, the detectable conjugate of the present invention can be administered by intramuscular injection to a local target tissue, subcutaneous injection, or the like.

In another embodiment, the present invention also relates to use of an anti-human CEACAM5 antibody Fab fragment for the production of a conjugate including the anti-human CEACAM5 antibody Fab fragment of the present invention, a peptide linker, and/or a ligand. In an embodiment, the present invention also relates to use of an anti-human CEACAM5 antibody Fab fragment for the production of a diagnostic composition including the conjugate of the present invention.

In addition, in an embodiment when the diagnostic composition of the present invention including a metal radioisotope is provided, it may be labeled with the metal radioisotope immediately before the use of the composition, and may be provided as a diagnostic composition including the metal radioisotope.

### 8. Pharmaceutical composition and method for treatment

In the present invention, about 0.001 mg/kg to 100 mg/kg can be used based on the mass of one or more conjugate Fab fragments of the present invention including a metal radioisotope such as ⁹⁰Y or ¹⁷⁷Lu.

A pharmaceutical composition including the conjugate of the present invention can be used for the treatment of a cancer. A cancer that is expected to be able to be treated with a pharmaceutical composition including the conjugate of the present invention is a cancer expressing human CEACAM5, and examples thereof include colorectal cancer, breast cancer, lung cancer, thyroid cancer, and a cancer resulting from metastasis thereof. Alternatively, a cancer that is expected to be able to be treated with a pharmaceutical composition including the conjugate of the present invention is a cancer expressing human MUC1, and examples thereof include breast cancer, lung cancer, colorectal cancer, bladder cancer, skin cancer, thyroid cancer, gastric cancer, pancreatic cancer, kidney cancer, ovarian cancer, or cervical cancer. In an embodiment, the cancer is breast cancer or bladder cancer.

The present invention includes a pharmaceutical composition including the conjugate of the present invention for treating colorectal cancer or a cancer resulting from metastasis of colorectal cancer. In addition, the present invention includes a method for treating colorectal cancer or a cancer resulting from metastasis of colorectal cancer, comprising a step of administering a therapeutically effective amount of the conjugate of the present invention. In addition, the present invention includes a method for inducing cell death of a cancer cell of colorectal cancer or a cancer resulting from metastasis of colorectal cancer, comprising a step of administering a therapeutically effective amount of the conjugate of the present invention.

The pharmaceutical composition for treating a cancer can also be used in the diagnosis of a cancer. For example, the pharmaceutical composition for treating colorectal cancer or a cancer resulting from metastasis of colorectal cancer can also be used for the diagnosis of the cancer.

In addition, the present invention includes a conjugate of the present invention for use in the treatment of colorectal cancer or a cancer resulting from metastasis of colorectal cancer. Further, the present invention includes use of a conjugate of the present invention for the production of a pharmaceutical composition for treating colorectal cancer or a cancer resulting from metastasis of colorectal cancer.

In another embodiment, the present invention also relates to use of an anti-human CEACAM5 antibody Fab fragment for the production of a pharmaceutical composition including the conjugate of the present invention.

In the above embodiments, a biomolecule can be used instead of an anti-human CEACAM5 antibody Fab fragment. The conjugate of the present invention can be provided as a diagnostic composition or a pharmaceutical composition for a disease associated with a biomolecule.

The present invention has been described in general, and specific Examples referred to for obtaining further understanding will be provided here. However, these are for purposes of illustration and are not intended to limit the present invention.

### EXAMPLES

The following abbreviations may be used in the following Examples and in the tables given later.

Gd/DOTA: 3arm DOTA labeled with Gd, Gd/4arm DOTA: 4arm DOTA labeled with Gd, MS: mass spectrometry, ESI+: m/z value (ionization method ESI, unless otherwise specified [M+H]+), ESI-: m/z value (ionization method ESI, unless otherwise specified [M-H]-), APCI/ESI+: m/z value (ionization method APCI/ESI, APCI/ESI means simultaneous measurement of APCI and ESI. Unless otherwise specified [M+H]+), APCI/ESI-: m/z value (ionization method APCI/ESI, unless otherwise specified [M+H]-), Ex-No: conjugate number, SNo: Production Example number, Str: chemical structural formula, Me: methyl, Et: ethyl, tBu: tert-butyl, 1,3-Ph: 1,3-phenylene, 1,4-Ph: 1,4-phenylene, diph-Ala: diphenylalanine, and naph-Ala: 3-(2-naphthyl)alanine.

### (Example 1: Preparation of anti-human CEACAM5 antibody Fab fragment)

T84.66, which is an anti-human CEACAM5 antibody derived from a mouse, was humanized with reference to the method disclosed in the literature (Protein Eng. Des. Sel.;2004;17:481-489), and then a molecular model of the humanized antibody constructed according to the literature (Proteins: Structure, Function, and Bioinformatics;2014;82:1624-1635) was used to design an antibody having a variable region where the affinity was expected not to be attenuated even by binding of a labeling portion.

A heavy chain fragment gene was formed by connecting a gene encoding a signal sequence (Protein Engineering;1987;1:499-505) to the 5' side of the heavy chain variable region gene of the antibody, and a human Igγ1 Fab region gene (consisting of the nucleotide sequence of nucleotides 364 to 678 of SEQ ID NO: 1) to the 3' side, and this heavy chain fragment gene was inserted into the GS vector pEE6.4 (Lonza). In addition, a light chain gene was formed by connecting a gene encoding a signal sequence to the 5' side of the light chain variable region gene of the antibody, and a human IgK constant region gene (consisting of the nucleotide sequence of nucleotides 337 to 657 of SEQ ID NO: 3) to the 3' side, and this light chain gene was inserted into the GS vector pEE12.4 (Lonza). The above-described pEE vectors into which the heavy chain fragment gene and the light chain gene of the antibody, respectively, were inserted were subjected to restriction enzyme cleavage with NotI and PvuI, and ligation was carried out using the ligation kit TAKARA Ligation Kit Ver2.1 (Takara Bio Inc.) to construct a GS vector into which both the heavy chain fragment gene and the light chain gene were inserted.

Using the above-described GS vector into which both the heavy chain fragment gene and the light chain gene were inserted, antibody expression was carried out by two methods, transient expression and constitutive expression. For transient expression, Expi293F cells (Thermo Fisher Scientific Inc.) cultured to about 3 million cells/mL in Expi293 Expression Medium (Thermo Fisher Scientific Inc.) were transfected with the above-described GS vector into which both the heavy chain fragment gene and the light chain gene were inserted, using ExpiFectamine 293 Transfection Kit (Thermo Fisher Scientific Inc.), and cultured for 5 to 7 days. The culture supernatant was purified using KappaSelect (GE Healthcare Japan Corporation) to obtain a Fab fragment. For constitutive expression, CHOK1SV cells (Lonza) were transfected with the above-described GS vector into which both the heavy chain fragment gene and the light chain gene were inserted linearized with PvuI, by an electroporation method using Gene Pulser (Bio-Rad Laboratories, Inc.). The day after transfection, methionine sulfoximine was added and the cells were cultured for 5 to 7 days. The cells were seeded in a semi-solid medium containing methyl cellulose, and after colonization, cells having a high Fab fragment expression level were obtained using ClonePix FL (Molecular Devices, LLC). The culture supernatant of the cells was purified using Capto L (GE Healthcare Japan Corporation), Q Sepharose Fast Flow (GE Healthcare Japan Corporation), and BioPro S75 (YMC Co., Ltd.) to obtain a Fab fragment.

The nucleotide sequence encoding the heavy chain fragment of the prepared anti-human CEACAM5 antibody Fab fragment (referred to as PB009-01 or Fab²) is shown in SEQ ID NO: 1, and the amino acid sequence encoded thereby is shown in SEQ ID NO: 2, the nucleotide sequence encoding the light chain of PB009-01 is shown in SEQ ID NO: 3, and the amino acid sequence encoded thereby is shown in SEQ ID NO: 4. The heavy chain variable region of PB009-01 consists of the amino acid sequence of amino acids 1 to 121 of SEQ ID NO: 2, and CDR1, CDR2, and CDR3 of the heavy chain consist of the amino acid sequences of amino acids 31 to 35, 50 to 66, and 99 to 110, respectively, of SEQ ID NO: 2. The light chain variable region of PB009-01 consists of the amino acid sequence of amino acids 1 to 112 of SEQ ID NO: 4, and CDR1, CDR2, and CDR3 of the light chain consist of the amino acid sequences of amino acids 24 to 38, 54 to 60, and 93 to 101, respectively, of SEQ ID NO: 4.

The variable regions and CDR sequences were determined according to Kabat numbering (Kabat et al., 1991, Sequences of Proteins of Immunological Interest, 5th Ed., United States Public Health Service, National Institute of Health, Bethesda).

### (Example 2: Preparation of anti-human CEACAM5 antibody Fab fragment conjugate)

The present Example discloses Production Examples of the conjugate. In presentation of each Production Example in the present Example, "Example 2" is followed by one hyphen followed by a "conjugate number." For example, "Example 2-11" shows that it is the Production Example of conjugate 11 in the present Example. In addition, Fab² in the present Example is the anti-human CEACAM5 antibody Fab fragment (PB009-01/Fab²) obtained in Example 1, and "p-Fab" shows that Fab² is bound to p labeling portions enclosed by [ ] or ( ) via p amino groups thereof to form a conjugate. In some of the following Examples, the number of labeling portions (Y-S₁-X) bound to Fab² of each conjugate which has been confirmed by MS analysis is described, but the result does not show that a conjugate having a number of labeling portions bound other than the above number is not included. It will be easy to understand that there may still be a conjugate having a number of labeling portions bound whose presence has not been able to be confirmed because of the accuracy of the MS analysis equipment. In addition, in the structural formulas in the present Example, the structural formula of DOTA to which Gd is bound schematically shows DOTA labeled with Gd.

### (Example 2-11. Synthesis of ([Gd/DOTA-NH-CH₂-(1,3-Ph)-C(=O)-Asp-Gly-Lys^{∗}-Z₂(#N)]p-Fab²))

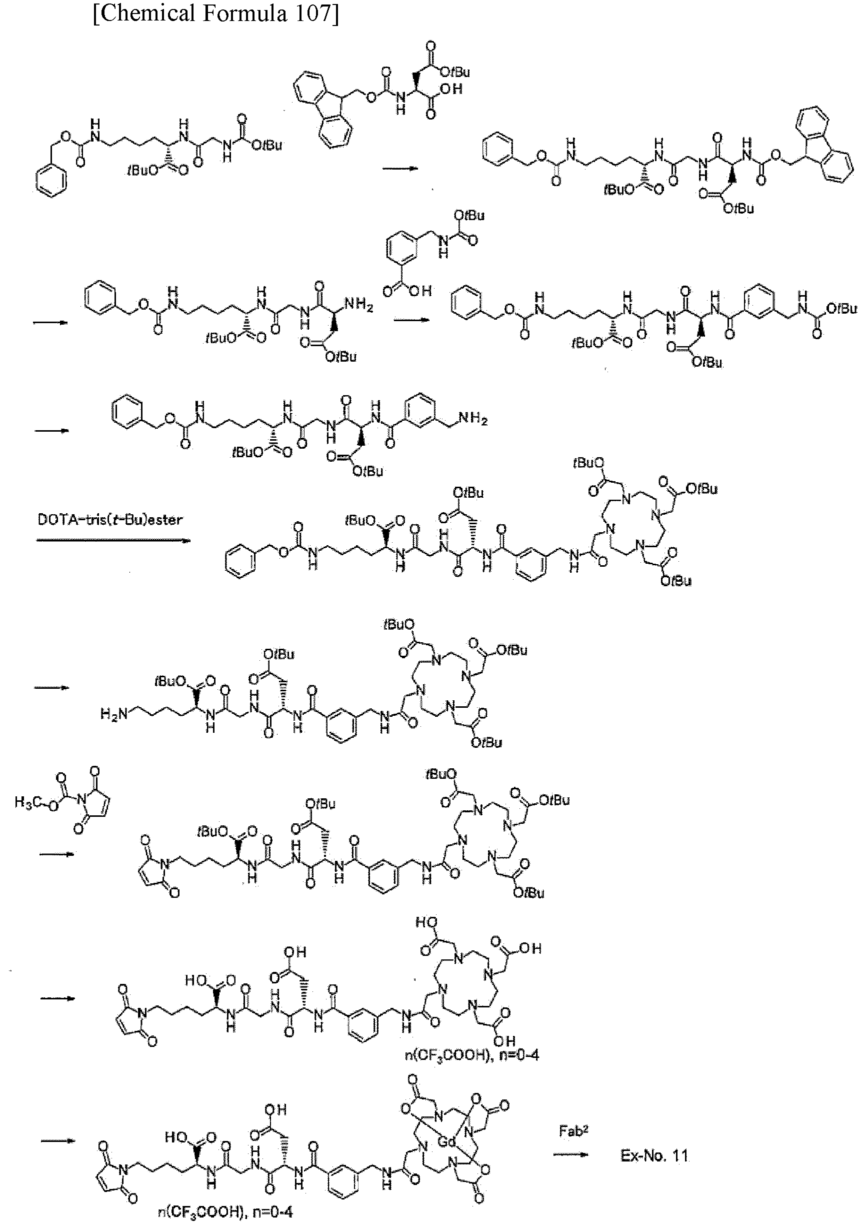

(i) Synthesis of tert-butyl O⁴-tert-butyl-N-[(9H-fluoren-9-ylmethoxy)carbonyl]-L-α-aspartylglycyl-N⁶-[(benzyloxy)carbonyl]-L-lysinate
   Trifluoroacetic acid (hereinafter, abbreviated as TFA) (3 mL) was added to a solution of tert-butyl N-(tert-butoxycarbonyl)glycyl-N⁶-[(benzyloxy)carbonyl]-L-lysinate (1.00 g) in dichloromethane (6 mL) under ice cooling, and the resulting mixture was stirred at the same temperature for 2 hours. The mixture was concentrated under reduced pressure, then a saturated sodium hydrogen carbonate aqueous solution was added, and the resulting mixture was extracted twice with dichloromethane. The combined organic layers were washed with a saturated sodium chloride aqueous solution, and then dried over anhydrous sodium sulfate, filtered, and then concentrated. To this residue, O⁴-tert-butyl hydrogen N-[(9H-fluoren-9-ylmethoxy)carbonyl]-L-aspartate (920 mg), dichloromethane (10 mL), diisopropylethylamine (hereinafter, abbreviated as DIPEA) (2 mL), 1-(Dimethylamino)-N,N-dimethyl-1-[(3H-[1,2,3]triazolo[4,5-b]pyridin-3-yl)oxy]methaniminium hexafluoridophosphate(1-) (hereinafter, abbreviated as HATU) (1.2 g) was added, and the resulting mixture was stirred at room temperature for 16 hours. A saturated sodium hydrogen carbonate aqueous solution was added, and the resulting mixture was extracted twice with dichloromethane. The combined organic layers were washed with a saturated sodium chloride aqueous solution, and then dried over anhydrous sodium sulfate, filtered, and then concentrated to obtain the title compound (2.86 g). MS (ESI+); 809.5 [M+Na]+
(ii) Synthesis of tert-butyl O⁴-tert-butyl-L-α-aspartylglycyl-N⁶-[(benzyloxy)carbonyl]-L-lysinate
   Morpholine (6 mL) was added to a solution of tert-butyl O⁴-tert-butyl-N-[(9H-fluoren-9-ylmethoxy)carbonyl]-L-α-aspartylglycyl-N⁶-[(benzyloxy)carbonyl]-L-lysinate (2.86 g) in tetrahydrofuran (hereinafter, abbreviated as THF) (10 mL), and the resulting mixture was stirred at room temperature for 1 hour. The mixture was cooled in an ice bath, then the resulting solid was filtered, and the residue was washed with methanol. The filtrate was concentrated under reduced pressure, and then the residue was purified by silica gel column chromatography (solvent gradient; 0 → 4% methanol/chloroform) to obtain the title compound (1.04 g). MS (ESI+); 565.5
(iii) Synthesis of tert-butyl N-(3-{[(tert-butoxycarbonyl)amino]methyl}benzoyl)-O⁴-tert-butyl-L-α-aspartylglycyl-N⁶-[(benzyloxy)carbonyl]-L-lysinate
   A mixture of tert-butyl O⁴-tert-butyl-L-α-aspartylglycyl-N⁶-[(benzyloxy)carbonyl]-L-lysinate (570 mg), dichloromethane (6 mL), 3-{[(tert-butoxycarbonyl)amino]methyl}benzoic acid (305 mg), DIPEA (520 µL), and HATU (575 mg) was stirred at room temperature for 43 hours. The reaction mixture was concentrated under reduced pressure, and then the residue was purified by silica gel column chromatography (solvent gradient; 0 → 80% ethyl acetate/hexane) to obtain the title compound (712 mg). MS (ESI+); 798.6
(iv) Synthesis of tert-butyl N-[3-(aminomethyl)benzoyl]-O⁴-tert-butyl-L-α-aspartylglycyl-N⁶-[(benzyloxy)carbonyl]-L-lysinate
   TFA (2 mL) was added to a solution of tert-butyl N-(3-{[(tert-butoxycarbonyl)amino]methyl}benzoyl)-O⁴-tert-butyl-L-α-aspartylglycyl-N⁶-[(benzyloxy)carbonyl]-L-lysinate (712 mg) in dichloromethane (2 mL) under ice cooling, and the resulting mixture was stirred at the same temperature for 7 hours. Triethylamine and amino silica gel were added, the resulting mixture was concentrated under reduced pressure, and then the residue was purified by silica gel column chromatography (amino silica gel, solvent gradient; 0 → 2% methanol/chloroform) to obtain the title compound (495 mg). MS (ESI+); 698.4
(v) Synthesis of tert-butyl O⁴-tert-butyl-N-[3-({2-[4,7,10-tris(2-tert-butoxy-2-oxoethyl)-1,4,7,10-tetraazacyclododecan-1-yl]acetamidolmethyl)benzoyl]-L-α-aspartylglycyl-N⁶-[(benzyloxy)carbonyl]-L-lysinate
   A mixture of tert-butyl N-[3-(aminomethyl)benzoyl]-O⁴-tert-butyl-L-α-aspartylglycyl-N⁶-[(benzyloxy)carbonyl]-L-lysinate (495 mg), N,N-dimethylformamide (hereinafter, abbreviated as DMF) (5 mL), [4,7,10-tris(2-tert-butoxy-2-oxoethyl)-1,4,7,10-tetraazacyclododecan-1-yl]acetic acid (hereinafter, abbreviated as DOTA-tris(t-Bu) ester) (446 mg), DIPEA (400 µL), and HATU (404 mg) was stirred at room temperature for 66 hours. The reaction mixture was concentrated under reduced pressure, and then the residue was purified by silica gel column chromatography (solvent gradient; 0 → 20% methanol/chloroform) to obtain the title compound (387 mg). MS (ESI+); 1275.5 [M+Na]+
(vi) Synthesis of tert-butyl O⁴-tert-butyl-N-[3-({2-[4,7,10-tris(2-tert-butoxy-2-oxoethyl)-1,4,7,10-tetraazacyclododecan-1-yl]acetamido}methyl)benzoyl]-L-**α-**aspartylglycyl-L-lysinate
   A mixture of tert-butyl O⁴-tert-butyl-N-[3-({2-[4,7,10-tris(2-tert-butoxy-2-oxoethyl)-1,4,7,10-tetraazacyclododecan-1-yl]acetamido}methyl)benzoyl]-L-**α-**aspartylglycyl-N⁶-[(benzyloxy)carbonyl]-L-lysinate (387 mg), 10% palladium on carbon (water content of 50%, 65 mg), and ethanol (4 mL) was stirred under a hydrogen atmosphere (1 atm) at room temperature for 5 hours. The mixture was filtered using Celite and concentrated. 10% palladium on carbon (50% wet with water, 650 mg) and ethanol (8 mL) were added to the residue, and the resulting mixture was stirred under a hydrogen atmosphere (1 atm) at room temperature for 20 hours. The mixture was filtered using Celite and then concentrated to obtain the title compound (336 mg). MS (ESI+); 1140.5 [M+Na]+
(vii) Synthesis of tert-butyl O⁴-tert-butyl-N-[3-({2-[4,7,10-tris(2-tert-butoxy-2-oxoethyl)-1,4,7,10-tetraazacyclododecan-1-yl]acetamido}methyl)benzoyl]-L-**α**-aspartylglycyl-6-(2,5-dioxo-2,5-dihydro-1H-pyrrol-1-yl)-L-norleucinate
   A solution of methyl 2,5-dioxo-2,5-dihydro-1H-pyrrole-1-carboxylate (56 mg) and THF (5 mL) was added to a mixture of tert-butyl O⁴-tert-butyl-N-[3-({2-[4,7,10-tris(2-tert-butoxy-2-oxoethyl)-1,4,7,10-tetraazacyclododecan-1-yl]acetamido}methyl)benzoyl]-L-**α-**aspartylglycyl-L-lysinate (336 mg) and a saturated sodium hydrogen carbonate aqueous solution (2.5 mL) under ice cooling, and the resulting mixture was stirred at the same temperature for 2 hours. AIM sodium hydroxide aqueous solution (60 µL) was added under ice cooling, and then the resulting mixture was stirred at room temperature for 1 hour. Ethyl acetate and a 10% citric acid aqueous solution were added, and then the organic layer was separated. The aqueous layer was extracted with 10% methanol/chloroform, and the collected organic layer was dried over anhydrous sodium sulfate and filtered. The filtrate was concentrated under reduced pressure, and then the residue was purified by silica gel column chromatography (solvent gradient; 0 → 20% methanol/chloroform) to obtain the title compound (341 mg). MS (ESI+); 1198.5
(viii) Synthesis of N-[3-({2-[4,7,10-tris(carboxymethyl)-1,4,7,10-tetraazacyclododecan-1-yl]acetamido}methyl)benzoyl]-L-**α**-aspartylglycyl-6-(2,5-dioxo-2,5-dihydro-1H-pyrrol-1-yl)-L-norleucine
   TFA (2 mL) was added to a solution of tert-butyl O⁴-tert-butyl-N-[3-({2-[4,7,10-tris(2-tert-butoxy-2-oxoethyl)-1,4,7,10-tetraazacyclododecan-1-yl]acetamido}methyl)benzoyl]-L-**α**-aspartylglycyl-6-(2,5-dioxo-2,5-dihydro-1H-pyrrol-1-yl)-L-norleucinate (341 mg) in dichloromethane (2 mL), and the resulting mixture was stirred at room temperature for 5 hours. The mixture was concentrated under reduced pressure, and then the residue was purified by reverse phase column chromatography (solvent gradient; 0 → 20% acetonitrile/0.1% TFA aqueous solution) to obtain the title compound (99.6 mg). MS (ESI+); 918.3
(ix) Synthesis of [N-{3-[(2-{4,7,10-tris[(carboxy-**κ**O)methyl]-1,4,7,10-tetraazacyclododecan-1-yl-**κ**⁴N¹,N⁴,N⁷,N¹⁰}acetamido-**κ**O)methyl]benzoyl}-L-**α**-aspartylglycyl-6-(2,5-dioxo-2,5-dihydro-1H-pyrrol-1-yl)-L-norleucinato(3-)]gadolinium
   A sodium hydrogen carbonate aqueous solution (0.1 M, 800 µL) was added to a mixture of N-[3-({2-[4,7,10-tris(carboxymethyl)-1,4,7,10-tetraazacyclododecan-1-yl]acetamido}methyl)benzoyl]-L-**α**-aspartylglycyl-6-(2,5-dioxo-2,5-dihydro-1H-pyrrol-1-yl)-L-norleucine (20.0 mg), water (2 mL), and gadolinium chloride (6 mg), and the resulting mixture was stirred at room temperature for 10 minutes. A sodium hydrogen carbonate aqueous solution (0.1 M, 60 µL) was added, and the resulting mixture was stirred at room temperature for 1 hour. An ethylenediaminetetraacetic acid (hereinafter, abbreviated as EDTA) aqueous solution (0.5 M, 300 µL) was added, and the resulting mixture was stirred at room temperature for 10 minutes. The reaction mixture was purified by reverse phase column chromatography (solvent gradient; 0 → 25% acetonitrile/0.1% TFA aqueous solution) to obtain the title compound (12.0 mg). MS (ESI-); 1071.4
(x) Synthesis of conjugate No. 11
   [N-{3-[(2-{4,7,10-tris[(carboxy-**κ**O)methyl]-1,4,7,10-tetraazacyclododecan-1-yl-**κ**⁴N¹,N⁴,N⁷,N¹⁰}acetamido-**κ**O)methyl]benzoyl}-L-**α**-aspartylglycyl-6-(2,5-dioxo-2,5-dihydro-1H-pyrrol-1-yl)-L-norleucinato(3-)]gadolinium (1 mg) was dissolved in DMSO (40 µL). 40 µL of the resulting solution was dispensed, a 0.1 M borate buffer (40 µL) was added, and a 0.1 M sodium carbonate aqueous solution was added so as to provide a pH of 6.6.

The solution previously prepared was added to a 4.45 mg/mL Fab² borate buffer (160 µL), and the resulting mixture was incubated at 30°C for 2 hours. A 0.05 M EDTA aqueous solution (40 µL) was added, and then the resulting mixture was incubated at 30°C for 10 minutes. The mixture was purified through a PD-10 column, and the resulting solution was recovered using an Amicon Ultra-0.5 mL centrifugal filter (Merck Millipore). The recovered solution was washed with phosphate buffered saline 7 times repeatedly, finally concentrated, and then filtered through a membrane filter to obtain a conjugate. It was confirmed by MS analysis that the conjugate was a mixture of a conjugate in which one [Gd/DOTA-NH-CH₂-(1,3-Ph)-C(=O)-Asp-Gly-Lys^{∗}-Z₂(#N)] having a molecular weight of 1073 was bound to one Fab² having a molecular weight of 47.9 kDa, a conjugate in which two such molecules were bound thereto, and a conjugate in which three such molecules were bound thereto.

### (Example 2-12. Synthesis of ([Gd/4arm-DOTA-CH₂-(1,4-Ph)-NH-C(=S)-Gly-Phe-Lys^{∗}-Z₂(#N)]p-Fab²))

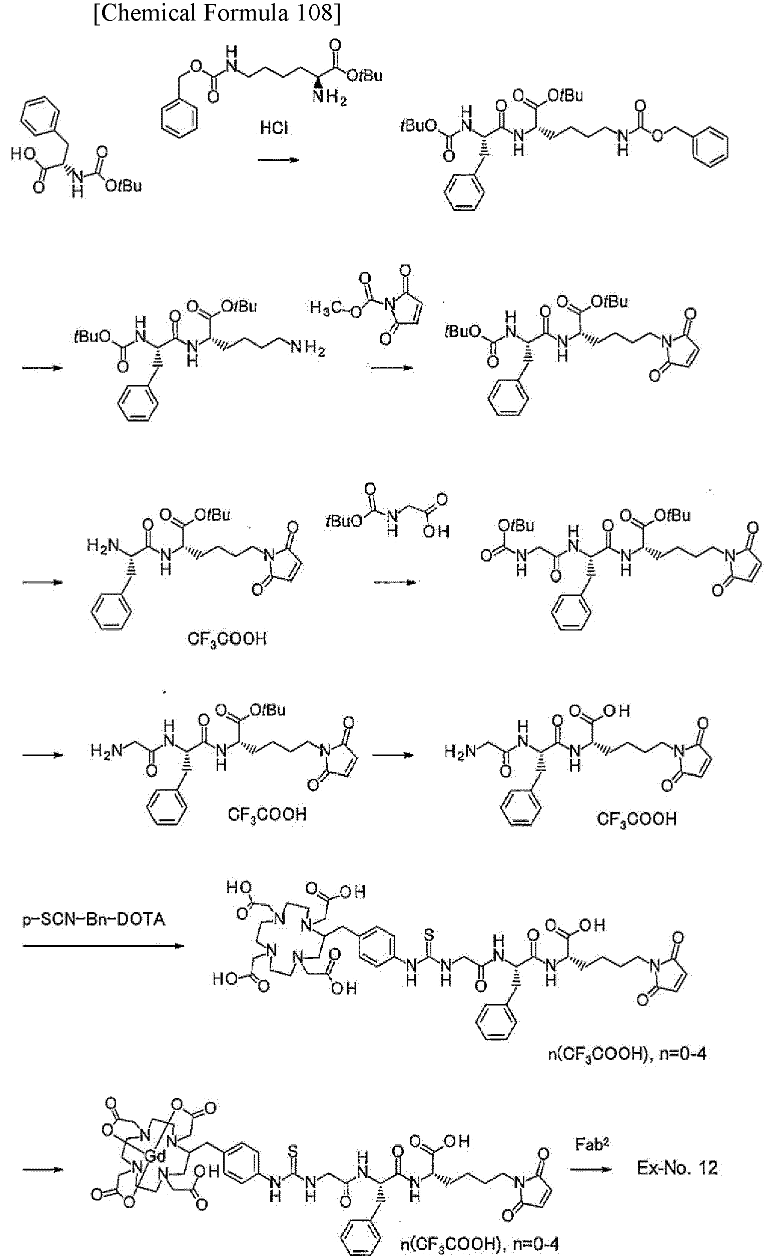

(i) Synthesis of tert-butyl N-(tert-butoxycarbonyl)-L-phenylalanyl-N⁶-[(benzyloxy)carbonyl]-L-lysinate
   tert-butyl N⁶-[(benzyloxy)carbonyl]-L-lysinate monohydrochloride (2.1 g), Et₃N (2.4 mL), and HATU (2.5 g) were sequentially added to a liquid mixture of N-(tert-butoxycarbonyl)-L-phenylalanine (1.5 g) in dichloromethane (30 ml), and the resulting mixture was reacted overnight at room temperature. The reaction mixture was concentrated and then purified through a silica gel column (solvent gradient; 10 → 50% ethyl acetate/hexane) to obtain the title compound (3.16 g). MS (ESI+): 606.4 [M+Na]+
(ii) Synthesis of tert-butyl N-(tert-butoxycarbonyl)-L-phenylalanyl-L-lysinate
   A mixture of ethanol (60 ml) of tert-butyl N-(tert-butoxycarbonyl)-L-phenylalanyl-N⁶-[(benzyloxy)carbonyl]-L-lysinate (3150 mg), and 10% palladium on carbon (water content of 50%, 1000 mg) was stirred under a hydrogen atmosphere (1 atm) at room temperature for 4 and a half hours. The starting materials remained, and thus the system was purged with argon, then the mixture was filtered through Celite, and the filtrate was concentrated. The residue was dissolved in methanol (60 ml), 10% palladium on carbon (50% wet with water, 1000 mg) was added, and the resulting mixture was stirred under a hydrogen atmosphere (1 atm) at room temperature for 5 hours. The disappearance of the starting material was confirmed, then the system was purged with argon, the mixture was filtered through Celite, and the filtrate was concentrated to obtain the title compound (2520 mg). MS (ESI+): 450.4
(iii) Synthesis of tert-butyl N-(tert-butoxycarbonyl)-L-phenylalanyl-6-(2,5-dioxo-2,5-dihydro-1H-pyrrol-1-yl)-L-norleucinate
   A solution of methyl 2,5-dioxo-2,5-dihydro-1H-pyrrole-1-carboxylate (340 mg) in THF (12 ml) was added to a suspension of tert-butyl N-(tert-butoxycarbonyl)-L-phenylalanyl-L-lysinate (825 mg) in a saturated sodium hydrogen carbonate aqueous solution (6 ml) under ice cooling. The resulting mixture was stirred at the same temperature for 2 hours, and then a 1 M sodium hydroxide aqueous solution (0.36 ml) was added, and the resulting mixture was stirred at room temperature for 1 hour. The mixture was diluted with ethyl acetate and water and then acidified with a 10% citric acid aqueous solution. The organic layer was separated, washed with a saturated sodium hydrogen carbonate aqueous solution and saturated brine, dried over magnesium sulfate, and filtered, and then the filtrate was concentrated under reduced pressure. The residue was purified through a silica gel column (solvent gradient; 0 → 8% methanol/chloroform) to obtain the title compound (743 mg). MS (ESI+): 552.4 [M+Na]+
(iv) Synthesis of tert-butyl L-phenylalanyl-6-(2,5-dioxo-2,5-dihydro-1H-pyrrol-1-yl)-L-norleucinate mono(trifluoroacetate)
   TFA (2 ml) was added dropwise to a solution of tert-butyl N-(tert-butoxycarbonyl)-L-phenylalanyl-6-(2,5-dioxo-2,5-dihydro-1H-pyrrol-1-yl)-L-norleucinate (325 mg) in dichloromethane (4 ml) under ice cooling. The resulting mixture was stirred at the same temperature for 1 hour. The mixture was concentrated under reduced pressure to obtain a crude product of the title compound (418 mg). MS (ESI+): 430.4
(v) Synthesis of tert-butyl N-(tert-butoxycarbonyl)glycyl-L-phenylalanyl-6-(2,5-dioxo-2,5-dihydro-1H-pyrrol-1-yl)-L-norleucinate
   N-(tert-butoxycarbonyl)glycine (118 mg), triethylamine (hereinafter, abbreviated as TEA) (0.26 ml), and HATU (256 mg) were sequentially added to a mixture of tert-butyl L-phenylalanyl-6-(2,5-dioxo-2,5-dihydro-1H-pyrrol-1-yl)-L-norleucinate mono(trifluoroacetate) (415 mg) in dichloromethane (10 ml), and the resulting mixture was stirred overnight at room temperature. The mixture was concentrated under reduced pressure and then purified through a silica gel column (solvent gradient; 0 → 30% acetone/chloroform) to obtain the title compound (206 mg). MS (ESI+): 609.4 [M+Na)+
(vi) Synthesis of tert-butyl glycyl-L-phenylalanyl-6-(2,5-dioxo-2,5-dihydro-1H-pyrrol-1-yl)-L-norleucinate mono(trifluoroacetate)
   Using tert-butyl N-(tert-butoxycarbonyl)glycyl-L-phenylalanyl-6-(2,5-dioxo-2,5-dihydro-1H-pyrrol-1-yl)-L-norleucinate (205 mg), a crude product of the title compound (223 mg) was obtained in the same manner as in Example 2-12 (iv) above. MS (ESI+): 487.4
(vii) Synthesis of glycyl-L-phenylalanyl-6-(2,5-dioxo-2,5-dihydro-1H-pyrrol-1-yl)-L-norleucine mono(trifluoroacetate)
   TFA (1 ml) was added to a mixture of tert-butyl glycyl-L-phenylalanyl-6-(2,5-dioxo-2,5-dihydro-1H-pyrrol-1-yl)-L-norleucinate mono(trifluoroacetate) (9 mg) in dichloromethane (1 ml), and the resulting mixture was stirred at room temperature for 1 hour. The mixture was concentrated under reduced pressure to obtain a crude product of the title compound (8 mg). MS (ESI+): 431.3
(viii) Synthesis of N-[(4-{[1,4,7,10-tetrakis(carboxymethyl)-1,4,7,10-tetraazacyclododecan-2-yl]methyl}phenyl)carbamothioyl] glycyl-L-phenylalanyl-6-(2,5-dioxo-2,5-dihydro-1H-pyrrol-1-yl)-L-norleucine
   DIPEA (35 µL) was added to a mixture of glycyl-L-phenylalanyl-6-(2,5-dioxo-2,5-dihydro-1H-pyrrol-1-yl)-L-norleucine mono(trifluoroacetate) (8 mg) and 2,2',2",2'''-{2-[(4-isothiocyanatophenyl)methyl]-1,4,7,10-tetraazacyclododecane-1,4,7,10-tetrayl}tetraacetic acid (abbreviated as p-SCN-Bn-DOTA) (8 mg) in DMF (1 ml), and the resulting mixture was stirred at room temperature for 1.5 hours. The mixture was diluted with a 1% TFA aqueous solution (about 5 ml) and purified by reverse phase column chromatography (solvent gradient 5 → 50% acetonitrile/0.1% TFA aqueous solution) to obtain the title compound (13 mg). MS (ESI+): 982.3
(ix) Synthesis of hydrogen [N-{[4-({1,4,7,10-tetrakis[(carboxy-**κ**O)methyl]-1,4,7,10-tetraazacyclododecan-2-yl-**κ**⁴N¹,N⁴,N⁷,N¹⁰}methyl)phenyl]carbamothioyl}glycyl-L-phenylalanyl-6-(2,5-dioxo-2,5-dihydro-1H-pyrrol-1-yl)-L-norleucinato(4-)]gadolinate(1-)
   N-[(4-{[1,4,7,10-tetrakis(carboxymethyl)-1,4,7,10-tetraazacyclododecan-2-yl]methyl}phenyl)carbamothioyl]glycyl-L-phenylalanyl-6-(2,5-dioxo-2,5-dihydro-1H-pyrrol-1-yl)-L-norleucine (12 mg) was diluted with water (5 ml), and gadolinium chloride (4 mg) was added. The pH of the mixture was adjusted to 5 to 6 with 0.1 M sodium hydrogen carbonate, and the mixture was stirred at room temperature for 1 hour. A 0.5 M EDTA aqueous solution (80 µL) was added to the mixture, and the resulting mixture was stirred at room temperature for 5 minutes. TFA (10 µL) was added, and then the resulting mixture was purified by reverse phase column chromatography (solvent gradient 5 → 50% acetonitrile/0.1% TFA aqueous solution) to obtain the title compound (5 mg). MS (ESI+): 1137.0
(x) Synthesis of conjugate No. 12
   Using hydrogen [N-{[4-({1,4,7,10-tetrakis[(carboxy-**κ**O)methyl]-1,4,7,10-tetraazacyclododecan-2-yl-**κ**⁴N¹,N⁴,N⁷,N¹⁰}methyl)phenyl]carbamothioyl}glycyl-L-phenylalanyl-6-(2,5-dioxo-2,5-dihydro-1H-pyrrol-1-yl)-L-norleucinato(4-)]gadolinate(1-), a conjugate was obtained in the same manner as in step (x) of Example 2-11 above. It was confirmed by MS analysis that the conjugate was a mixture of a conjugate in which one [Gd/4arm-DOTA-CH₂-(1,4-Ph)-NH-C(=S)-Gly-Phe-Lys^{∗}-Z₂(#N)] having a molecular weight of 1136 was bound to one Fab² having a molecular weight of 47.9 kDa, a conjugate in which two such molecules were bound thereto, and a conjugate in which three such molecules were bound thereto.

### (Example 2-13. Synthesis of ([Gd/DOTA-NH-CH₂-(1,3-Ph)-C(=O)-Met-Gly-Lys^{∗}-Z₂(#N)]p-Fab²))

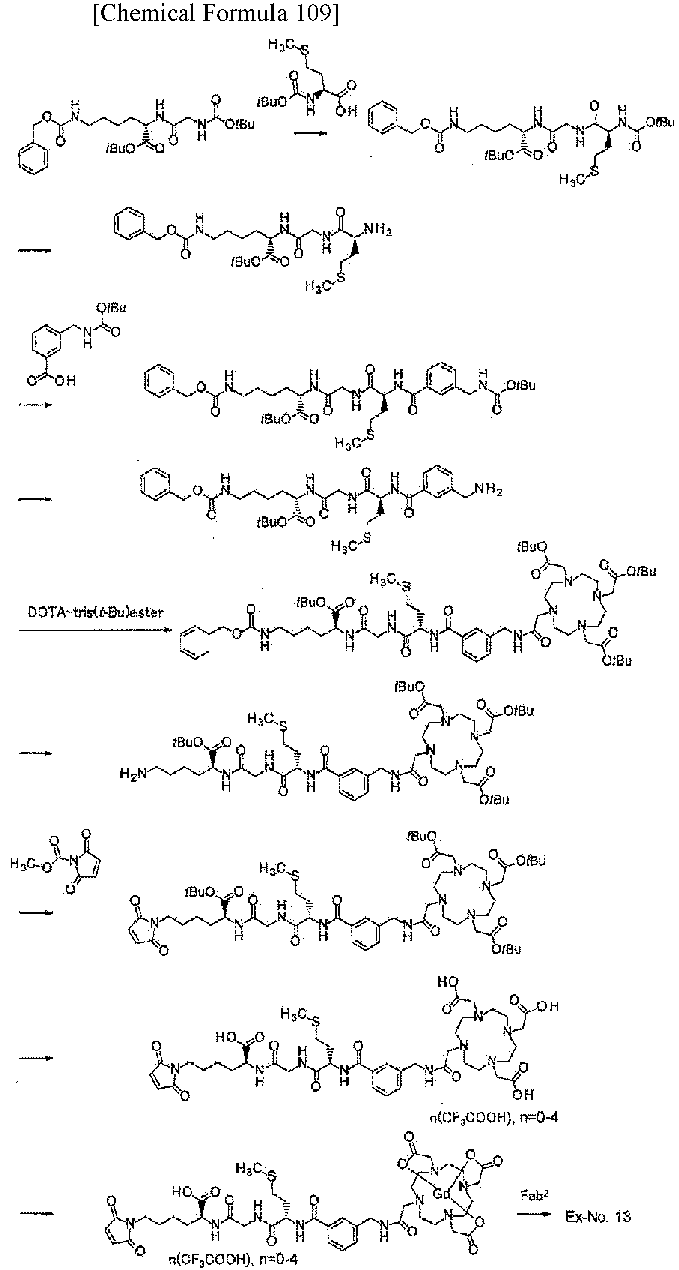

(i) Synthesis of tert-butyl N-(tert-butoxycarbonyl)-L-methionylglycyl-N⁶-[(benzyloxy)carbonyl]-L-lysinate
   TFA (3 mL) was added to a solution of tert-butyl N-(tert-butoxycarbonyl)glycyl-N⁶-[(benzyloxy)carbonyl]-L-lysinate (1.00 g) in dichloromethane (6 mL) under ice cooling, and the resulting mixture was stirred at the same temperature for 2 hours. The mixture was concentrated under reduced pressure, then a saturated sodium hydrogen carbonate aqueous solution was added, and the resulting mixture was extracted twice with dichloromethane. The combined organic layers were washed with a saturated sodium chloride aqueous solution, and then dried over anhydrous sodium sulfate, filtered, and then concentrated. N-(tert-butoxycarbonyl)-L-methionine (556 mg), dichloromethane (10 mL), DIPEA (2 mL), and HATU (1.16 g) were added to this residue, and the resulting mixture was stirred at room temperature for 1 hour. The reaction solution was concentrated, then a saturated sodium hydrogen carbonate aqueous solution was added to the residue, and the resulting mixture was extracted twice with dichloromethane. The combined organic layers were washed with a 1 M sodium hydroxide aqueous solution and a saturated sodium chloride aqueous solution, and then dried over anhydrous sodium sulfate, filtered, and then concentrated to obtain the title compound (1.70 g). MS (ESI+); 625.5
(ii) Synthesis of tert-butyl L-methionylglycyl-N⁶-[(benzyloxy)carbonyl]-L-lysinate
   TFA (4 mL) was added to a solution of tert-butyl N-(tert-butoxycarbonyl)-L-methionylglycyl-N⁶-[(benzyloxy)carbonyl]-L-lysinate (1.70 g) in dichloromethane (10 mL) under ice cooling, and the resulting mixture was stirred at the same temperature for 7 hours. The reaction mixture was concentrated under reduced pressure, and then the residue was purified by silica gel column chromatography (amino silica gel, solvent gradient; 0 → 4% methanol/chloroform) to obtain the title compound (663 mg). MS (ESI+); 525.4
(iii) Synthesis of tert-butyl N-(3-{[(tert-butoxycarbonyl)amino]methyl}benzoyl)-L-methionylglycyl-N⁶-[(benzyloxy)carbonyl]-L-lysinate
   A mixture of tert-butyl L-methionylglycyl-N⁶-[(benzyloxy)carbonyl]-L-lysinate (663 mg), dichloromethane (6 mL), 3-{[(tert-butoxycarbonyl)amino]methyl}benzoic acid (480 mg), DIPEA (700 µL), and HATU (960 mg) was stirred at room temperature for 39 hours. The reaction mixture was concentrated under reduced pressure, and then the residue was purified by silica gel column chromatography (solvent gradient; 0 → 100% ethyl acetate/hexane) to obtain the title compound (935 mg). MS (ESI+); 758.5
(iv) Synthesis of tert-butyl N-[3-(aminomethyl)benzoyl]-L-methionylglycyl-N⁶-[(benzyloxy)carbonyl]-L-lysinate
   TFA (2 mL) was added to a solution of tert-butyl N-(3-{[(tert-butoxycarbonyl)amino]methyl}benzoyl)-L-methionylglycyl-N⁶-[(benzyloxy)carbonyl]-L-lysinate (935 mg) in dichloromethane (2 mL) under ice cooling, and the resulting mixture was stirred at the same temperature for 1 hour. Et₃N and amino silica gel were added, the resulting mixture was concentrated under reduced pressure, and then the residue was purified by silica gel column chromatography (amino silica gel, solvent gradient; 0 → 10% methanol/chloroform) to obtain the title compound (260 mg). MS (ESI+); 658.5
(v) Synthesis of tert-butyl N-[3-({2-[4,7,10-tris(2-tert-butoxy-2-oxoethyl)-1,4,7,10-tetraazacyclododecan-1-yl]acetamido}methyl)benzoyl]-L-methionylglycyl-N⁶-[(benzyloxy)carbonyl]-L-lysinate
   Using tert-butyl N-[3-(aminomethyl)benzoyl]-L-methionylglycyl-N⁶-[(benzyloxy)carbonyl]-L-lysinate (260 mg), the title compound (353 mg) was obtained in the same manner as in step (v) of Example 2-11 above. MS (ESI-); 1210.4
(vi) Synthesis of tert-butyl N-[3-({2-[4,7,10-tris(2-tert-butoxy-2-oxoethyl)-1,4,7,10-tetraazacyclododecan-1-yl]acetamido}methyl)benzoyl]-L-methionylglycyl-L-lysinate
   Using tert-butyl N-[3-({2-[4,7,10-tris(2-tert-butoxy-2-oxoethyl)-1,4,7,10-tetraazacyclododecan-1-yl]acetamido}methyl)benzoyl]-L-methionylglycyl-N⁶-[(benzyloxy)carbonyl]-L-lysinate (353 mg), the title compound (245 mg) was obtained in the same manner as in step (vi) of Example 2-11 above. MS (ESI+): 1078.8
(vii) Synthesis of tert-butyl N-[3-({2-[4,7,10-tris(2-tert-butoxy-2-oxoethyl)-1,4,7,10-tetraazacyclododecan-1-yl]acetamido}methyl)benzoyl]-L-methionylglycyl-6-(2,5-dioxo-2,5-dihydro-1H-pyrrol-1-yl)-L-norleucinate
   Using tert-butyl N-[3-({2-[4,7,10-tris(2-tert-butoxy-2-oxoethyl)-1,4,7,10-tetraazacyclododecan-1-yl]acetamido}methyl)benzoyl]-L-methionylglycyl-L-lysinate (245 mg), the title compound (139 mg) was obtained in the same manner as in step (vii) of Example 2-11 above. MS (ESI+): 1158.8
(viii) Synthesis of N-[3-({2-[4,7,10-tris(carboxymethyl)-1,4,7,10-tetraazacyclododecan-1-yl]acetamido}methyl)benzoyl]-L-methionylglycyl-6-(2,5-dioxo-2,5-dihydro-1H-pyrrol-1-yl)-L-norleucine
   Using tert-butyl N-[3-({2-[4,7,10-tris(2-tert-butoxy-2-oxoethyl)-1,4,7,10-tetraazacyclododecan-1-yl]acetamido}methyl)benzoyl]-L-methionylglycyl-6-(2,5-dioxo-2,5-dihydro-1H-pyrrol-1-yl)-L-norleucinate (341 mg), the title compound (38.2 mg) was obtained in the same manner as in step (viii) of Example 2-11 above. MS (ESI+): 934.4
(ix) Synthesis of [N-{3-[(2-{4,7,10-tris[(carboxy-**κ**O)methyl]-1,4,7,10-tetraazacyclododecan-1-yl-**κ**⁴N¹,N⁴,N⁷,N¹⁰}acetamido-**κ**O)methyl]benzoyl}-L-methionylglycyl-6-(2,5-dioxo-2,5-dihydro-1H-pyrrol-1-yl)-L-norleucinato(3-)]gadolinium
   Using N-[3-({2-[4,7,10-tris(carboxymethyl)-1,4,7,10-tetraazacyclododecan-1-yl]acetamido}methyl)benzoyl]-L-methionylglycyl-6-(2,5-dioxo-2,5-dihydro-1H-pyrrol-1-yl)-L-norleucine (28 mg), the title compound (13.8 mg) was obtained in the same manner as in step (ix) of Example 2-11 above. MS (ESI+): 1089.2
(x) Synthesis of conjugate No. 13
   Using [N-{3-[(2-{4,7,10-tris[(carboxy-**κ**O)methyl]-1,4,7,10-tetraazacyclododecan-1-yl-**κ**⁴N¹,N⁴,N⁷,N¹⁰}acetamido-**κ**O)methyl]benzoyl}-L-methionylglycyl-6-(2,5-dioxo-2,5-dihydro-1H-pyrrol-1-yl)-L-norleucinato(3-)]gadolinium, a conjugate was obtained in the same manner as in step (x) of Example 2-11 above. It was confirmed by MS analysis that the conjugate was a mixture of a conjugate in which one [Gd/DOTA-NH-CH₂-(1,3-Ph)-C(=O)-Met-Gly-Lys^{∗}-Z₂(#N)] having a molecular weight of 1089 was bound to one Fab² having a molecular weight of 47.9 kDa, a conjugate in which two such molecules were bound thereto, and a conjugate in which three such molecules were bound thereto.

### (Example 2-15. Synthesis of ([Gd/DOTA-NH-(CH₂CH₂O)₃-CH₂-C(=O)-NH-CH₂-(1,3-Ph)-C(=O)-Gly-Lys^{∗}-Z₂(#N)]p-Fab²))

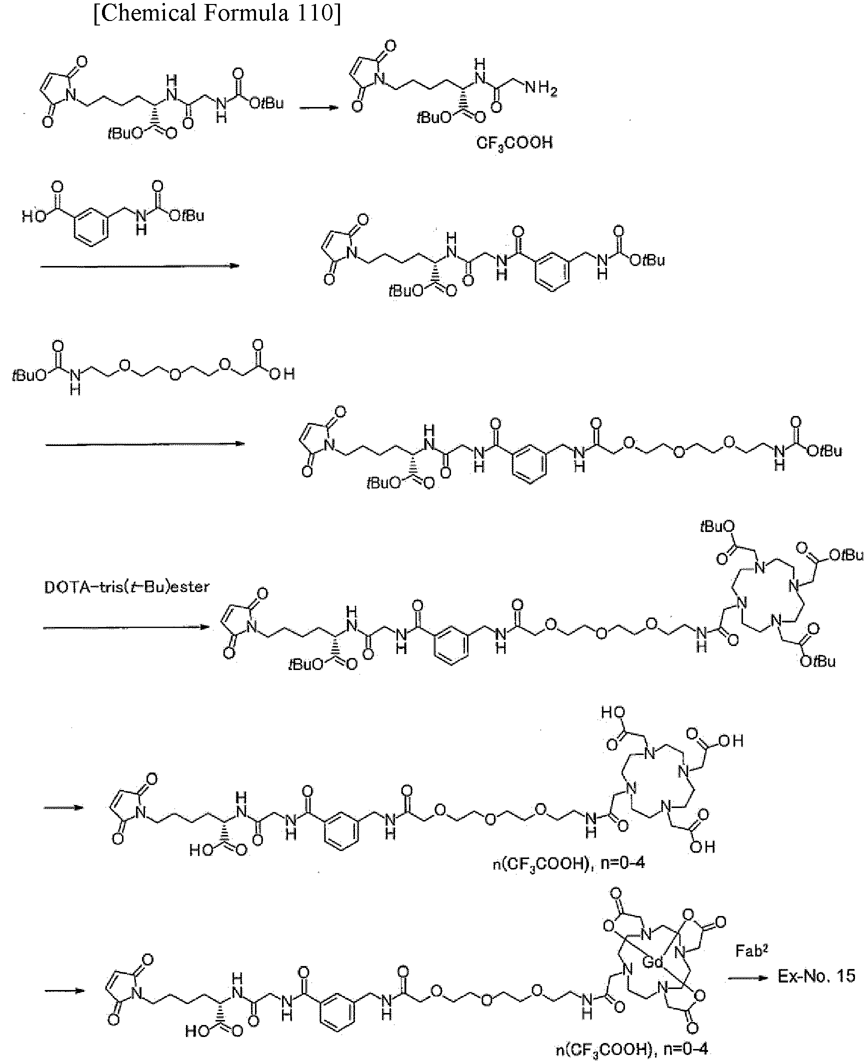

(i) Synthesis of tert-butyl glycyl-6-(2,5-dioxo-2,5-dihydro-1H-pyrrol-1-yl)-L-norleucinate mono(trifluoroacetate)
   TFA (4 ml) was added to a solution of tert-butyl N-(tert-butoxycarbonyl)glycyl-6-(2,5-dioxo-2,5-dihydro-1H-pyrrol-1-yl)-L-norleucinate (400 mg) in dichloromethane (4 mL) under ice cooling, and the resulting mixture was stirred under ice cooling for 1 hour. The reaction mixture was concentrated under reduced pressure to obtain the title compound (642 mg). MS (ESI+); 340.4
(ii) Synthesis of tert-butyl N-(3-{[(tert-butoxycarbonyl)amino]methyl}benzoyl)glycyl-6-(2,5-dioxo-2,5-dihydro-1H-pyrrol-1-yl)-L-norleucinate
   3-{[(tert-butoxycarbonyl)amino]methyl}benzoic acid (228 mg), DIPEA (1.5 mL), and HATU (345 mg) were added to a mixture of tert-butyl glycyl-6-(2,5-dioxo-2,5-dihydro-1H-pyrrol-1-yl)-L-norleucinate mono(trifluoroacetate) (642 mg) and dichloromethane (6 mL) under ice cooling, and the resulting mixture was stirred at room temperature for 2 hours. The reaction mixture was concentrated under reduced pressure, and then the residue was purified by silica gel column chromatography (solvent gradient; 0 → 4% methanol/chloroform) to obtain the title compound (489 mg). MS (ESI+); 573.4
(iii) Synthesis of tert-butyl N-[3-(17,17-dimethyl-3,15-dioxo-5,8,11,16-tetraoxa-2,14-diazaoctadecan-1-yl)benzoyl]glycyl-6-(2,5-dioxo-2,5-dihydro-1H-pyrrol-1-yl)-L-norleucinate
   TFA (2 ml) was added to a mixture of tert-butyl N-(3-{[(tert-butoxycarbonyl)amino]methyl}benzoyl)glycyl-6-(2,5-dioxo-2,5-dihydro-1H-pyrrol-1-yl)-L-norleucinate (489 mg), anisole (280 µL), and dichloromethane (5 mL) under ice cooling, and the resulting mixture was stirred at the same temperature for 1 hour. The reaction mixture was concentrated under reduced pressure, and then dichloromethane (7 mL) was added, and 2,2-dimethyl-4-oxo-3,8,11,14-tetraoxa-5-azahexadecan-16-oic acid (289 mg), DIPEA (1.5 mL), and HATU (487 mg) were added under ice cooling, and the resulting mixture was stirred at room temperature for 2 hours. The reaction mixture was concentrated under reduced pressure, and then the residue was purified by silica gel column chromatography (solvent gradient; 0 → 1% methanol/chloroform) to obtain the title compound (427 mg). MS (ESI+); 762.5
(iv) Synthesis of tert-butyl N-(3-{3,15-dioxo-16-[4,7,10-tris(2-tert-butoxy-2-oxoethyl)-1,4,7,10-tetraazacyclododecan-1-yl]-5,8,11-trioxa-2,14-diazahexadecan-1-yl}benzoyl)glycyl-6-(2,5-dioxo-2,5-dihydro-1H-pyrrol-1-yl)-L-norleucinate
   TFA (1.3 mL) was added to a mixture of tert-butyl N-[3-(17,17-dimethyl-3,15-dioxo-5,8,11,16-tetraoxa-2,14-diazaoctadecan-1-yl)benzoyl]glycyl-6-(2,5-dioxo-2,5-dihydro-1H-pyrrol-1-yl)-L-norleucinate (427 mg), anisole (200 µL), and dichloromethane (4 mL) under ice cooling, and the resulting mixture was stirred at the same temperature for 1 hour. The reaction mixture was concentrated under reduced pressure, and then DMF (6 mL) was added, and DOTA-tris(t-Bu) ester (353 mg), DIPEA (0.96 mL), and HATU (320 mg) were added under ice-cooling, and the resulting mixture was stirred at room temperature for 2 hours. The reaction mixture was concentrated under reduced pressure, and then the residue was purified by silica gel column chromatography (solvent gradient; 0 → 10% methanol/chloroform) to obtain the title compound (863 mg). MS (ESI+): 1238.9 [M+Na]+
(v) Synthesis of N-(3-{3,15-dioxo-16-[4,7,10-tris(carboxymethyl)-1,4,7,10-tetraazacyclododecan-1-yl]-5,8,11-trioxa-2,14-diazahexadecan-1-yl}benzoyl)glycyl-6-(2,5-dioxo-2,5-dihydro-1H-pyrrol-1-yl)-L-norleucine
   Using tert-butyl N-(3-{3,15-dioxo-16-[4,7,10-tris(2-tert-butoxy-2-oxoethyl)-1,4,7,10-tetraazacyclododecan-1-yl]-5,8,11-trioxa-2,14-diazahexadecan-1-yl}benzoyl)glycyl-6-(2,5-dioxo-2,5-dihydro-1H-pyrrol-1-yl)-L-norleucinate (863 mg), the title compound (40.0 mg) was obtained in the same manner as in step (viii) of Example 2-11 above. MS (ESI+): 992.5
(vi) Synthesis of [N-{3-[3-oxo-15-(oxo-**κ**O)-16-{4,7,10-tris[(carboxy-**κ**O)methyl]-1,4,7,10-tetraazacyclododecan-1-yl-**κ**⁴N¹,N⁴,N⁷,N¹⁰}-5,8,11-trioxa-2,14-diazahexadecan-1-yl]benzoyl}glycyl-6-(2,5-dioxo-2,5)-dihydro-1H-pyrrol-1-yl)-L-norleucinato(3-)]gadolinium
   Using N-(3-{3,15-dioxo-16-[4,7,10-tris(carboxymethyl)-1,4,7,10-tetraazacyclododecan-1-yl]-5,8,11-trioxa-2,14-diazahexadecan-1-yl}benzoyl)glycyl-6-(2,5-dioxo-2,5-dihydro-1H-pyrrol-1-yl)-L-norleucine (40 mg), the title compound (15.9 mg) was obtained in the same manner as in step (ix) of Example 2-11 above. MS (ESI-); 1145.0
(vii) Synthesis of conjugate No. 15 [0262] Using [N-{3-[3-oxo-15-(oxo-**κ**O)-16-{4,7,10-tris[(carboxy-**κ**O)methyl]-1,4,7,10-tetraazacyclododecan-1-yl-**κ**⁴N¹,N⁴,N⁷,N¹⁰}-5,8,11-trioxa-2,14-diazahexadecan-1-yl]benzoyl}glycyl-6-(2,5-dioxo-2,5)-dihydro-1H-pyrrol-1-yl)-L-norleucinato(3-)]gadolinium, a conjugate was obtained in the same manner as in step (x) of Example 2-11 above. It was confirmed by MS analysis that the conjugate was a mixture of a conjugate in which one [Gd/DOTA-NH-(CH₂CH₂O)₃-CH₂-C(=O)-NH-CH₂-(1,3-Ph)-C(=O)-Gly-Lys^{∗}-Z₂(#N)] having a molecular weight of 1147 was bound to one Fab² having a molecular weight of 47.9 kDa, a conjugate in which two such molecules were bound thereto, and a conjugate in which three such molecules were bound thereto.

### (Example 2-24. Synthesis of ([Gd/DOTA-NH-CH₂-(1,3-Ph)-C(=O)-Met-Gly-Lys^{∗}-Z₂(#S)]p-Fab²))

### Synthesis of conjugate No. 24 (conjugation via iminothiolane)

A 0.1 M borate buffer (5 µL) of 2 mg/mL 2-iminothiolane (hereinafter, abbreviated as 2-IT) was added to a 4.45 mg/mL Fab² borate buffer (160 µL), and the resulting mixture was incubated at 37°C for 40 minutes. Excess 2-IT was washed with phosphate buffered saline using an Amicon Ultra-0.5 mL centrifugal filter 3 times repeatedly, and finally concentrated.

[N-{3-[(2-{4,7,10-tris[(carboxy-**κ**O)methyl]-1,4,7,10-tetraazacyclododecan-1-yl-**κ**⁴N¹,N⁴,N⁷,N¹⁰}acetamido-**κ**O)methyl]benzoyl}-L-methionylglycyl-6-(2,5-dioxo-2,5-dihydro-1H-pyrrol-1-yl)-L-norleucinato(3-)]gadolinium (1 mg) synthesized in Example 2-13 (ix) was dissolved in DMSO (40 µL). A 0.1 M borate buffer (40 µL) was added to the resulting solution, and a 0.1 M sodium carbonate aqueous solution was added so as to provide a pH of 6.0.

A linker solution prepared was added to the obtained filtrate containing an antibody, and the resulting mixture was incubated at 30°C for 2 hours. An EDTA-containing phosphate buffered saline (pH 6.0) was added, and then the resulting mixture was incubated at 30°C for 10 minutes. The mixture was purified using a PD-10 column, and the resulting solution was recovered using an Amicon Ultra-0.5 mL centrifugal filter. The recovered solution was washed with phosphate buffered saline 7 times, finally concentrated, and then filtered through a membrane filter to obtain a conjugate. It was confirmed by MS analysis that the conjugate was a mixture of a conjugate in which one [Gd/DOTA-NH-CH₂-(1,3-Ph)-C(=O)-Met-Gly-Lys^{∗}-Z₂(#S)] having a molecular weight of 1190 was bound to one Fab² having a molecular weight of 47.9 kDa and a conjugate in which two such molecules were bound thereto.

### (Example 2-29. Synthesis of ([Gd/DOTA-NH-(CH₂CH₂O)₃-CH₂-C(=O)-NH-CH₂-(1,3-Ph)-C(=O)-Met-Gly-Lys^{∗}-Z₂(#N)]p-Fab²))

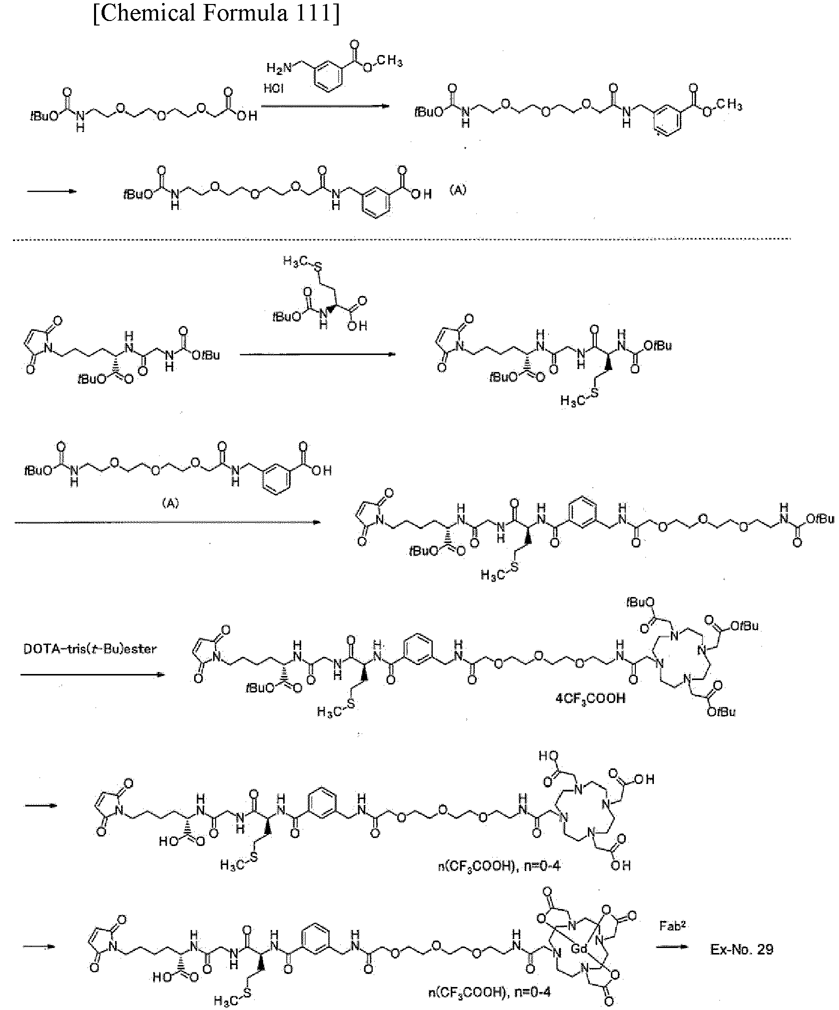

(i) Synthesis of methyl 3-(17,17-dimethyl-3,15-dioxo-5,8,11,16-tetraoxa-2,14-diazaoctadecan-1-yl)benzoate
   methyl 3-(Aminomethyl)benzoate monohydrochloride (286 mg), HATU (590 mg), and Et₃N (900 µL) were added to a solution of 2,2-dimethyl-4-oxo-3,8,11,14-tetraoxa-5-azahexadecan-16-oic acid (396 mg) in dichloromethane (10 mL), and the resulting mixture was stirred at room temperature for 2 hours. The reaction mixture was concentrated under reduced pressure, and the residue was purified by silica gel column chromatography (solvent gradient; 2 → 6% methanol/chloroform) to obtain the title compound (598 mg). MS (ESI+); 455.2
(ii) Synthesis of 3-(17,17-dimethyl-3,15-dioxo-5,8,11,16-tetraoxa-2,14-diazaoctadecan-1-yl)benzoic acid
   AIM sodium hydroxide aqueous solution (4 mL) and water (5 mL) were added to a solution of methyl 3-(17,17-dimethyl-3,15-dioxo-5,8,11,16-tetraoxa-2,14-diazaoctadecan-1-yl)benzoate (597 mg) in THF (15 mL), and the resulting mixture was stirred at room temperature for 5 hours. The reaction solution was diluted with water, 10% citric acid was added, then the resulting mixture was extracted with ethyl acetate, and the collected organic layer was washed with saturated brine. The organic layer was dried over anhydrous magnesium sulfate and filtered, and then the filtrate was concentrated under reduced pressure to obtain the title compound (662 mg). MS (ESI+); 441.1
(iii) Synthesis of tert-butyl N-(tert-butoxycarbonyl)-L-methionylglycyl-6-(2,5-dioxo-2,5-dihydro-1H-pyrrol-1-yl)-L-norleucinate
   TFA (3 ml) was added to a mixture of tert-butyl N-(tert-butoxycarbonyl)glycyl-6-(2,5-dioxo-2,5-dihydro-1H-pyrrol-1-yl)-L-norleucinate (1.63 g) and dichloromethane (5 mL) under ice cooling, and the resulting mixture was stirred at the same temperature for 1 hour. The reaction mixture was concentrated under reduced pressure. DIPEA (5 mL) was added to a mixture of N-(tert-butoxycarbonyl)-L-methionine (1.11 g), dichloromethane (8 mL), and HATU (2.12 g), and the resulting mixture was stirred at room temperature for 5 minutes. A solution of the residue obtained in the previous reaction in dichloromethane (4 mL) was added to this reaction mixture, and the resulting mixture was stirred at room temperature for 20 hours. The reaction mixture was concentrated under reduced pressure, and then the residue was purified by silica gel column chromatography (solvent gradient; 0 → 5% methanol/chloroform) to obtain the title compound (1.63 g). MS (APCI/ESI+); 571.3
(iv) Synthesis of tert-butyl N-[3-(17,17-dimethyl-3,15-dioxo-5,8,11,16-tetraoxa-2,14-diazaoctadecan-1-yl)benzoyl]-L-methionylglycyl-6-(2,5-dioxo-2,5-dihydro-1H-pyrrol-1-yl)-L-norleucinate
   TFA (2 ml) was added to a mixture of tert-butyl N-(tert-butoxycarbonyl)-L-methionylglycyl-6-(2,5-dioxo-2,5-dihydro-1H-pyrrol-1-yl)-L-norleucinate (150 mg) and dichloromethane (2 ml) under ice cooling, and the resulting mixture was stirred at the same temperature for 1 hour. The reaction mixture was concentrated under reduced pressure. DIPEA (360 µL) was added to a mixture of 3-(17,17-dimethyl-3,15-dioxo-5,8,11,16-tetraoxa-2,14-diazaoctadecan-1-yl)benzoic acid (120 mg), dichloromethane (3 mL), and HATU (150 mg), and the resulting mixture was stirred at room temperature for 5 minutes. A solution of the residue obtained in the previous reaction in dichloromethane (2 mL) was added to this reaction mixture, and the resulting mixture was stirred at room temperature for 1 hour. The reaction mixture was concentrated under reduced pressure, and then the residue was purified by silica gel column chromatography (solvent gradient; 0 → 5% methanol/chloroform) to obtain the title compound (113 mg). MS (ESI+); 893.4
(v) Synthesis of tert-butyl N-(3-{3,15-dioxo-16-[4,7,10-tris(2-tert-butoxy-2-oxoethyl)-1,4,7,10-tetraazacyclododecan-1-yl)]-5,8,11-trioxa-2,14-diazahexadecan-1-yl}benzoyl)-L-methionylglycyl-6-(2,5-dioxo-2,5-dihydro-1H-pyrrol-1-yl)-L-norleucinate tetrakis(trifluoroacetate)
   TFA (2 mL) was added to a mixture of tert-butyl N-[3-(17,17-dimethyl-3,15-dioxo-5,8,11,16-tetraoxa-2,14-diazaoctadecan-1-yl)benzoyl]-L-methionylglycyl-6-(2,5-dioxo-2,5-dihydro-1H-pyrrol-1-yl)-L-norleucinate (113 mg) and dichloromethane (2 mL) under ice cooling, and the resulting mixture was stirred at the same temperature for 1 hour and then concentrated under reduced pressure to obtain a crude product. DIPEA (0.22 mL) was added to a mixture of DOTA-tris(t-Bu) ester (80 mg), HATU (80 mg), and dimethylacetamide (hereinafter, abbreviated as DMAc) (1 mL) at room temperature, the resulting mixture was stirred for 10 minutes, then a mixture of the previously obtained crude product in DMAc (1 mL) was added, and the resulting mixture was stirred at the same temperature for 2 hours. The reaction mixture was purified by reverse phase column chromatography (solvent gradient; 0 → 50% acetonitrile/0.1% TFA aqueous solution) to obtain the title compound (178 mg). MS (ESI-); 1345.8
(vi) Synthesis of N-(3-{3,15-dioxo-16-[4,7,10-tris(carboxymethyl)-1,4,7,10-tetraazacyclododecan-1-yl]-5,8,11-trioxa-2,14-diazahexadecan-1-yl}benzoyl)-L-methionylglycyl-6-(2,5-dioxo-2,5-dihydro-1H-pyrrol-1-yl)-L-norleucine
   Using tert-butyl N-(3-{3,15-dioxo-16-[4,7,10-tris(2-tert-butoxy-2-oxoethyl)-1,4,7,10-tetraazacyclododecan-1-yl]-5,8,11-trioxa-2,14-diazahexadecan-1-yl}benzoyl)-L-methionylglycyl-6-(2,5-dioxo-2,5-dihydro-1H-pyrrol-1-yl)-L-norleucinate tetrakis(trifluoroacetate) (178 mg), the title compound (60.0 mg) was obtained in the same manner as in step (viii) of Example 2-11 above. MS (APCI/ESI+); 1123.4
(vii) Synthesis of [N-{3-[3-oxo-15-(oxo-**κ**O)-16-{4,7,10-tris[(carboxy-**κ**O)methyl]-1,4,7,10-tetraazacyclododecan-1-yl-**κ**⁴N¹,N⁴,N⁷,N¹⁰}-5,8,11-trioxa-2,14-diazahexadecan-1-yl]benzoyl}-L-methionylglycyl-6-(2,5-dioxo-2,5-dihydro-1H-pyrrol-1-yl)-L-norleucinato(3-)]gadolinium
   Using N-(3-{3,15-dioxo-16-[4,7,10-tris(carboxymethyl)-1,4,7,10-tetraazacyclododecan-1-yl]-5,8,11-trioxa-2,14-diazahexadecan-1-yl}benzoyl)-L-methionylglycyl-6-(2,5-dioxo-2,5-dihydro-1H-pyrrol-1-yl)-L-norleucine (40 mg), the title compound (10.3 mg) was obtained in the same manner as in step (ix) of Example 2-11 above. MS (ESI+): 1278.3
(viii) Synthesis of conjugate No. 29
   Using [N-{3-[3-oxo-15-(oxo-**κ**O)-16-{4,7,10-tris[(carboxy-**κ**O)methyl]-1,4,7,10-tetraazacyclododecan-1-yl-**κ**⁴N¹,N⁴,N⁷,N¹⁰}-5,8,11-trioxa-2,14-diazahexadecan-1-yl]benzoyl}-L-methionylglycyl-6-(2,5-dioxo-2,5-dihydro-1H-pyrrol-1-yl)-L-norleucinato(3-)]gadolinium, a conjugate was obtained in the same manner as in step (x) of Example 2-11 above. It was confirmed by MS analysis that the conjugate was a mixture of a conjugate in which one [Gd/DOTA-NH-(CH₂CH₂O)₃-CH₂-C(=O)-NH-CH₂-(1,3-Ph)-C(=O)-Met-Gly-Lys^{∗}-Z₂(#N)] having a molecular weight of 1278 was bound to one Fab² having a molecular weight of 47.9 kDa and a conjugate in which two such molecules were bound thereto.

### (Example 2-33. Synthesis of ([Gd/DOTA-[2,5-(1,2,3,4-tetrahydroisoquinoline)]-C(=O)-Met-Gly-Lys^{∗}-Z₂(#N)]p-Fab²))

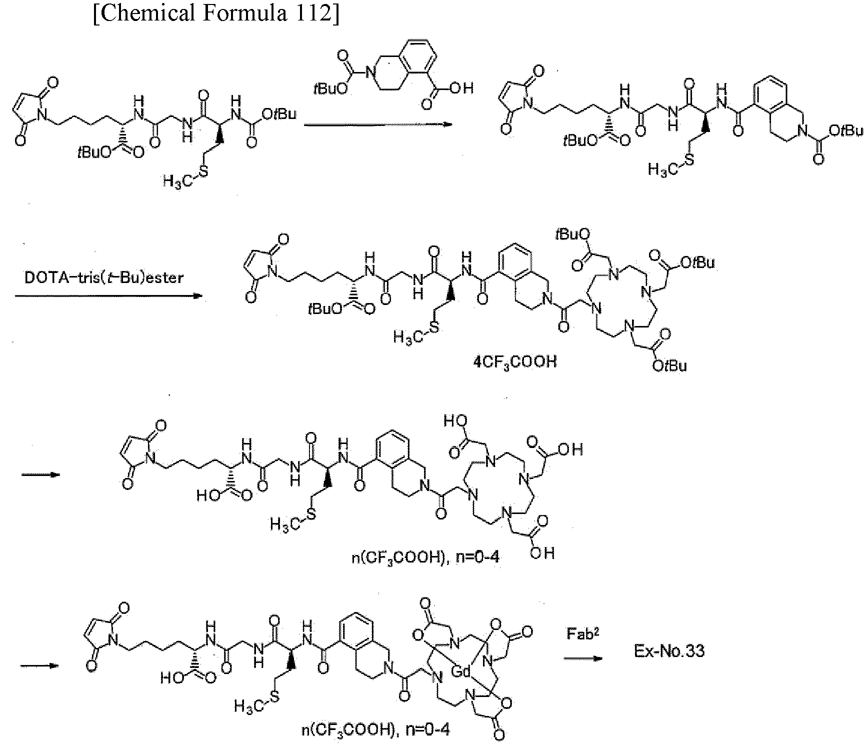

(i) Synthesis of tert-butyl N-[2-(tert-butoxycarbonyl)-1,2,3,4-tetrahydroisoquinoline-5-carbonyl]-L-methionylglycyl-6-(2,5-dioxo-2,5-dihydro-1H-pyrrol-1-yl)-L-norleucinate
   TFA (2 mL) was added to a mixture of tert-butyl N-(tert-butoxycarbonyl)-L-methionylglycyl-6-(2,5-dioxo-2,5-dihydro-1H-pyrrol-1-yl)-L-norleucinate (150 mg) and dichloromethane (2 mL) under ice cooling, and the resulting mixture was stirred at the same temperature for 1 hour. The reaction mixture was concentrated under reduced pressure. A solution of the residue obtained in the previous reaction in dichloromethane (2 mL) was added to a mixture of 2-(tert-butoxycarbonyl)-1,2,3,4-tetrahydroisoquinoline-5-carboxylic acid (88 mg), dichloromethane (2 mL), HATU (150 mg), and DIPEA (360 µL), and the resulting mixture was stirred at room temperature for 1 hour. The reaction mixture was concentrated under reduced pressure, and then the residue was purified by silica gel column chromatography (solvent gradient; 0 → 6% methanol/chloroform) to obtain the title compound (231 mg). MS (APCI/ESI+); 752.2 [M+Na]+
(ii) Synthesis of tert-butyl N-(2-{[4,7,10-tris(2-tert-butoxy-2-oxoethyl)-1,4,7,10-tetraazacyclododecan-1-yl]acetyl}-1,2,3,4-tetrahydroisoquinoline-5-carbonyl)-L-methionylglycyl-6-(2,5-dioxo-2,5-dihydro-1H-pyrrol-1-yl)-L-norleucinate tetrakis(trifluoroacetate)
   Using tert-butyl N-[2-(tert-butoxycarbonyl)-1,2,3,4-tetrahydroisoquinoline-5-carbonyl]-L-methionylglycyl-6-(2,5-dioxo-2,5-dihydro-1H-pyrrol-1-yl)-L-norleucinate (231 mg), the title compound (222 mg) was obtained in the same manner as in step (v) of Example 2-29 above. MS (ESI-); 1182.7
(iii) Synthesis of N-(2-{[4,7,10-tris(carboxymethyl)-1,4,7,10-tetraazacyclododecan-1-yl]acetyl}-1,2,3,4-tetrahydroisoquinoline-5-carbonyl)-L-methionylglycyl-6-(2,5-dioxo-2,5-dihydro-1H-pyrrol-1-yl)-L-norleucine
   Using tert-butyl N-(2-{[4,7,10-tris(2-tert-butoxy-2-oxoethyl)-1,4,7,10-tetraazacyclododecan-1-yl]acetyl}-1,2,3,4-tetrahydroisoquinoline-5-carbonyl)-L-methionylglycyl-6-(2,5-dioxo-2,5-dihydro-1H-pyrrol-1-yl)-L-norleucinate tetrakis(trifluoroacetate) (222 mg), the title compound (53 mg) was obtained in the same manner as in step (viii) of Example 2-11 above. MS (APCI/ESI+); 960.2
(iv) Synthesis of {N-[2-({4,7,10-tris[(carboxy-**κ**O)methyl]-1,4,7,10-tetraazacyclododecan-1-yl-**κ**⁴N¹,N⁴,N⁷,N¹⁰}acetyl-**κ**O)-1,2,3,4-tetrahydroisoquinoline-5-carbonyl]-L-methionylglycyl-6-(2,5-dioxo-2,5-dihydro-1H-pyrrol-1-yl)-L-norleucinato(3-)}gadolinium
   Using N-(2-{[4,7,10-tris(carboxymethyl)-1,4,7,10-tetraazacyclododecan-1-yl]acetyl}-1,2,3,4-tetrahydroisoquinoline-5-carbonyl)-L-methionylglycyl-6-(2,5-dioxo-2,5-dihydro-1H-pyrrol-1-yl)-L-norleucine (22 mg), the title compound (7.0 mg) was obtained in the same manner as in step (ix) of Example 2-11 above. MS (ESI+): 1115.3
(v) Synthesis of conjugate No. 33
   Using the compound of (iv), a conjugate was obtained in the same manner as in step (x) of Example 2-11 above. It was confirmed by MS analysis that the conjugate was a mixture of a conjugate in which one [Gd/DOTA-[2,5-(1,2,3,4-tetrahydroisoquinoline)]-C(=O)-Met-Gly-Lys^{∗}-Z₂(#N)] having a molecular weight of 1115 was bound to one Fab² having a molecular weight of 47.9 kDa and a conjugate in which two such molecules were bound thereto.

### (Example 2-35. Synthesis of ([Gd/DOTA-NH-(CH₂CH₂O)₃-CH₂-C(=O)-NH-CH₂-(1,3-Ph)-C(=O)-Met-Gly-Lys^{∗}-Z₂(#S)]p-Fab²))

### Synthesis of conjugate No. 35

Using [N-{3-[3-oxo-15-(oxo-KO)-16-{4,7,10-tris[(carboxy-**κ**O)methyl]-1,4,7,10-tetraazacyclododecan-1-yl-**κ**⁴N¹,N⁴,N⁷,N¹⁰}-5,8,11-trioxa-2,14-diazahexadecan-1-yl]benzoyl}-L-methionylglycyl-6-(2,5-dioxo-2,5-dihydro-1H-pyrrol-1-yl)-L-norleucinato(3-)]gadolinium synthesized in Example 2-29 (vii), a conjugate was obtained in the same manner as in the step of Example 2-24 above. It was confirmed by MS analysis that the conjugate was a conjugate in which one [Gd/DOTA-NH-(CH₂CH₂O)₃-CH₂-C(=O)-NH-CH₂-(1,3-Ph)-C(=O)-Met-Gly-Lys^{∗}-Z₂(#S)] having a molecular weight of 1380 was bound to one Fab² having a molecular weight of 47.9 kDa.

### (Example 2-40. Synthesis of [Gd/4arm-DOTA-CH₂-(1,4-Ph)-NH-C(=S)-NH-CH₂-(1,3-Ph)-C(=O)-Met-Gly-Lys^{∗}-Z₂(#S)]p-Fab²))

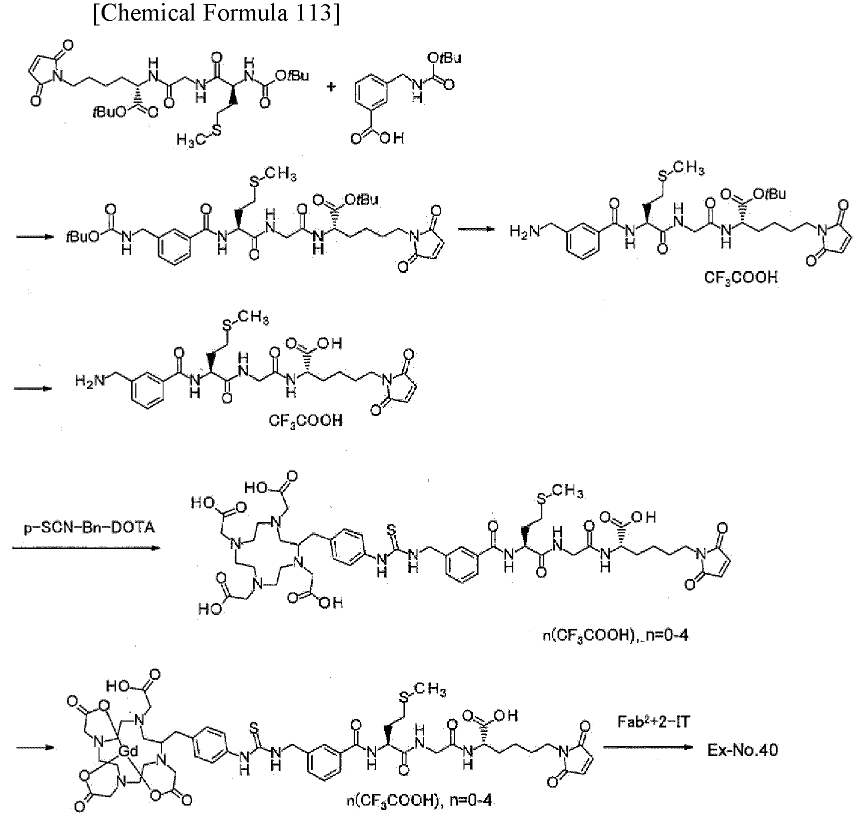

(i) Synthesis of tert-butyl N-(3-{[(tert-butoxycarbonyl)amino]methyl}benzoyl)-L-methionylglycyl-6-(2,5 -dioxo-2,5 -dihydro-1H-pyrrol-1-yl)-L-norleucinate Using tert-butyl N-(tert-butoxycarbonyl)-L-methionylglycyl-6-(2,5-dioxo-2,5-dihydro-1H-pyrrol-1-yl)-L-norleucinate (690 mg), the title compound (710 mg) was obtained in the same manner as in step (i) of Example 2-33 above. MS (ESI+): 726.5 [M+Na]+
(ii) Synthesis of tert-butyl N-[3-(aminomethyl)benzoyl]-L-methionylglycyl-6-(2,5-dioxo-2,5-dihydro-1H-pyrrol-1-yl)-L-norleucinate mono(trifluoroacetate)
   Using tert-butyl N-(3-{[(tert-butoxycarbonyl)amino]methyl}benzoyl)-L-methionylglycyl-6-(2,5-dioxo-2,5-dihydro-1H-pyrrol-1-yl)-L-norleucinate (41 mg), a crude product of the title compound (42 mg) was obtained in the same manner as in Example 2-12 (iv) above. MS (ESI+): 604.3
(iii) Synthesis of N-[3-(aminomethyl)benzoyl]-L-methionylglycyl-6-(2,5-dioxo-2,5-dihydro-1H-pyrrol-1-yl)-L-norleucine mono(trifluoroacetate)
   TFA (2 ml) was added to a mixture of tert-butyl N-[3-(aminomethyl)benzoyl]-L-methionylglycyl-6-(2,5 -dioxo-2,5 -dihydro-1H-pyrrol-1-yl)-L-norleucinate mono(trifluoroacetate) (41 mg) in dichloromethane (4 ml), and the resulting mixture was stirred at room temperature for 1 hour. The mixture was concentrated under reduced pressure and then azeotropically dried with toluene to obtain a crude product (43 mg). 16 mg of the crude product was purified by reverse phase column chromatography (solvent gradient; 5 → 50% acetonitrile/0.1% TFA aqueous solution) to obtain the title compound (11 mg). MS (ESI+): 548.2
(iv) Synthesis of N-[3-({[(4-{[1,4,7,10-tetrakis(carboxymethyl)-1,4,7,10-tetraazacyclododecan-2-yl]methyl}phenyl)carbamothioyl]amino}methyl)benzoyl]-L-methionylglycyl-6-(2,5-dioxo-2,5-dihydro-1H-pyrrol-1-yl)-L-norleucine
   Using N-[3-(aminomethyl)benzoyl]-L-methionylglycyl-6-(2,5-dioxo-2,5-dihydro-1H-pyrrol-1-yl)-L-norleucine mono(trifluoroacetate) (11 mg), the title compound (17 mg) was obtained in the same manner as in step (viii) of Example 2-12 above. MS (ESI+): 1099.5
(v) Synthesis of hydrogen [N-{3-[({[4-({1,4,7,10-tetrakis[(carboxy-**κ**O)methyl]-1,4,7,10-tetraazacyclododecan-2-yl-**κ**⁴N¹,N⁴,N⁷,N¹⁰}methyl)phenyl]carbamothioyl}amino)methyl]benzoyl}-L-methionylglycyl-6-(2,5-dioxo-2,5-dihydro-1H-pyrrol-1-yl)-L-norleucinato(4-)]gadolinate(1-)
   Using N-[3-({[(4-{[1,4,7,10-tetrakis(carboxymethyl)-1,4,7,10-tetraazacyclododecan-2-yl]methyl}phenyl)carbamothioyl]amino}methyl)benzoyl]-L-methionylglycyl-6-(2,5-dioxo-2,5-dihydro-1H-pyrrol-1-yl)-L-norleucine (16 mg), the title compound (6 mg) was obtained in the same manner as in step (ix) of Example 2-12 above. MS (ESI+): 1254.6
(vi) Synthesis of conjugate No. 40
   Using hydrogen [N-{3-[({[4-({1,4,7,10-tetrakis[(carboxy-**κ**O)methyl]-1,4,7,10-tetraazacyclododecan-2-yl-**κ**⁴N¹,N⁴,N⁷,N¹⁰}methyl)phenyl]carbamothioyl}amino)methyl]benzoyl}-L-methionylglycyl-6-(2,5-dioxo-2,5-dihydro-1H-pyrrol-1-yl)-L-norleucinato(4-)]gadolinate(1-), a conjugate was obtained in the same manner as in the step of Example 2-24 above. It was confirmed by MS analysis that the conjugate was a mixture of a conjugate in which one [Gd/4arm-DOTA-CH₂-(1,4-Ph)-NH-C(=S)-NH-CH₂-(1,3-Ph)-C(=O)-Met-Gly-Lys^{∗}-Z₂(#S)] having a molecular weight of 1355 was bound to one Fab² having a molecular weight of 47.9 kDa and a conjugate in which two such molecules were bound thereto.

### (Example 2-47: Synthesis of ([Gd/DOTA]p-Fab²))

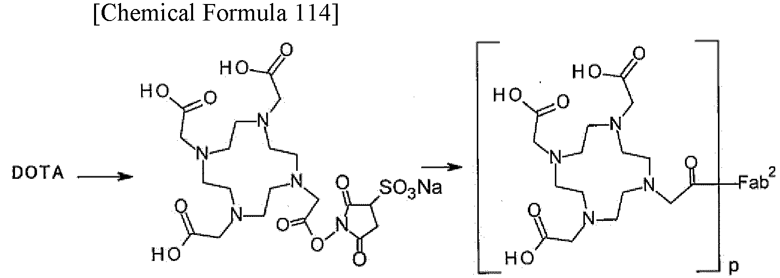

AIM sodium hydroxide aqueous solution (80 µL) was added to a mixed solution of 1,4,7,10-tetraazacyclododecane-1,4,7,10-tetraacetic acid (DOTA) (16 mg) and water (810 µL) under ice cooling to adjust the pH to 6. Sodium 1-hydroxy-2,5-dioxopyrrolidine-3-sulfonate (2.3 mg) dissolved in water (117 µL) was added to the obtained solution (239 µL) under ice cooling. Thereafter, a 3-{[(ethylimino)methylidene]amino}-N,N-dimethylpropan-1-amine monohydrochloride (hereinafter, abbreviated as EDC HCl) aqueous solution (8.3 µL, 25 mg/mL) was added, and the resulting mixture was stirred for 30 minutes under ice cooling to prepare an N-hydroxysulfosuccinimidyl DOTA solution. Before the addition of Fab², a 0.2 M disodium hydrogen phosphate aqueous solution (pH 9) (40 µL) was added to adjust the pH to 7.
(i) The prepared N-hydroxysulfosuccinimidyl DOTA solution (200 µL) was added to a 0.1 M disodium hydrogen phosphate aqueous solution (390 µL) of 17.8 mg/mL Fab² (60 µL), and the resulting mixture was incubated at 4°C for 23 hours. The excess linker was washed with a 10 mM phosphate buffer using an Amicon Ultra-0.5 mL centrifugal filter 3 times repeatedly, washed with a 0.3 M ammonium acetate buffer, finally concentrated, and then filtered through a membrane filter to obtain a conjugate. 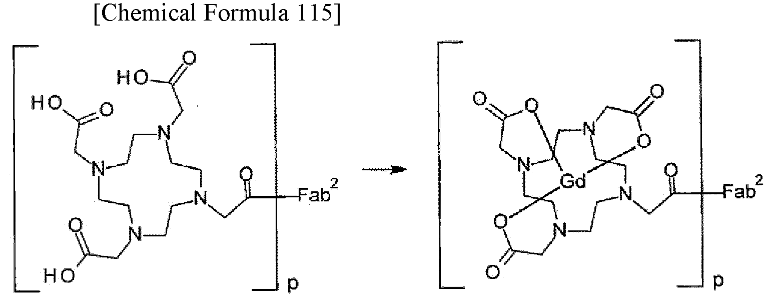
(ii) A 0.3 M ammonium acetate buffer containing the conjugate prepared in (i) was diluted with the same buffer, and the pH was adjusted to 6.62 using a 0.25 M Acetate buffer. A GdCl₃ aqueous solution (35 µL) prepared to 0.057 M was added to the Fab² solution prepared to 2.8 mg/ml, and the resulting mixture was incubated at 37°C for 0.5 hours. After the reaction, 0.05 M EDTA (525 µL) was added, and thereafter the resulting mixture was purified using a PD-10 column, and the resulting solution was recovered using an Amicon Ultra-0.5 mL centrifugal filter. The recovered solution was washed with phosphate buffered saline 5 times, finally concentrated, and then filtered through a membrane filter to obtain a conjugate. It was confirmed by MS analysis that the conjugate was a mixture of a conjugate in which one or two [Gd/DOTA] having a molecular weight of 542 were bound to one Fab² having a molecular weight of 47.9 kDa.

### (Example 2-48. Synthesis of ([Gd/4arm-DOTA-CH₂-(1,4-Ph)-NH-C(=S)]p-Fab²))

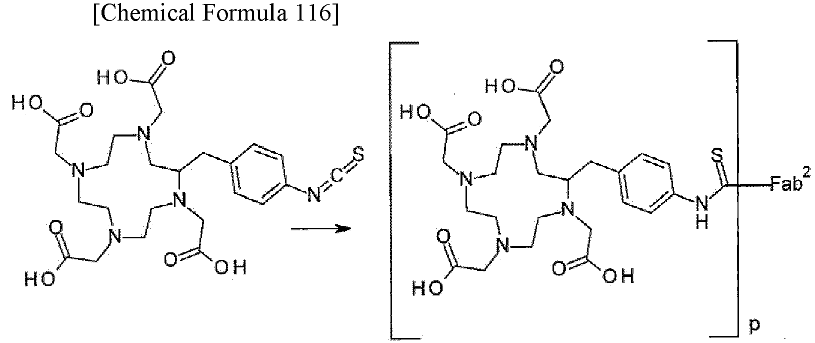

(i) For binding of DOTA, which is a chelating agent, to Fab², p-SCN-Bn-DOTA (Macrocyclics, Inc.) was used. A 0.1 M sodium carbonate solution (pH 9.0) was added to a Fab² solution prepared to 2.54 mg/mL with phosphate buffered saline (pH 7.4) and glycerin to adjust the pH to 8.8 to 9.5. P-SCN-Bn-DOTA was added thereto, and the resulting mixture was incubated at 37°C for 2 hours. After the reaction, the reaction product was recovered using an Amicon Ultra-0.5 mL centrifugal filter to purify a conjugate. It was confirmed by MS analysis that the conjugate was a conjugate in which one or two [4arm-DOTA-CH₂-(1,4-Ph)-NH-C(=S)] having a molecular weight of 553 were bound to one of Fab² having a molecular weight of 47.9 kDa. 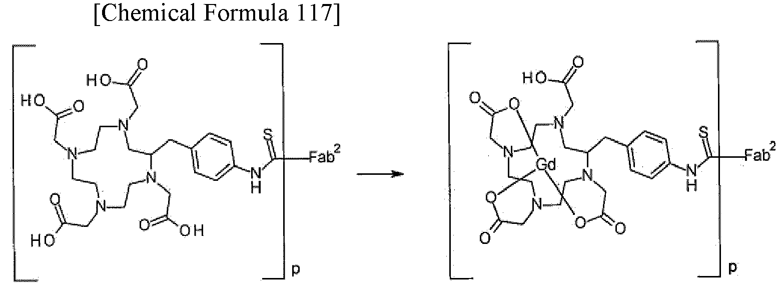
(ii) Using the conjugate prepared in (i), a conjugate was obtained in the same manner as in step (ii) of Example 2-47 above. It was confirmed by MS analysis that the conjugate was a conjugate in which one [Gd/4arm-DOTA-CH₂-(1,4-Ph)-NH-C(=S)] having a molecular weight of 706 was bound to one of Fab² having a molecular weight of 47.9 kDa.

### (Example 2-60. Synthesis of ([DFO-C(=O)-CH₂O-(1,3-Ph)-C(=O)-Gly-Tyr^{∗}-CH₂-C(=O)-NH-(CH₂)₂-Z₁(#S)]p-Fab²))

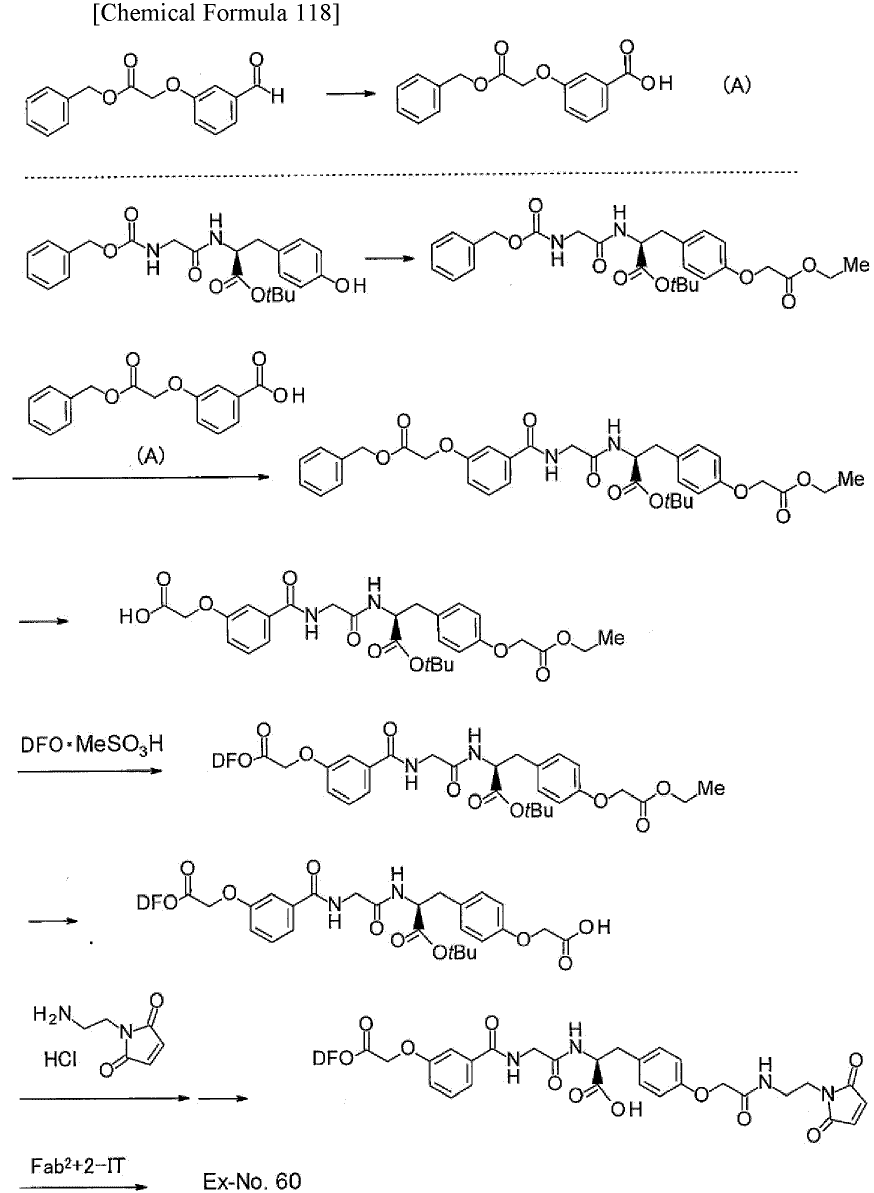

(i) Synthesis of 3-[2-(benzyloxy)-2-oxoethoxy]benzoic acid 2-Methylbut-2-ene (6 mL), sodium dihydrogen phosphate dihydrate (3.35 g), and sodium chlorite (3.64 g) were added to a mixture of benzyl (3-formylphenoxy)acetate (2.90 g), 2-methylpropan-2-ol (60 mL), and water (30 mL) at room temperature, and the resulting mixture was stirred for 2 hours. Ethyl acetate and 1 M hydrochloric acid (60 mL) were added to the reaction liquid, and the resulting mixture was extracted with ethyl acetate. The organic layer was washed with water and a saturated sodium chloride aqueous solution, dried over anhydrous magnesium sulfate, and filtered. The filtrate was concentrated to obtain the title compound (2.93 g). MS (ESI-); 285.1
(ii) Synthesis of tert-butyl N-[(benzyloxy)carbonyl]glycyl-O-(2-ethoxy-2-oxoethyl)-L-tyrosinate
   tert-butyl N-[(benzyloxy)carbonyl]glycyl-L-tyrosinate (2.49 g) was dissolved in acetone (50 mL), and ethyl bromoacetate (2.05 g) and potassium carbonate (2.41 g) were added, and the resulting mixture was stirred at room temperature for 4 hours. The insoluble matter was filtered off, the filtrate was concentrated, and the obtained residue was purified by silica gel column chromatography (ethyl acetate:hexane = 30:70 → 60:40) to obtain the title compound (2.99 g). MS (ESI+): 537.4 [M+Na]+
(iii) Synthesis of tert-butyl N-{3-[2-(benzyloxy)-2-oxoethoxy]benzoyl}glycyl-O-(2-ethoxy-2-oxoethyl)-L-tyrosinate
   tert-butyl N-[(benzyloxy)carbonyl]glycyl-O-(2-ethoxy-2-oxoethyl)-L-tyrosinate (2.75 g) was dissolved in ethanol (55 mL), 10% palladium on carbon (water content of 50%, 550 mg) was added under an argon atmosphere, and the resulting mixture was stirred under a hydrogen atmosphere (1 atm) overnight at room temperature. The system was purged with argon, then the insoluble matter was filtered off, the filtrate was concentrated, the residue was dissolved in dichloromethane (55 mL), 3-[2-(benzyloxy)-2-oxoethoxy]benzoic acid (1.99 g), HATU (2.84 g), and Et₃N (1.5 mL) were added, and the resulting mixture was stirred at room temperature for 1 hour. The reaction liquid was concentrated and purified by silica gel column chromatography (ethyl acetate:hexane = 30:70 → 70:30) to obtain the title compound (3.07 g). MS (ESI-); 647.4
(iv) Synthesis of tert-butyl N-[3-(carboxymethoxy)benzoyl]glycyl-O-(2-ethoxy-2-oxoethyl)-L-tyrosinate
   tert-butyl N-{3-[2-(benzyloxy)-2-oxoethoxy]benzoyl}glycyl-O-(2-ethoxy-2-oxoethyl)-L-tyrosinate (3282 mg) was dissolved in THF (66 mL), 10% palladium on carbon (50% wet with water, 328 mg) was added under an argon atmosphere, and the resulting mixture was stirred under a hydrogen atmosphere overnight at room temperature. The reaction liquid was filtered through Celite, and the filtrate was concentrated to obtain the title compound (2.54 g). MS (ESI-); 557.4
(v) Synthesis of tert-butyl N-{3-[(9,20,31-trihydroxy-2,10,13,21,24,32-hexaoxo-3,9,14,20,25,31-hexaazatritriacontan-1-yl)oxy]benzoyl}glycyl-O-(2-ethoxy-2-oxoethyl)-L-tyrosinate
   DMF (5 mL) and Et₃N (0.16 mL) were added to N⁴-{5-[acetyl(hydroxy)amino]pentyl}-N¹-(5-{4-[(5-aminopentyl)(hydroxy)amino]-4-oxobutanamido}pentyl)-N¹-hydroxybutanediamide monomethanesulfonate (DFO.MeSO₃H) (500 mg), tert-butyl N-[3-(carboxymethoxy)benzoyl]glycyl-O-(2-ethoxy-2-oxoethyl)-L-tyrosinate (446 mg), EDC HCl (175 mg), and 1H-benzotriazol-1-ol (hereinafter, abbreviated as HOBt) (123 mg), and the resulting mixture was stirred overnight at room temperature. A 0.1% TFA aqueous solution (1 ml) and TFA (0.03 mL) were added to the reaction liquid, and the resulting mixture was purified by reverse phase column chromatography (0.1% TFA aqueous solution:acetonitrile = 95:5 → 0:100) to obtain the title compound (548 mg). MS (ESI-); 1099.7
(vi) Synthesis of tert-butyl N-{3-[(9,20,31-trihydroxy-2,10,13,21,24,32-hexaoxo-3,9,14,20,25,31-hexaazatritriacontan-1-yl)oxy]benzoyl}glycyl-O-(carboxymethyl)-L-tyrosinate
   Methanol (5 mL) and DMF (5 mL) were added to tert-butyl N-{3-[(9,20,31-trihydroxy-2,10,13,21,24,32-hexaoxo-3,9,14,20,25,31-hexaazatritriacontan-1-yl)oxy]benzoyl}glycyl-O-(2-ethoxy-2-oxoethyl)-L-tyrosinate (500 mg) and slightly heated for dissolution, a 1 M sodium hydroxide aqueous solution (600 µL) was added, and the resulting mixture was stirred overnight at room temperature. AIM sodium hydroxide aqueous solution (600 µL) was added, and the resulting mixture was stirred overnight at room temperature. TFA (90 µL) was added under ice cooling, methanol was distilled off under reduced pressure, and the obtained solution was purified by reverse phase column chromatography (0.1% TFA aqueous solution:acetonitrile = 95:5 → 0:100) to obtain the title compound (315 mg). MS (ESI-); 1071.6
(vii) Synthesis of tert-butyl N-{3-[(9,20,31-trihydroxy-2,10,13,21,24,32-hexaoxo-3,9,14,20,25,31-hexaazatritriacontan-1-yl)oxy]benzoyl}glycyl-O-(2-{[2-(2,5-dioxo-2,5-dihydro-1H-pyrrol-1-yl)ethyl]amino}-2-oxoethyl)-L-tyrosinate
   DMF (4 mL) was added to a mixture of tert-butyl N-{3-[(9,20,31-trihydroxy-2,10,13,21,24,32-hexaoxo-3,9,14,20,25,31-hexaazatritriacontan-1-yl)oxy]benzoyl}glycyl-O-(carboxymethyl)-L-tyrosinate (269 mg), EDC HCl (58 mg), and HOBt (41 mg), further, 1-(2-aminoethyl)-1H-pyrrole-2,5-dione monohydrochloride (44 mg) and Et₃N (35 µL) were added, and the resulting mixture was stirred at room temperature for 4 hours. A 0.1% TFA aqueous solution (1 mL) was added to the reaction liquid, and the resulting mixture was purified by reverse phase column chromatography (0.1% TFA aqueous solution:acetonitrile = 95:5 → 0:100) to obtain a mixture of the starting material carboxylic acid and the title compound (155 mg, about 6:4). This mixture was dissolved in DMSO (1 mL) and DMF (2 mL), EDC HCl (22 mg) and HOBt (15 mg) were added, the resulting mixture was stirred at room temperature for 5 minutes, then 1-(2-aminoethyl)-1H-pyrrole-2,5-dione monohydrochloride (15.5 mg), and Et₃N (12 µL) were added, and the resulting mixture was stirred at room temperature for 1 hour. A 0.1% TFA aqueous solution (1 mL) was added to the reaction liquid, and the resulting mixture was purified by reverse phase column chromatography (0.1% TFA aqueous solution:acetonitrile = 95:5 → 10:90) to obtain the title compound (118 mg). MS (ESI-); 1193.6
(viii) Synthesis of N-{3-[(9,20,31-trihydroxy-2,10,13,21,24,32-hexaoxo-3,9,14,20,25,31-hexaazatritriacontan-1-yl)oxy]benzoyl}glycyl-O-(2-{[2-(2,5-dioxo-2,5-dihydro-1H-pyrrol-1-yl)ethyl]amino}-2-oxoethyl)-L-tyrosine
   tert-butyl N-{3-[(9,20,31-trihydroxy-2,10,13,21,24,32-hexaoxo-3,9,14,20,25,31-hexaazatritriacontan-1-yl)oxy]benzoyl}glycyl-O-(2-{[2-(2,5-dioxo-2,5-dihydro-1H-pyrrol-1-yl)ethyl]amino}-2-oxoethyl)-L-tyrosinate (118 mg) was dissolved in TFA (1.2 mL), and the resulting solution was stirred at room temperature for 1.5 hours. The reaction liquid was concentrated, DMF (1.5 ml) and water (0.5 ml) were added, and the resulting mixture was purified by reverse phase column chromatography (0.1% TFA aqueous solution:acetonitrile = 95:5 → 10:90) to obtain the title compound (82 mg). MS (ESI-); 1137.5
(ix) Synthesis of conjugate No. 60
   A 2-IT solution prepared with a 0.1 M borate buffer was added to a Fab² solution prepared to 4.45 mg/mL with a 0.1 M borate buffer, and the resulting mixture was incubated at 37°C for 30 minutes. Excess 2-IT was washed with EDTA-containing phosphate buffered saline (pH 6.0) using an Amicon Ultra-0.5 mL centrifugal filter, finally concentrated, and then filtered through a membrane filter. N-{3-[(9,20,31-trihydroxy-2,10,13,21,24,32-hexaoxo-3,9,14,20,25,31-hexaazatritriacontan-1-yl)oxy]benzoyl}glycyl-O-(2-{[2-(2,5-dioxo-2,5-dihydro-1H-pyrrol-1-yl)ethyl)]amino}-2-oxoethyl)-L-tyrosine dissolved in DMF was added to the obtained filtrate, and the resulting mixture was diluted with a 0.1 M borate buffer (pH 8.5) and incubated at room temperature for 2 hours. The excess reagent was washed with EDTA-containing phosphate buffered saline (pH 6.0) using an Amicon Ultra-0.5 mL centrifugal filter, which was repeated 3 times, finally concentrated, and then filtered through a membrane filter.

Subsequently, a 2-iodoacetamide solution prepared to 10 mg/mL with phosphate buffered saline (pH 6.0) was added to the obtained supernatant, and then the resulting mixture was incubated at 37°C for 30 minutes. Excess iodoacetamide was washed with phosphate buffered saline using an Amicon Ultra-0.5 mL centrifugal filter 3 times repeatedly, finally concentrated, and then filtered through a membrane filter to purify a Fab²-bound conjugate. It was confirmed by MS analysis that the conjugate was a mixture of a conjugate in which one [DFO-C(=O)-CH₂O-(1,3-Ph)-C(=O)-Gly-Tyr^{∗}-CH₂-C(=O)-NH-(CH₂)₂-Z₁(#S)] having a molecular weight of 1241 was bound to one Fab² having a molecular weight of 47.9 kDa.

### (Example 2-61. Synthesis of ([DFO-C(=O)-(CH₂CH₂O)₄-(1,3-Ph)-C(=O)-Gly-Lys^{∗}-Z₂(#S)]ₚ-Fab²))

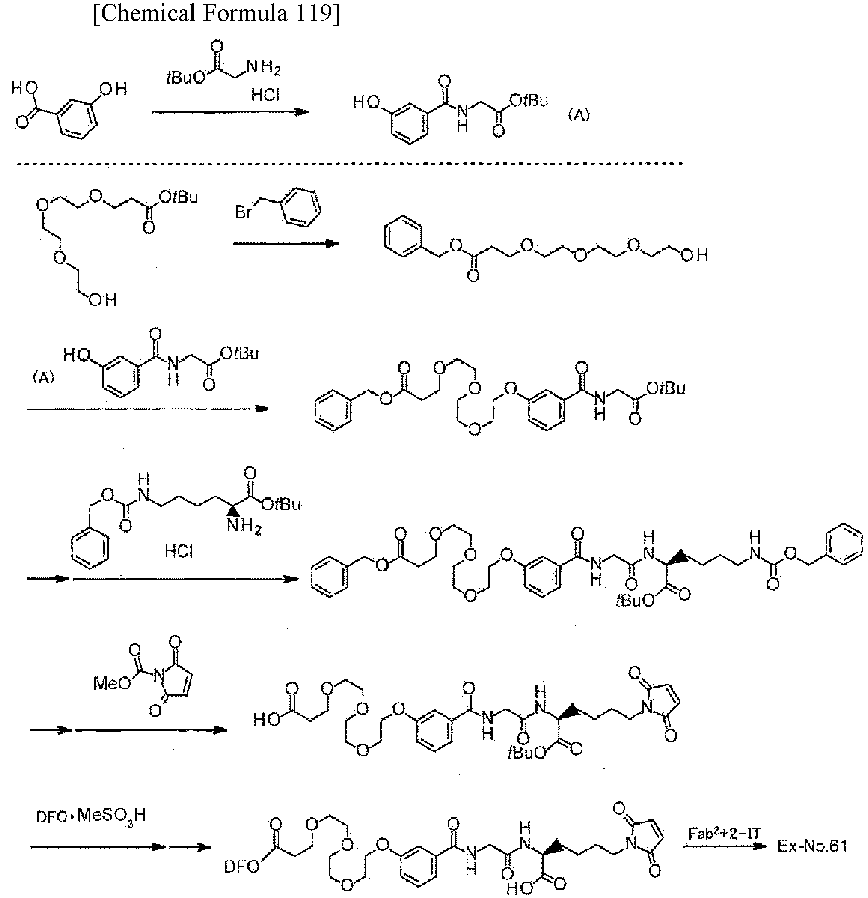

(i) Synthesis of tert-butyl N-(3-hydroxybenzoyl)glycinate
   HATU (3.3 g) and DIPEA (3 mL) were added to a mixture of 3-hydroxybenzoic acid (1.0 g) and tert-butyl glycinate monohydrochloride (1.2 g) in DMF (10 mL) under ice cooling, and the resulting mixture was stirred at room temperature for 1 hour. Water and ethyl acetate were added to the mixture, the resulting mixture was subjected to layer separation and extraction twice, the organic layer was washed with water and a saturated sodium chloride aqueous solution, then dried over anhydrous magnesium sulfate, and filtered, then the filtrate was concentrated under reduced pressure, ethyl acetate was added to the obtained solid, the resulting mixture was stirred at room temperature, then the insoluble matter was collected by filtration, and the filtrate was concentrated. The residue was purified by silica gel column chromatography (hexane/ethyl acetate = 95/5 → 50/50), fractions of the desired product were collected, concentrated, and dried under reduced pressure to obtain the title compound (1.23 g). MS (ESI+): 274.2 [M+Na]+
(ii) Synthesis of benzyl 3-{2-[2-(2-hydroxyethoxy)ethoxy]ethoxy}propanoate
   4 M hydrogen chloride/dioxane (10 mL) was added to tert-butyl 3-{2-[2-(2-hydroxyethoxy)ethoxy]ethoxy}propanoate (3.0 g) under ice cooling, and then the resulting mixture was stirred at room temperature for 1 hour. The mixture was concentrated and then azeotropically dried twice with toluene, then methanol (20 mL) and a 1 M sodium hydroxide aqueous solution (13 mL) were added at room temperature, and the resulting mixture was stirred at the same temperature for 1 and a half hours. The mixture was concentrated, then THF (10 mL), methanol (10 mL), and benzyl bromide (1.8 mL) were added, and the resulting mixture was stirred at room temperature for 3 days. The mixture was concentrated, and the residue was purified by silica gel column chromatography (hexane/ethyl acetate = 95/5 → 0/100 → chloroform/methanol = 90/10) to obtain the title compound (2.19 g). MS (ESI+): 313.2
(iii) Synthesis of tert-butyl N-{3-[(3-oxo-1-phenyl-2,6,9,12-tetraoxatetradecan-14-yl)oxy]benzoyl}glycinate
   tert-butyl N-(3-Hydroxybenzoyl)glycinate (915 mg), benzyl 3-{2-[2-(2-hydroxyethoxy)ethoxy]ethoxy}propanoate (1.38 g), diethyl azodicarboxylate (40% toluene solution, about 2.2 M, 3.4 mL), and THF (20 mL) were added, then triphenylphosphine (2.0 g) was added in portions at room temperature, and then the resulting mixture was stirred overnight at a bath temperature of 60°C. Magnesium chloride (1.5 g) and toluene (20 mL) were added, and the resulting mixture was heated and stirred at a bath temperature of 60°C for 2 hours. The mixture was allowed to cool to room temperature, then the precipitated solid was removed by filtration, and the solvent was distilled off. The residue was purified by silica gel column chromatography (silica gel; hexane/ethyl acetate = 95/5 → 20/80) and then purified again by silica gel column chromatography (amino silica gel; hexane/ethyl acetate = 95/5 → 50/50) to obtain the title compound (1.12 g). MS (ESI+): 546.4
(iv) Synthesis of N-{3-[(3-oxo-1-phenyl-2,6,9,12-tetraoxatetradecan-14-yl)oxy]benzoyl}glycine
   tert-butyl N-{3-[(3-Oxo-1-phenyl-2,6,9,12-tetraoxatetradecan-14-yl)oxy]benzoyl}glycinate (1.11 g) was dissolved in 4 M hydrogen chloride/dioxane (5 mL) at room temperature, and the resulting solution was stirred at the same temperature for 2 hours. The mixture was concentrated and dried under reduced pressure to obtain the title compound (1.12 g). MS (ESI+): 490.3
(v) Synthesis of tert-butyl N-{3-[(3-oxo-1-phenyl-2,6,9,12-tetraoxatetradecan-14-yl)oxy]benzoyl}glycyl-N⁶-[(benzyloxy)carbonyl]-L-lysinate
   HATU (1.2 g) and DIPEA (1.4 mL) were added to a solution of N-{3-[(3-oxo-1-phenyl-2,6,9,12-tetraoxatetradecan-14-yl)oxy]benzoyl}glycine (1300 mg), tert-butyl N⁶-[(benzyloxy)carbonyl]-L-lysinate monohydrochloride (1.0 g) in DMF (20 mL) under ice cooling, and the resulting mixture was stirred at room temperature for 2 hours. Water and ethyl acetate were added to the mixture for layer separation and extraction, the organic layer was washed with water and saturated brine, then dried over anhydrous magnesium sulfate, and filtered, and then the filtrate was concentrated under reduced pressure. The residue was purified by silica gel column chromatography (amino silica gel; hexane/ethyl acetate = 90/10 → 0/100) to obtain the title compound (1.14 g). MS (ESI+): 808.5
(vi) Synthesis of tert-butyl N-[3-(2-{2-[2-(2-carboxyethoxy)ethoxy]ethoxy}ethoxy)benzoyl]glycyl-L-lysinate
   10% Palladium on carbon (50% wet with water, 200 mg) was added to a mixture of tert-butyl N-{3-[(3-oxo-1-phenyl-2,6,9,12-tetraoxatetradecan-14-yl)oxy]benzoyl}glycyl-N⁶-[(benzyloxy)carbonyl]-L-lysinate (1.12 g), Et₃N (20 µL), and ethanol (20 mL) at room temperature, and the resulting mixture was stirred under a hydrogen atmosphere overnight at the same temperature. The mixture was stirred at room temperature for 30 minutes or more in an open system, then the reaction liquid was filtered through Celite, and the filtrate was concentrated to obtain the title compound (825 mg). MS (ESI+): 584.5
(vii) Synthesis of tert-butyl N-[3-(2-{2-[2-(2-carboxyethoxy)ethoxy]ethoxy}ethoxy)benzoyl]glycyl-6-(2,5-dioxo-2,5-dihydro-1H-pyrrol-1-yl)-L-norleucinate
   methyl 2,5-Dioxo-2,5-dihydro-1H-pyrrole-1-carboxylate (150 mg) and Et₃N (500 µL) were added to a mixture of tert-butyl N-[3-(2-{2-[2-(2-carboxyethoxy)ethoxy]ethoxy}ethoxy)benzoyl]glycyl-L-lysinate (350 mg), THF (3 mL), and DMF (1 mL) at room temperature, and the resulting mixture was stirred at a bath temperature of 60°C for 4 days. The mixture was cooled to room temperature, and then TFA (300 µL) and water (500 µl) were added to neutralize the mixture. Appropriate amounts of water and DMF were added to the mixture, and the resulting mixture was purified by reverse phase column chromatography (0.1% TFA aqueous solution:acetonitrile = 95:5 → 0:100). Fractions of the desired product were collected, concentrated, and dried under reduced pressure to obtain the title compound (250 mg). MS (ESI+): 664.5
(viii) Synthesis of tert-butyl N-{3-[(19,30,41-trihydroxy-12,20,23,31,34,42-hexaoxo-3,6,9-trioxa-13,19,24,30,35,41-hexaazatritetracontan-1-yl)oxy]benzoyl}glycyl-6-(2,5-dioxo-2,5-dihydro-1H-pyrrol-1-yl)-L-norleucinate
   EDC HCl (140 mg), HOBt (100 mg), and DIPEA (200 µL) were added to a mixture of N⁴-{5-[acetyl(hydroxy)amino]pentyl}-N¹-(S-{4-[(5-aminopentyl)(hydroxy)amino]-4-oxobutaneamido}pentyl)-N¹-hydroxybutanediamide monomethanesulfonate (DFO.MeSO₃H) (240 mg), tert-butyl N-[3-(2-{2-[2-(2-carboxyethoxy)ethoxy]ethoxy}ethoxy)benzoyl]glycyl-6-(2,5-dioxo-2,5-dihydro-1H-pyrrol-1-yl)-L-norleucinate (243 mg) in DMF (3 mL) and DMSO (1 mL) under ice cooling, and the resulting mixture was stirred at room temperature for 3 hours. TFA (100 µL) and water (500 µL) were added, and the resulting solution was purified by reverse phase column chromatography (0.1% TFA aqueous solution:acetonitrile = 95:5 → 10:90) and freeze-dried to obtain the title compound (207 mg). MS (ESI+): 1228.5 [M+Na]+
(ix) Synthesis of N-{3-[(19,30,41-trihydroxy-12,20,23,31,34,42-hexaoxo-3,6,9-trioxa-13,19,24,30,35,41-hexaazatritetracontan-1-yl)oxy]benzoyl}glycyl-6-(2,5-dioxo-2,5-dihydro-1H-pyrrol-1-yl)-L-norleucine
   TFA (1 mL) was added to tert-butyl N-{3-[(19,30,41-trihydroxy-12,20,23,31,34,42-hexaoxo-3,6,9-trioxa-13,19,24,30,35,41-hexaazatritetracontan-1-yl)oxy]benzoyl}glycyl-6-(2,5-dioxo-2,5-dihydro-1H-pyrrol-1-yl)-L-norleucinate (202 mg) at room temperature, and the resulting mixture was stirred at the same temperature for 1 hour. The mixture was concentrated, DMF (4 ml) and water (500 µL) were added thereto, and the resulting mixture was purified by reverse phase column chromatography (0.1% TFA aqueous solution:acetonitrile = 95:5 → 10:90) and freeze-dried to obtain the title compound (137 mg). MS (ESI-); 1148.7
(x) Synthesis of conjugate No. 61
   Using N-{3-[(19,30,41-trihydroxy-12,20,23,31,34,42-hexaoxo-3,6,9-trioxa-13,19,24,30,35,41-hexaazatritetracontan-1-yl)oxy]benzoyl}glycyl-6-(2,5-dioxo-2,5-dihydro-1H-pyrrol-1-yl)-L-norleucine, a conjugate was obtained in the same manner as in step (ix) of Example 2-60 above. It was confirmed by MS analysis that the conjugate was a mixture of a conjugate in which one [DFO-C(=O)-(CH₂CH₂O)₄-(1,3-Ph)-C(=O)-Gly-Lys^{∗}-Z₂(#S)] having a molecular weight of 1252 was bound to one Fab² having a molecular weight of 47.9 kDa and a conjugate in which two such molecules were bound thereto.

### (Example 2-18. Synthesis of ([Gd/DOTA-NH-CH₂-(1,4-Ph)-NH-C(=O)-Met-Ile-NH-(CH₂)₂-Z₁(#N)]p-Fab²))

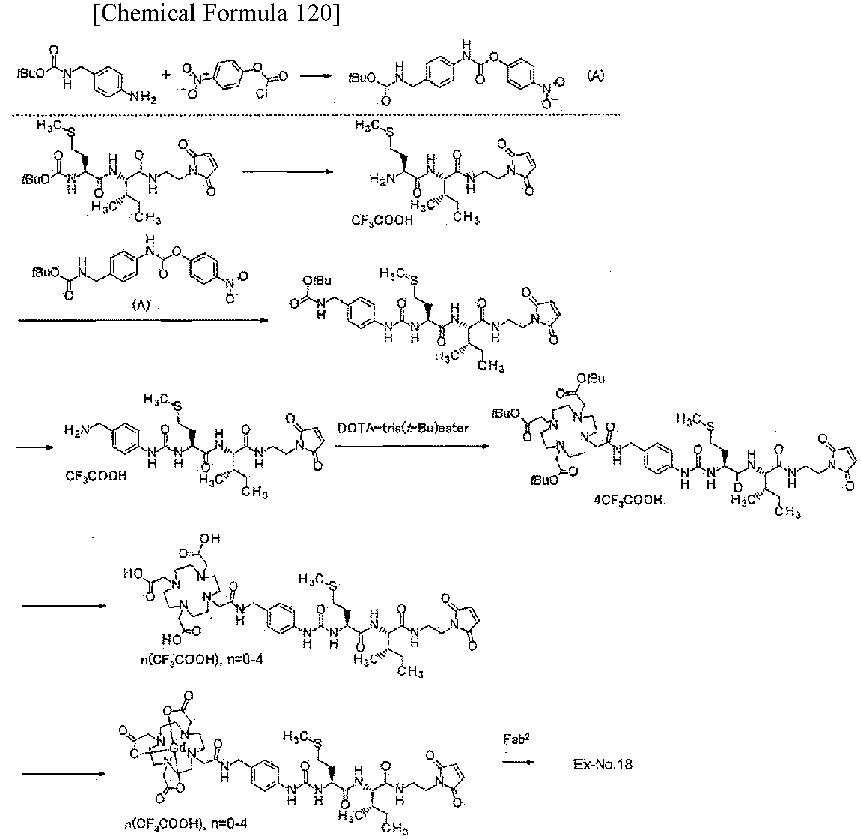

(i) Synthesis of 4-nitrophenyl (4-{[(tert-butoxycarbonyl)amino]methyl}phenyl)carbamate
   A solution of 4-nitrophenyl carbonochloridate (952 mg) in dichloromethane (10 ml) was ice-cooled under a nitrogen atmosphere, a mixed solution of tert-butyl [(4-aminophenyl)methyl]carbamate (1000 mg) and pyridine (430 µL) in dichloromethane (10 mL) was added dropwise, and then the resulting mixture was stirred at the same temperature for 1 hour. The reaction mixture was concentrated under reduced pressure, and the residue was purified by silica gel column chromatography (solvent gradient; 10 → 100% ethyl acetate/hexane) to obtain a crude product. Ethyl acetate was added to the crude product, and the resulting mixture was stirred to triturate the solid. The solid was collected by filtration, washed with ethyl acetate, and then dried under reduced pressure to obtain the title compound (861 mg). MS (ESI+): 410.3 [M+Na]+
(ii) Synthesis of L-methionyl-N¹-[2-(2,5-dioxo-2,5-dihydro-1H-pyrrol-1-yl)ethyl]-L-isoleucinamide mono(trifluoroacetate)
   TFA (2 ml) was added dropwise to a solution of N-(tert-butoxycarbonyl)-L-methionyl-N¹-[2-(2,5-dioxo-2,5-dihydro-1H-pyrrol-1-yl)ethyl]-L-isoleucinamide (257 mg) in dichloromethane (4 ml) under ice cooling. The resulting mixture was stirred at the same temperature for 1 hour. The mixture was concentrated under reduced pressure, diisopropyl ether was added, decantation was carried out twice, and the precipitated solid was washed to obtain a crude product of the title compound (248 mg). MS (ESI+): 385.3
(iii) Synthesis of N-[(4-{[(tert-butoxycarbonyl)amino]methyl}phenyl)carbamoyl]-L-methionyl-N¹-[2-(2,5-dioxo-2,5-dihydro-1H-pyrrol-1-yl)ethyl]-L-isoleucinamide
   TEA (59 µL) was added to a mixed solution of 4-nitrophenyl (4-{[(tert-butoxycarbonyl)amino]methyl}phenyl)carbamate (54 mg) and L-methionyl-N¹-[2-(2,5-dioxo-2,5-dihydro)-1H-pyrrol-1-yl)ethyl]-L-isoleucinamide mono(trifluoroacetate) (70 mg) in dichloromethane (5 ml), and the resulting mixture was stirred at room temperature for 30 minutes. The reaction mixture was concentrated under reduced pressure, and the residue was purified by silica gel column chromatography (solvent gradient; 2 → 6% methanol/chloroform) to obtain the title compound (55 mg). MS (ESI+): 633.5
(iv) Synthesis of N-{[4-(aminomethyl)phenyl]carbamoyl}-L-methionyl-N¹-[2-(2,5-dioxo-2,5-dihydro-1H-pyrrol-1-yl)ethyl]-L-isoleucinamide mono(trifluoroacetate)
   TFA (2 ml) was added dropwise to a solution of N-[(4-{[(tert-butoxycarbonyl)amino]methyl}phenyl)carbamoyl]-L-methionyl-N¹-[2-(2,5-dioxo-2,5-dihydro-1H-pyrrol-1-yl)ethyl]-L-isoleucinamide (54 mg) in dichloromethane (4 ml) under ice cooling, and the resulting mixture was stirred at the same temperature for 1 hour. The mixture was concentrated under reduced pressure, diisopropyl ether was added, decantation was carried out twice, and the precipitated solid was washed to obtain a crude product of the title compound (65 mg). MS (ESI+): 555.4 [M+Na]+
(v) Synthesis of N-{[4-({2-[4,7,10-tris(2-tert-butoxy-2-oxoethyl)-1,4,7,10-tetraazacyclododecan-1-yl]acetamido}methyl)phenyl]carbamoyl}-L-methionyl-N¹-[2-(2,5-dioxo-2,5-dihydro-1H-pyrrol-1-yl)ethyl]-L-isoleucinamide tetrakis(trifluoroacetate)
   HATU (66 mg) and DIPEA (45 µL) were added to a mixed solution of N-{[4-(aminomethyl)phenyl]carbamoyl}-L-methionyl-N¹-[2-(2,5-dioxo-2,5-dihydro-1H-pyrrol-1-yl)ethyl]-L-isoleucinamide mono(trifluoroacetate) (64 mg) and DOTA-tris(t-Bu) ester (50 mg) in DMAc (2 mL), and the resulting mixture was stirred at room temperature for 2 hours. A 1% TFA aqueous solution (about 4 ml) and acetonitrile (about 1 ml) were added to the reaction mixture, then a diluted solution thereof was purified by reverse phase column chromatography (solvent gradient; 20 → 100% 0.1% TFA acetonitrile/0.1% TFA aqueous solution) to obtain the title compound (92 mg). MS (ESI+): 1109.4 [M+Na]+
(vi) Synthesis of N-{[4-({2-[4,7,10-tris(carboxymethyl)-1,4,7,10-tetraazacyclododecan-1-yl]acetamido}methyl)phenyl]carbamoyl}-L-methionyl-N¹-[2-(2,5-dioxo-2,5-dihydro-1H-pyrrol-1-yl)ethyl]-L-isoleucinamide tetrakis(trifluoroacetate)
   TFA (4 mL) was added to a solution of N-{[4-({2-[4,7,10-tris(2-tert-butoxy-2-oxoethyl)-1,4,7,10-tetraazacyclododecan-1-yl]acetamido}methyl)phenyl]carbamoyl}-L-methionyl-N¹-[2-(2,5-dioxo-2,5-dihydro-1H-pyrrol-1-yl)ethyl]-L-isoleucinamide tetrakis(trifluoroacetate) (90 mg) in dichloromethane (4 mL), and the resulting mixture was stirred overnight at room temperature. The mixture was concentrated under reduced pressure, and then the residue was purified by reverse phase column chromatography (solvent gradient; 5 → 50% acetonitrile/0.1% TFA aqueous solution) to obtain the title compound (57 mg). MS (ESI+): 919.4
(vii) Synthesis of N-({4-[(2-{4,7,10-tris[(carboxy-**κ**O)methyl]-1,4,7,10-tetraazacyclododecan-1-yl-**κ**⁴N¹,N⁴,N⁷,N¹⁰}acetamido-**κ**O)methyl]phenyl}carbamoyl)-L-methionyl-N¹-[2-(2,5-dioxo-2,5-dihydro-1H-pyrrol-1-yl)ethyl]-L-isoleucinamidato(3-)]gadolinium
   Gadolinium chloride (8 mg) was added to a mixture of N-{[4-({2-[4,7,10-tris(carboxymethyl)-1,4,7,10-tetraazacyclododecan-1-yl]acetamido}methyl)phenyl]carbamoyl}-L-methionyl-N¹-[2-(2,5-dioxo-2,5-dihydro-1H-pyrrol-1-yl)ethyl]-L-isoleucinamide tetrakis(trifluoroacetate) (22 mg) and water (3 mL), a 0.1 M sodium hydrogen carbonate aqueous solution was added to adjust the pH to 5 to 6, and the resulting mixture was stirred at room temperature for 1 hour. TFA (10 µL) was added to the reaction mixture, and then the solution thereof was purified by reverse phase column chromatography (solvent gradient 5 → 50% acetonitrile/0.1% TFA aqueous solution) to obtain the title compound (16 mg). MS (ESI-); 1072.1
(viii) Synthesis of conjugate No. 18
   [N-({4-[(2-{4,7,10-tris[(carboxy-**κ**O)methyl]-1,4,7,10-tetraazacyclododecan-1-yl-**κ**⁴N¹,N⁴,N⁷,N¹⁰}acetamido-**κ**O)methyl]phenyl}carbamoyl)-L-methionyl-N¹-[2-(2,5-dioxo-2,5-dihydro-1H-pyrrol-1-yl)ethyl]-L-isoleucinamidato(3-)]gadolinium (1 mg) synthesized in Example 2-18 (vii) was dissolved in DMSO (40 µL). A 0.1 M borate buffer (40 µL) was added to the resulting solution, and the pH was adjusted to 7.3 using a 0.1 M sodium carbonate aqueous solution and a 0.25 M acetate buffer.
   40 µL of the solution previously prepared was added to a 4.45 mg/mL Fab² borate buffer (160 µL), and the resulting mixture was incubated at 30°C for 2 hours. The mixture was purified using a PD-10 column, and the resulting solution was recovered using an Amicon Ultra-0.5 mL centrifugal filter. The recovered solution was washed with phosphate buffered saline 7 times, finally concentrated, and then filtered through a membrane filter to obtain a conjugate. It was confirmed by MS analysis that the conjugate was a mixture of a conjugate in which one [Gd/DOTA-NH-CH₂-(1,4-Ph)-NH-C(=O)-Met-Ile-NH-(CH₂)₂-Z₁(#N)] having a molecular weight of 1074 was bound to one Fab² having a molecular weight of 47.9 kDa, a conjugate in which two such molecules were bound thereto, and a conjugate in which three such molecules were bound thereto.

### (Example 2-66. Synthesis of ([Gd/DOTA-NH-CH₂-(1,4-Ph)-NH-C(=O)-Met-Ile-NH-(CH₂)₂-(A-4 and/or A-5)]p-Fab²))

(i) Synthesis of conjugate No. 66
   [N-({4-[(2-{4,7,10-tris[(carboxy-**κ**O)methyl]-1,4,7,10-tetraazacyclododecan-1-yl-**κ**⁴N¹,N⁴,N⁷,N¹⁰}acetamido-**κ**O)methyl]phenyl}carbamoyl)-L-methionyl-N¹-[2-(2,5-dioxo-2,5-dihydro-1H-pyrrol-1-yl)ethyl]-L-isoleucinamidato(3-)]gadolinium (1 mg) synthesized in Example 2-18 (vii) was dissolved in DMSO (40 µL). A 0.1 M borate buffer (40 µL) was added to the resulting solution, and the pH was adjusted to 6.5 using a 0.1 M sodium carbonate aqueous solution.

The solution previously prepared was added to a 4.45 mg/mL Fab² borate buffer (320 µL), and the resulting mixture was incubated at 30°C for 2 hours. 10 µL of 0.05 M EDTA was added, and the resulting mixture was incubated at 30°C for 10 minutes. Using an Amicon Ultra-15 mL centrifugal filter, the mixture was washed twice with phosphate buffered saline, and the resulting solution was recovered.

A 20 mM bis tris propane buffer solution (pH 9.5) was added to the recovered solution, diluted to a 5-fold amount, supported on a 5 mL HiTrap Q column (GE Healthcare), and allowed to stand at room temperature for 6 hours. The column was washed using a 20 mM bis tris propane buffer solution (pH 9.5) and a 1 M sodium chloride aqueous solution, and the support solution was recovered. The buffer solution was exchanged with phosphate buffered saline using an Amicon Ultra-15 mL centrifugal filter, then the solution was purified using a PD-10 column, and the resulting solution was recovered using an Amicon Ultra-15 mL centrifugal filter. The recovered solution was washed twice with phosphate buffered saline, finally concentrated, and then filtered through a membrane filter to obtain a conjugate. It was confirmed by MS analysis that the conjugate was a mixture of a conjugate in which one [Gd/DOTA-NH-CH₂-(1,4-Ph)-NH-C(=O)-Met-Ile-NH-(CH₂)₂-(A-4 and/or A-5)] having a molecular weight of 1092 was bound to one Fab² having a molecular weight of 47.9 kDa, a conjugate in which two such molecules were bound thereto, a conjugate in which three such molecules were bound thereto, and a conjugate in which four such molecules were bound thereto.

### (Example 2-67. Synthesis of ([Gd/DOTA-NH-CH₂-(1,4-Ph)-NH-C(=O)-Met-Ile-NH-(CH₂)₂-Z₁(#S)]p-Fab²))

(i) Synthesis of conjugate No. 67
   [N-({4-[(2-{4,7,10-tris[(carboxy-**κ**O)methyl]-1,4,7,10-tetraazacyclododecan-1-yl-**κ**⁴N¹,N⁴,N⁷,N¹⁰}acetamido-**κ**O)methyl]phenyl}carbamoyl)-L-methionyl-N¹-[2-(2,5-dioxo-2,5-dihydro-1H-pyrrol-1-yl)ethyl]-L-isoleucinamidato(3-)]gadolinium (2 mg) synthesized in Example 2-18 (vii) was dissolved in DMSO (80 µL), a 0.1 M borate buffer (80 µL) was added, and then the pH was adjusted to 7.3 using a 0.1 M sodium carbonate aqueous solution.

A 2 mg/mL 2-IT solution (10 µL) prepared using a 0.1 M borate buffer was added to a 4.45 mg/mL Fab² borate buffer (320 µL), and the resulting mixture was incubated at 37°C for 40 minutes. Excess 2-IT was washed with phosphate buffered saline using an Amicon Ultra-15 mL centrifugal filter and concentrated to 200 µL, then the solution previously prepared was added, and the resulting mixture was incubated at 30°C for 2 hours. 0.05 M EDTA (10 µL) was added, the resulting mixture was incubated at 30°C for 10 minutes, then the mixture was purified using a PD-10 column, and the resulting solution was recovered using an Amicon Ultra-15 mL centrifugal filter. The recovered solution was washed twice with phosphate buffered saline, finally concentrated, and then filtered through a membrane filter to obtain a conjugate.

It was confirmed by MS analysis that the conjugate was a mixture of a conjugate in which one [Gd/DOTA-NH-CH₂-(1,4-Ph)-NH-C(=O)-Met-Ile-NH-(CH₂)₂-Z₁(#S)] having a molecular weight of 1175 was bound to one Fab² having a molecular weight of 47.9 kDa, a conjugate in which two such molecules were bound thereto, and a conjugate in which three such molecules were bound thereto.

### (Example 2-68. Synthesis of ([Gd/DOTA-NH-CH₂-(1,4-Ph)-NH-C(=O)-Met-Ile-NH-(CH₂)₂-(A-3)]p-Fab²))

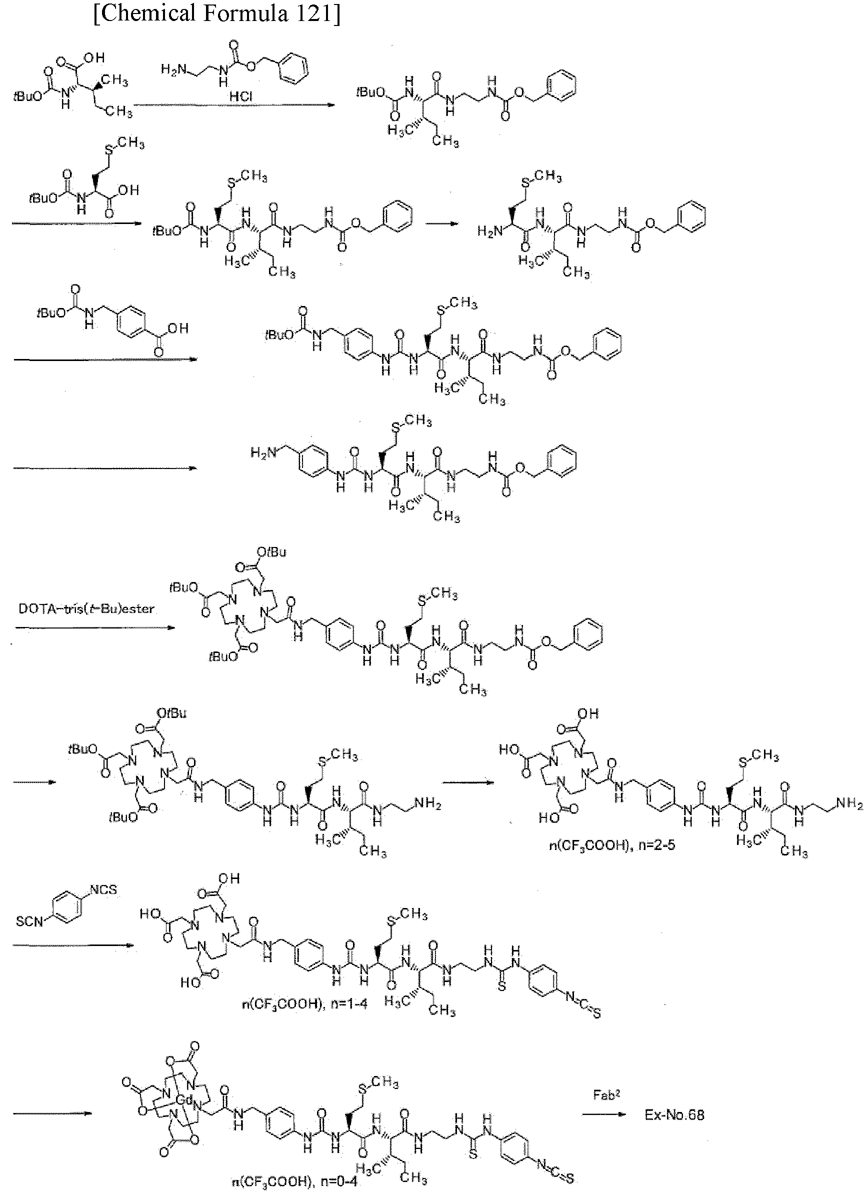

(I) Synthesis of N¹-(2-{[(benzyloxy)carbonyl]amino}ethyl)-N²-(tert-butoxycarbonyl)-L-isoleucinamide
   DIPEA (6.6 mL) and benzyl (2-aminoethyl) carbamate monohydrochloride (3.29 g) were sequentially added to a mixed solution of N-(tert-butoxycarbonyl)-L-isoleucine (3.00 g) and HATU (5.92 g) in dichloromethane (30 ml), and the resulting mixture was stirred at room temperature for 1 hour. A saturated sodium hydrogen carbonate aqueous solution was added to the reaction mixture, and the resulting mixture was extracted twice with dichloromethane. The combined organic layers were washed with a saturated sodium chloride aqueous solution, and then dried over anhydrous sodium sulfate, filtered, and then concentrated. 200 mL of an ethyl acetate/dichloromethane (1:2) solution was added to the residue, and then the resulting mixture was concentrated to give a 100 mL solution, which was then ice-cooled. The precipitated solid was collected by filtration and washed with an ethyl acetate/dichloromethane (1:1) solution to obtain a solid. The filtrate was concentrated again and then ice-cooled, and the precipitated solid was collected by filtration and washed with an ethyl acetate/dichloromethane (1:1) solution to obtain a solid. The obtained solids were combined and dried under reduced pressure to obtain the title compound (4.10 g). MS (ESI+): 408.3
(ii) Synthesis of N-(tert-butoxycarbonyl)-L-methionyl-N¹-(2-{[(benzyloxy)carbonyl]amino}ethyl)-L-isoleucinamide
   TFA (7.51 mL) was added to a solution of N¹-(2-{[(benzyloxy)carbonyl]amino}ethyl)-N²-(tert-butoxycarbonyl)-L-isoleucinamide (2.00 g) in dichloromethane (10 mL) under ice cooling, and the resulting mixture was stirred at the same temperature for 1 hour. The mixture was concentrated under reduced pressure, then N-(tert-butoxycarbonyl)-L-methionine (1.35 g), DIPEA (4.2 mL), dichloromethane (20 mL), and HATU (2.24 g) were added to this residue, and the resulting mixture was stirred at room temperature for 1 hour. The precipitated solid was collected by filtration, washed with dichloromethane and methanol, and then dried under reduced pressure to obtain the title compound (1.58 g). MS (ESI+): 539.4
(iii) Synthesis of L-methionyl-N¹-(2-{[(benzyloxy)carbonyl]amino}ethyl)-L-isoleucinamide
   TFA (3 ml) was added dropwise to a solution of N-(tert-butoxycarbonyl)-L-methionyl-N¹-(2-{[(benzyloxy)carbonyl]amino}ethyl)-L-isoleucinamide (1.05 g) in dichloromethane (6 ml) under ice cooling. The resulting mixture was stirred at the same temperature for 2 hours. The mixture was concentrated under reduced pressure, dichloromethane and a saturated sodium hydrogen carbonate aqueous solution were added, and the resulting mixture was extracted twice with dichloromethane. The combined organic layers were washed with a saturated sodium chloride aqueous solution, and then dried over anhydrous sodium sulfate, filtered, and then concentrated to obtain a crude product of the title compound (690 mg). MS (ESI+): 439.4
(iv) Synthesis of N-[(4-{[(tert-butoxycarbonyl)amino]methyl}phenyl)carbamoyl]-L-methionyl-N¹-(2-{[(benzyloxy)carbonyl]amino}ethyl)-L-isoleucinamide
   diphenyl Phosphoraziate (680 µL) was added to a mixed solution of 4-{[(tert-butoxycarbonyl)amino]methyl}benzoic acid (395 mg), TEA (660 µL), and toluene (20 mL) under an argon atmosphere, and the resulting mixture was stirred at room temperature for 1 hour and then stirred at a bath temperature of 100°C for 2 hours. The reaction solution was allowed to cool to room temperature, a solution of L-methionyl-N¹-(2-{[(benzyloxy)carbonyl]amino}ethyl)-L-isoleucinamide (690 mg) in THF (7 mL) was added, and the resulting mixture was stirred at room temperature for 3 hours. The reaction solution was concentrated under reduced pressure, a sodium hydrogen carbonate aqueous solution and ethyl acetate were added, and the resulting solid was filtered, washed with ethyl acetate, and then dried under reduced pressure to obtain the title compound (790 mg). MS (ESI+): 687.5 (v) Synthesis of N-{[4-(aminomethyl)phenyl]carbamoyl}-L-methionyl-N¹-(2-{[(benzyloxy)carbonyl]amino}ethyl)-L-isoleucinamide
   TFA (2 ml) was added dropwise to a solution of N-[(4-{[(tert-butoxycarbonyl)amino]methyl}phenyl)carbamoyl]-L-methionyl-N¹-(2-{[(benzyloxy)carbonyl]amino}ethyl)-L-isoleucinamide (790 mg) in dichloromethane (3 ml) under ice cooling. The resulting mixture was stirred at the same temperature for 1 hour. The mixture was concentrated under reduced pressure, and a saturated sodium hydrogen carbonate aqueous solution was added. The precipitated solid was collected by filtration, washed with water, and then dried under reduced pressure to obtain a crude product of the title compound (720 mg). MS (ESI+): 587.6
(vi) Synthesis of N-{[4-({2-[4,7,10-tris(2-tert-butoxy-2-oxoethyl)-1,4,7,10-tetraazacyclododecan-1-yl]acetamido}methyl)phenyl]carbamoyl}-L-methionyl-N¹-(2-{[(benzyloxy)carbonyl]amino}ethyl)-L-isoleucinamide
   HATU (280 mg) was added to a mixed solution of N-{[4-(aminomethyl)phenyl]carbamoyl}-L-methionyl-N¹-(2-{[(benzyloxy)carbonyl]amino}ethyl)-L-isoleucinamide (360 mg), DOTA-tris(t-Bu) ester (386 mg), and DIPEA (320 µL) in DMAc (2 mL), and the resulting mixture was stirred at room temperature for 2 hours. The reaction mixture was purified by silica gel column chromatography (solvent gradient; 0 → 20% methanol/chloroform) to obtain the title compound (683 mg). MS (ESI+); 1141.9
(vii) Synthesis of N-{[4-({2-[4,7,10-tris(2-tert-butoxy-2-oxoethyl)-1,4,7,10-tetraazacyclododecan-1-yl]acetamido}methyl)phenyl]carbamoyl}-L-methionyl-N¹-(2-aminoethyl)-L-isoleucinamide
   A mixture of N-{[4-({2-[4,7,10-tris(2-tert-butoxy-2-oxoethyl)-1,4,7,10-tetraazacyclododecan-1-yl]acetamido}methyl)phenyl]carbamoyl}-L-methionyl-N¹-(2-{[(benzyloxy)carbonyl]amino}ethyl)-L-isoleucinamide (683 mg), 10% palladium on carbon (50% wet with water, 382 mg), and methanol (10 mL) was stirred under a hydrogen atmosphere (1 atm) at room temperature for 18 hours. The mixture was filtered to remove the insoluble matter and then concentrated. 10% palladium on carbon (water content of 50%, 382 mg) and methanol (10 mL) were added to the residue, and the resulting mixture was stirred under a hydrogen atmosphere (1 atm) at room temperature for 2 hours. The mixture was filtered to remove the insoluble matter and then concentrated to obtain the title compound (417 mg). MS (ESI+); 1007.7
(viii) Synthesis of N-{[4-({2-[4,7,10-tris(carboxymethyl)-1,4,7,10-tetraazacyclododecan-1-yl]acetamido}methyl)phenyl]carbamoyl}-L-methionyl-N¹-(2-aminoethyl)-L-isoleucinamide pentakis(trifluoroacetate)
   A mixed solution of N-{[4-({2-[4,7,10-tris(2-tert-butoxy-2-oxoethyl)-1,4,7,10-tetraazacyclododecan-1-yl]acetamidolmethyl)phenyl]carbamoyl}-L-methionyl-N¹-(2-aminoethyl)-L-isoleucinamide (417 mg), water (210 µL), tri(propan-2-yl)silane (210 µL), and TFA (8 mL) was stirred at room temperature for 3 hours. The mixture was concentrated under reduced pressure, and then the residue was purified by reverse phase column chromatography (solvent gradient; 0 → 33% acetonitrile/0.1% TFA aqueous solution) to obtain the title compound (255 mg). MS (ESI+); 839.7
(ix) Synthesis of N-{[4-({2-[4,7,10-tris(carboxymethyl)-1,4,7,10-tetraazacyclododecan-1-yl]acetamido}methyl)phenyl]carbamoyl}-L-methionyl-N¹-(2-{[(4-isothiocyanatophenyl)carbamothioyl]amino}ethyl)-L-isoleucinamide tetrakis(trifluoroacetate)
   TEA (200 µL) was added to a solution of N-{[4-({2-[4,7,10-tris(carboxymethyl)-1,4,7,10-tetraazacyclododecan-1-yl]acetamido}methyl)phenyl]carbamoyl}-L-methionyl-N¹-(2-aminoethyl)-L-isoleucinamide pentakis(trifluoroacetate) (255 mg) in DMAc (2 mL) under ice cooling, and the resulting mixture was stirred at the same temperature for 10 minutes. A solution of 1,4-diisothiocyanatobenzene (174 mg) in DMAc (2 mL) was added to the solution, and the resulting mixture was stirred at the same temperature for 1 hour. The reaction solution was purified by reverse phase column chromatography (solvent gradient; 0 → 50% acetonitrile/0.1% TFA aqueous solution) to obtain the title compound (136 mg). MS (ESI+); 1031.4
(x) Synthesis of N-({4-[(2-{4,7,10-tris[(carboxy-**κ**O)methyl]-1,4,7,10-tetraazacyclododecan-1-yl-**κ**⁴N¹,N⁴,N⁷,N¹⁰}acetamido-**κ**O)methyl]phenyl}carbamoyl)-L-methionyl-N¹-[2-{[(4-isothiocyanatophenyl)carbamothioyl]amino}ethyl]-L-isoleucinamidato(3-)]gadolinium
   Gadolinium chloride (10 mg) and a 0.1 M sodium hydrogen carbonate aqueous solution (1.7 mL) were added to a mixture of N-{[4-({2-[4,7,10-tris(carboxymethyl)-1,4,7,10-tetraazacyclododecan-1-yl]acetamido}methyl)phenyl]carbamoyl}-L-methionyl-N¹-[2-{[(4-isothiocyanatophenyl)carbamothioyl]amino}ethyl]-L-isoleucinamide tetrakis(trifluoroacetate) (36 mg) and water (1.8 mL) to adjust the pH to 5.4, and the resulting mixture was stirred at room temperature for 30 minutes. The reaction mixture was purified by reverse phase column chromatography (solvent gradient; 0 → 50% acetonitrile/0.1% TFA aqueous solution) to obtain the title compound (33.0 mg). MS (ESI-); 1184.3
(xi) Synthesis of conjugate No. 68
   [N-({4-[(2-{4,7,10-tris[(carboxy-**κ**O)methyl]-1,4,7,10-tetraazacyclododecan-1-yl-**κ**⁴N¹,N⁴,N⁷,N¹⁰}acetamido-**κ**O)methyl]phenyl}carbamoyl)-L-methionyl-N¹-[2-{[(4-isothiocyanatophenyl)carbamothioyl]amino}ethyl]-L-isoleucinamidato(3-)]gadolinium (1 mg) synthesized in Example 2-68 (x) was dissolved in DMSO (310 µL).

30 µL of the solution previously prepared was added to a 4.45 mg/mL Fab² phosphate buffered saline solution (320 µL), the pH was adjusted to about 9.0 using a 0.1 M sodium carbonate aqueous solution, and the resulting mixture was incubated at 37°C for 24 hours. The mixture was purified using a PD-10 column, and the resulting solution was recovered using an Amicon Ultra-15 mL centrifugal filter. The recovered solution was washed twice with phosphate buffered saline, finally concentrated, and then filtered through a membrane filter to obtain a conjugate. It was confirmed by MS analysis that the conjugate was a mixture of a conjugate in which one [Gd/DOTA-NH-CH₂-(1,4-Ph)-NH-C(=O)-Met-Ile-NH-(CH₂)₂-(A-3)] having a molecular weight of 1187 was bound to one Fab² having a molecular weight of 47.9 kDa, a conjugate in which two such molecules were bound thereto, and a conjugate in which three such molecules were bound thereto.

### (Example 2-69. Synthesis of ([Gd/DOTA-[2,5-(1,2,3,4-tetrahydroisoquinoline)]-C(=O)-Met-Gly-Lys^{∗}-C(=S)-NH-(1,4-Ph)-NH-C(=S)]p-Fab²))

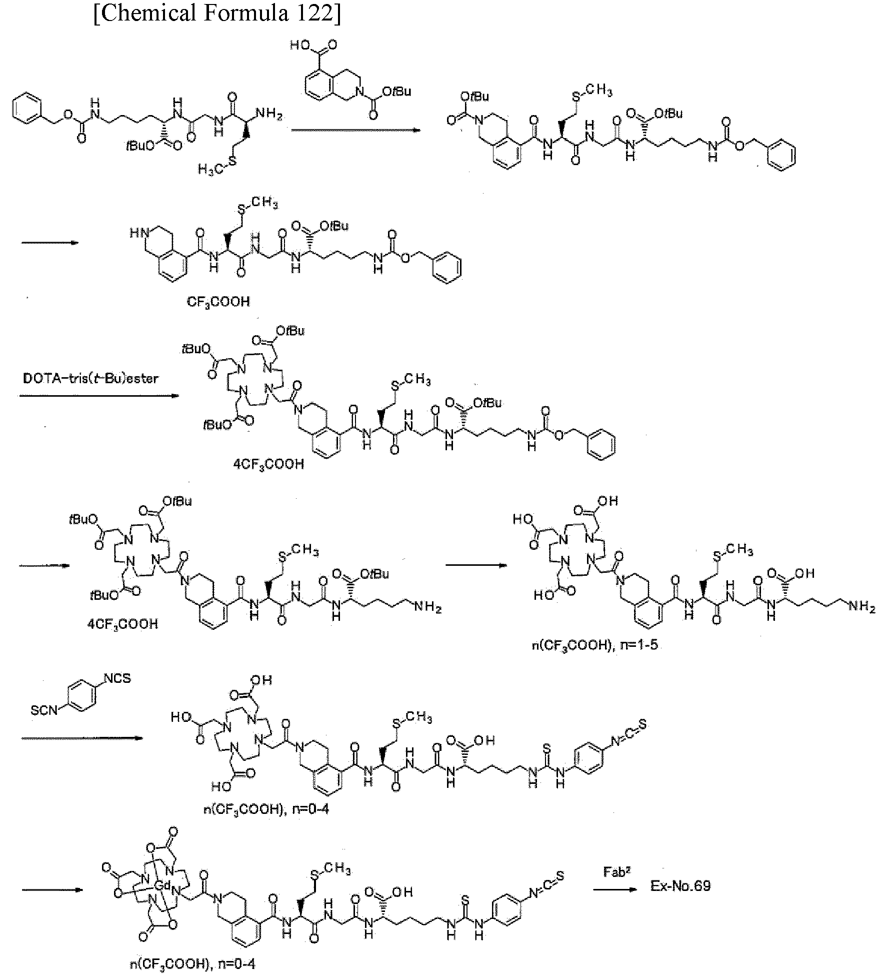

(i) Synthesis of tert-butyl N-[2-(tert-butoxycarbonyl)-1,2,3,4-tetrahydroisoquinoline-5-carbonyl]-L-methionylglycyl-N⁶-[(benzyloxy)carbonyl]-L-lysinate
   TEA (0.24 mL) and HATU (241 mg) were added to a mixed solution of tert-butyl L-methionylglycyl-N⁶-[(benzyloxy)carbonyl]-L-lysinate (303 mg) and 2-(tert-butoxycarbonyl)-1,2,3,4-tetrahydroisoquinoline-5-carboxylic acid (160 mg) in dichloromethane (10 mL), and the resulting mixture was stirred at room temperature for 1 hour. The reaction mixture was concentrated under reduced pressure, and then the residue was purified by silica gel column chromatography (solvent gradient; 0 → 4% methanol/chloroform) to obtain the title compound (191 mg). MS (ESI+); 784.4
(ii) Synthesis of tert-butyl N-(1,2,3,4-tetrahydroisoquinoline-5-carbonyl)-L-methionylglycyl-N⁶-[(benzyloxy)carbonyl]-L-lysinate mono(trifluoroacetate)
   TFA (1 ml) was added dropwise to a solution of tert-butyl N-[2-(tert-butoxycarbonyl)-1,2,3,4-tetrahydroisoquinoline-5-carbonyl]-L-methionylglycyl-N⁶-[(benzyloxy)carbonyl]-L-lysinate (190 mg) in dichloromethane (3 ml) under ice cooling. The resulting mixture was stirred at the same temperature for 1 hour and then concentrated under reduced pressure to obtain a crude product of the title compound (262 mg). MS (ESI+): 684.5
(iii) Synthesis of tert-butyl N-(2-{[4,7,10-tris(2-tert-butoxy-2-oxoethyl)-1,4,7,10-tetraazacyclododecan-1-yl]acetyl}-1,2,3,4-tetrahydroisoquinoline-5-carbonyl)-L-methionylglycyl-N⁶-[(benzyloxy)carbonyl]-L-lysinate tetrakis(trifluoroacetate)
   HATU (184 mg) and DIPEA (124 µL) were added to a solution of DOTA-tris(t-Bu) ester (138 mg) in DMAc (1 mL), and the resulting mixture was stirred at room temperature for 5 minutes. A solution of tert-butyl N-(1,2,3,4-tetrahydroisoquinoline-5-carbonyl)-L-methionylglycyl-N⁶-[(benzyloxy)carbonyl]-L-lysinate mono(trifluoroacetate) (261 mg) in DMAc (1 mL) was added to the reaction solution, and the resulting mixture was stirred at room temperature for 2 hours. The reaction mixture was diluted with a 1% TFA aqueous solution (about 1 ml), and then the resulting solution was purified by reverse phase column chromatography (solvent gradient; 20 → 100% acetonitrile/0.1% TFA aqueous solution) to obtain the title compound (231 mg). MS (ESI+); 1260.7 [M+Na]+
(iv) Synthesis of tert-butyl N-(2-{[4,7,10-tris(2-tert-butoxy-2-oxoethyl)-1,4,7,10-tetraazacyclododecan-1-yl]acetyl}-1,2,3,4-tetrahydroisoquinoline-5-carbonyl)-L-methionylglycyl-L-lysinate tetrakis(trifluoroacetate)
   A mixture of tert-butyl N-(2-{[4,7,10-tris(2-tert-butoxy-2-oxoethyl)-1,4,7,10-tetraazacyclododecan-1-yl]acetyl}-1,2,3,4-tetrahydroisoquinoline-5-carbonyl)-L-methionylglycyl-N⁶-[(benzyloxy)carbonyl]-L-lysinate tetrakis(trifluoroacetate) (230 mg), 10% palladium on carbon (50% wet with water, 300 mg), and methanol (10 mL) was stirred at room temperature for 10 minutes. The mixture was filtered to remove the insoluble matter, and then the filtrate was concentrated. 10% palladium on carbon (50% wet with water, 300 mg) and methanol (10 mL) were added to the residue, and then the resulting mixture was stirred under a hydrogen atmosphere (3 atm) at room temperature for 18 hours. The mixture was filtered to remove the insoluble matter and then concentrated to obtain the title compound (152 mg). MS (ESI+); 1104.5
(v) Synthesis of N-(2-{[4,7,10-tris(carboxymethyl)-1,4,7,10-tetraazacyclododecan-1-yl]acetyl}-1,2,3,4-tetrahydroisoquinoline-5-carbonyl)-L-methionylglycyl-L-lysine pentakis(trifluoroacetate)
   TFA (1.5 mL) was added to a solution of tert-butyl N-(2-{[4,7,10-tris(2-tert-butoxy-2-oxoethyl)-1,4,7,10-tetraazacyclododecan-1-yl]acetyl}-1,2,3,4-tetrahydroisoquinoline-5-carbonyl)-L-methionylglycyl-L-lysinate tetrakis(trifluoroacetate) (152 mg) in dichloromethane (1 mL), and the resulting mixture was stirred at room temperature for 4 hours. The mixture was concentrated under reduced pressure, and then the residue was purified by reverse phase column chromatography (solvent gradient; 0 → 33% acetonitrile/0.1% TFA aqueous solution) to obtain the title compound (76 mg). MS (ESI+); 880.4
(vi) Synthesis of N-(2-{[4,7,10-tris(carboxymethyl)-1,4,7,10-tetraazacyclododecan-1-yl]acetyl}-1,2,3,4-tetrahydroisoquinoline-5-carbonyl)-L-methionylglycyl-N⁶-[(4-isothiocyanatophenyl)carbamothioyl]-L-lysine tetrakis(trifluoroacetate)
   TFA (60 µL) was added to a solution of N-(2-{[4,7,10-tris(carboxymethyl)-1,4,7,10-tetraazacyclododecan-1-yl]acetyl}-1,2,3,4-tetrahydroisoquinoline-5-carbonyl)-L-methionylglycyl-L-lysine pentakis(trifluoroacetate) (76 mg) and 1,4-diisothiocyanatobenzene (50 mg) in DMAc (1 mL) under ice cooling, and the resulting mixture was stirred at room temperature for 1 hour. The reaction solution was purified by reverse phase column chromatography (solvent gradient; 0 → 50% acetonitrile/0.1% TFA aqueous solution) to obtain the title compound (46 mg). MS (ESI-); 1070.6
(vii) Synthesis of {N-[2-({4,7,10-tris[(carboxy-κO)methyl]-1,4,7,10-tetraazacyclododecan-1-yl-κ⁴N¹,N⁴,N⁷,N¹⁰}acetyl-κO)-1,2,3,4-tetrahydroisoquinoline-5-carbonyl]-L-methionylglycyl-N⁶-[(4-isothiocyanatophenyl)carbamothioyl]-L-lysinato(3-)}gadolinium
   Gadolinium chloride (8 mg) and a 0.1 M sodium hydrogen carbonate aqueous solution (700 µL) were added to a mixture of N-(2-{[4,7,10-tris(carboxymethyl)-1,4,7,10-tetraazacyclododecan-1-yl]acetyl}-1,2,3,4-tetrahydroisoquinoline-5-carbonyl)-L-methionylglycyl-N⁶-[(4-isothiocyanatophenyl)carbamothioyl]-L-lysine tetrakis(trifluoroacetate) (16 mg) and water (800 µL), and the resulting mixture was stirred at room temperature for 30 minutes. The reaction mixture was purified by reverse phase column chromatography (solvent gradient; 0 → 40% acetonitrile/0.1% TFA aqueous solution) to obtain the title compound (7.4 mg). MS (ESI-); 1225.3
(viii) Synthesis of conjugate No. 69
   {N-[2-({4,7,10-tris[(carboxy-κO)methyl]-1,4,7,10-tetraazacyclododecan-1-yl-κ⁴N¹,N⁴,N⁷,N¹⁰}acetyl-κO)-1,2,3,4-tetrahydroisoquinoline-5-carbonyl]-L-methionylglycyl-N⁶-[(4-isothiocyanatophenyl)carbamothioyl]-L-lysinato(3-)}gadolinium (1 mg) synthesized in Example 2-69 (vii) was dissolved in DMSO (310 µL).

30 µL of the solution previously prepared was added to a 4.45 mg/mL Fab² phosphate buffered saline solution (320 µL), the pH was adjusted to about 9.0 using a 0.1 M sodium carbonate aqueous solution, and the resulting mixture was incubated at 37°C for 24 hours. The mixture was purified using a PD-10 column, and the resulting solution was recovered using an Amicon Ultra-15 mL centrifugal filter. The recovered solution was washed twice with phosphate buffered saline, finally concentrated, and then filtered through a membrane filter to obtain a conjugate. It was confirmed by MS analysis that the conjugate was a mixture of a conjugate in which one [Gd/DOTA-[2,5-(1,2,3,4-tetrahydroisoquinoline)]-C(=O)-Met-Gly-Lys^{∗}-C(=S)-NH-(1,4-Ph)-NH-C(=S)] having a molecular weight of 1228 was bound to one Fab² having a molecular weight of 47.9 kDa, a conjugate in which two such molecules were bound thereto, a conjugate in which three such molecules were bound thereto, a conjugate in which four such molecules were bound thereto, and a conjugate in which five such molecules were bound thereto.

The conjugates obtained in the above Production Examples are shown in Tables 1 and 2.

**[Table 1]**

| (Y-S₁-X) ₚ-Fab² | | | |
|---|---|---|---|
| Ex-No | Y | S₁ | X |
| 11 | Gd/DOTA | -NH-CH₂-(1, 3-Ph) -C (=O)- | -Asp-Gly-Lys∗-Z₂(#N)- |
| 12 | Gd/4arm-DOTA | -CH₂-(1,4-Ph)-NH-C(=S)- | -Gly-Phe-Lys∗-Z₂(#N)- |
| 13 | Gd/DOTA | -NH-CH₂-(1,3-Ph)-C(=O)- | -Met-Gly-Lys∗-Z₂(#N)- |
| 15 | Gd/DOTA | -NH-(CH₂CH₂O)₃-CH₂-C(=O)-NH-CH₂-( 1, 3-Ph) -C (=0) - | -Gly-Lys∗-Z₂(#N)- |
| 18 | Gd/DOTA | -NH-CH₂-(1, 4-Ph) -NH-C (=O)- | -Met-Ile-NH- (CH₂)₂-Z₁( #N)- |
| 24 | Gd/DOTA | -NH-CH₂-(1,3-Ph)-C(=O)- | -Met-Gly-Lys∗-Z₂(#S)- |
| 29 | Gd/DOTA | -NH-(CH₂CH₂O)₃-CH₂-C(=O)-NH-CH₂-( 1,3-Ph)-C(=O)- | -Met-Gly-Lys∗-Z₂(#N) - |
| 33 | Gd/DOTA | 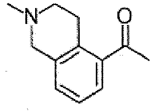 | -Met-Gly-Lys∗-Z₂(#N)- |
| 35 | Gd/DOTA | -NH-(CH₂CH₂O)₃-CH₂-C(=O)-NH-CH₂( 1,3-Ph)-C (=O)- | -Met-Gly-Lys∗-Z₂(#S)- |
| 40 | Gd/4arm-DOTA | -CH₂-(1,4-Ph)-NH-C(=S)-NH-CH₂-(1 ,3-Ph)-C(=O)- | -Met-Gly-LyS∗-Z₂(#S)- |
| 47 | Gd/DOTA | - | - |

**[Table 2]**

| (Y-S₁-X) ₚ-Fab² | | | |
|---|---|---|---|
| Ex-No | Y | S₁ | X |
| 48 | Gd/4arm-DOTA | -CH₂-(1, 4-Ph) -NH-C(=S)- | - |
| 60 | DFO | -C(=O)-CH₂O-(1, 3-Ph) -C (=O)- | -Gly-Tyr∗-CH₂-C(=O)-N H-(CH₂)₂-Z₁(#S)- |
| 61 | DFO | -C(=O)-(CH₂CH₂O)₄-(1,3-Ph)-C(=O) | -Gly-Lys∗-Z₂(#S)- |
| 66 | Gd/DOTA | -NH-CH₂-(1,4-Ph)-NH-C(=O)- | -Met-Ile-NH-(CH₂)₂-(A-4 and/or A-5)- |
| 67 | Gd/DOTA | -NH-CH₂-(1, 4-Ph)-NH-C(=O)- | -Met-Ile-NH- (CH₂)₂-Z₁( #S)- |
| 68 | Gd/DOTA | -NH-CH₂-(1,4-Ph)-NH-C(=O)- | -Met-Ile-NH-(CH₂)₂-(A-3)- |
| 69 | Gd/DOTA | 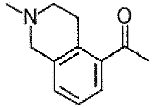 | -Met-Gly-Lys∗-Z₃- |

In the above tables and Tables 15 to 18 given later, - represents a bond, and symbols (a) to (q) in the S₁ column represent spacers represented by the following formulas (a) to (q), respectively.

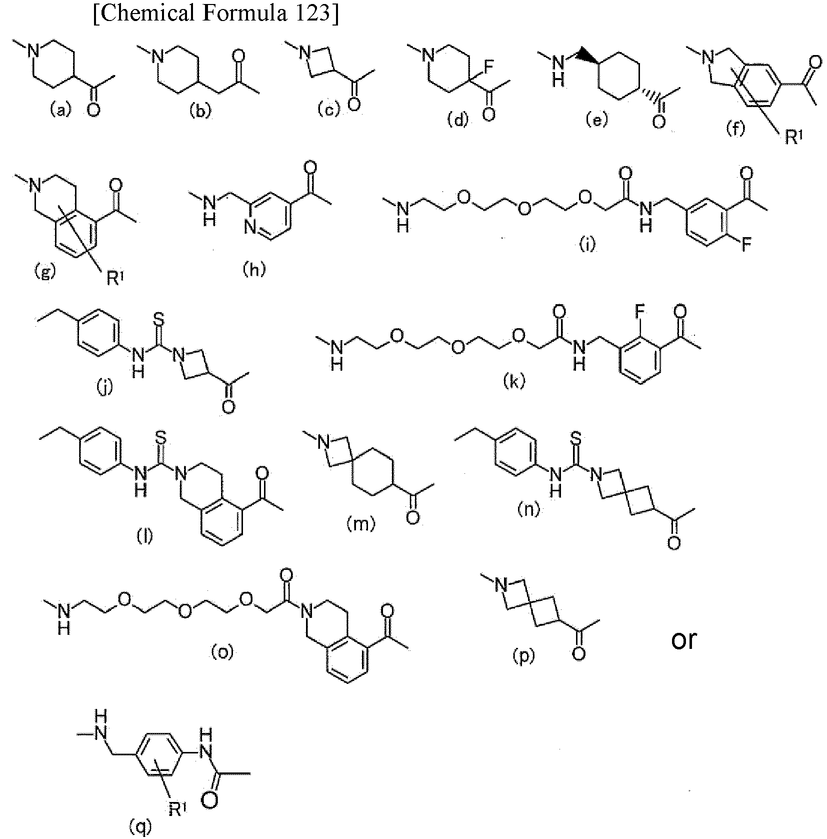

The compounds of Production Example Nos. A1 to A43 and B1 to B4 in Tables 3 to 14 were synthesized using the same methods as in the above Production Examples or methods known to those skilled in the art, and these compounds were used to obtain the conjugates shown in Tables 15 to 18 below.

**[Table 3]**

| SNo | Str | MS |
|---|---|---|
| A1 | 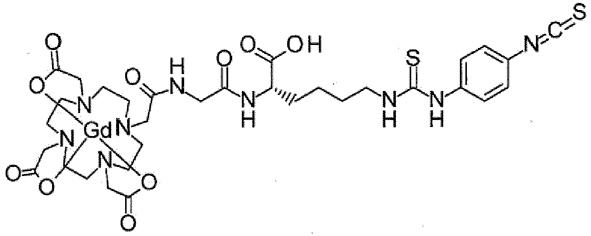 | ESI+; 937. 8 |
| A2 | 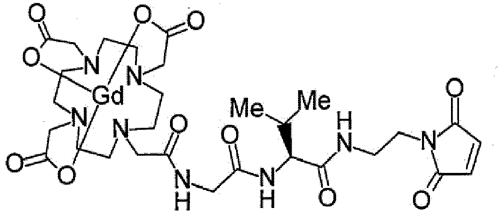 | ESI+; 838.4 |
| A3 | 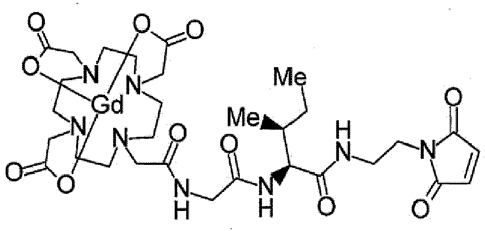 | ESI+; 852.4 |
| A4 | 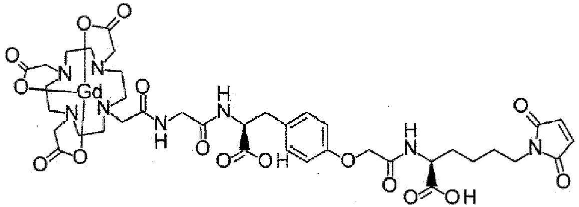 | ESI+; 1046. 1 |

**[Table 4]**

| SNo | Str | MS |
|---|---|---|
| A5 | 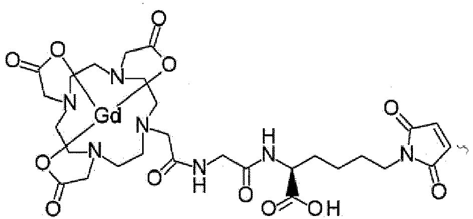 | ESI+; 825. 1 |
| A6 | 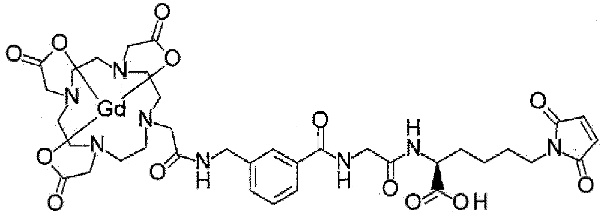 | ESI+; 958.4 |
| A7 | 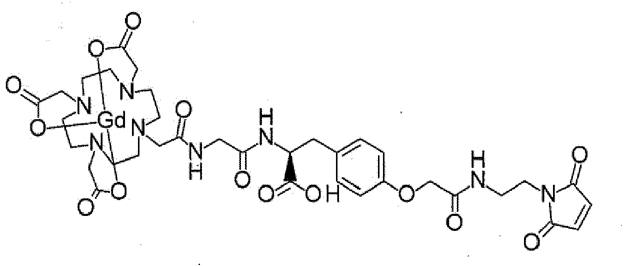 | ESI+; 959.8 |
| A8 | 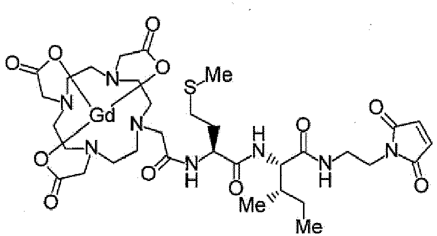 | ESI+; 926.2 |

**[Table 5]**

| SNo | Str | MS |
|---|---|---|
| A9 | 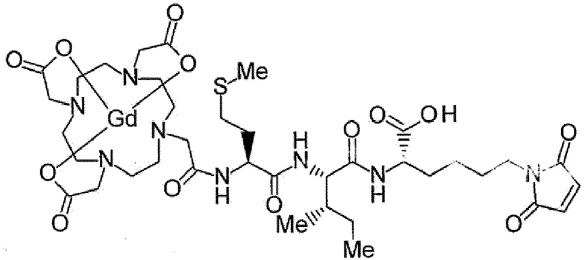 | ESI-; 1010.2 |
| A10 | 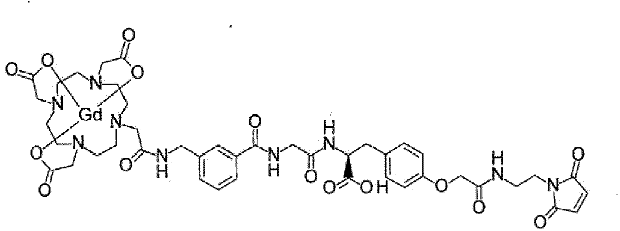 | ESI-; 1090. 7 |
| A11 | 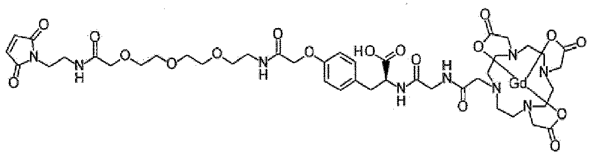 | APCI/ESI+; 1149.9 |
| A12 | 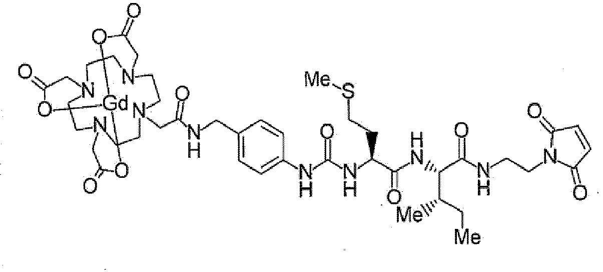 | ESI-; 1072. 1 |

**[Table 6]**

| SNo | Str | MS |
|---|---|---|
| A13 | 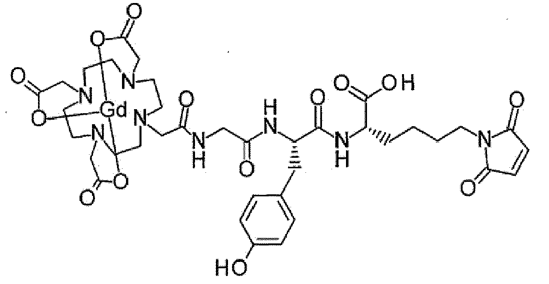 | ESI+; 988.4 |
| A14 | 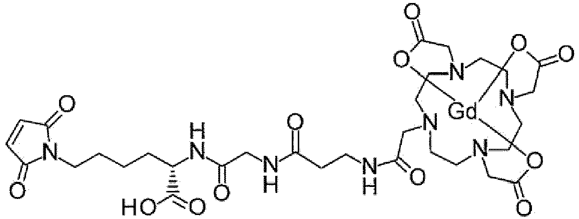 | ESI+; 896.1 |
| A15 | 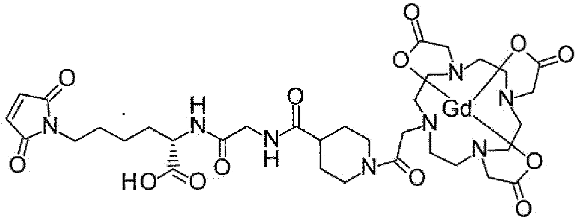 | ESI+; 936.3 |
| A16 | 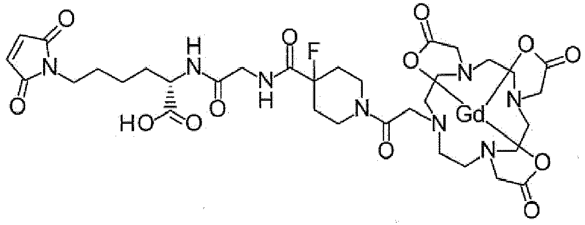 | ESI+; 954. 3 |

**[Table 7]**

| SNo | Str | MS |
|---|---|---|
| A17 | 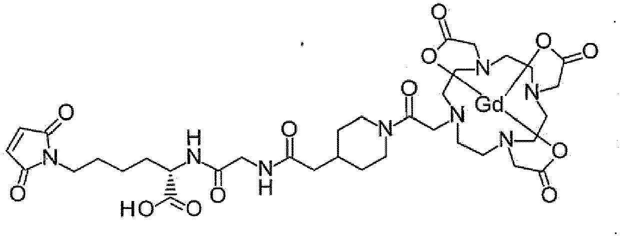 | ESI+; 950.3 |
| A18 | 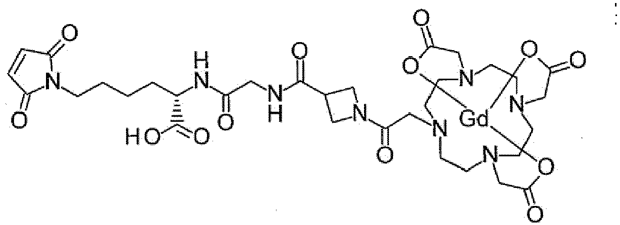 | ESI+; 908. 2 |
| A19 | 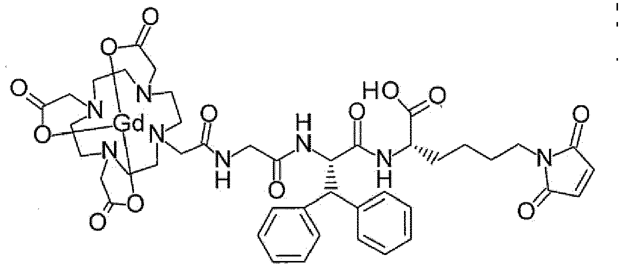 | ESI+; 1048.4 |
| A20 | 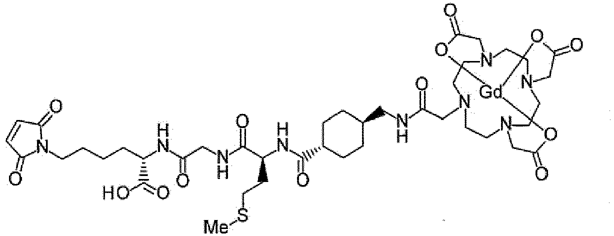 | ESI+; 1095.3 |

**[Table 8]**

| SNo | Str | MS |
|---|---|---|
| A21 | 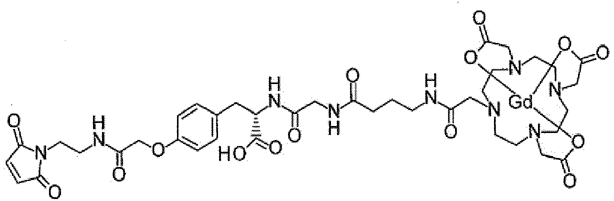 | APCI/ESI+; 1045.1 |
| A22 | 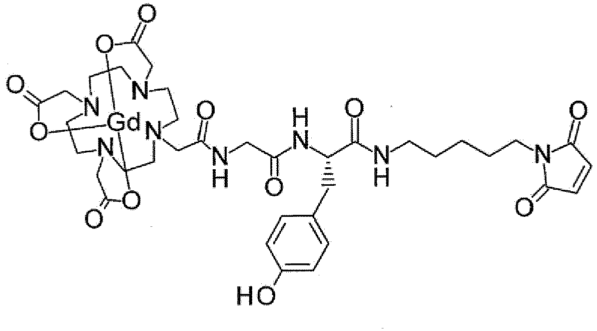 | ESI+; 944.2 |
| A23 | 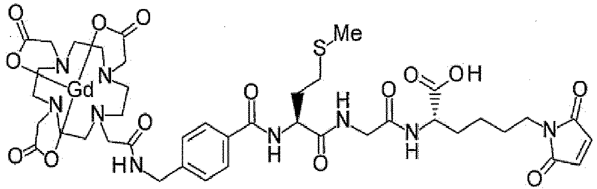 | ESI+; 1089.1 |
| A24 | 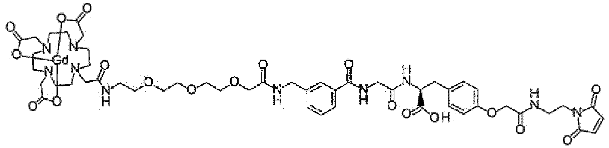 | ESI-; 1280 .4 |

**[Table 9]**

| SNo | Str | MS |
|---|---|---|
| A25 | 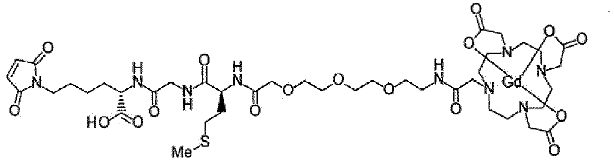 | ESI-; 1143 .5 |
| A26 | 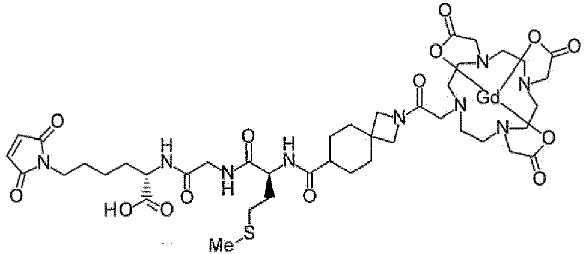 | ESI+; 1107.3 |
| A27 | 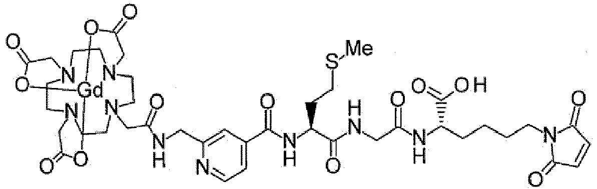 | ESI+; 1090.4 |
| A28 | 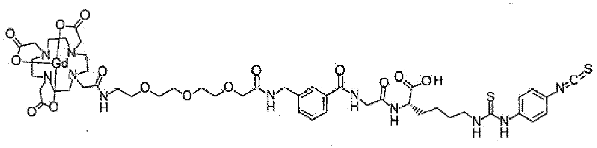 | ESI+; 1259.5 |

**[Table 10]**

| SNo | Str | MS |
|---|---|---|
| A29 | 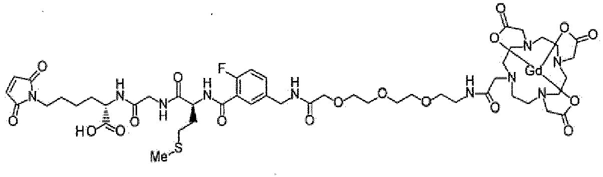 | ESI+; 1296.5 |
| A30 | 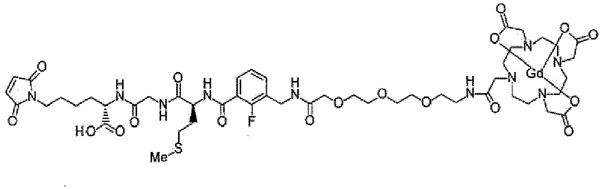 | ESI-; 1294.2 |
| A31 | 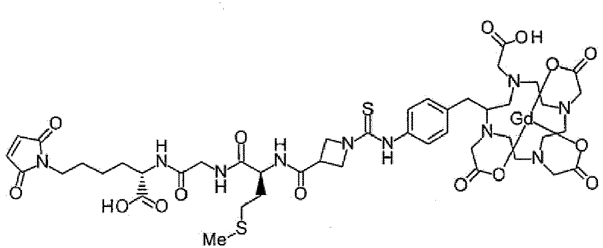 | ESI-; 1201.6 |
| A32 | 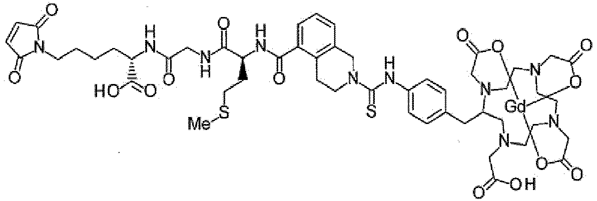 | ESI-; 1277.7 |

**[Table 11]**

| SNo | Str | MS |
|---|---|---|
| A33 | 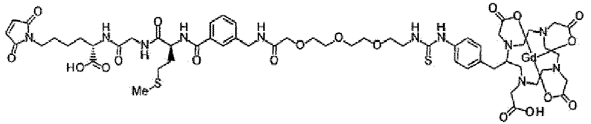 | APCI/ESI-; 1441.1 |
| A34 | 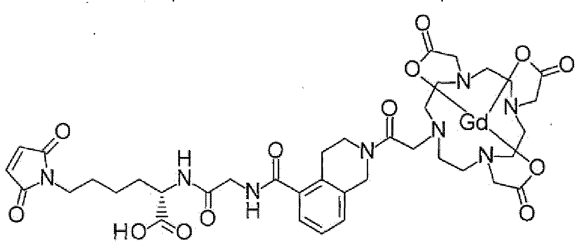 | ESI+; 984.1 |
| A35 | 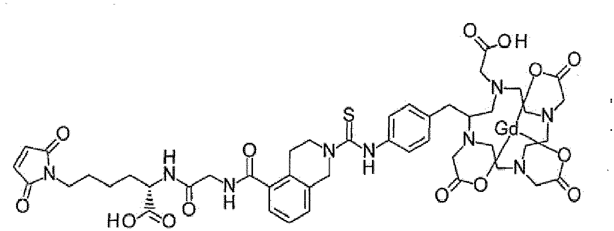 | ESI-; 1147.2 |
| A36 | 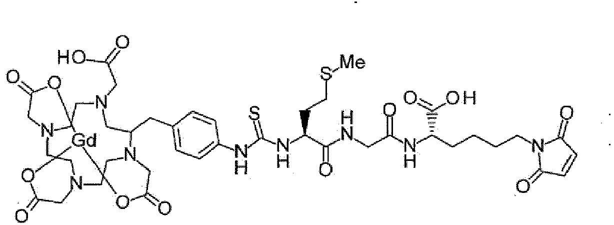 | ESI+; 1120.9 |

**[Table 12]**

| SNo | Str | MS |
|---|---|---|
| A37 | 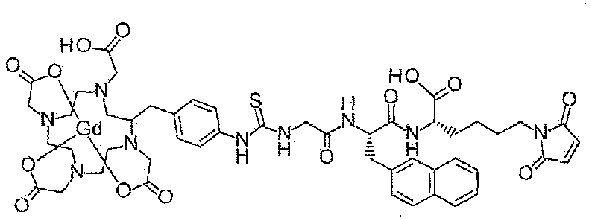 | ESI+; 1187.9 |
| A38 | 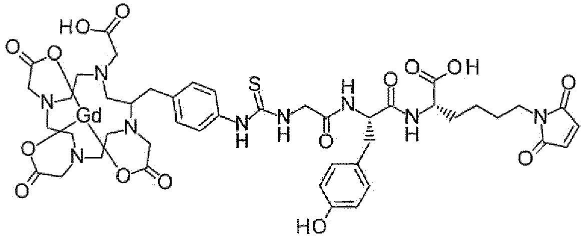 | ESI+; 1153.5 |
| A39 | 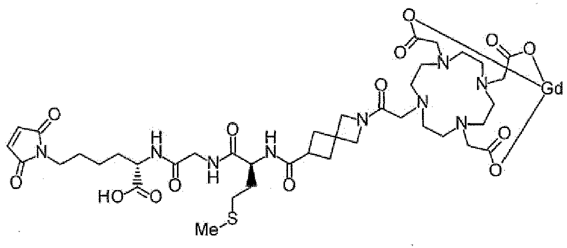 | ESI-; 1077.4 |
| A40 | 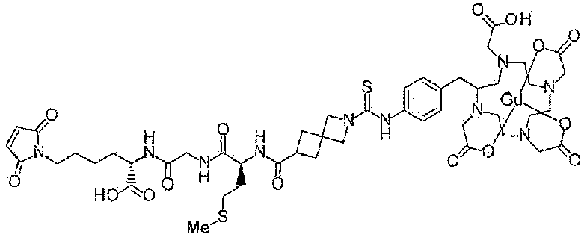 | ESI-; 1242.4 |

**[Table 13]**

| SNo | Str | MS |
|---|---|---|
| A41 | 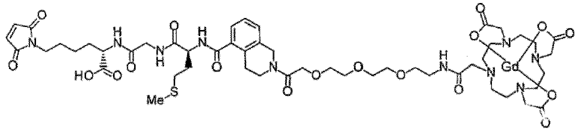 | ESI+; 1304.6 |
| A42 | 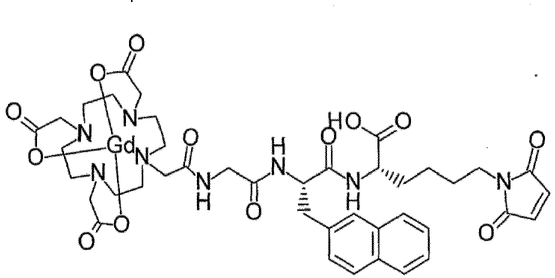 | ESI+; 1022.3 |
| A43 | 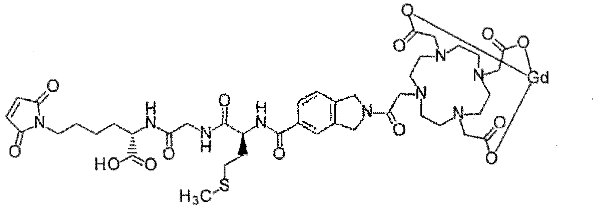 | ESI+; 1101.3 |

**[Table 14]**

| SNo | Str | MS |
|---|---|---|
| B1 | 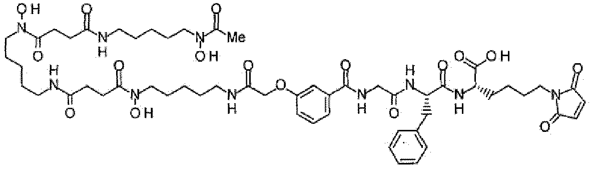 | ESI-; 1149.6 |
| B2 | 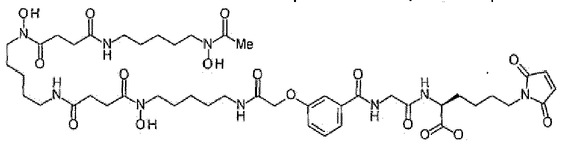 | ESI-;1002.6 |
| B3 | 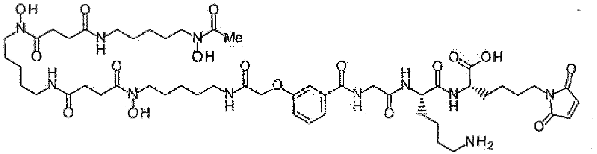 | ESI-; 1130.7 |
| B4 | 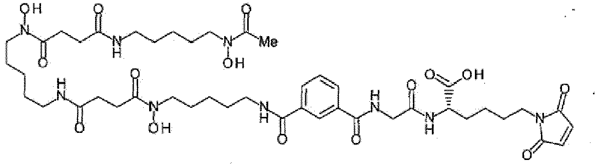 | ESI-; 972.6 |

**[Table 15]**

| (Y-S₁-X) ₚ-Fab² | | | |
|---|---|---|---|
| Ex-No | Y | S₁ | X |
| 1 | Gd/DOTA | - | -Gly-Lys∗-C(=S)-NH-(1,4-Ph)-NH-C(=S) |
| 2 | Gd/DOTA | - | -Gly-Val-NH-(CH₂)₂-Z₁(#N)- |
| 3 | Gd/DOTA | - | -Gly-Ile-NH-(CH₂)₂-Z₁(#N)- |
| 4 | Gd/DOTA | - | -Gly-Tyr∗-CH₂-C(=0) -Lys∗-Z₂(#N)- |
| 5 | Gd/DOTA | - | -Gly-Lys∗-Z₂(#S)- |
| 6 | Gd/DOTA | -NH-CH₂-(1, 3-Ph) - C (=O)- | -Gly-Lys∗-Z₂(#S)- |
| 7 | Gd/DOTA | - | -Gly-Tyr∗-CH₂-C(=O)-NH-(CH₂)₂-Z₁(#S)- |
| 8 | Gd/DOTA | - | -Met-Ile-NH-(CH₂)₂-Z₁(#S)- |
| 9 | Gd/DOTA | -NH-CH₂-(1,3-Ph) - C(=O)- | -Gly-Lys∗-Z₂(#N)- |
| 10 | Gd/DOTA | - | -Met-Ile-Lys∗-Z₂(#N)- |
| 14 | Gd/DOTA | -NH-CH₂-(1,3-Ph)-C(=O)- | -Gly-Tyr∗-CH₂-C(=0)-NH-(CH₂)₂-Z₁(#N) - |
| 17 | Gd/DOTA | - | -Gly-Tyr∗-CH₂-C(=O)-NH-(CH₂O)₃CH₂-C(= O)-NH-(CH₂)₂-Z₁(#N)- |

**[Table 16]**

| (Y-S₁-X) ₚ-Fab² | | | |
|---|---|---|---|
| Ex-No | Y | S₁ | X |
| 18 | Gd/DOTA | -NH-CH₂-(1,4-Ph)-NH-C(=O)- | -Met-Ile-NH-(CH₂)₂-Z₁(#N)- |
| 19 | Gd/DOTA | - | -Gly-Tyr-Lys∗-Z₂(#N)- |
| 20 | Gd/DOTA | - | -Gly-Lys∗-Z₂(#N)- |
| 21 | Gd/DOTA | - | -Gly-Tyr∗-CH₂-C(=O)-NH-(CH₂)₂-Z₁(#N)- |
| 22 | Gd/DOTA | -NH-(CH₂)₂-C(=O)- | -Gly-Lys∗-Z₂(#N)- |
| 23 | Gd/DOTA | (a) | -Gly-Lys∗-Z₂(#N)- |
| 25 | Gd/DOTA | -NH-(CH₂CH₂O)₃-CH₂-C(=O)-NH-CH₂-(1,3 -Ph)-C(=O)- | -Gly-Lys∗-Z₂(#S)- |
| 26 | Gd/DOTA | (d) | -Gly-Lys∗-Z₂(#N)- |
| 27 | Gd/DOTA | (b) | -Gly-Lys∗-Z₂(#N)- |
| 28 | Gd/DOTA | (c) | -Gly-Lys∗-Z₂(#N)- |
| 30 | Gd/DOTA | - | -Gly-(diph-Ala)-Lys∗-Z₂(#S)- |
| 31 | Gd/DOTA | - | -Gly-(naph-Ala)-Lys∗-Z₂(#S)- |
| 32 | Gd/DOTA | (e) | -Met-Gly-Lys∗-Z₂(#N)- |
| 34 | Gd/DOTA | 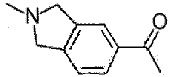 | -Met-Gly-Lys∗-Z₂(#N)- |

**[Table 17]**

| (Y-S₁)-X) ₚ-Fab² | | | |
|---|---|---|---|
| Ex-No . | Y | S₁ | X |
| 36 | Gd/DOTA | -NH-(CH₂)₃-C(=O)- | -Gly-Tyr∗-CH₂-C(=0) -NH-(CH₂)₂-Z₁(#N)- |
| 37 | Gd/DOTA | - | -Gly-Tyr-NH-(CH₂)₅-Z₁(#S)- |
| 38 | Gd/DOTA | -NH-CH₂-(1, 4-Ph) -C (=0) - | -Met-Gly-Lys∗-Z₂(#S)- |
| 39 | Gd/DOTA | -NH-(CH₂CH₂O)₃-CH₂-C(=O)-NH-CH₂-(1,3-Ph) -C (=O)- | -Gly-Tyr∗-CH₂-C(=O)-NH-(CH₂)₂-Z₁(#S) |
| 41 | Gd/DOTA | -NH-(CH₂CH₂O)₃-CH₂-C(=O)- | -Met-Gly-Lys∗-Z₂(#N)- |
| 42 | Gd/DOTA | (m) | -Met-Gly-Lys∗-Z₂(#N)- |
| 43 | Gd/DOTA | (h) | -Met-Gly-Lys∗-Z₂(#N) - |
| 44 | Gd/DOTA | -NH-(CH₂CH₂O)₃-CH₂-C(=O)-NH-CH₂-(1, 3-Ph)-C(=O)- | -Gly-Lys∗-C(=S)-NH-(1,4-Ph)-N H-C (=S) - |
| 45 | Gd/DOTA | (i) | -Met-Gly-Lys∗-Z₂(#N)- |
| 46 | Gd/DOTA | (k) | -Met-Gly-Lys∗-Z₂(#N)- |
| 49 | Gd/4arm-DO TA | (j) | -Met-Gly-Lys∗-Z₂(#N)- |
| 50 | Gd/4arm-DO TA | (l) | -Met-Gly-Lys∗-Z₂(#N)- |

**[Table 18]**

| (Y-S₁-X) ₚ-Fab² | | | |
|---|---|---|---|
| Ex-No | Y | S₁ | X |
| 51 | Gd/4arm-DOTA | -CH₂-(1,4-Ph)-NH-C(=S)-NH-(CH₂CH₂O)₃-CH₂-C(=O)-NH-CH₂-(1,3-Ph) -C (=O)- | -Met-Gly-Lys∗-Z₂(#N)- |
| 52 | Gd/DOTA | 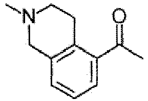 | -Gly-Lys∗-Z₂(#N)- |
| 53 | Gd/4arm-DOTA | (1) | -Gly-Lys∗-Z₂(#N)- |
| 54 | Gd/4arm-DOTA | -CH₂-(1,4-Ph)-NH-C(=S)- | -Met-Gly-Lys∗-Z₂(#N) - |
| 55 | Gd/4arm-DOTA | -CH₂-(1,4-Ph)-NH-C(=S)- | -Gly-(naph-Ala)-Lys∗-Z₂(# N)- |
| 56 | Gd/4arm-DOTA | -CH₂-(1,4-Ph)-NH-C(=S)- | -Gly-Tyr-Lys∗-Z₂(#N)- |
| 57 | Gd/DOTA | (p) | -Met-Gly-Lys∗-Z₂(#N)- |
| 58 | Gd/4arm-DOTA | (n) | -Met-Gly-Lys∗-Z₂(#N)- |
| 59 | Gd/DOTA | (o) | -Met-Gly-Lys∗-Z₂(#N)- |
| 62 | DFO | -C(=O)-CH₂O-(1,3-Ph)-C(=O) | -Gly-Lys∗-Z₂(#S)- |
| 63 | DFO | -C(=O)-CH₂O-(1,3-Ph)-C(=O) | -Gly-Phe-Lys∗-Z₂(#S)- |
| 64 | DFO | -C(=O)-(1,3-Ph)-C (=O)- | -Gly-Lys∗-Z₂(#S)- |
| 65 | DFO | -C(=O)-CH₂O-(1, 3-Ph) -C (=0) | -Gly-Lys-Lys∗-Z₂(#S)- |

The MS analyses of the Example compounds shown in Tables 15 to 18 are shown in Tables 19 to 23 below.

**[Table 19]**

| Ex-No | MS |
|---|---|
| 1 | It was confirmed by MS analysis that the conjugate was a mixture of a conjugate in which one low molecular weight molecule having a molecular weight of 937 was bound to one Fab² having a molecular weight of 47.9 kDa and a conjugate in which two such molecules were bound thereto. |
| 2 | It was confirmed by MS analysis that the conjugate was a mixture of a conjugate in which one low molecular weight molecule having a molecular weight of 838 was bound to one Fab² having a molecular weight of 47.9 kDa and a conjugate in which two such molecules were bound thereto. |
| 3 | It was confirmed by MS analysis that the conjugate was a mixture of a conjugate in which one low molecular weight molecule having a molecular weight of 852 was bound to one Fab² having a molecular weight of 47.9 kDa, a conjugate in which two such molecules were bound thereto, and a conjugate in which three such molecules were bound thereto. |
| 4 | It was confirmed by MS analysis that the conjugate was a mixture of a conjugate in which one low molecular weight molecule having a molecular weight of 1046 was bound to one Fab² having a molecular weight of 47.9 kDa, a conjugate in which two such molecules were bound thereto, and a conjugate in which three such molecules were bound thereto. |
| 5 | It was confirmed by MS analysis that the conjugate was a mixture of a conjugate in which one low molecular weight molecule having a molecular weight of 926 was bound to one Fab² having a molecular weight of 47.9 kDa and a conjugate in which two such molecules were bound thereto. |
| 6 | It was confirmed by MS analysis that the conjugate was a mixture of a conjugate in which one low molecular weight molecule having a molecular weight of 1059 was bound to one Fab² having a molecular weight of 47.9 kDa and a conjugate in which two such molecules were bound thereto. |
| 7 | It was confirmed by MS analysis that the conjugate was a mixture of a conjugate in which one low molecular weight molecule having a molecular weight of 1061 was bound to one Fab² having a molecular weight of 47.9 kDa, a conjugate in which two such molecules were bound thereto, and a conjugate in which three such molecules were bound thereto. |
| 8 | It was confirmed by MS analysis that the conjugate was a mixture of a conjugate in which one low molecular weight molecule having a molecular weight of 1027 was bound to one Fab² having a molecular weight of 47.9 kDa and a conjugate in which two such molecules were bound thereto. |
| 9 | It was confirmed by MS analysis that the conjugate was a mixture of a conjugate in which one low molecular weight molecule having a molecular weight of 958 was bound to one Fab² having a molecular weight of 47.9 kDa, a conjugate in which two such molecules were bound thereto, and a conjugate in which three such molecules were bound thereto. |
| 10 | It was confirmed by MS analysis that the conjugate was a mixture of a conjugate in which one low molecular weight molecule having a molecular weight of 1012 was bound to one Fab² having a molecular weight of 47.9 kDa and a conjugate in which two such molecules were bound thereto. |
| 1 4 | It was confirmed by MS analysis that the conjugate was a mixture of a conjugate in which one low molecular weight molecule having a molecular weight of 1093 was bound to one Fab² having a molecular weight of 47.9 kDa, a conjugate in which two such molecules were bound thereto, and a conjugate in which three such molecules were bound thereto. |
| 17 | It was confirmed by MS analysis that the conjugate was a mixture of a conjugate in which one low molecular weight molecule having a molecular weight of 1149 was bound to one Fab² having a molecular weight of 47.9 kDa and a conjugate in which two such molecules were bound thereto. |

**[Table 20]**

| Ex-No | MS |
|---|---|
| 18 | It was confirmed by MS analysis that the conjugate was a mixture of a conjugate in which one low molecular weight molecule having a molecular weight of 1074 was bound to one Fab² having a molecular weight of 47.9 kDa, a conjugate in which two such molecules were bound thereto, and a conjugate in which three such molecules were bound thereto. |
| 19 | It was confirmed by MS analysis that the conjugate was a mixture of a conjugate in which one low molecular weight molecule having a molecular weight of 988 was bound to one Fab² having a molecular weight of 47.9 kDa, a conjugate in which two such molecules were bound thereto, a conjugate in which three such molecules were bound thereto, and a conjugate in which four such molecules were bound thereto. |
| 20 | It was confirmed by MS analysis that the conjugate was a mixture of a conjugate in which one low molecular weight molecule having a molecular weight of 825 was bound to one Fab² having a molecular weight of 47.9 kDa, a conjugate in which two such molecules were bound thereto, and a conjugate in which three such molecules were bound thereto. |
| 21 | It was confirmed by MS analysis that the conjugate was a mixture of a conjugate in which one low molecular weight molecule having a molecular weight of 960 was bound to one Fab² having a molecular weight of 47.9 kDa, a conjugate in which two such molecules were bound thereto, a conjugate in which three such molecules were bound thereto, and a conjugate in which four such molecules were bound thereto. |
| 22 | It was confirmed by MS analysis that the conjugate was a mixture of a conjugate in which one low molecular weight molecule having a molecular weight of 896 was bound to one Fab² having a molecular weight of 47.9 kDa, a conjugate in which two such molecules were bound thereto, and a conjugate in which three such molecules were bound thereto. |
| 23 | It was confirmed by MS analysis that the conjugate was a mixture of a conjugate in which one low molecular weight molecule having a molecular weight of 936 was bound to one Fab² having a molecular weight of 47.9 kDa, a conjugate in which two such molecules were bound thereto, and a conjugate in which three such molecules were bound thereto. |
| 25 | It was confirmed by MS analysis that the conjugate was a mixture of a conjugate in which one low molecular weight molecule having a molecular weight of 1248 was bound to one Fab² having a molecular weight of 47.9 kDa and a conjugate in which two such molecules were bound thereto. |
| 26 | It was confirmed by MS analysis that the conjugate was a mixture of a conjugate in which one low molecular weight molecule having a molecular weight of 954 was bound to one Fab² having a molecular weight of 47.9 kDa, a conjugate in which two such molecules were bound thereto, and a conjugate in which three such molecules were bound thereto. |
| 27 | It was confirmed by MS analysis that the conjugate was a mixture of a conjugate in which one low molecular weight molecule having a molecular weight of 950 was bound to one Fab² having a molecular weight of 47.9 kDa, a conjugate in which two such molecules were bound thereto, and a conjugate in which three such molecules were bound thereto. |
| 28 | It was confirmed by MS analysis that the conjugate was a mixture of a conjugate in which one low molecular weight molecule having a molecular weight of 908 was bound to one Fab² having a molecular weight of 47.9 kDa, a conjugate in which two such molecules were bound thereto, and a conjugate in which three such molecules were bound thereto. |
| 30 | It was confirmed by MS analysis that the conjugate was a mixture of a conjugate in which one low molecular weight molecule having a molecular weight of 1149 was bound to one Fab² having a molecular weight of 47.9 kDa, a conjugate in which two such molecules were bound thereto, and a conjugate in which three such molecules were bound thereto. |

**[Table 21]**

| Ex-No | MS |
|---|---|
| 31 | It was confirmed by MS analysis that the conjugate was a mixture of a conjugate in which one low molecular weight molecule having a molecular weight of 1123 was bound to one Fab² having a molecular weight of 47.9 kDa and a conjugate in which two such molecules were bound thereto. |
| 32 | It was confirmed by MS analysis that the conjugate was a mixture of a conjugate in which one low molecular weight molecule having a molecular weight of 1095 was bound to one Fab² having a molecular weight of 47.9 kDa and a conjugate in which two such molecules were bound thereto. |
| 34 | It was confirmed by MS analysis that the conjugate was a mixture of a conjugate in which one low molecular weight molecule having a molecular weight of 1101 was bound to one Fab² having a molecular weight of 47.9 kDa and a conjugate in which two such molecules were bound thereto. |
| 36 | It was confirmed by MS analysis that the conjugate was a mixture of a conjugate in which one low molecular weight molecule having a molecular weight of 1045 was bound to one Fab² having a molecular weight of 47.9 kDa, a conjugate in which two such molecules were bound thereto, and a conjugate in which three such molecules were bound thereto. |
| 37 | It was confirmed by MS analysis that the conjugate was a mixture of a conjugate in which one low molecular weight molecule having a molecular weight of 1045 was bound to one Fab² having a molecular weight of 47.9 kDa, a conjugate in which two such molecules were bound thereto, and a conjugate in which three such molecules were bound thereto. |
| 38 | It was confirmed by MS analysis that the conjugate was a mixture of a conjugate in which one low molecular weight molecule having a molecular weight of 1190 was bound to one Fab² having a molecular weight of 47.9 kDa, a conjugate in which two such molecules were bound thereto, and a conjugate in which three such molecules were bound thereto. |
| 39 | It was confirmed by MS analysis that the conjugate was a mixture of a conjugate in which one low molecular weight molecule having a molecular weight of 1384 was bound to one Fab² having a molecular weight of 47.9 kDa, a conjugate in which two such molecules were bound thereto, and a conjugate in which three such molecules were bound thereto. |
| 41 | It was confirmed by MS analysis that the conjugate was a mixture of a conjugate in which one low molecular weight molecule having a molecular weight of 1145 was bound to one Fab² having a molecular weight of 47.9 kDa, a conjugate in which two such molecules were bound thereto, a conjugate in which three such molecules were bound thereto, and a conjugate in which four such molecules were bound thereto. |
| 42 | It was confirmed by MS analysis that the conjugate was a conjugate in which one low molecular weight molecule having a molecular weight of 1107 was bound to one Fab² having a molecular weight of 47.9 kDa. |
| 43 | It was confirmed by MS analysis that the conjugate was a mixture of a conjugate in which one low molecular weight molecule having a molecular weight of 1090 was bound to one Fab² having a molecular weight of 47.9 kDa and a conjugate in which two such molecules were bound thereto. |
| 44 | It was confirmed by MS analysis that the conjugate was a mixture of a conjugate in which one low molecular weight molecule having a molecular weight of 1259 was bound to one Fab² having a molecular weight of 47.9 kDa, a conjugate in which two such molecules were bound thereto, and a conjugate in which three such molecules were bound thereto. |

**[Table 22]**

| Ex-No | MS |
|---|---|
| 45 | It was confirmed by MS analysis that the conjugate was a mixture of a conjugate in which one low molecular weight molecule having a molecular weight of 1296 was bound to one Fab² having a molecular weight of 47.9 kDa and a conjugate in which two such molecules were bound thereto. |
| 46 | It was confirmed by MS analysis that the conjugate was a mixture of a conjugate in which one low molecular weight molecule having a molecular weight of 1296 was bound to one Fab² having a molecular weight of 47.9 kDa and a conjugate in which two such molecules were bound thereto. |
| 49 | It was confirmed by MS analysis that the conjugate was a mixture of a conjugate in which one low molecular weight molecule having a molecular weight of 1203 was bound to one Fab² having a molecular weight of 47.9 kDa and a conjugate in which two such molecules were bound thereto. |
| 50 | It was confirmed by MS analysis that the conjugate was a mixture of a conjugate in which one low molecular weight molecule having a molecular weight of 1280 was bound to one Fab² having a molecular weight of 47.9 kDa and a conjugate in which two such molecules were bound thereto. |
| 51 | It was confirmed by MS analysis that the conjugate was a mixture of a conjugate in which one low molecular weight molecule having a molecular weight of 1443 was bound to one Fab² having a molecular weight of 47.9 kDa. |
| 52 | It was confirmed by MS analysis that the conjugate was a mixture of a conjugate in which one low molecular weight molecule having a molecular weight of 984 was bound to one Fab² having a molecular weight of 47.9 kDa, a conjugate in which two such molecules were bound thereto, and a conjugate in which three such molecules were bound thereto. |
| 53 | It was confirmed by MS analysis that the conjugate was a mixture of a conjugate in which one low molecular weight molecule having a molecular weight of 1148 was bound to one Fab² having a molecular weight of 47.9 kDa and a conjugate in which two such molecules were bound thereto. |
| 54 | It was confirmed by MS analysis that the conjugate was a mixture of a conjugate in which one low molecular weight molecule having a molecular weight of 1121 was bound to one Fab² having a molecular weight of 47.9 kDa. |
| 55 | It was confirmed by MS analysis that the conjugate was a mixture of a conjugate in which one low molecular weight molecule having a molecular weight of 1187 was bound to one Fab² having a molecular weight of 47.9 kDa and a conjugate in which two such molecules were bound thereto. |
| 56 | It was confirmed by MS analysis that the conjugate was a mixture of a conjugate in which one low molecular weight molecule having a molecular weight of 1153 was bound to one Fab² having a molecular weight of 47.9 kDa and a conjugate in which two such molecules were bound thereto. |
| 57 | It was confirmed by MS analysis that the conjugate was a mixture of a conjugate in which one low molecular weight molecule having a molecular weight of 1079 was bound to one Fab² having a molecular weight of 47.9 kDa, a conjugate in which two such molecules were bound thereto, and a conjugate in which three such molecules were bound thereto. |
| 58 | It was confirmed by MS analysis that the conjugate was a mixture of a conjugate in which one low molecular weight molecule having a molecular weight of 1244 was bound to one Fab² having a molecular of 47.9 kDa. |

**[Table 23]**

| Ex-No | MS |
|---|---|
| 59 | It was confirmed by MS analysis that the conjugate was a mixture of a conjugate in which one low molecular weight molecule having a molecular weight of 1304 was bound to one Fab² having a molecular weight of 47.9 kDa and a conjugate in which two such molecules were bound thereto. |
| 6 2 | It was confirmed by MS analysis that the conjugate was a conjugate in which one low molecular weight molecule having a molecular weight of 1106 was bound to one Fab² having a molecular weight of 47.9 kDa. |
| 63 | It was confirmed by MS analysis that the conjugate was a mixture of a conjugate in which one low molecular weight molecule having a molecular weight of 1253 was bound to one Fab² having a molecular weight of 47.9 kDa and a conjugate in which two such molecules were bound thereto. |
| 64 | It was confirmed by MS analysis that the conjugate was a mixture of a conjugate in which one low molecular weight molecule having a molecular weight of 1076 was bound to one Fab² having a molecular weight of 47.9 kDa, a conjugate in which two such molecules were bound thereto, and a conjugate in which three such molecules were bound thereto. |
| 65 | It was confirmed by MS analysis that the conjugate was a mixture of a conjugate in which one low molecular weight molecule having a molecular weight of 1234 was bound to one Fab² having a molecular weight of 47.9 kDa and a conjugate in which two such molecules were bound thereto. |

### Example 3-1: Evaluation of binding activity of CEACAM5 antibody Fab fragment conjugate

Each anti-human CEACAM5 antibody Fab fragment conjugate prepared in Example 2 was subjected to ELISA to evaluate the binding activity thereof to CEACAM5. In the present test, a phosphate buffer (Nacalai Tesque Inc., 0.1 mol/L-Phosphate Buffer Solution (pH 7.2)) prepared to 10 mM by diluting 10-fold with distilled water was used as a solvent for an antigen immobilization liquid, and 20X PBS/Tween 20 Buffer (Thermo Fisher Scientific Inc., 28352) diluted 20-fold with distilled water was used as a wash liquid. In addition, for bovine serum albumin (BSA) used in the present test, 30% Bovine Serum Albumin solution (Sigma-Aldrich, Inc., A9576-50ML) was added in an appropriate proportion for use. CEACAM5 (R&D Systems, 4128-CM-050) was diluted with a 10 mM phosphate buffer (pH 7.2) to 0.1 µg/mL, and added to a Nunc MaxiSorp White 384 plate (Nunc, 4603272) at 30 µL per well, and the plate was incubated overnight at 4°C for immobilization. The CEACAM5 immobilization liquid was removed by reverse centrifugation, and then blocking was carried out by adding a PBS/Tween 20 buffer containing 5.0% BSA. Thereafter, the blocking solution was removed by reverse centrifugation, a solution of each CEACAM5 antibody Fab² fragment conjugate described above or a Fab² fragment having no labeling portion (PB009-01) as a control at about 10000 ng/mL was diluted in 14 steps by 3-fold dilution using a PBS/Tween 20 buffer containing 1.0% BSA, and 30 µL was added per well and incubated at room temperature for 60 minutes. The plate was washed 3 times with a PBS/Tween 20 buffer, and Horseradish Peroxidase (HRP)-labeled goat anti-human IgG (H + L chain) antibody (MBL Life Sciences, 206) diluted 1000-fold using a PBS/Tween 20 buffer containing 5.0% BSA was added at 30 µL per well and incubated at room temperature for 30 minutes. The plate was washed 3 times with a PBS/Tween 20 buffer, and ECL Prime Western Blotting DETECTION Reagent (GE Healthcare, RPN2232) as a substrate was added at 30 µL per well. The substrate was incubated at room temperature for 15 minutes, and then a signal value thereof was measured using an Envision counter (PerkinElmer, Inc.).

The test for each antibody was carried out in duplicate, and an EC₅₀ value was calculated using a 4-parameter logistic curve model. The geometric mean (Geometric mean), the standard deviation (Geometric SD factor), and the lower limit (Lower) and the upper limit (Upper) of the 95% confidence interval (95% CI of geo. mean) of the EC₅₀ values (nM) for 9 runs for PB009-01 are shown in Table 24. In addition, the EC₅₀ value of each conjugate run in duplicate is shown in Table 25. Ex-No in the table shows a conjugate number in Example 2.

**[Table 24]**

| | **PB009-01** |
|---|---|
| **Geometric mean (nM)** | 0.20 |
| **Geometric SD factor** | 1.45 |
| **Lower 95% CI of geo. mean (nM)** | 0.15 |
| **Upper 95% CI of geo. mean (nM)** | 0.27 |

**[Table 25]**

| **Ex-No** | **EC₅₀(nM)** | | **Ex-No** | **EC₅₀(nM)** | | **Ex-No** | **EC₅₀(nM)** |
|---|---|---|---|---|---|---|---|
| 1 | 0.16 | | 20 | 0.22 | | 39 | 0.20 |
| 2 | 0.14 | | 21 | 0.27 | | 40 | 0.22 |
| 3 | 0.13 | | 22 | 0.32 | | 41 | 0.21 |
| 4 | 0.15 | | 23 | 0.33 | | 42 | 0.21 |
| 5 | 0.14 | | 24 | 0.25 | | 43 | 0.23 |
| 6 | 0.14 | | 25 | 0.32 | | 44 | 0.29 |
| 7 | 0.17 | | 26 | 0.30 | | 45 | 0.23 |
| 8 | 0.11 | | 27 | 0.46 | | 46 | 0.22 |
| 9 | 0.10 | | 28 | 0.36 | | 47 | 0.25 |
| 10 | 0.11 | | 29 | 0.30 | | 48 | 0.21 |
| 11 | 0.14 | | 30 | 0.31 | | 54 | 0.23 |
| 12 | 0.11 | | 31 | 0.30 | | 55 | 0.26 |
| 13 | 0.14 | | 32 | 0.37 | | 56 | 0.26 |
| 14 | 0.13 | | 33 | 0.33 | | 57 | 0.29 |
| 15 | 0.13 | | 34 | 0.38 | | 58 | 0.28 |
| 17 | 0.23 | | 35 | 0.43 | | 59 | 0.30 |
| 18 | 0.24 | | 36 | 0.38 | | 60 | 0.18 |
| 19 | 0.26 | | 37 | 0.23 | | 61 | 0.18 |
| | | | 38 | 0.18 | | 64 | 0.20 |

### Example 3-2:

Each anti-human CEACAM5 antibody Fab fragment conjugate prepared in Example 2 was subjected to ELISA to evaluate the binding activity thereof to CEACAM5. In the present test, a phosphate buffer (Nacalai Tesque Inc., 0.1 mol/L-Phosphate Buffer Solution (pH 7.2)) prepared to 10 mM by diluting 10-fold with distilled water was used as a solvent for an antigen immobilization liquid, and 20X PBS/Tween 20 Buffer (Thermo Fisher Scientific Inc., 28352) diluted 20-fold with distilled water was used as a wash liquid. In addition, for bovine serum albumin (BSA) used in the present test, 30% Bovine Serum Albumin solution (Sigma-Aldrich, Inc., A9576-50ML) was added in an appropriate proportion for use. CEACAM5 (R&D Systems, 4128-CM-050) was diluted with a 10 mM phosphate buffer (pH 7.2) to 0.1 µg/mL, and added to a Nunc MaxiSorp White 384 plate (Nunc, 4603272) at 30 µL per well, and the plate was incubated overnight at 4°C for immobilization. The CEACAM5 immobilization liquid was removed by reverse centrifugation, and then blocking was carried out by adding a PBS/Tween 20 buffer containing 5.0% BSA. Thereafter, the blocking solution was removed by reverse centrifugation, a solution of each CEACAM5 antibody Fab² fragment conjugate described above or a Fab² fragment having no labeling portion (PB009-01) as a control at about 10000 ng/mL was diluted in 14 steps by 3-fold dilution using a PBS/Tween 20 buffer containing 1.0% BSA, and 30 µL was added per well and incubated at room temperature for 60 minutes. The plate was washed 3 times with a PBS/Tween 20 buffer, and Horseradish Peroxidase (HRP)-labeled goat anti-human IgG (H + L chain) antibody (MBL Life Sciences, 206) diluted 1000-fold using a PBS/Tween 20 buffer containing 5.0% BSA was added at 30 µL per well and incubated at room temperature for 30 minutes. The plate was washed 3 times with a PBS/Tween 20 buffer, and ECL Prime Western Blotting DETECTION Reagent (GE Healthcare, RPN2232) as a substrate was added at 30 µL per well. The substrate was incubated at room temperature for 15 minutes, and then a signal value thereof was measured using an Envision counter (PerkinElmer, Inc.).

The test for each antibody was carried out in duplicate, and the EC₅₀ value was calculated using a 4-parameter logistic curve model. The EC₅₀ value (nM) for each of 2 runs for PB009-01 is shown in Table 26. In addition, the EC₅₀ value of each conjugate run in duplicate is shown in Table 27. Ex-No in the table shows a conjugate number in Example 2.

**[Table 26]**

| PB009-01 (nM) | |
|---|---|
| 0.14 | |
| 0.21 | |

**[Table 27]**

| Ex-No. | EC50 (nM) |
|---|---|
| 66 | 0.16 |
| 68 | 0.31 |

### Example 4-1: Kidney accumulation evaluation test of Gd-labeled conjugates (normal mice)

The conjugate includes one having a peptide linker having an amino acid sequence cleaved by a renal brush border membrane enzyme or a lysosomal enzyme as a peptide linker of "X." The conjugate of the present invention having such a linker is specifically cleaved at the linker moiety by any of these enzymes present in the kidneys, and thus it is expected that the accumulation of the labeling portion in the kidneys is reduced. Evaluation results of the kidney accumulation of such a conjugate of the present invention are shown below.

### (Test method)

A Gd-labeled conjugate of the conjugate containing a peptide linker produced in one of the above Examples was administered from the tail vein of mice (BALB/c or BALB/c nu/nu) so that the protein mass was 0.02 mg (100 µL) per animal. After about 24 hours, the mice were sacrificed and the kidneys were removed, and the amount of Gd in the kidneys was measured using ICP-MS. The present test was carried out in 3 cases in each group, the mean value of the 3 cases was calculated, and the amount of Gd contained per kidney after administration was shown as a percentage (% of dose/tissue) of the total amount of Gd administered. The results are shown in Table 28 below. Ex-No in the table shows a conjugate number in Example 2. In addition, the same test was carried out using Ex-No. 47 ([Gd/DOTA]p-Fab) produced in Production Example 2-47, which did not contain a peptide linker, as a control conjugate, and the geometric mean (Geometric mean), the standard deviation (Geometric SD factor), and the lower limit value (Lower) and the upper limit value (Upper) of the 95% confidence interval (95% CI of geo. mean) for 5 runs were calculated.

### (Results)

The results are shown in Table 29 below. Based on the percentage of the control conjugate (% of dose/tissue), the proportion of decrease in the amount of Gd contained in the kidneys of each of the conjugates having a peptide linker was determined and shown in Table 28. As shown in Table 28, the accumulation of the labeling portion in the kidneys was 39.6 to 82.9% points lower in the conjugates having a peptide linker than in the control conjugate.

**[Table 28]**

| **Ex-No** | **% of dose/tissue** | **Proportion of decrease (%)** |
|---|---|---|
| 11 | 14.8 | 52.2 |
| 12 | 9.9 | 68.0 |
| 13 | 15.4 | 50.3 |
| 15 | 17.1 | 44.8 |
| 24 | 12.9 | 58.3 |
| 25 | 14.2 | 54.1 |
| 29 | 6.8 | 78.0 |
| 31 | 18.7 | 39.6 |
| 33 | 5.3 | 82.9 |
| 34 | 11.9 | 61.6 |
| 35 | 7.0 | 77.4 |
| 40 | 8.4 | 72.9 |

**[Table 29]**

| | **Ex-No 47** |
|---|---|
| **Geometric mean (% of dose/tissue)** | 31.0 |
| **Geometric SD factor** | 1.2 |
| **Lower 95% CI of geo. mean (% of dose/tissue)** | 25.3 |
| **Upper 95% CI of geo. mean (% of dose/tissue)** | 37.9 |

### Example 4-2: Kidney accumulation evaluation test of Gd-labeled conjugates (normal mice)

### (Test method)

A Gd-labeled conjugate of the conjugate containing a peptide linker produced in one of the above Examples was administered from the tail vein of mice (BALB/c or BALB/c nu/nu) so that the protein mass was 0.02 mg (100 µL) per animal. After about 24 hours, the mice were sacrificed and the kidneys were removed, and the amount of Gd in the kidneys was measured using ICP-MS. The present test was carried out in 3 cases in each group, the mean value of the 3 cases was calculated, and the amount of Gd contained per kidney after administration was shown as a percentage (% of dose/tissue) of the total amount of Gd administered. The results are shown in Table 30 below. Ex-No in the table shows a conjugate number in Example 2. In addition, the same test was carried out using Ex-No 47 ([Gd/DOTA]p-Fab) produced in Production Example 2, which did not contain a peptide linker, as a control conjugate.

### (Results)

The results are shown in Table 30 below. Based on the percentage of the control conjugate (% of dose/tissue), the proportion of decrease in the amount of Gd contained in the kidneys of each of the conjugates having a peptide linker was determined and shown in Table 30. As shown in Table 30, the accumulation of the labeling portion in the kidneys was 92.15 to 95.38% points lower in the conjugates having a peptide linker than in control conjugate 47.

**[Table 30]**

| Renal residual amount (normal mice) | | |
|---|---|---|
| Ex-No | % of dose/tissue | Proportion of decrease (%) |
| 66 | 2.43 | 92.15 |
| 68 | 1.43 | 95. 38 |
| 4 7 | 31.33 | - |

### Example 4-3: Kidney accumulation evaluation test of Gd-labeled conjugates (normal mice)

### (Test method)

A Gd-labeled conjugate of the conjugate containing a peptide linker produced in one of the above Examples was administered from the tail vein of mice (BALB/c or BALB/c nu/nu) so that the protein mass was 0.02 mg (100 µL) per animal. After about 24 hours, the mice were sacrificed and the kidneys were removed, and the amount of Gd in the kidneys was measured using ICP-MS. The present test was carried out in 3 cases in each group, the mean value of the 3 cases was calculated, and the amount of Gd contained per kidney after administration was shown as a percentage (% of dose/tissue) of the total amount of Gd administered. The same test was carried out using Ex-No. 47 produced in Production Example 2 above, which did not contain a peptide linker, as a control conjugate, and the mean of the renal residual amounts for 5 runs for Ex-No. 47 is shown in Table 31.

### (Results)

Based on the percentage of the control conjugate (% of dose/tissue), the proportion (%) of decrease in the amount of Gd contained in the kidneys of each of the conjugates having a peptide linker was determined and shown in Table 32. As shown in Table 32, the accumulation of the labeling portion in the kidneys was 21.00 to 94.66% points lower in the conjugates having a peptide linker than in the control conjugate.

**[Table 31]**

| Renal residual amount (normal mice) | |
|---|---|
| | Renal residual amount (% of dose/tissue) |
| Mean | 29.33 |

**[Table 32]**

| Renal residual amount (normal mice) | | |
|---|---|---|
| Ex-No. | % of dose/tissue | **Proportion of decrease** (%) |
| 8 | 23.17 | 21.00 |
| 10 | 20.10 | 31.46 |
| 18 | 2.60 | 91.13 |
| 3 2 | 10.10 | 65.56 |
| 38 | 13.33 | 54.54 |
| 4 1 | 21.67 | 26.12 |
| 4 3 | 8.97 | 69.42 |
| 45 | 12.00 | 59.08 |
| 46 | 11.33 | 61.35 |
| 67 | 1.57 | 94.66 |
| 6 9 | 7.13 | 75.68 |

### Example 4-4: Kidney accumulation evaluation test of Gd-labeled conjugates (tumor-bearing mice) (Test method)

A Gd-labeled conjugate of the conjugate containing a peptide linker produced in one of the above Examples was administered from the tail vein of tumor-bearing mice so that the protein mass was 0.02 mg (100 µL) per animal. As the tumor-bearing mice used in the present Example, mice (BALB/c nu/nu) that were in a tumor-bearing state brought about by transplanting human colorectal cancer cell line LS174T cells (ATCC(registered trademark); CL-188) at 2.0 to 5.0 × 10⁶ cells/mouse subcutaneously on the back before administration of the sample were used. 24 hours after administration of the sample, the mice were sacrificed, the kidneys and the tumor were removed, and the amounts of Gd in the kidneys and in the tumor were measured using ICP-MS. The present test was carried out in 3 cases in each group, and the mean value of the 3 cases was calculated. The amount of Gd contained per kidney after administration was shown as a percentage of the amount of Gd administered (% of dose/tissue), and the amount of Gd contained per g of tumor tissue was shown as a percentage of the total amount of Gd administered (% of dose/g). The results are shown in Tables 35 and 36. Ex-No in the tables shows a conjugate number in Example 2. In addition, the same test was carried out using Ex-No. 47 produced in Production Example 2-47, which did not contain a peptide linker, as a control conjugate. The means for 2 runs for Ex-No. 47 are shown in Tables 33 and 34.

### (Results)

As shown in Tables 35 and 36, the accumulation of the labeling portion in the kidneys was 52.26 to 77.68% points lower in the conjugates having a peptide linker than in the control conjugate.

**[Table 33]**

| Renal residual amount (tumor-bearing mice) | |
|---|---|
| | Renal residual amount (% of dose/tissue) |
| Mean | 25.83 |

**[Table 34]**

| Tumor accumulation | |
|---|---|
| | Amount of tumor accumulation (% of dose/g) |
| Mean | 4.92 |

**[Table 35]**

| Renal residual amount (tumor-bearing mice) | | |
|---|---|---|
| Ex-No. | % of dose/tissue | Proportion of decrease (%) |
| 18 | 5.77 | 77.68 |
| 24 | 12.33 | 52.26 |
| 29 | 8.40 | 67.48 |
| 33 | 7.30 | 71.74 |
| 35 | 9.40 | 63.61 |

**[Table 36]**

| Amount of tumor accumulation (tumor-bearing mice) | | |
|---|---|---|
| | Ex-No. | % of dose/g |
| | 18 | 10.63 |
| | 24 | 10.50 |
| | 29 | 4.37 |
| | 33 | 4.53 |
| | 35 | 6.90 |

### (Example 5-1: Preparation of anti-human MUC1 antibody Fab⁵ fragment conjugate)

The present Example discloses Production Examples of the conjugate. In presentation of each Production Example in the present Example, "Example 5" is followed by one hyphen followed by a "conjugate number." For example, "Example 5-101" shows that it is the Production Example of conjugate 101 in the present Example. In addition, as Fab⁵ in the present Example, (P10-2) disclosed in International Publication WO2018/092885 was used. "p-Fab⁵" shows that Fab⁵ is bound to p labeling portions enclosed by [ ] or ( ) via p amino groups thereof to form a conjugate. In some of the following Examples, the number of labeling portions) bound to Fab⁵ of each conjugate which can be confirmed by MS analysis is described, but the result does not show that a conjugate having a number of labeling portions bound other than the above number is not included. It will be easy to understand that there may still be a conjugate having a number of labeling portions bound whose presence has not been able to be confirmed because of the accuracy of the MS analysis equipment. In addition, in the structural formulas in the present Example, the structural formula of DOTA to which Gd is bound schematically shows DOTA labeled with Gd.

### (Example 5-101. Synthesis of ([Gd/DOTA-NH-CH₂-(1,4-Ph)-NH-C(=O)-Met-Ile-NH-(CH₂)₂-Z₁(#N)]p-Fab⁵))

(i) Synthesis of conjugate No. 101
   [N-({4-[(2-{4,7,10-tris[(carboxy-κO)methyl]-1,4,7,10-tetraazacyclododecan-1-yl-κ⁴N¹,N⁴,N⁷,N¹⁰}acetamido-κO)methyl]phenyl}carbamoyl)-L-methionyl-N¹-[2-(2,5-dioxo-2,5-dihydro-1H-pyrrol-1-yl)ethyl]-L-isoleucinamidato(3-)]gadolinium (1 mg) synthesized in Example 2-18 (vii) was dissolved in DMSO (40 µL). A 0.1 M borate buffer (40 µL) was added to the resulting solution, and the pH was adjusted to 6.6 using a 0.25 M acetate buffer.

80 µL of the solution previously prepared was added to a 5.2 mg/mL Fab⁵ borate buffer (240 µL), and the resulting mixture was incubated at 30°C for 2 hours. 15 µL of 0.05 M EDTA was added, and the resulting mixture was incubated at 30°C for 10 minutes. The mixture was purified using a PD-10 column, and the resulting solution was recovered using an Amicon Ultra-0.5 mL centrifugal filter. The recovered solution was washed with phosphate buffered saline 7 times, finally concentrated, and then filtered through a membrane filter to obtain a conjugate. It was confirmed by MS analysis that the conjugate was a mixture of a conjugate in which one [Gd/DOTA-NH-CH₂-(1,4-Ph)-NH-C(=O)-Met-Ile-NH-(CH₂)₂-Z₁(#N)] having a molecular weight of 1074 was bound to one Fab⁵ having a molecular weight of 47.5 kDa and a conjugate in which two such molecules were bound thereto.

### (Example 5-104. Synthesis of ([Gd/DOTA-NH-CH₂-(1,4-Ph)-NH-C(=O)-Met-Ile-NH-(CH₂)₂-(A-4 and/or A-5)]p-Fab⁵))

(i) Synthesis of conjugate No. 104
   [N-({4-[(2-{4,7,10-tris[(carboxy-κO)methyl]-1,4,7,10-tetraazacyclododecan-1-yl-κ⁴N¹,N⁴,N⁷,N¹⁰}acetamido-κO)methyl]phenyl}carbamoyl)-L-methionyl-N¹-[2-(2,5-dioxo-2,5-dihydro-1H-pyrrol-1-yl)ethyl]-L-isoleucinamidato(3-)]gadolinium (1 mg) synthesized in Example 2-18 (vii) was dissolved in DMSO (40 µL). A 0.1 M borate buffer (40 µL) was added to the resulting solution, and the pH was adjusted to 10.2 using a 0.1 M sodium carbonate aqueous solution.

The whole of the solution previously prepared was added to a 5.2 mg/mL Fab⁵ borate buffer (240 µL), and the resulting mixture was incubated at 30°C for 2 hours. The mixture was recovered using an Amicon Ultra-15 mL centrifugal filter and washed with phosphate buffered saline.

Buffer solution exchange was carried out twice using 3 mL of a HEPES-NaOH buffer (pH = 4.5) and concentration was carried out to make a 100 µL solution with pH = 4.52, and then the solution was incubated at 45°C for 30 minutes.

The solution was purified using a PD-10 column, and the resulting solution was recovered using an Amicon Ultra-15 mL centrifugal filter. The recovered solution was washed with phosphate buffered saline 3 times, finally concentrated, and then filtered through a membrane filter to obtain a conjugate. It was confirmed by MS analysis that the conjugate was a mixture of a conjugate in which one [Gd/DOTA-NH-CH₂-(1,4-Ph)-NH-C(=O)-Met-Ile-NH-(CH₂)₂-(A-4 and/or A-5)] having a molecular weight of 1092 was bound to one Fab⁵ having a molecular weight of 47.5 kDa, a conjugate in which two such molecules were bound thereto, and a conjugate in which three such molecules were bound thereto.

### (Example 5-107. Synthesis of ([Gd/DOTA-NH-CH₂-(1,4-Ph)-NH-C(=O)-Met-Ile-NH-(CH₂)₂-Z₁(#S)]p-Fab⁵))

(i) Synthesis of conjugate No. 107
   [N-({4-[(2-{4,7,10-tris[(carboxy-κO)methyl]-1,4,7,10-tetraazacyclododecan-1-yl-κ⁴N¹,N⁴,N⁷,N¹⁰}acetamido-κO)methyl]phenyl}carbamoyl)-L-methionyl-N¹-[2-(2,5-dioxo-2,5-dihydro-1H-pyrrol-1-yl)ethyl]-L-isoleucinamidato(3-)]gadolinium (0.75 mg) synthesized in Example 2-18 (vii) was dissolved in DMSO (40 µL), a 0.1 M borate buffer (40 µL) was added, and then the pH was adjusted to 8.2 using a 0.1 M sodium carbonate aqueous solution and a 0.25 M acetate buffer.

A 2 mg/mL 2-IT solution (7.5 µL) prepared using a 0.1 M borate buffer was added to a 5.2 mg/mL Fab⁵ borate buffer (240 µL), and the resulting mixture was incubated at 37°C for 40 minutes. Excess 2-IT was washed with phosphate buffered saline using an Amicon Ultra-0.5 mL centrifugal filter and concentrated, then the solution previously prepared was added, and the resulting mixture was incubated at 30°C for 2 hours. The mixture was purified using a PD-10 column, and the resulting solution was recovered using an Amicon Ultra-15 mL centrifugal filter. The recovered solution was washed twice with phosphate buffered saline, finally concentrated, and then filtered through a membrane filter to obtain a conjugate.

It was confirmed by MS analysis that the conjugate was a mixture of a conjugate in which one [Gd/DOTA-NH-CH₂-(1,4-Ph)-NH-C(=O)-Met-Ile-NH-(CH₂)₂-Z₁(#S)] having a molecular weight of 1175 was bound to one Fab⁵ having a molecular weight of 47.5 kDa, a conjugate in which two such molecules were bound thereto, and a conjugate in which three such molecules were bound thereto.

### (Example 5-112. Synthesis of ([Gd/DOTA-NH-CH₂-(1,4-Ph)-NH-C(=O)-Met-Ile-NH-(CH₂)₂-(A-3)]p-Fab⁵))

(i) Synthesis of conjugate No. 112
   [N-({4-[(2-{4,7,10-tris[(carboxy-κO)methyl]-1,4,7,10-tetraazacyclododecan-1-yl-κ⁴N¹,N⁴,N⁷,N¹⁰}acetamido-κO)methyl]phenyl}carbamoyl)-L-methionyl-N¹-[2-{[(4-isothiocyanatophenyl)carbamothioyl]amino}ethyl]-L-isoleucinamidato(3-)]gadolinium (0.46 mg) synthesized in Example 2-68 (x) was dissolved in DMSO (155 µL). 30 µL of a 0.1 M sodium carbonate aqueous solution and the solution previously prepared was added to a Fab⁵ solution (240 µL) prepared to 5.2 mg/mL using a borate buffer and a glycerin solution to adjust the pH to 8.8, and the resulting mixture was incubated at 37°C for 2 hours. The mixture was purified using a PD-10 column, and the resulting solution was recovered using an Amicon Ultra-15 mL centrifugal filter. The recovered solution was washed twice with phosphate buffered saline, finally concentrated, and then filtered through a membrane filter to obtain a conjugate. It was confirmed by MS analysis that the conjugate was a mixture of a conjugate in which one [Gd/DOTA-NH-CH₂-(1,4-Ph)-NH-C(=O)-Met-Ile-NH-(CH₂)₂-(A-3)] having a molecular weight of 1187 was bound to one Fab⁵ having a molecular weight of 47.5 kDa and a conjugate in which two such molecules were bound thereto.

### (Example 5-113. Synthesis of ([Gd/DOTA-[2,5-(1,2,3,4-tetrahydroisoquinoline)]-C(=O)-Met-Gly-Lys^{∗}-C(=S)-NH-(1,4-Ph)-NH-C(=S)]p-Fab⁵))

(i) Synthesis of conjugate No. 113
   {N-[2-({4,7,10-tris[(carboxy-κO)methyl]-1,4,7,10-tetraazacyclododecan-1-y-κ⁴N¹,N⁴,N⁷,N¹⁰}acetyl-κO)-1,2,3,4-tetrahydroisoquinoline-5-carbonyl]-L-methionylglycyl-N⁶-[(4-isothiocyanatophenyl)carb amothioyl] -L-lysinato(3-)}gadolinium (0.22 mg) synthesized in Example 2-69 (vii) was dissolved in DMSO (72 µL).

30 µL of a 0.1 M sodium carbonate aqueous solution and the solution previously prepared was added to a Fab⁵ solution (240 µL) prepared to 5.2 mg/mL using a borate buffer and a glycerin solution to adjust the pH to 9.3, and the resulting mixture was incubated at 37°C for 2 hours. The mixture was purified using a PD-10 column, and the resulting solution was recovered using an Amicon Ultra-15 mL centrifugal filter. The recovered solution was washed twice with phosphate buffered saline, finally concentrated, and then filtered through a membrane filter to obtain a conjugate. It was confirmed by MS analysis that the conjugate was a mixture of a conjugate in which one [Gd/DOTA-[2,5-(1,2,3,4-tetrahydroisoquinoline)]-C(=O)-Met-Gly-Lys^{∗}-C(=S)-NH-(1,4-Ph)-NH-C(=S)] having a molecular weight of 1228 was bound to one Fab⁵ having a molecular weight of 47.5 kDa and a conjugate in which two such molecules were bound thereto.

The chemical structural formulas (Str) of the conjugates obtained in the above Production Examples are shown in Tables 37 and 38.

**[Table 37]**

| Ex- No | Str |
|---|---|
| 101 | 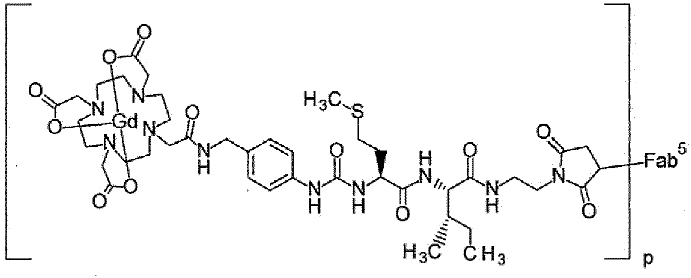 |
| 104 | 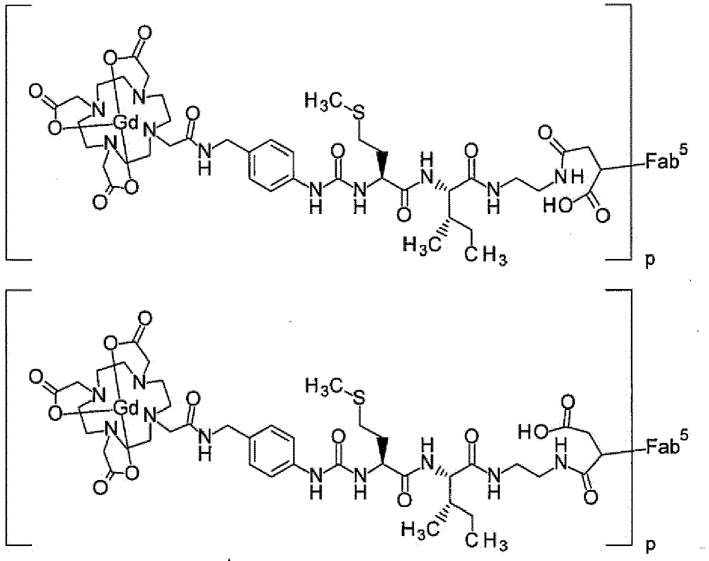 |

**[Table 38]**

| Ex-No | Str |
|---|---|
| 107 | 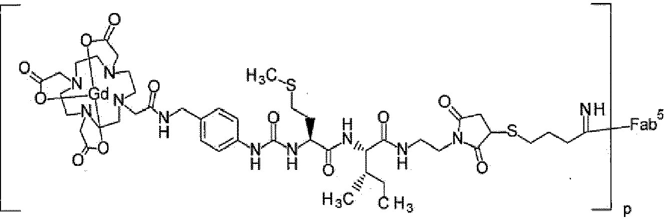 |
| 112 | 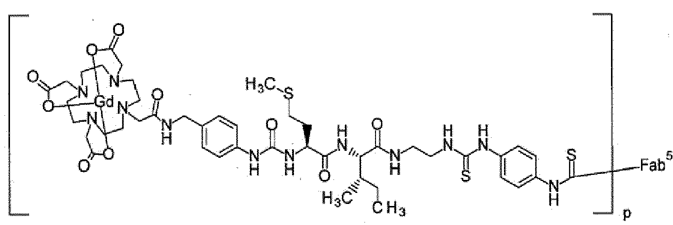 |
| 113 | 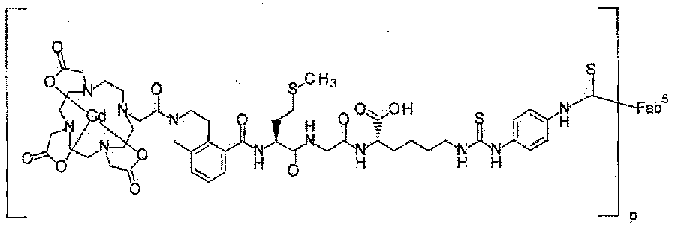 |

The compounds of Production Example No. C1 and C2 in Table 39 were synthesized using the same methods as in the above Production Examples, or methods known to those skilled in the art, and these compounds were used or the compounds synthesized in Example 2 were used to obtain conjugates with Fab⁵ shown in Tables 40 and 41 below.

**[Table 39]**

| SNo | Str | MS | Same production method |
|---|---|---|---|
| C1 | 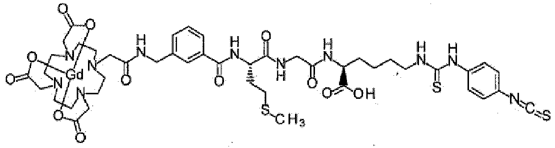 | ESI-; 1199 .2 | As in Ex Nos. 68 and 69 |
| C2 | 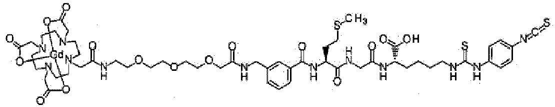 | ESI-; 1388 .3 | As in Ex Nos. 68 and 69 |

**[Table 40]**

| Ex-No | Str |
|---|---|
| 102 | 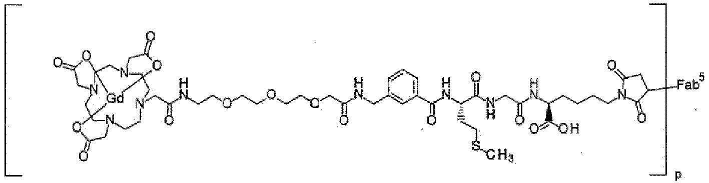 |
| 103 | 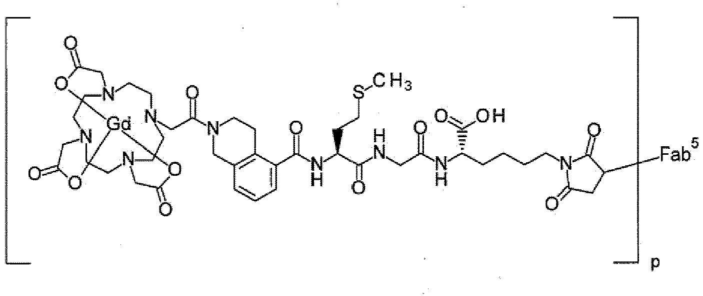 |
| 105 | 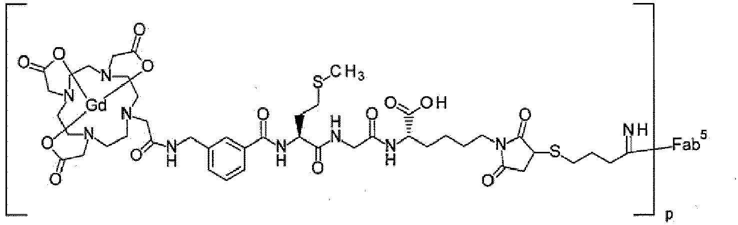 |
| 106 | 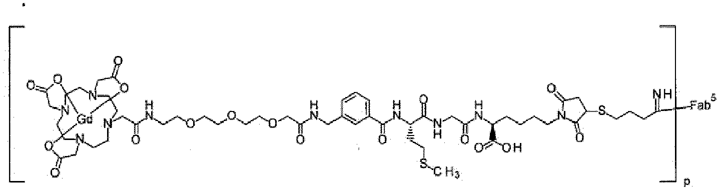 |

**[Table 41]**

| Ex-No | Str |
|---|---|
| 108 | 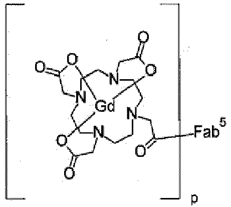 |
| 109 | 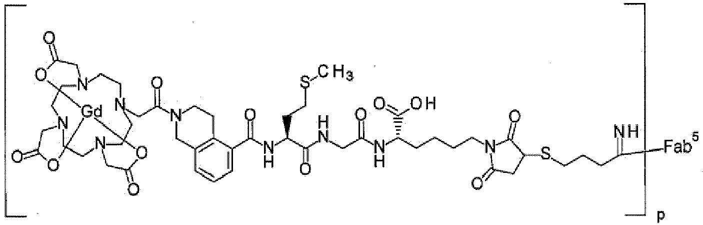 |
| 110 | 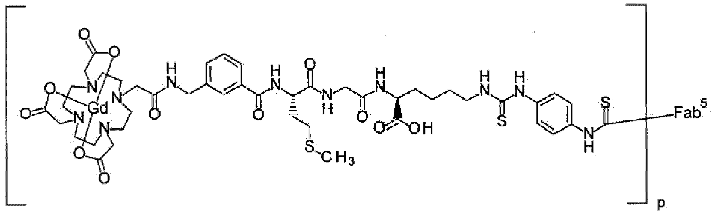 |
| 111 | 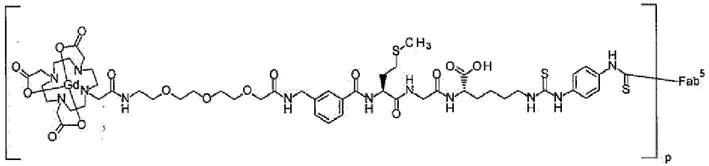 |

The MS analyses of the Example compounds shown in Tables 40 and 41 are shown in the table below.

**[Table 42]**

| Ex-No | MS |
|---|---|
| 102 | It was confirmed by MS analysis that the conjugate was a mixture of a conjugate in which one low molecular weight molecule having a molecular weight of 1278 was bound to one Fab⁵ having a molecular weight of 47.5 kDa and a conjugate in which two such molecules were bound thereto. |
| 103 | It was confirmed by MS analysis that the conjugate was a mixture of a conjugate in which one low molecular weight molecule having a molecular weight of 1115 was bound to one Fab⁵ having a molecular weight of 47.5 kDa and a conjugate in which two such molecules were bound thereto. |
| 105 | It was confirmed by MS analysis that the conjugate was a mixture of a conjugate in which one low molecular weight molecule having a molecular weight of 1190 was bound to one Fab⁵ having a molecular weight of 47.5 kDa, a conjugate in which two such molecules were bound thereto, and a conjugate in which three such molecules were bound thereto. |
| 106 | It was confirmed by MS analysis that the conjugate was a mixture of a conjugate in which one low molecular weight molecule having a molecular weight of 1380 was bound to one Fab⁵ having a molecular weight of 47.5 kDa, a conjugate in which two such molecules were bound thereto, and a conjugate in which three such molecules were bound thereto. |
| 108 | It was confirmed by MS analysis that the conjugate was a mixture of a conjugate in which one low molecular weight molecule having a molecular weight of 542 was bound to one Fab⁵ having a molecular weight of 47.5 kDa, a conjugate in which two such molecules were bound thereto, and a conjugate in which three such molecules were bound thereto. |
| 109 | It was confirmed by MS analysis that the conjugate was a mixture of a conjugate in which one low molecular weight molecule having a molecular weight of 1216 was bound to one Fab⁵ having a molecular weight of 47.5 kDa and a conjugate in which two such molecules were bound thereto. |
| 110 | It was confirmed by MS analysis that the conjugate was a mixture of a conjugate in which one low molecular weight molecule having a molecular weight of 1201 was bound to one Fab⁵ having a molecular weight of 47.5 kDa and a conjugate in which two such molecules were bound thereto. |
| 111 | It was confirmed by MS analysis that the conjugate was a mixture of a conjugate in which one low molecular weight molecule having a molecular weight of 1391 was bound to one Fab⁵ having a molecular weight of 47.5 kDa and a conjugate in which two such molecules were bound thereto. |

The Example compound in Table 43 was obtained using the same method as in the above Production Examples or a method known to those skilled in the art.

**[Table 43]**

| Ex- No | Str | Same production method |
|---|---|---|
| 114 | 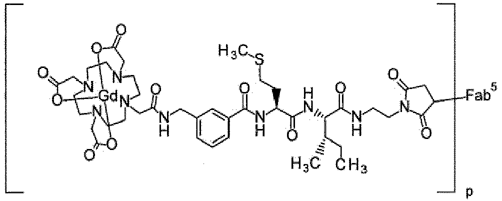 | As in Ex Nos. 15 and 18 |

The MS analysis of the Example compound shown in Table 43 is shown in Table 44 below.

**[Table 44]**

| Ex-No | MS |
|---|---|
| 114 | It was confirmed by MS analysis that the conjugate was a mixture of a conjugate in which one low molecular weight molecule having a molecular weight of 1059 was bound to one Fab⁵ having a molecular weight of 47.5 kDa. |

Further, the conjugates in Tables 45 to 50 can be obtained using the same methods as in the above Production Examples or methods known to those skilled in the art.

**[Table 45]**

| Ex-No | Str |
|---|---|
| P1 | 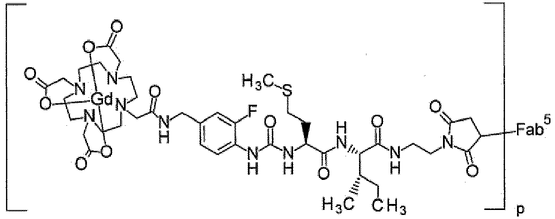 |
| P2 | 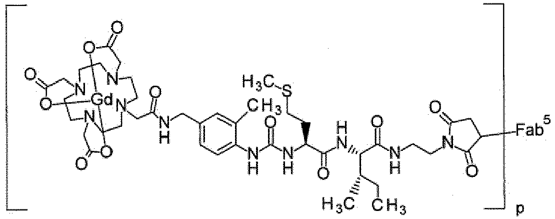 |
| P3 | 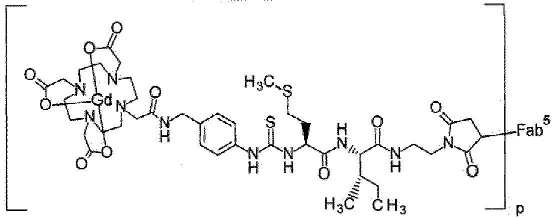 |
| P4 | 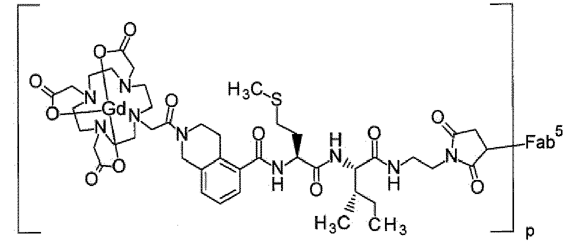 |

**[Table 46]**

| Ex-No | Str |
|---|---|
| P5 | 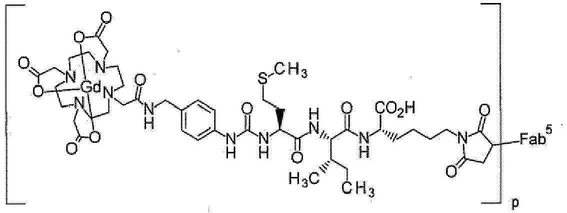 |
| P6 | 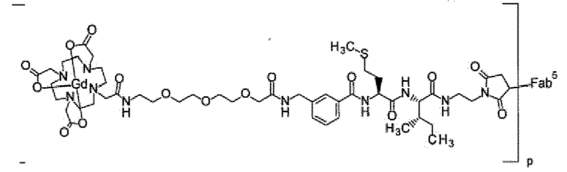 |
| P7 | 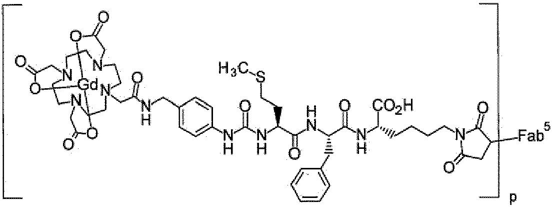 |
| P8 | 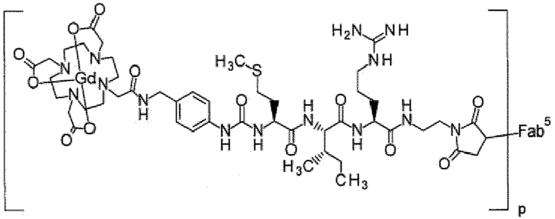 |

**[Table 47]**

| Ex-No | Str |
|---|---|
| P9 | 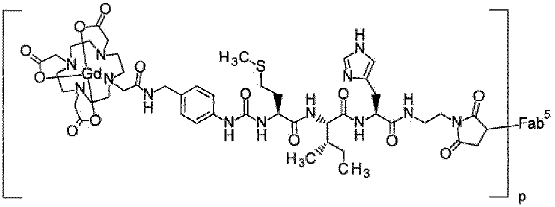 |
| P10 | 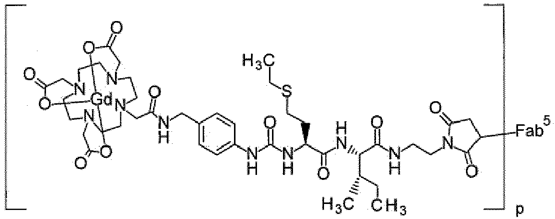 |
| P11 | 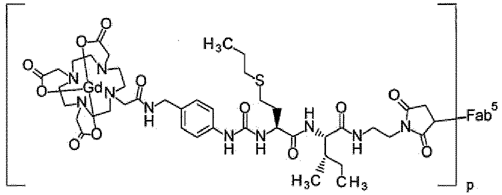 |
| P12 | 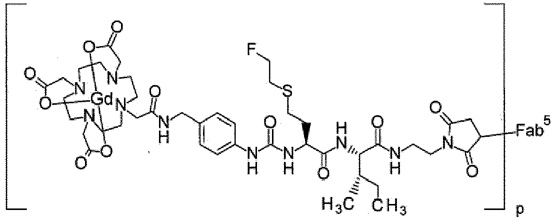 |

**[Table 48]**

| Ex-No | Str |
|---|---|
| P13 | 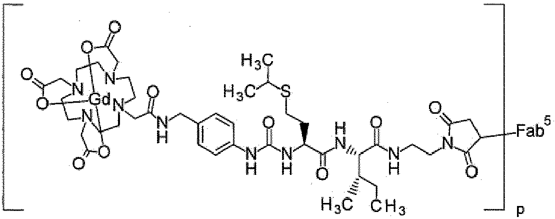 |
| P14 | 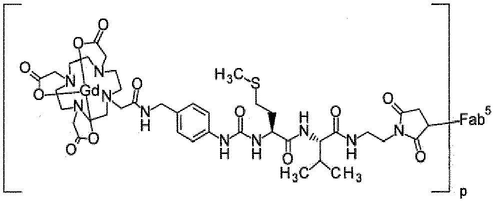 |
| P15 | 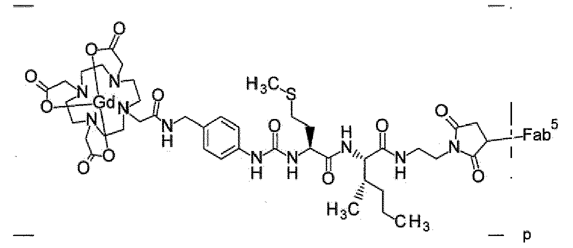 |
| P16 | 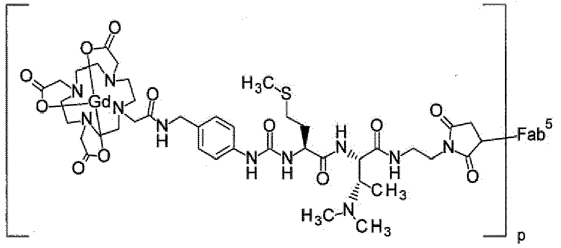 |

**[Table 49]**

| Ex-No | Str |
|---|---|
| P17 | 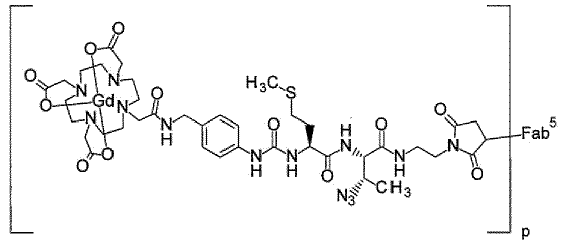 |
| P18 | 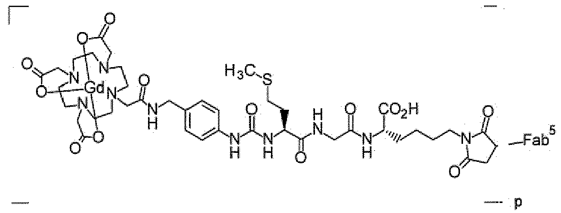 |
| P19 | 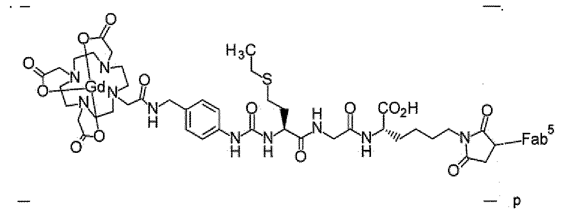 |
| P20 | 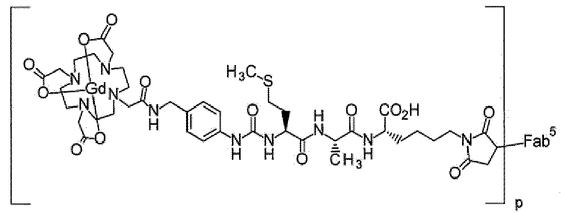 |

**[Table 50]**

| Ex-No | Str |
|---|---|
| P21 | 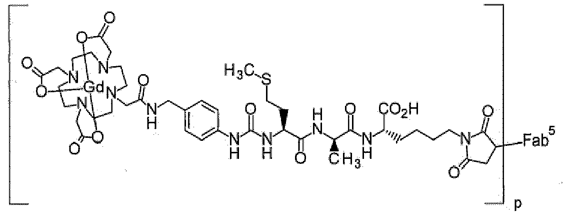 |

### Example 6-1: Evaluation of binding activity of anti-human MUC1 antibody Fab⁵ fragment conjugate

Each anti-human MUC1 antibody Fab⁵ fragment conjugate prepared by the method of Example 5 was subjected to ELISA to evaluate the binding activity thereof to human cancer-specific MUC1. In the present test, 20X PBS/Tween 20 Buffer (Thermo Fisher Scientific Inc., 28352) diluted 20-fold with distilled water was used as a wash liquid. In addition, for bovine serum albumin (BSA) used in the present test, 30% Bovine Serum Albumin solution (Sigma-Aldrich, Inc., A9576-50ML) was added in an appropriate proportion for use. A human cancer-specific MUC1 peptide (PTL 1) at 0.5 µmol/L was added to a Nunc MaxiSorp White 384 plate (Nunc, 460372) at 30 µL per well, and the plate was incubated overnight at 4°C for immobilization. The MUC1 peptide was removed by reverse centrifugation, and then blocking was carried out by adding a PBS/Tween 20 buffer containing 5.0% BSA. Thereafter, the blocking solution was removed by reverse centrifugation, a solution of each anti-human MUC1 antibody Fab⁵ fragment conjugate (Ex-No. 104, 108, or 112) described above at about 10000 ng/mL was diluted in 14 steps by 3-fold dilution using a PBS/Tween 20 buffer containing 1.0% BSA, and 30 µL was added per well and incubated at room temperature for 60 minutes. The plate was washed 3 times with a PBS/Tween 20 buffer, and Horseradish Peroxidase-labeled goat anti-human IgK antibody (Southern Biotechnology Associates, Inc.) diluted 10000-fold using a PBS/Tween 20 buffer containing 5.0% BSA was added at 30 µL per well and incubated at room temperature for 30 minutes. The plate was washed 3 times with a PBS/Tween 20 buffer, and ECL Prime Western Blotting DETECTION Reagent (GE Healthcare, RPN2232) as a substrate was added at 30 µL per well. The substrate was incubated at room temperature for 15 minutes, and then a signal value thereof was measured using an Envision counter (PerkinElmer, Inc.).

The test for each antibody was carried out in duplicate, and the EC₅₀ value was calculated using a 4-parameter logistic curve model. The EC₅₀ value (nM) for each of 3 runs for P10-2 is shown in Table 51. In addition, the EC₅₀ value of each conjugate run in duplicate is shown in Table 52.

**[Table 51]**

| P10―2 (nM) |
|---|
| 0.06 |
| 0.06 |
| 0.14 |

**[Table 52]**

| Ex―No. | EC50 (nM) |
|---|---|
| 104 | 0.08 |
| 112 | 0.15 |
| 108 | 0.07 |

### Example 6-2: Evaluation of binding activity of anti-human MUC1 antibody Fab⁵ fragment conjugate

Each anti-human MUC1 antibody Fab⁵ fragment conjugate prepared by the method of Example 5 was subjected to ELISA to evaluate the binding activity thereof to human cancer-specific MUC1. In the present test, 20X PBS/Tween 20 Buffer (Thermo Fisher Scientific Inc., 28352) diluted 20-fold with distilled water was used as a wash liquid. In addition, for bovine serum albumin (BSA) used in the present test, 30% Bovine Serum Albumin solution (Sigma-Aldrich, Inc., A9576-50ML) was added in an appropriate proportion for use. A human cancer-specific MUC1 peptide (PTL 1) at 0.5 µmol/L was added to a Nunc MaxiSorp White 384 plate (Nunc, 460372) at 30 µL per well, and the plate was incubated overnight at 4°C for immobilization. The MUC1 peptide was removed by reverse centrifugation, and then blocking was carried out by adding a PBS/Tween 20 buffer containing 5.0% BSA. Thereafter, the blocking solution was removed by reverse centrifugation, a solution of each anti-human MUC1 antibody Fab⁵ fragment conjugate (Ex-No. 101-113) described above at about 10000 ng/mL was diluted in 14 steps by 3-fold dilution using a PBS/Tween 20 buffer containing 1.0% BSA, and 30 µL was added per well and incubated at room temperature for 60 minutes. The plate was washed 3 times with a PBS/Tween 20 buffer, and Horseradish Peroxidase-labeled goat anti-human Igκ antibody (Southern Biotechnology Associates, Inc.) diluted 10000-fold using a PBS/Tween 20 buffer containing 5.0% BSA was added at 30 µL per well and incubated at room temperature for 30 minutes. The plate was washed 3 times with a PBS/Tween 20 buffer, and ECL Prime Western Blotting DETECTION Reagent (GE Healthcare, RPN2232) as a substrate was added at 30 µL per well. The substrate was incubated at room temperature for 15 minutes, and then a signal value thereof was measured using an Envision counter (PerkinElmer, Inc.).

The test for each antibody was carried out in duplicate, and the EC₅₀ value was calculated using a 4-parameter logistic curve model. The EC₅₀ value (nM) for each of 3 runs for P10-2 is shown in Table 53. In addition, the EC₅₀ value of each conjugate run in duplicate is shown in Table 54.

**[Table 53]**

| P10―2 (nM) |
|---|
| 0.06 |
| 0.06 |
| 0.14 |

**[Table 54]**

| Ex―No. | EC50 (nM) |
|---|---|
| 101 | 0.08 |
| 102 | 0.07 |
| 103 | 0.08 |
| 105 | 0.11 |
| 106 | 0.10 |
| 107 | 0.09 |
| 108 | 0.07 |
| 109 | 0.08 |
| 110 | 0.19 |
| 111 | 0.16 |
| 113 | 0.19 |

### Example 7: Kidney accumulation evaluation test of Gd-labeled conjugates (normal mice)

### (Test method)

A Gd-labeled conjugate of the conjugate containing a peptide linker produced in one of the above Examples was administered from the tail vein of mice (BALB/c or BALB/c nu/nu) so that the protein mass was 0.02 mg (100 µL) per animal. After about 24 hours, the mice were sacrificed and the kidneys were removed, and the amount of Gd in the kidneys was measured using ICP-MS. The present test was carried out in 3 cases in each group, the mean value of the 3 cases was calculated, and the amount of Gd contained per kidney after administration was shown as a percentage (% of dose/tissue) of the total amount of Gd administered. The same test was carried out using Ex-No 108 produced in Production Example 5, which did not contain a peptide linker, as a control conjugate. The mean for 2 runs for Ex-No 108 is shown in Tables 55.

### (Results)

The results are shown in Table 56. Based on the amount of Gd contained in the kidneys of the control conjugate, the proportion of decrease in the amount of Gd contained in the kidneys of each of the conjugates having a peptide linker was determined. As shown in Table 56, the accumulation of the labeling portion in the kidneys was 91.52 to 93.03% points lower in the conjugates having a peptide linker than in the control conjugate.

**[Table 55]**

| Renal residual amount (normal mice) | |
|---|---|
| | Ex-No. 108 |
| Mean (% of dose/tissue) | 33.00 |

**[Table 56]**

| Renal residual amount (normal mice) | | |
|---|---|---|
| Ex―No. | % of dose/tissue | Proportion of decrease (%) |
| 104 | 2.30 | 93.03 |
| 112 | 2.80 | 91.52 |

### Example 8: Kidney accumulation evaluation test of Gd-labeled conjugates (tumor-bearing mice)

### (Test method)

A Gd-labeled conjugate of the conjugate containing a peptide linker produced in one of the above Examples was administered from the tail vein of the tumor-bearing mice so that the protein mass was 0.02 mg (100 µL) per animal. As the tumor-bearing mice used in the present Example, mice (BALB/c nu/nu) that were in a tumor-bearing state brought about by transplanting human breast cancer cell line MDA-MB-468 (ATCC(registered trademark); HTB-132) at 5.0 × 10⁶ cells/mouse subcutaneously on the back before administration of the sample were used. 24 hours after administration of the sample, the mice were sacrificed, the kidneys and the tumor were removed, and the amounts of Gd in the kidneys and in the tumor were measured using ICP-MS. The present test was carried out in 3 cases in each group, and the mean value of the 3 cases was calculated. The amount of Gd contained per kidney after administration was shown as a percentage of the amount of Gd administered (% of dose/tissue), and the amount of Gd contained per g of tumor tissue was shown as a percentage of the total amount of Gd administered (% of dose/g). The results are shown in Tables 59 and 60. Ex-No in the tables shows a conjugate number in Example 5. In addition, the same test was carried out using Ex-No. 108 produced in Production Example 5, which did not contain a peptide linker, as a control conjugate. The means for 2 runs for Ex-No. 108 are shown in Tables 57 and 58.

### (Results)

As shown in Tables 57 to 60, the accumulation of the labeling portion in the kidneys was 88.08 to 90.87% points lower in the conjugates having a peptide linker than in the control conjugate.

**[Table 57]**

| Renal residual amount | |
|---|---|
| | Renal residual amount (% of dose/tissue) |
| Mean | 34.67 |

**[Table 58]**

| Tumor accumulation | |
|---|---|
| | Amount of tumor accumulation (% of dose/g) |
| Mean | 0.92 |

**[Table 59]**

| Renal residual amount (tumor-bearing mice) | | |
|---|---|---|
| Ex-No. | % of dose/tissue | Proportion of decrease (%) |
| 104 | 3.17 | 90.87 |
| 112 | 4.13 | 88.08 |

**[Table 60]**

| Amount of tumor accumulation (tumor-bearing mice) | |
|---|---|
| Ex―No. | % of dose/g |
| 104 | 1.04 |
| 112 | 1.27 |

### Example 9: Preparation of anti-human MUC1 antibody Fab⁵ fragment conjugate (Example 9-115: Synthesis of [Gd/DOTA]p-Fab⁵)

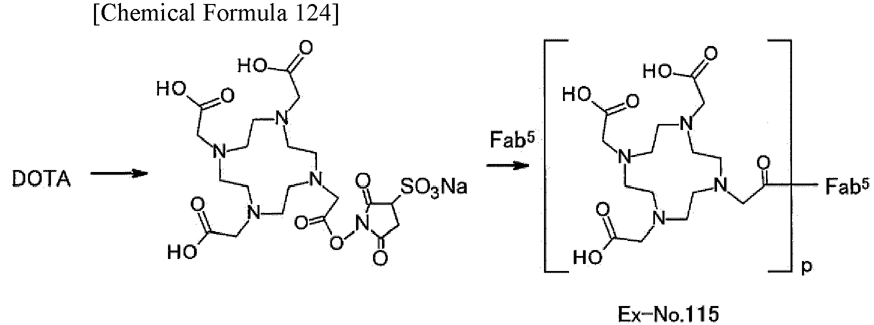

A 1 M sodium hydroxide aqueous solution (80 µL) was added to a mixed solution of 1,4,7,10-tetraazacyclododecane-1,4,7,10-tetraacetic acid (DOTA) (16 mg) and water (810 µL) under ice cooling to adjust the pH to 5.5 to 6. Sodium 1-hydroxy-2,5-dioxopyrrolidine-3-sulfonate (2.3 mg) dissolved in water (117 µL) was added to the obtained solution (239 µL) under ice cooling. Thereafter, an EDC HCl aqueous solution (8.3 µL, 25 mg/mL) was added, and the resulting mixture was stirred under ice cooling for 30 minutes to prepare an N-hydroxysulfosuccinimidyl DOTA solution. Before the addition of Fab⁵, a 0.2 M disodium hydrogen phosphate aqueous solution (pH 9) (40 µL) was added to adjust the pH to 7.

The prepared N-hydroxysulfosuccinimidyl DOTA solution (300 µL) was added to a 0.1 M disodium hydrogen phosphate aqueous solution (585 µL) of 20.8 mg/mL Fab⁵ (90 µL), and the resulting mixture was incubated at 4°C for 23 hours. The excess linker was washed with a 10 mM phosphate buffer using an Amicon Ultra-15 mL centrifugal filter twice repeatedly, washed with a 0.3 M ammonium acetate buffer, finally concentrated, and then filtered through a membrane filter to obtain conjugate Ex-No. 115. It was confirmed by MS analysis that the conjugate was a mixture of a conjugate in which one [DOTA] having a molecular weight of 387 was bound to one Fab⁵ having a molecular weight of 47.5 kDa, a conjugate in which two such molecules were bound thereto, a conjugate in which three such molecules were bound thereto, and a conjugate in which four such molecules were bound thereto.

### (Example 9-116. Synthesis of [DOTA-NH-CH₂-(1,4-Ph)-NH-C(=O)-Met-Ile-NH-(CH₂)₂-(A-3)]p-Fab⁵)

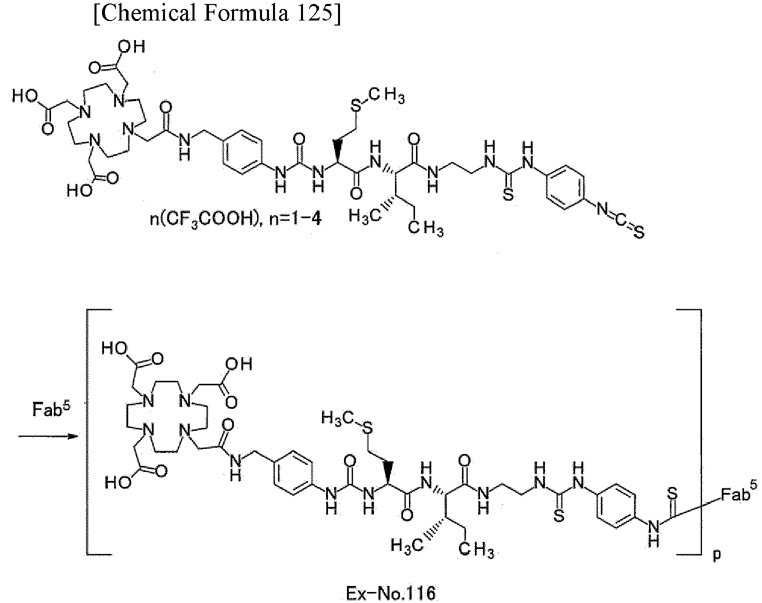

N-{[4-({2-[4,7,10-tris(carboxymethyl)-1,4,7,10-tetraazacyclododecan-1-yl]acetamido}methyl)phenyl]carbamoyl}-L-methionyl-N¹-(2-{[(4-isothiocyanatophenyl)carbamothioyl]amino}ethyl)-L-isoleucinamide tetrakis(trifluoroacetate) (1 mg) synthesized in Example 2-68 (ix) was dissolved in DMSO (336 µL).

30 µL of the previously prepared solution was added to a 5.2 mg/mL Fab⁵ phosphate buffered saline solution (240 µL), the pH was adjusted to about 8.0 using a 0.1 M sodium carbonate aqueous solution, and the resulting mixture was incubated at 30°C for 2 hours. The mixture was purified using a PD-10 column, and the resulting solution was recovered using an Amicon Ultra-15 mL centrifugal filter. The recovered solution was washed twice with phosphate buffered saline, finally concentrated, and then filtered through a membrane filter to obtain conjugate Ex-No. 116. The same operation was carried out again, and the obtained conjugate Ex-No. 116 was mixed. It was confirmed by MS analysis that the conjugate was a mixture of a conjugate in which one [DOTA-NH-CH₂-(1,4-Ph)-NH-C(=O)-Met-Ile-NH-(CH₂)₂-(A-3)] having a molecular weight of 1032 was bound to one Fab⁵ having a molecular weight of 47.5 kDa, a conjugate in which two such molecules were bound thereto, a conjugate in which three such molecules were bound thereto, and a conjugate in which four such molecules were bound thereto.

### Example 10: Study of common marmoset pharmacokinetics of metal complex compounds

### Preparation Example 1 of ⁶⁴Cu labeled protein

139 µL of a 0.1 mol/L sodium acetate buffer (pH 6.5) was added to 10 µL (19.8 MBq, FUJIFILM Toyama Chemical Co., Ltd.) of a 0.05 mol/L hydrochloric acid solution of [⁶⁴Cu]CuCl₂ and admixed. Further, 51 µL of the protein conjugate prepared in Example 9-115 was added, and this mixture was incubated at room temperature for 60 minutes for reaction. The reaction liquid was added to an ultrafiltration membrane (Amicon Ultra 10K, Millipore) and further, a 50 mmol/L sodium acetate buffer was added to carry out ultrafiltration purification to obtain ⁶⁴Cu-protein conjugate solution (A) of interest. 140 µL of the protein conjugate and 145 µL of a PBS solution were mixed into the obtained solution, and the resulting mixture was filtered using a syringe filter (Millex-GV 0.22 µm, Millipore) to prepare a ⁶⁴Cu-protein conjugate (A)-containing PBS solution (11.5 MBq, 19.17 MBq/mg).

### Preparation Example 2 of ⁶⁴Cu labeled protein

285 µL of a 0.1 mol/L sodium acetate buffer (pH 6.5) was added to 20 µL (37.1 MBq, FUJIFILM Toyama Chemical Co., Ltd.) of a 0.05 mol/L hydrochloric acid solution of [⁶⁴Cu]CuCl₂ and admixed. Further, 94.7 µL of the protein conjugate prepared in Example 9-116 was added, and this mixture was incubated at room temperature for 60 minutes for reaction. The reaction liquid was added to an ultrafiltration membrane (Amicon Ultra 10K, Millipore) and further, a 50 mmol/L sodium acetate buffer was added to carry out ultrafiltration purification to obtain ⁶⁴Cu-protein conjugate solution (B) of interest. 260 µL of the protein conjugate and 377 µL of a PBS solution were mixed into the obtained solution, and the resulting mixture was filtered using a syringe filter (Millex-GV 0.22 µm, Millipore) to prepare a ⁶⁴Cu-protein conjugate (B)-containing PBS solution (31.5 MBq,
26.27 MBq/mg).

### PET/CT imaging

A common marmoset (2 years old) was anesthetized with isoflurane, and 175 to 185 µL of a PBS solution containing ⁶⁴Cu-protein conjugate solution (A) or (B) was administered from the tail vein. After administration, under anesthesia, images were acquired using a PET/CT imaging apparatus (PET: Clairvivo PET, manufactured by Shimadzu Corporation, CT: Aquilion, manufactured by TOSHIBA).

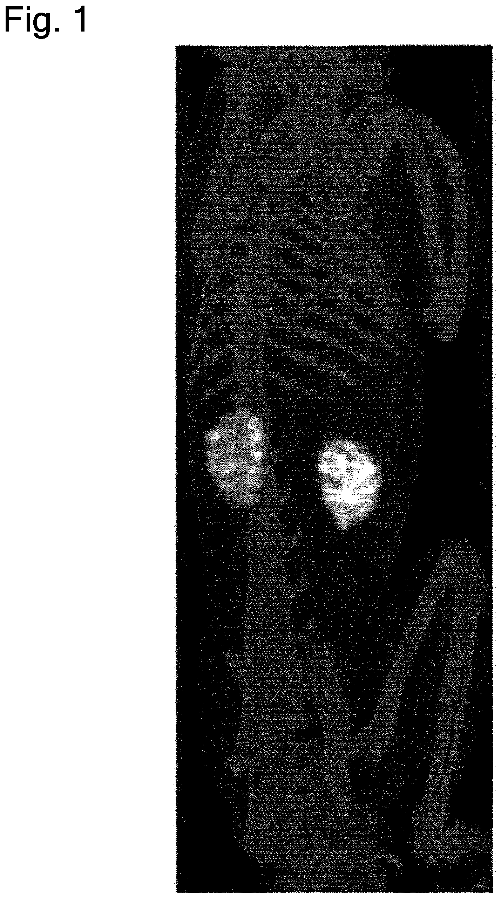

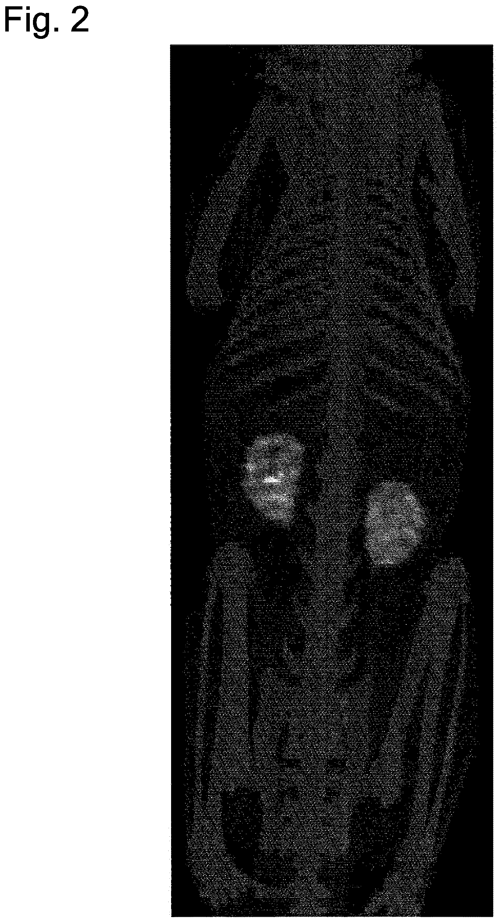

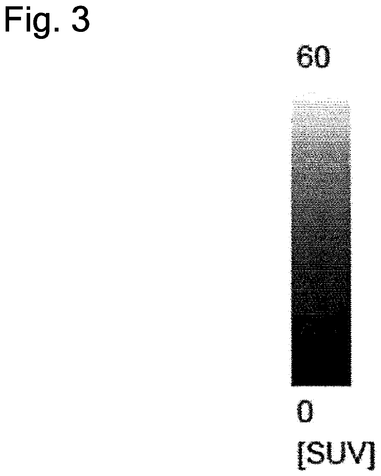

Fig. 1 shows a PET/CT image obtained about 3 hours after the administration of a PBS solution containing ⁶⁴Cu-protein conjugate solution (A). In addition, Fig. 2 shows a PET/CT image obtained about 3 hours after the administration of a PBS solution containing ⁶⁴Cu-protein conjugate solution (B). Fig. 3 shows SUV. The SUV is a value obtained by dividing the radioactivity per g of tissue by the administered radioactivity per g of body weight, that is, a value represented by SUV = radioactivity per g of tissue / administered radioactivity per g of body weight. As the radioactivity, a value corrected for attenuation is used. It was observed that the accumulation in the kidneys was lower in FIG. 2 than in FIG. 1.

### INDUSTRIAL APPLICABILITY

The present invention includes a conjugate having excellent binding activity to human CEACAM5 and is expected to be useful for diagnosis and/or treatment of a cancer involving human CEACAM5.

In addition, the present invention includes a conjugate having excellent binding activity to MUC1 and is expected to be useful for diagnosis and/or treatment of a cancer involving MUC1.

In addition, a conjugate consisting of 3arm DOTA, a specific spacer, a specific peptide linker, and a biomolecule, which is a conjugate of the present invention, is decomposed in the kidneys and excreted, and thus is expected to be useful for diagnosis and/or treatment of a disease associated with the biomolecule.

### SEQUENCE LISTING FREE TEXT

In the numerical heading <223> of the following sequence listing, a description of a typical "Artificial Sequence" is provided. Specifically, the nucleotide sequences represented by SEQ ID NOs: 1 and 3 in the sequence listing are the nucleotide sequences of the heavy chain fragment and the light chain of PB009-01, respectively, and the amino acid sequences represented by SEQ ID NOs: 2 and 4 are the amino acid sequences of the heavy chain fragment and the light chain encoded by SEQ ID NOs: 1 and 3, respectively. In addition, the nucleotide sequences represented by SEQ ID NOs: 5 and 9 in the sequence listing are the nucleotide sequences of the heavy chain fragment and the light chain of P10-1, respectively, and the amino acid sequences represented by SEQ ID NOs: 6 and 10 are the amino acid sequences of the heavy chain fragment and the light chain encoded by SEQ ID NOs: 5 and 9, respectively. The nucleotide sequences represented by SEQ ID NOs: 7 and 9 in the sequence listing are the nucleotide sequences of the heavy chain fragment and the light chain of P10-2, respectively, and the amino acid sequences represented by SEQ ID NOs: 8 and 10 are the amino acid sequences of the heavy chain fragment and the light chain encoded by SEQ ID NOs: 7 and 9, respectively. SEQ ID NOs: 11 and 15 are the heavy variable region and the light chain variable region of P10-1, respectively, and the amino acid sequences represented by SEQ ID NOs: 12 and 16 are the amino acid sequences of the heavy chain variable region and the light chain variable region encoded by SEQ ID NOs: 11 and 15, respectively. SEQ ID NOs: 13 and 15 are the heavy variable region and the light chain variable region of P10-2, respectively, and the amino acid sequences represented by SEQ ID NOs: 14 and 16 are the amino acid sequences of the heavy chain variable region and the light chain variable region encoded by SEQ ID NOs: 13 and 15, respectively.

## Claims

1. A conjugate represented by the following formula (I):
(Y-S₁-X)ₚ-Fab¹ (I)
wherein
Fab¹ is an anti-human CEACAM5 antibody Fab fragment selected from the group consisting of
(a) an anti-human CEACAM5 antibody Fab fragment comprising a heavy chain fragment including a heavy chain variable region consisting of the amino acid sequence of amino acids 1 to 121 of SEQ ID NO: 2 and a light chain including a light chain variable region consisting of the amino acid sequence of amino acids 1 to 112 of SEQ ID NO: 4, and
(b) an anti-human CEACAM5 antibody Fab fragment comprising a heavy chain fragment including a heavy chain variable region which consists of the amino acid sequence of amino acids 1 to 121 of SEQ ID NO: 2 and in which glutamic acid of amino acid 1 of SEQ ID NO: 2 is modified to pyroglutamic acid, and a light chain including a light chain variable region consisting of the amino acid sequence of amino acids 1 to 112 of SEQ ID NO: 4,
the Fab¹ is bound to X via p amino groups or thiol groups in the Fab¹;
X is a peptide linker or a bond;
S₁ is a spacer or a bond;
Y is a ligand; and
p is a natural number of 1 to 25 and represents the number of (Y-S₁-X) bound to Fab¹; provided that when X is a bond, S₁ is -CH₂-(1,4-phenylene)-NH-C(=S)- or a bond, and Y is 1,4,7,10-tetraazacyclododecane-1,4,7,10-tetraacetic acid.

2. The conjugate according to claim 1, wherein Fab¹ is selected from the group consisting of
(a) an anti-human CEACAM5 antibody Fab fragment comprising a heavy chain fragment consisting of the amino acid sequence shown in SEQ ID NO: 2 and a light chain consisting of the amino acid sequence shown in SEQ ID NO: 4, and
(b) an anti-human CEACAM5 antibody Fab fragment comprising a heavy chain fragment which consists of the amino acid sequence shown in SEQ ID NO: 2 and in which glutamic acid of amino acid 1 of SEQ ID NO: 2 is modified to pyroglutamic acid, and a light chain consisting of the amino acid sequence shown in SEQ ID NO: 4.

3. The conjugate according to claim 2, wherein Fab¹ includes a heavy chain fragment consisting of the amino acid sequence shown in SEQ ID NO: 2 and a light chain consisting of the amino acid sequence shown in SEQ ID NO: 4.

4. The conjugate according to any one of claims 1 to 3, wherein X is a peptide linker including a peptide consisting of 2 to 4 amino acids having an amino acid sequence cleaved by a renal brush border membrane enzyme or a lysosomal enzyme.

5. The conjugate according to any one of claims 1 to 4, wherein S₁ is
- C(=O)-CH₂O-(1,3-phenylene)-C(=O)-,
- C(=O)-(CH₂CH₂O)₄-(1,3-phenylene)-C(=O)-,
- C(=O)-(1,3-phenylene)-C(=O)-,
- NH-CH₂-(1,3-phenylene)-C(=O)-,
- NH-(CH₂)₂-C(=O)-,
- CH₂-(1,4-phenylene)-NH-C(=S)-, -NH-(CH₂CH₂O)₃-CH₂-C(=O)-NH-CH₂-(1,3-phenylene)-C(=O)-,
- NH-CH₂-(1,4-phenylene)-NH-C(=O)-,
- NH-(CH₂)₃-C(=O)-,
- NH-(CH₂CH₂O)₃-CH₂-C(=O)-,
- NH-CH₂-(1,4-phenylene)-C(=O)-,
- CH₂-(1,4-phenylene)-NH-C(=S)-NH-CH₂-(1,3-phenylene)-C(=O)-,
- CH₂-(1,4-phenylene)-NH-C(=S)-NH-(CH₂CH₂O)₃-CH₂-C(=O)-NH-CH₂-(1,3-phenylene)-C(=O)-,
a spacer represented by any of the following formulas (a) to (q), or a bond, 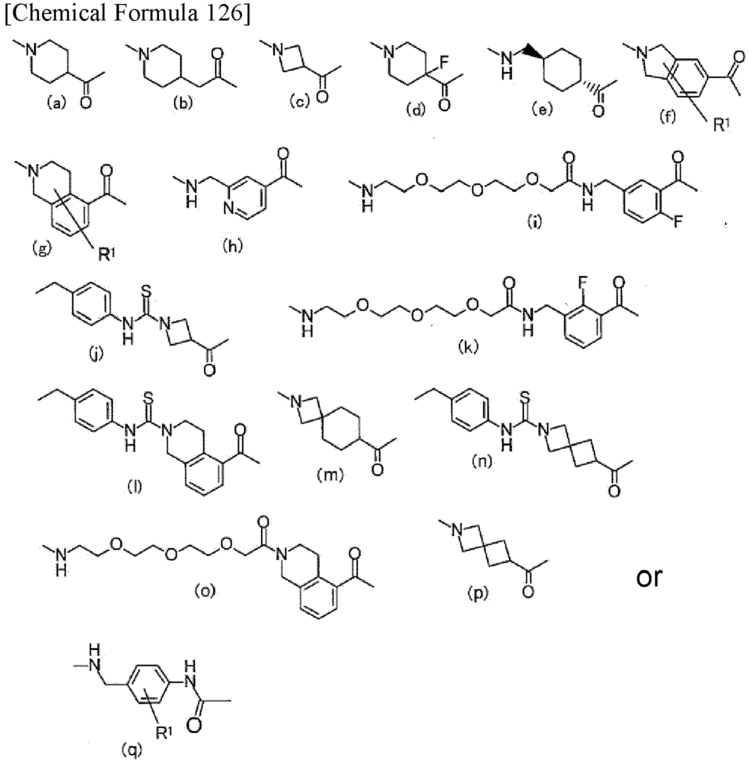 wherein R¹ is a hydrogen atom, a halogen, or C₁₋₆ alkyl, or halo C₁₋₆ alkyl; and
X is a peptide linker selected from the group consisting of
(1) -Met-Ile-NH-(CH₂)₂-Z₁-,
(2) -Gly-Lys^{∗}-Z₂-,
(3) -Gly-Phe-Lys^{∗}-Z₂-,
(4) -Met-Val-Lys^{∗}-Z₂-,
(5) -Gly-Tyr^{∗}-CH₂-C(=O)-NH-(CH₂)₂-Z₁-,
(6) -Gly-Lys-Lys^{∗}-Z₂-,
(7) -Gly-Arg-Lys^{∗}-Z₂-,
(8)-Gly-Lys^{∗}-C(=S)-NH-(1,4-phenylene)-NH-C(=S)-,
(9)-Met-Ile-NH-(CH₂)₂-NH-C(=S)-NH-(1,4-phenylene)-NH-C(=S)-,
(10) -Asp-Gly-Lys^{∗}-Z₂-,
(11) -Met-Gly-Lys^{∗}-Z₂-,
(12) -Met-Ile-Lys^{∗}-Z₂-,
(13) -Gly-Tyr^{∗}-CH₂-C(=O)-Lys^{∗}-Z₂-,
(14) -Val-NH-(CH₂)₂-Z₁-,
(15) -Ile-NH-(CH₂)₂-Z₁-,
(16) -Gly-Val-NH-(CH₂)₂-Z₁-,
(17) -Gly-Ile-NH-(CH₂)₂-Z₁-,
(18) -Met-Phe-Lys^{∗}-Z₂-,
(19) -Gly-Tyr-Lys^{∗}-Z₂-,
(20) -Gly-Tyr^{∗}-CH₂-C(=O)-NH-(CH₂O)₃-CH₂-C(=O)-NH-(CH₂)₂-Z₁-,
(21) -Gly-(3-(2-naphthyl)alanine)-Lys^{∗}-Z₂-,
(22) -Gly-diphenylalanine-Lys^{∗}-Z₂-,
(23) -Gly-Tyr-NH-(CH₂)₅-Z₁-,
(24) -Met-Ile-NH-(CH₂)₂-(A-4)-,
(25) -Met-De-NH-(CH₂)₂-(A-5)-,
(26) -Met-Ile-NH-(CH₂)₂-(II-II)-,
(27) -Met-De-NH-(CH₂)₂-(A-3)-,
(28) -Met-Gly-Lys^{∗}-Z₃-, and
or
(29) a bond,
wherein Met represents methionine, Ile represents isoleucine, Gly represents glycine, Lys represents lysine, Phe represents phenylalanine, Val represents valine, Tyr represents tyrosine, Arg represents arginine, Asp represents aspartic acid, Z₁ represents a group represented by the following formula (II-I) or (II-II), -Lys^{∗}-Z₂- represents a group represented by the following formula (III-I) or (III-II), -Tyr^{∗}-CH₂- represents a group represented by the following formula (IV), -Ly^{∗}-C(=S)- represents a group represented by the following formula (V), -Lys^{∗}-Z₃- represents a group represented by the following formula (III-III), and group (A-3), (A-4), or (A-5) is as represented by the following formulas.

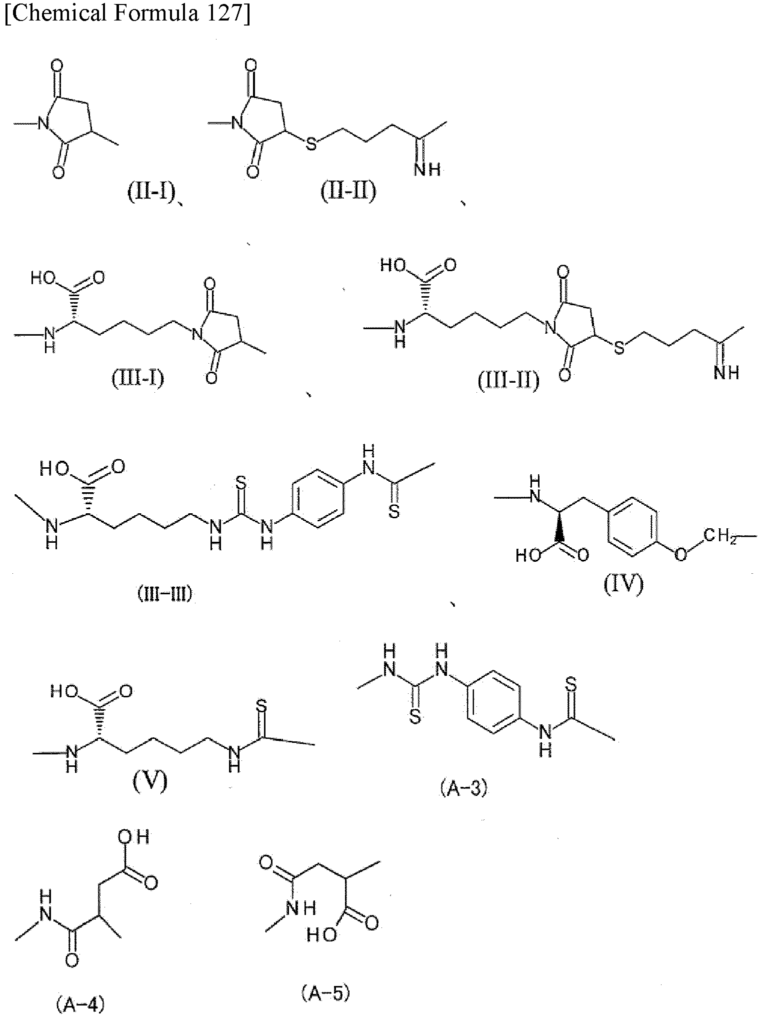

6. The conjugate according to any one of claims 1 to 5, wherein S₁ is -C(=O)-CH₂O-(1,3-phenylene)-C(=O)-, -C(=O)-(CH₂CH₂O)₄-(1,3-phenylene)-C(=O)-, or -C(=O)-(1,3-phenylene)-C(=O)-, and
X is a peptide linker selected from the group consisting of
(1) -Met-Ile-NH-(CH₂)₂-Z₁-,
(2) -Gly-Lys^{∗}-Z₂-,
(3) -Gly-Phe-Lys^{∗}-Z₂-,
(5) -Gly-Tyr^{∗}-CH₂-C(=O)-NH-(CH₂)₂-Z₁-,
(6) -Gly-Lys-Lys^{∗}-Z₂-, and
(8) -Gly-Lys^{∗}-C(=S)-NH-(1,4-phenylene)-NH-C(=S)-.

7. The conjugate according to any one of claims 1 to 5, wherein S₁ is -C(=O)-CH₂O-(1,3-phenylene)-C(=O)-, -C(=O)-(CH₂CH₂O)₄-(1,3-phenylene)-C(=O)-, -NH-CH₂-(1,3-phenylene)-C(=O)-, -NH-CH₂-(1,4-phenylene)-NH-C(=O)-, -NH-CH₂-(1,4-phenylene)-C(=O)-, -C(=O)-(1,3-phenylene)-C(=O)-, or the following formula 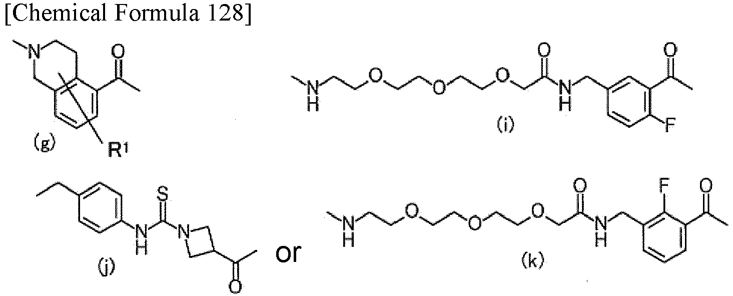 and X is a peptide linker selected from the group consisting of
(1) -Met-Ile-NH-(CH₂)₂-Z₁-,
(2) -Gly-Lys^{∗}-Z₂-,
(3) -Gly-Phe-Lys^{∗}-Z₂-,
(5) -Gly-Tyr^{∗}-CH₂-C(=O)-NH-(CH₂)₂-Z₁-,
(6) -Gly-Lys-Lys^{∗}-Z₂-,
(8) -Gly-Lys^{∗}-C(=S)-NH-(1,4-phenylene)-NH-C(=S)-,
(24) -Met-De-NH-(CH₂)₂-(A-4)-,
(25) -Met-De-NH-(CH₂)₂-(A-5)-,
(26) -Met-Ile-NH-(CH₂)₂-(II-II)-,
(27) -Met-Ile-NH-(CH₂)₂-(A-3)-, and
(28) -Met-Gly-Lys^{∗}-Z₃-.

8. The conjugate according to any one of claims 1 to 5, wherein S₁ is -NH-CH₂-(1,3-phenylene)-C(=O)-, -CH₂-(1,4-phenylene)-NH-C(=S)-, -NH-(CH₂CH₂O)₃-CH₂-C(=O)-NH-CH₂-(1,3-phenylene)-C(=O)-,-CH₂-(1,4-phenylene)-NH-C(=S)-NH-CH₂-(1,3-phenylene)-C(=O)-, -NH-CH₂-(1,4-phenylene)-NH-C(=O)-, a spacer represented by any of the following formulas (e) to (i) or (k), or a bond, 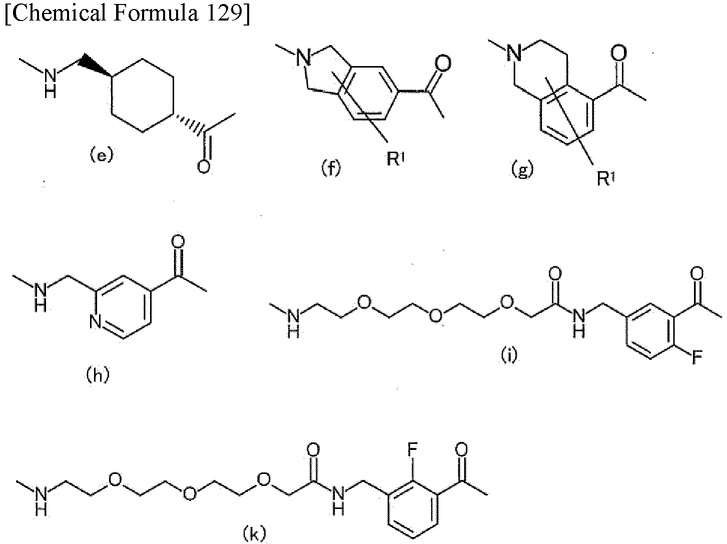 and X is a peptide linker selected from the group consisting of
(1) -Met-Ile-NH-(CH₂)₂-Z₁-,
(2) -Gly-Lys^{∗}-Z₂-,
(3) -Gly-Phe-Lys^{∗}-Z₂-,
(9)-Met-Ile-NH-(CH₂)₂-NH-C(=S)-NH-(1,4-phenylene)-NH-C(=S)-,
(10) -Asp-Gly-Lys^{∗}-Z₂-,
(11) -Met-Gly-Lys^{∗}-Z₂-,
(12) -Met-Ile-Lys^{∗}-Z₂-,
(21) -Gly-(3-(2-naphthyl)alanine)-Lys^{∗}-Z₂-,
(24) -Met-De-NH-(CH₂)₂-(A-4)-,
(25) -Met-De-NH-(CH₂)₂-(A-5)-,
(26) -Met-Ile-NH-(CH₂)₂-(II-II)-,
(27) -Met-Ile-NH-(CH₂)₂-(A-3)-, and
(28) -Met-Gly-Lys^{∗}-Z₃-.

9. The conjugate according to any one of claims 1 to 5, wherein X is a peptide linker selected from the group consisting of
(1) -Met-Ile-NH-(CH₂)₂-Z₁-,
(9)-Met-Ile-NH-(CH₂)₂-NH-C(=S)-NH-(l,4-phenylene)-NH-C(=S)-,
(12) -Met-Ile-Lys^{∗}-Z₂-,
(24) -Met-Ile-NH-(CH₂)₂-(A-4)-,
(25) -Met-De-NH-(CH₂)₂-(A-5)-,
(26) -Met-Ile-NH-(CH₂)₂-(II-II)-,
(27) -Met-De-NH-(CH₂)₂-(A-3)-.

10. The conjugate according to any one of claims 1 to 5, wherein X is a peptide linker selected from the group consisting of
(11) -Met-Gly-Lys^{∗}-Z₂-, and
(28) -Met-Gly-Lys^{∗}-Z₃-.

11. The conjugate according to any one of claims 1 to 10, wherein Y is deferoxamine or 1,4,7,10-tetraazacyclododecane-1,4,7,10-tetraacetic acid.

12. The conjugate according to claims 11, wherein Y is deferoxamine.

13. The conjugate according to claim 11, wherein Y is 1,4,7,10-tetraazacyclododecane-1,4,7,10-tetraacetic acid.

14. The conjugate according to claim 13, wherein Y is 3arm DOTA (1,4,7,10-tetraazacyclododecane-1,4,7,10-tetraacetic acid) or 4arm DOTA.

15. The conjugate according to claim 14, wherein Y is 3arm DOTA (1,4,7,10-tetraazacyclododecane-1,4,7,10-tetraacetic acid).

16. The conjugate according to any one of claims 11 or 13 to 15, wherein the conjugate is a conjugate selected from the group consisting of the compounds represented by the following formulas, and Fab¹is bound to an adjacent carbon atom via p amino groups or thiol groups in the Fab¹.

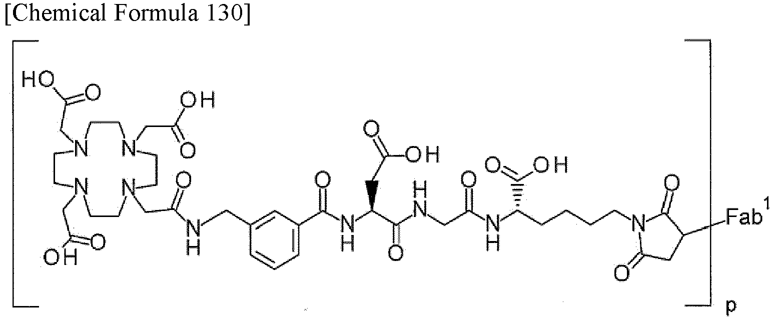

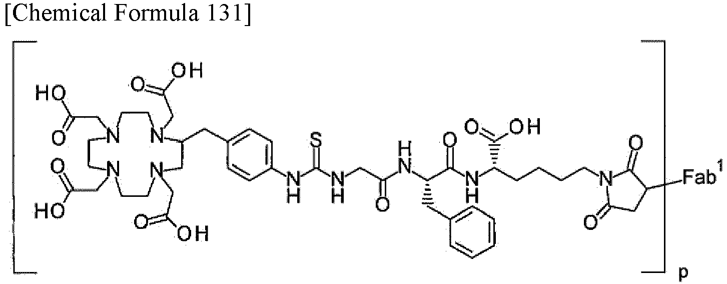

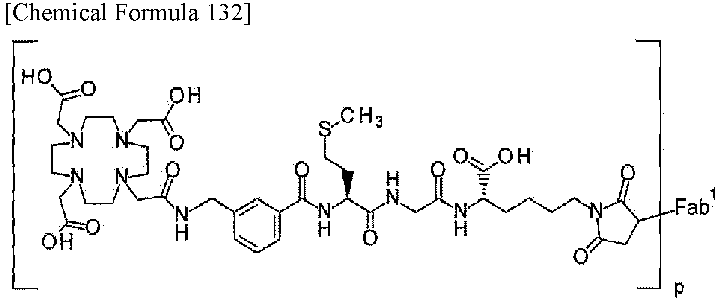

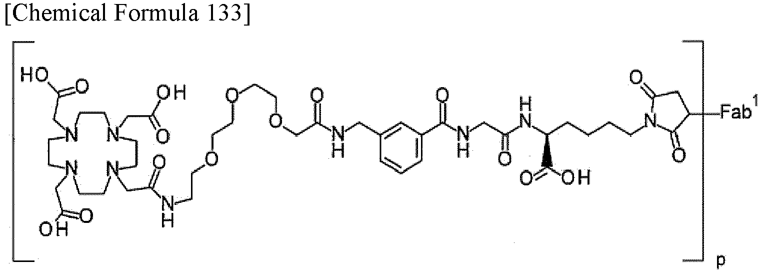

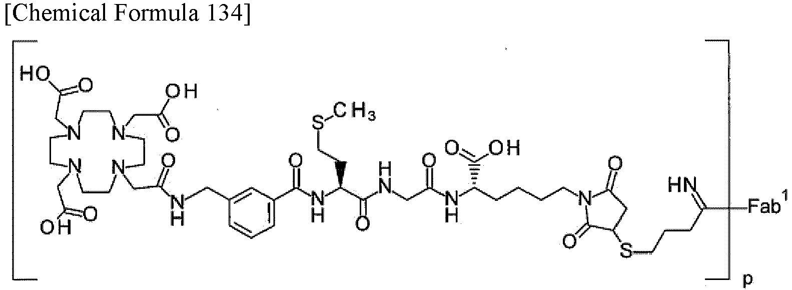

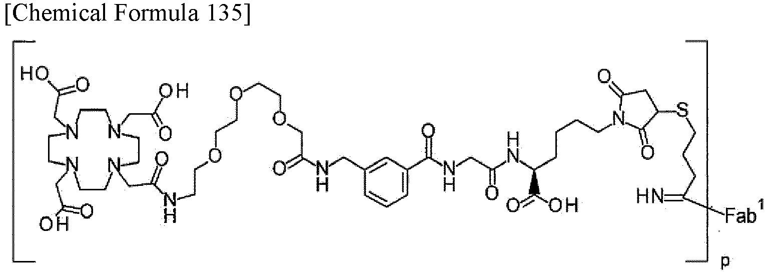

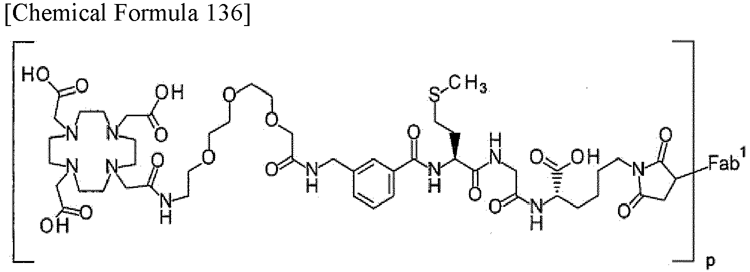

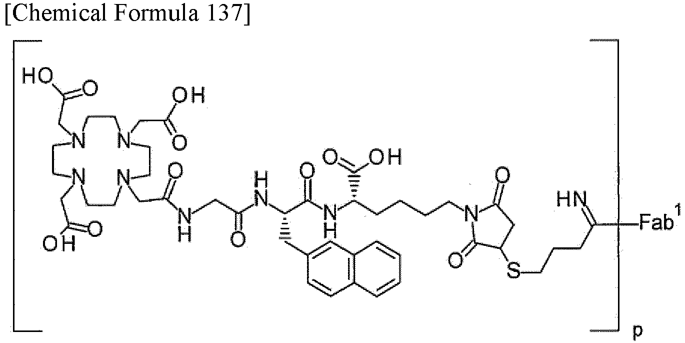

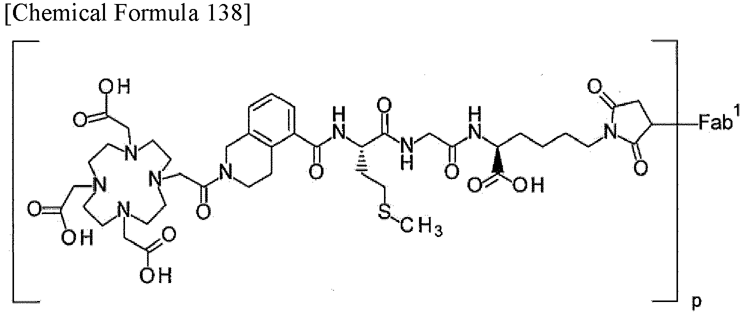

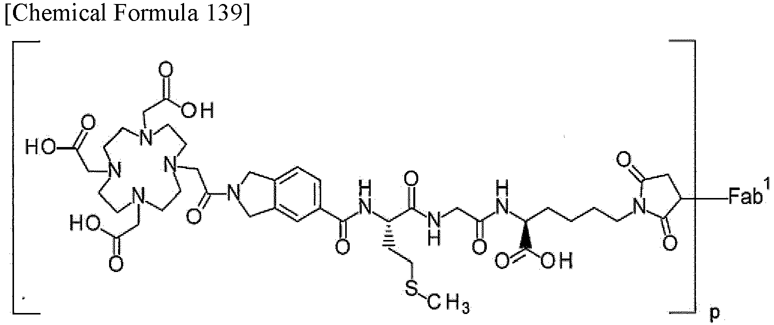

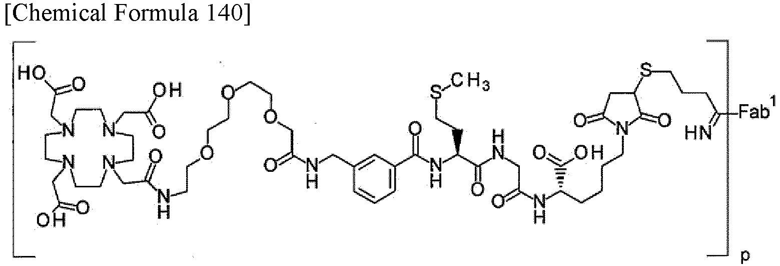

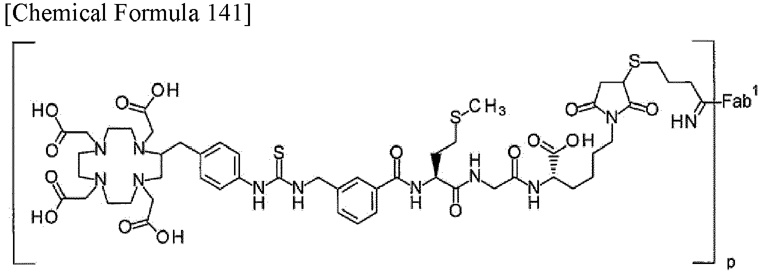

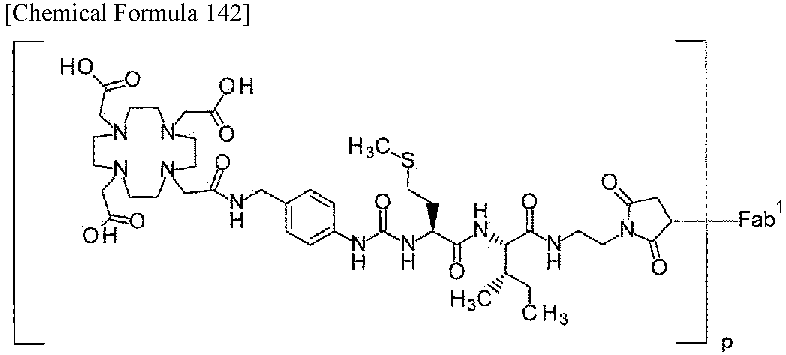

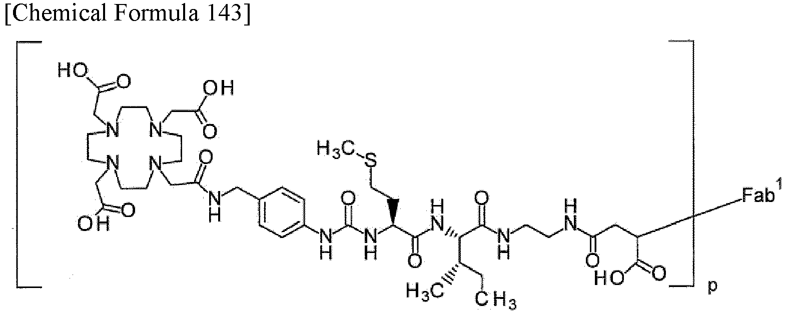

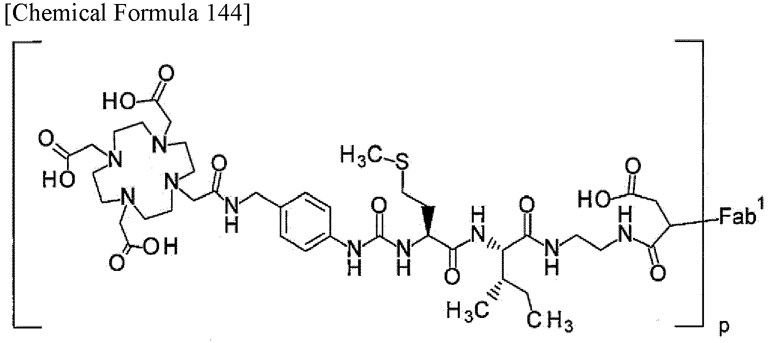

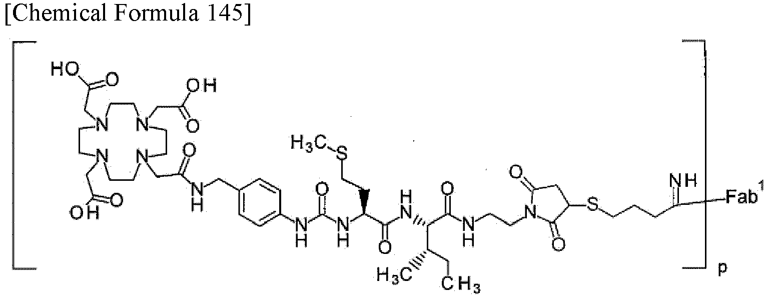

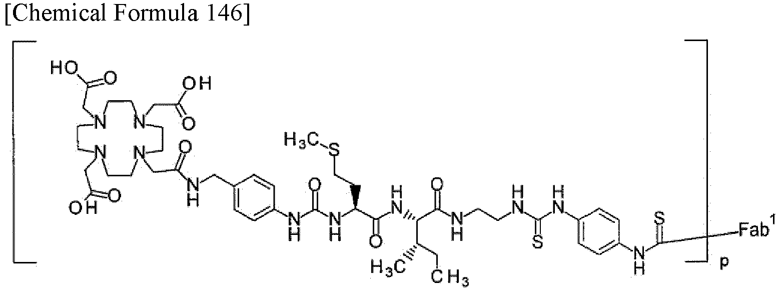

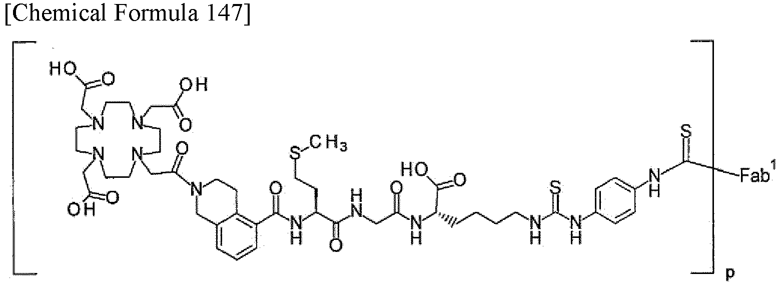

17. The conjugate according to any one of claims 11 or 13 to 15, wherein the conjugate is a conjugate selected from the group consisting of the compounds represented by the following formulas.

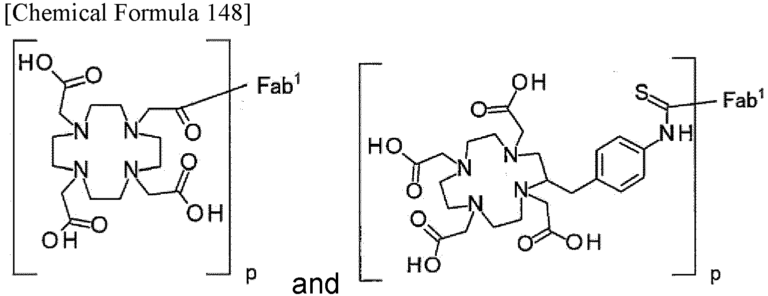

18. A conjugate represented by the following formula: 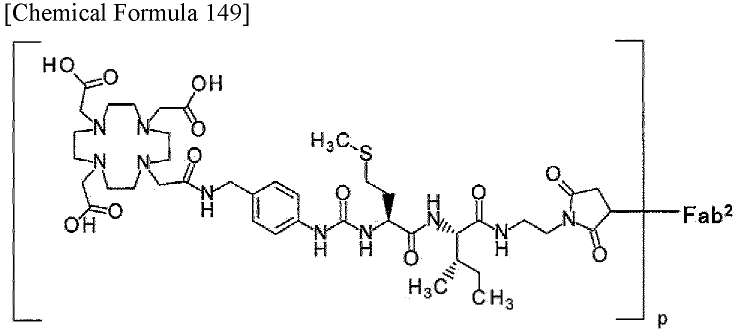 wherein Fab² is a Fab fragment including a heavy chain fragment consisting of the amino acid sequence shown in SEQ ID NO: 2 and a light chain consisting of the amino acid sequence shown in SEQ ID NO: 4;
p is a natural number of 1 to 25; and
Fab² is bound to an adjacent carbon atom via p amino groups or thiol groups in the Fab².

19. A conjugate represented by the following formula: 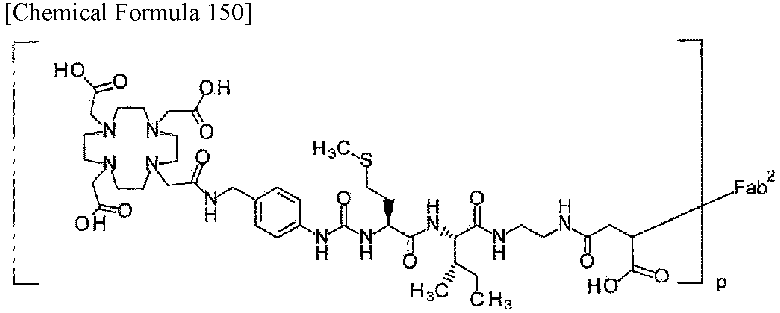 wherein Fab² is a Fab fragment including a heavy chain fragment consisting of the amino acid sequence shown in SEQ ID NO: 2 and a light chain consisting of the amino acid sequence shown in SEQ ID NO: 4;
p is a natural number of 1 to 25; and
Fab² is bound to an adjacent carbon atom via p amino groups or thiol groups in the Fab².

20. A conjugate represented by the following formula: 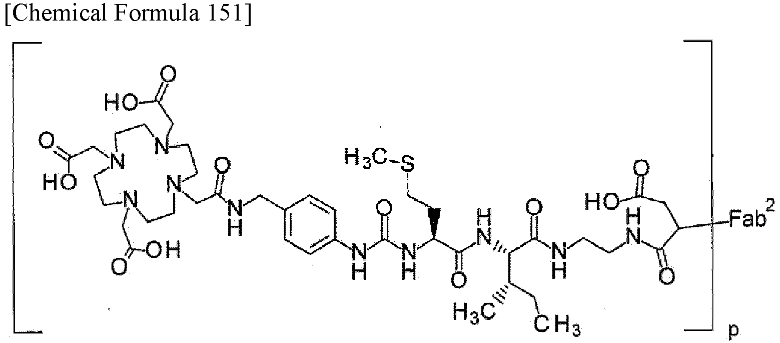 wherein Fab² is a Fab fragment including a heavy chain fragment consisting of the amino acid sequence shown in SEQ ID NO: 2 and a light chain consisting of the amino acid sequence shown in SEQ ID NO: 4;
p is a natural number of 1 to 25; and
Fab² is bound to an adjacent carbon atom via p amino groups or thiol groups in the Fab².

21. A conjugate represented by the following formula: 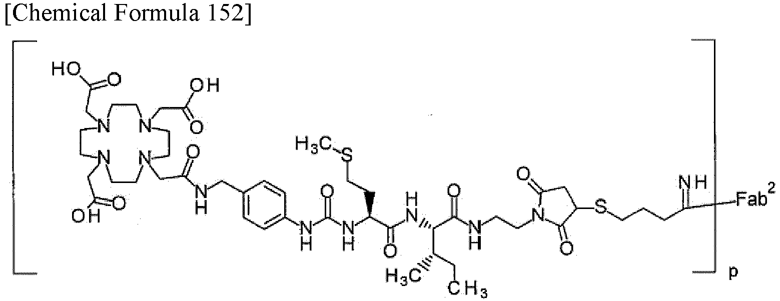 wherein Fab² is a Fab fragment including a heavy chain fragment consisting of the amino acid sequence shown in SEQ ID NO: 2 and a light chain consisting of the amino acid sequence shown in SEQ ID NO: 4;
p is a natural number of 1 to 25; and
Fab² is bound to an adjacent carbon atom via p amino groups or thiol groups in the Fab².

22. A conjugate represented by the following formula: 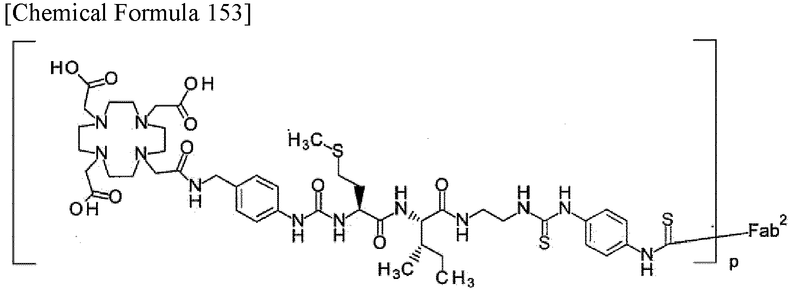 wherein Fab² is a Fab fragment including a heavy chain fragment consisting of the amino acid sequence shown in SEQ ID NO: 2 and a light chain consisting of the amino acid sequence shown in SEQ ID NO: 4;
p is a natural number of 1 to 25; and
Fab² is bound to an adjacent carbon atom via p amino groups or thiol groups in the Fab².

23. A conjugate represented by the following formula: 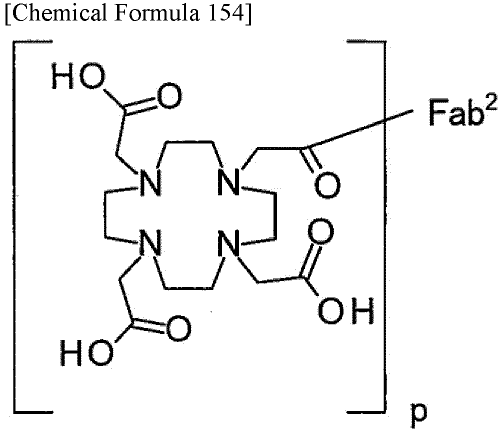 wherein Fab² is a Fab fragment including a heavy chain fragment consisting of the amino acid sequence shown in SEQ ID NO: 2 and a light chain consisting of the amino acid sequence shown in SEQ ID NO: 4;
p is a natural number of 1 to 25; and
Fab² is bound to an adjacent carbon atom via p amino groups or thiol groups in the Fab².

24. A conjugate represented by the following formula: 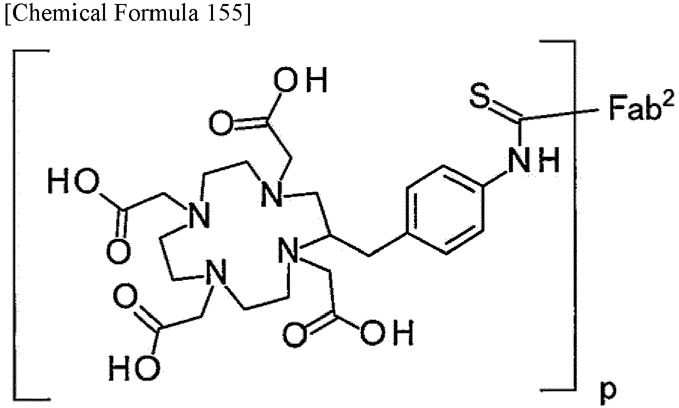 wherein Fab² is a Fab fragment including a heavy chain fragment consisting of the amino acid sequence shown in SEQ ID NO: 2 and a light chain consisting of the amino acid sequence shown in SEQ ID NO: 4;
p is a natural number of 1 to 25; and
Fab² is bound to an adjacent carbon atom via p amino groups or thiol groups in the Fab².

25. The conjugate according to any one of claims 1 to 24, wherein p is a natural number of 1 to 5.

26. The conjugate according to any one of claims 1 to 15, wherein a metal is coordinated to Y.

27. The conjugate according to any one of claims 16 to 24, wherein a metal is coordinated.

28. The conjugate according to claim 26 or 27, wherein the metal is a metal radioisotope.

29. The conjugate according to claim 28, wherein the metal is ⁸⁹Zr.

30. The conjugate according to claim 26 or 27, wherein the metal is a paramagnetic metal ion.

31. The conjugate according to claim 30, wherein the metal is Gd³⁺.

32. The conjugate according to any one of claims 26 to 31, wherein the conjugate is a PET tracer.

33. A diagnostic composition comprising one or more conjugates according to any one of claims 26 to 32 and a pharmaceutically acceptable carrier.

34. The diagnostic composition according to claim 33, wherein the diagnostic composition is used as an early diagnostic drug or a staging drug.

35. The diagnostic composition according to claim 33 or 34, wherein the diagnostic composition is used for diagnosing a cancer expressing human CEACAM5.

36. The diagnostic composition according to claim 35, wherein the cancer is colorectal cancer, breast cancer, lung cancer, thyroid cancer, or a cancer resulting from metastasis thereof.

37. A pharmaceutical composition comprising one or more conjugates according to any one of claims 26 to 31 and a pharmaceutically acceptable carrier.

38. The pharmaceutical composition according to claim 37, wherein the pharmaceutical composition is a pharmaceutical composition for treating a cancer expressing human CEACAM5.

39. The pharmaceutical composition according to claim 38, wherein the cancer is colorectal cancer, breast cancer, lung cancer, thyroid cancer, or a cancer resulting from metastasis thereof.

40. Use of the conjugate according to any one of claims 26 to 31 for production of a diagnostic composition for a cancer and/or a pharmaceutical composition for treating a cancer.

41. The conjugate according to any one of claims 26 to 31, wherein the conjugate is used for diagnosing a cancer and/or treating a cancer.

42. A method for diagnosing a cancer, comprising administering the conjugate according to any one of claims 26 to 31 to a subject.

43. A method for treating a cancer, comprising administering a therapeutically effective amount of the conjugate according to any one of claims 26 to 31 to a subject.
